(19) Europäisches Patentamt
European Patent Office
Office européen des brevets

(11) EP 4 574 816 A1

(12) EUROPEAN PATENT APPLICATION
published in accordance with Art. 153(4) EPC

(43) Date of publication:
**25.06.2025 Bulletin 2025/26**

(21) Application number: **23833572.3**

(22) Date of filing: **07.07.2023**

(51) International Patent Classification (IPC):
***C07D 237/32*** (2006.01) ***C07D 403/02*** (2006.01)
***C07D 403/14*** (2006.01) ***C07D 471/04*** (2006.01)
***A61K 31/502*** (2006.01) ***A61K 31/517*** (2006.01)
***A61P 35/00*** (2006.01)

(52) Cooperative Patent Classification (CPC):
**A61K 31/502; A61K 31/517; A61P 35/00; C07D 237/32; C07D 403/02; C07D 403/14; C07D 471/04**

(86) International application number:
**PCT/CN2023/106257**

(87) International publication number:
**WO 2024/008176 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.07.2022 CN 202210803253**
**22.07.2022 CN 202210866886**
**11.08.2022 CN 202210962185**
**05.09.2022 CN 202211079984**
**18.11.2022 CN 202211457720**

(71) Applicant: **Xizang Haisco Pharmaceutical Co., Ltd.**
**Lhoka, Tibet 856099 (CN)**

(72) Inventors:
- **LI, Yao**
**Lhoka Tibet 856099 (CN)**
- **ZHANG, Haoliang**
**Tibet 856099 (CN)**
- **SHI, Zongjun**
**Tibet 856099 (CN)**
- **WANG, Jiancheng**
**Lhoka Tibet 856099 (CN)**
- **WANG, Long**
**Lhoka, Tibet 856099 (CN)**
- **GUI, Naicheng**
**Lhoka, Tibet 856099 (CN)**
- **ZHAO, Jingzheng**
**Lhoka, Tibet 856099 (CN)**
- **HUANG, Shuai**
**Lhoka, Tibet 856099 (CN)**
- **TANG, Pingming**
**Lhoka, Tibet 856099 (CN)**
- **ZHANG, Chen**
**Lhoka, Tibet 856099 (CN)**
- **YAN, Pangke**
**Lhoka, Tibet 856099 (CN)**

(74) Representative: **IP TRUST SERVICES**
**Europole - Le Grenat**
**3, avenue Doyen Louis Weil**
**38000 Grenoble (FR)**

# (54) HETEROCYCLIC COMPOUND CAPABLE OF INHIBITING PRMT5 - MTA AND USE THEREOF

(57) A nitrogen-containing heterocyclic compound represented by formula (I-a), or a stereoisomer, a deuterated compound, a solvate, a pharmaceutically acceptable salt or a eutectic crystal thereof, a pharmaceutical composition comprising same, and a use thereof in the preparation of a drug for treating/preventing PRMT5•MTA-mediated diseases, wherein each group in formula (I-a) is as defined in the description.

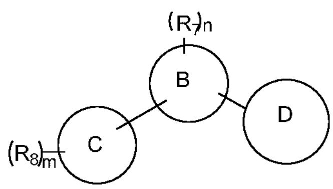

I-a

EP 4 574 816 A1

## Description

### Technical Field

The present invention relates to an inhibitor of a PRMT5•MTA complex and the use thereof in the preparation for treating tumours.

### Background Art

Protein arginine methylation catalysed by protein arginine methyltransferases (PRMTs) is an important post-translational modification, in which S-adenosylmethionine (SAM) is used as a methyl donor to methylate the nitrogen atoms of the arginine side chains of proteins. Based on the arginine methylation status, nine identified human PRMTs (PRMT1-9) are further subdivided into type I, type II and type III. Type I PRMTs catalyse the formation of monomethylarginine (MMA) and asymmetric dimethylarginine (aDMA), including PRMT1, PRMT2, PRMT3, PRMT4, PRMT6, and PRTM8; type II PRMTs catalyse the formation of MMA and symmetric dimethylarginine (sDMA), including PRMT5 and PRMT9; and type III PRMT catalyses the formation of MMA, including PRMT7. As a type II PRMT, PRMT5 transfers the methyl group from SAM to the guanidine moiety of arginine residues to form MMA and sDMA, thereby regulating the expression of target genes in an epigenetic manner or regulating key signalling molecules by post-translational methylation modification pathways.

The homozygous deletion of pl6/CDKN2a is common in cancer. These mutations often involve co-occurring deletions of adjacent genes, including the gene encoding methylthioadenosine phosphorylase (MTAP). It is estimated that approximately 15% of human cancers involve homozygous deletions of MTAP genes (e.g., see Firestone & Schramm (2017) J. Am. Chem Soc. 139(39): 13754-13760. doi: 10.1021/jacs.7b05803. Epub 2017 Sep 20).

In MTAP-deficient cells, methylthioadenosine (MTA) overaccumulates and binds to PRMT5 to form a PRMT5•MTA complex, thereby partially inhibiting PRMT5 enzyme activity and increasing the sensitivity of cell proliferation to PRMT5 deficiency or loss of PRMT5 activity. Therefore, MTAP deficiency reduces the methylation activity of PRMT5 and renders cells selectively dependent on PRMT5 activity. In MTAP-deficient cancers, the inhibition of PRMT5 activity synergistically engaged by MTA would provide therapeutic benefits in a variety of cancers.

Therefore, it is necessary to develop a new MTA-synergistic PRMT5 inhibitor that can inhibit PRMT5 activity under elevated MTA concentrations, especially in MTAP-deficient cells.

### Summary of the Invention

The present invention provides a compound represented by formula (I-a), formula (I-b), formula (I), formula (II-a) or formula (II), or a stereoisomer, a deuterated compound, a solvate or a pharmaceutically acceptable salt thereof, wherein the compound has the advantages of good activity, excellent physicochemical properties for preparation, high bioavailability, and low toxic and side effects.

The compound represented by formula (I-a), formula (I-b), formula (I), formula (II-a) or formula (II), or a stereoisomer, a deuterated compound, a solvate or a pharmaceutically acceptable salt thereof,

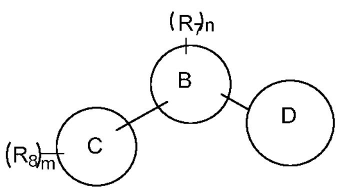
I-a

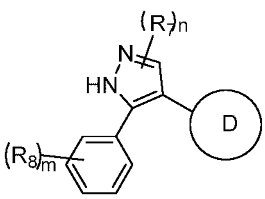
II-a

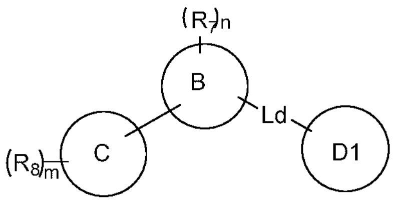
I-b

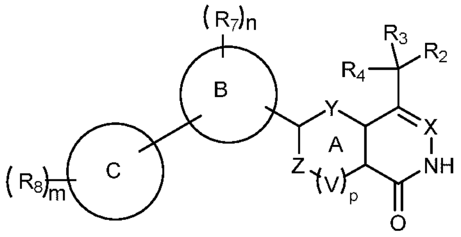

I

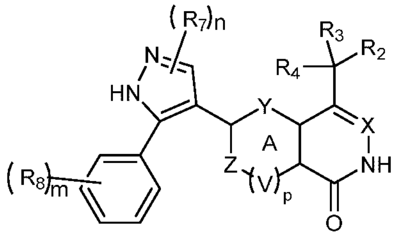

II

,

wherein D is selected from:

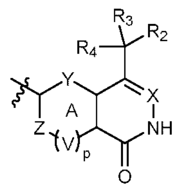

,

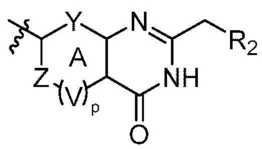

,

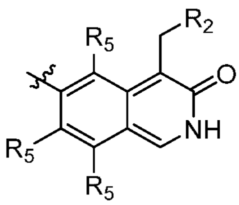

,

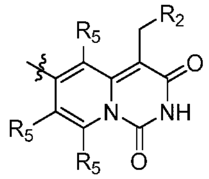

,

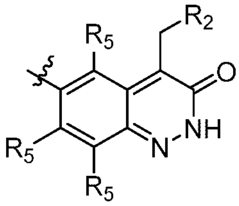

,

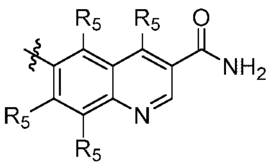

,

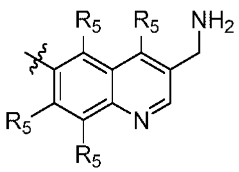

,

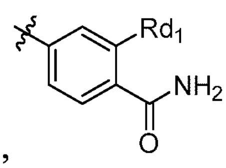

,

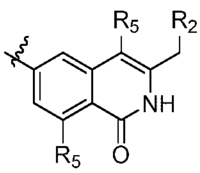

,

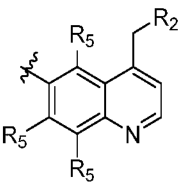

,

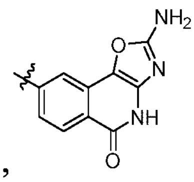

,

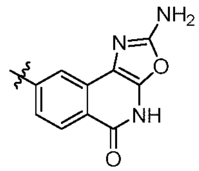

,

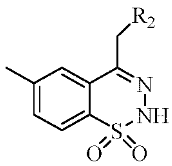

;

in some embodiments, D is selected from:

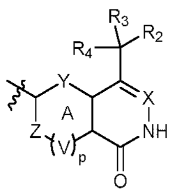

,

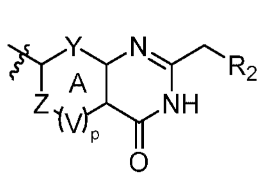

,

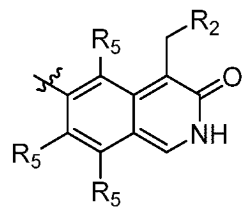

,

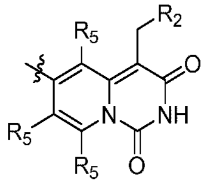

,

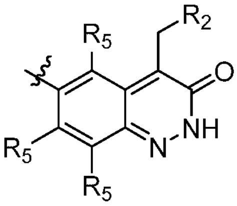 , 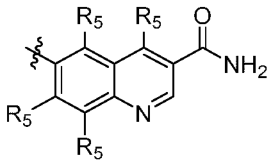 , 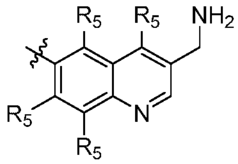 , 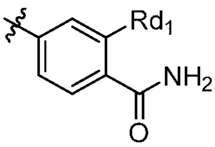 ,

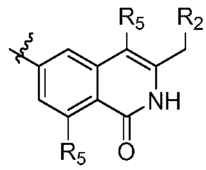 , 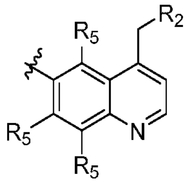 , 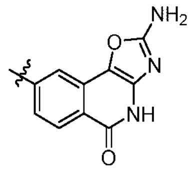 , 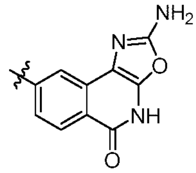 ;

in some embodiments, D is selected from:

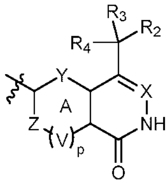 , 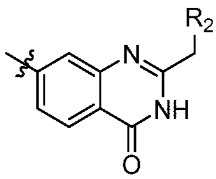 , 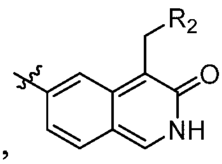 ,

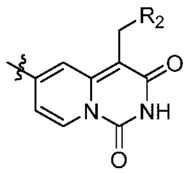 , 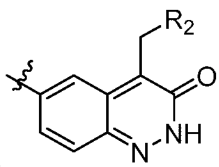 , 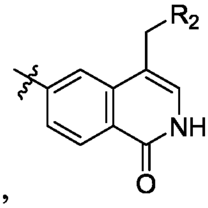 , 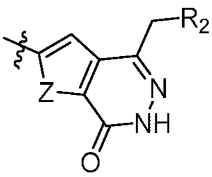 , 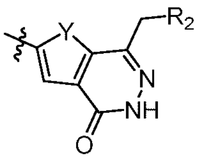 ,

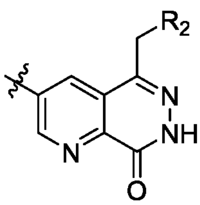 , 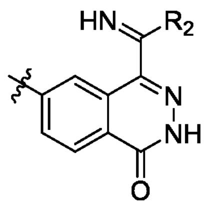 ;

in some embodiments, D is selected from

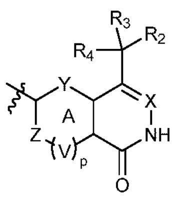 ;

in some embodiments, D is selected from

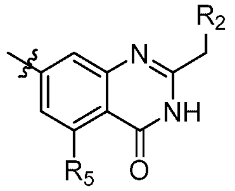,

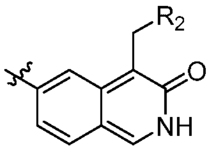, 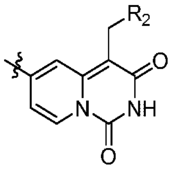, 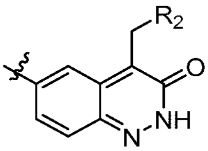, 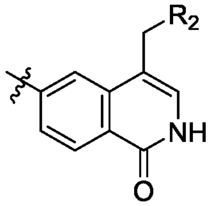, 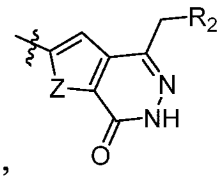,

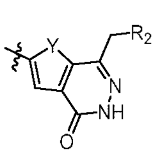, 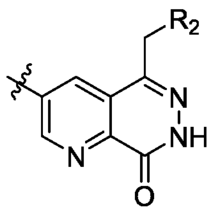, 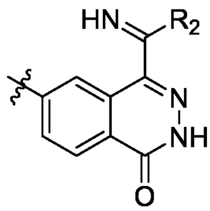, 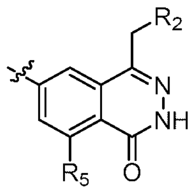, 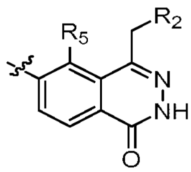,

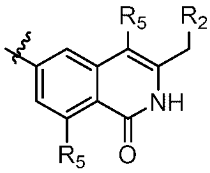, 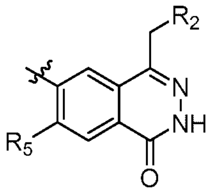, 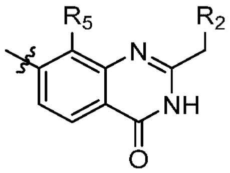, 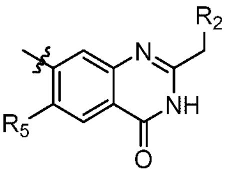,

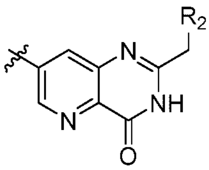, 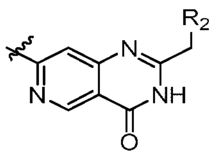, 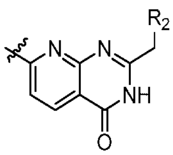, 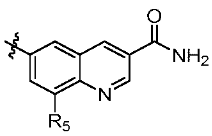, 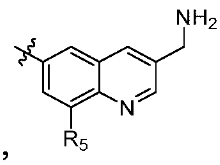,

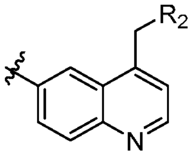, 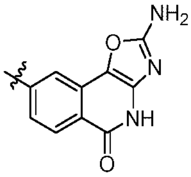, 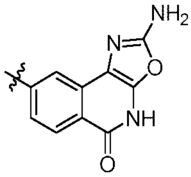;

in some embodiments, D is selected from

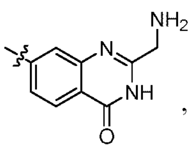, 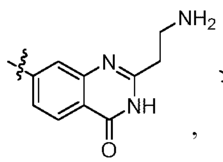, 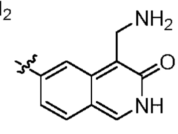, 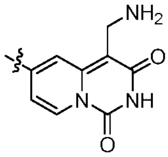, 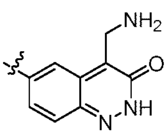,

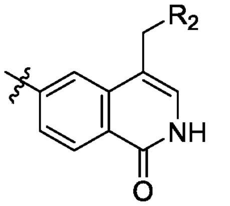 ,

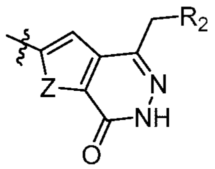 ,

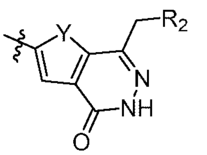 ,

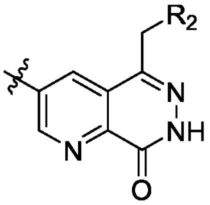 ,

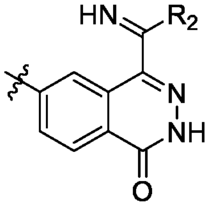 ,

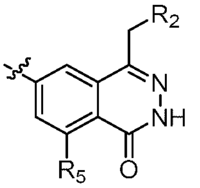 ,

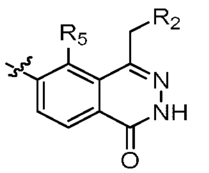 ,

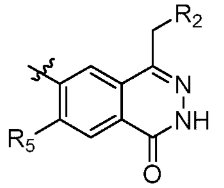 ,

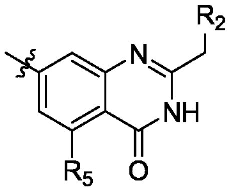 ,

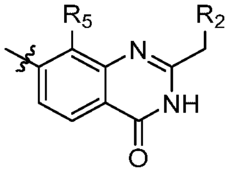 ,

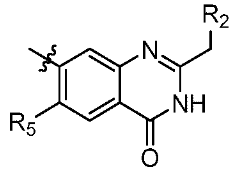 ,

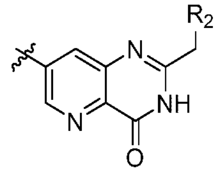 ,

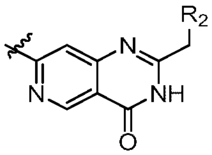 ,

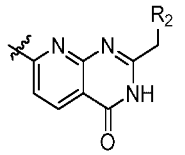 ,

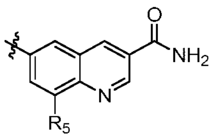 ,

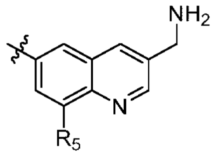 ,

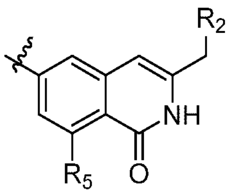 ,

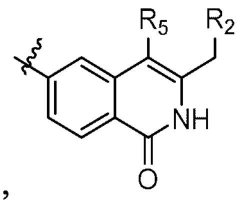 ,

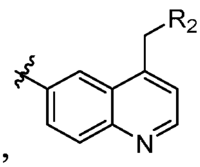 ,

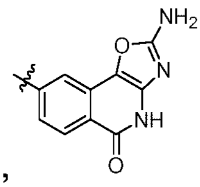 ,

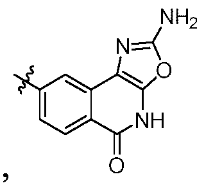

; in some embodiments, D is selected from

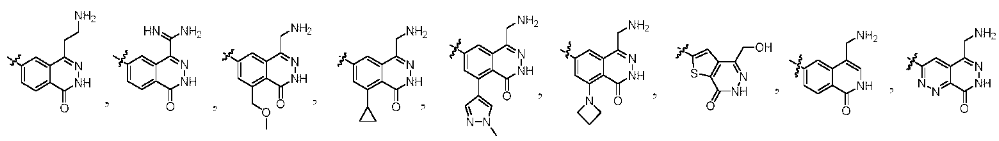
, , , , , , , , , ,

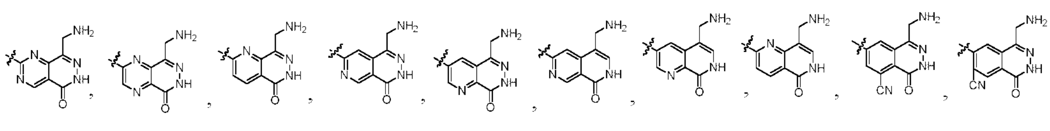
, , , , , , , , , ,

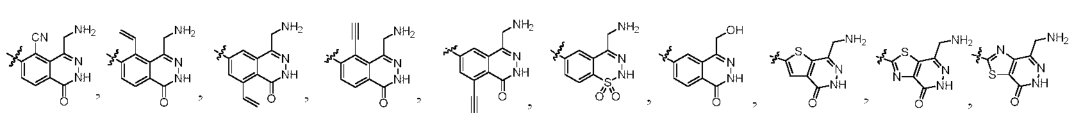
, , , , , , , , ,

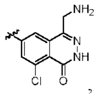, 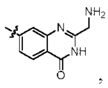, 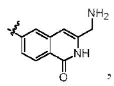, 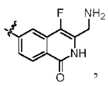, 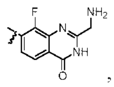, 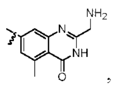, 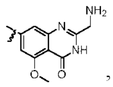, 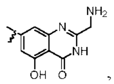, 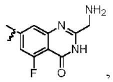,

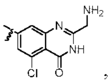, 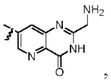, 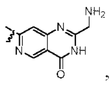, 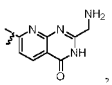, 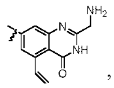, 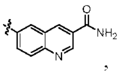, 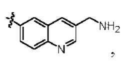, 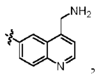,

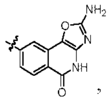, 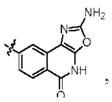, 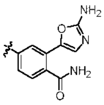,

or

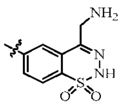;

in some embodiments, D is selected from

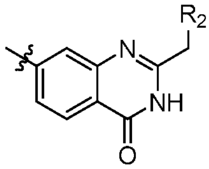, 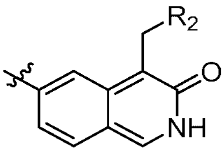,

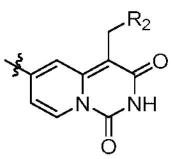, 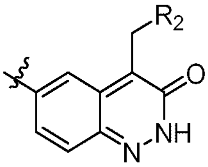, 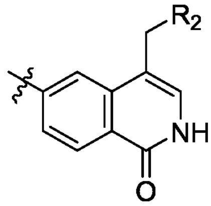, 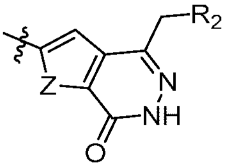,

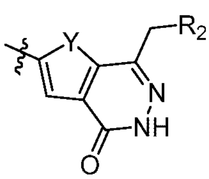, 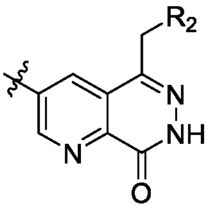, 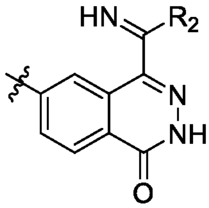, 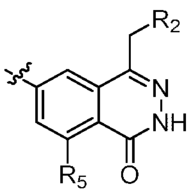, 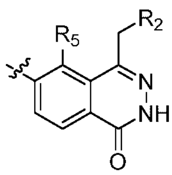,

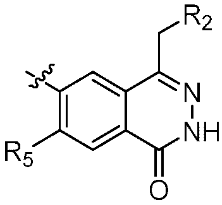;

in some embodiments, D is selected from

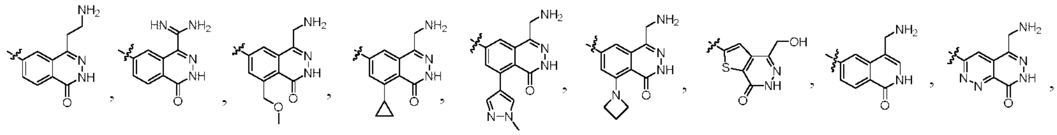
, , , , , , , , ,

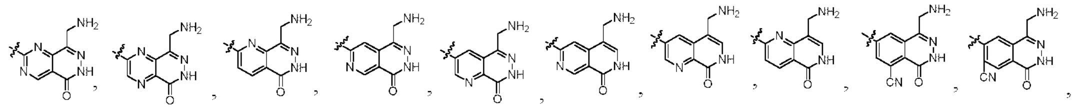
, , , , , , , , , ,

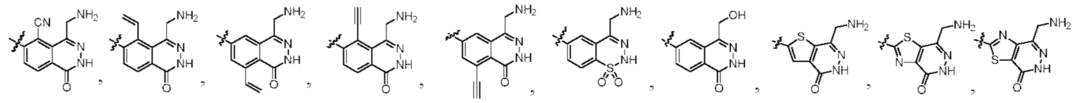
, , , , , , , , ,

; in some embodiments, D is selected from

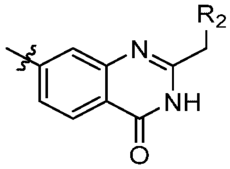
,
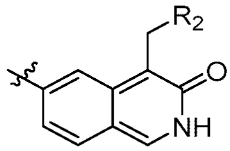
,

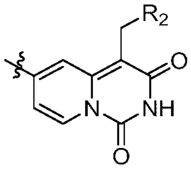
,
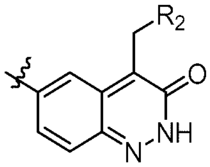
,
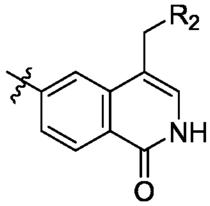
,
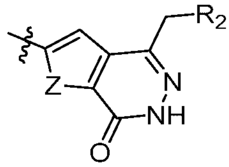
,

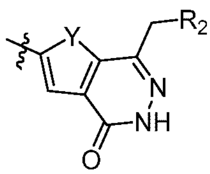
,
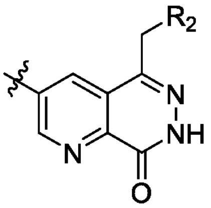
,
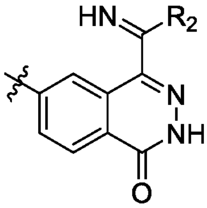
;

in some embodiments, D is selected from

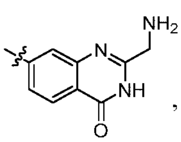
,
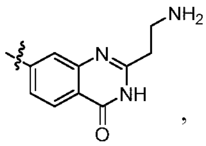
,
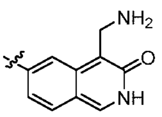
,
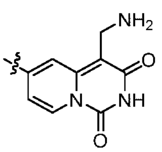
,
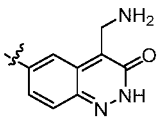
;

in some embodiments, D is selected from

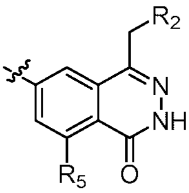
,
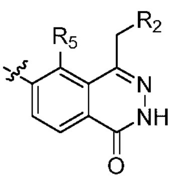
,

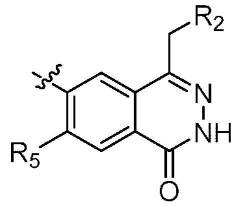
;

in some embodiments, D is selected from

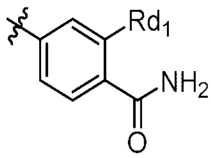
;

in some embodiments, D is selected from

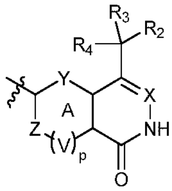
,
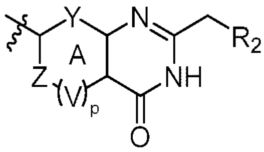
;

D1 is selected from:

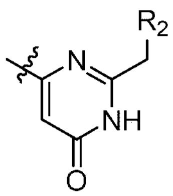
;

in some embodiments, D1 is selected from:

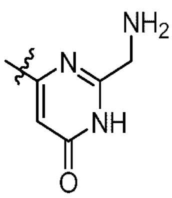
;

Ld is selected from $C_{1-4}$ alkyl, $C_{2-4}$ alkenyl or $C_{2-4}$ alkynyl; in some embodiments, Ld is selected from $C_{2-4}$ alkenyl or $C_{2-4}$ alkynyl; in some embodiments, Ld is selected from $C_{2-4}$ alkenyl; in some embodiments, Ld is selected from

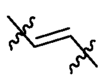
;

Rd1 is selected from 5-membered heteroaryl, wherein the heteroaryl is optionally substituted with 1-3 groups selected from halogen, D, CN, OH, $C_{1-4}$ alkyl, $C_{1-4}$ alkoxy and $NH_2$; in some embodiments, Rd1 is selected from 5-membered heteroaryl, wherein the heteroaryl is optionally substituted with 1-2 groups selected from halogen, D, CN, OH, $C_{1-2}$ alkyl, $C_{1-2}$ alkoxy and $NH_2$; in some embodiments, Rd1 is selected from 5-membered heteroaryl, wherein the heteroaryl is optionally substituted with 1-2 groups selected from $NH_2$; in some embodiments, Rd1 is selected from

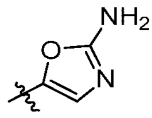 ;

X is selected from $CR_1$ or N; in some embodiments, X is $CR_1$; in some embodiments, X is N; in some embodiments, X is CH;

Y, Z and V are independently $CR_5$, $C(R_5)_2$, $NR_6$, N, O or S, and the total number of O and S is not greater than 1; in some embodiments, Y, Z and V are independently $CR_5$, N, O or S, and the total number of O and S is not greater than 1; in some embodiments, Y, Z and V are independently $CR_5$, N or S, and the total number of S is not greater than 1;

ring A is phenyl, 5- to 7-membered heterocycloalkyl containing 1-3 heteroatoms selected from N, O or S, or 5- to 6-membered heteroaryl containing 1-3 heteroatoms selected from N, O or S; in some embodiments, ring A is phenyl, or 5- to 6-membered heteroaryl containing 1-3 heteroatoms selected from N, O or S; in some embodiments, ring A is phenyl, thienyl, thiazolyl, pyrrolyl, imidazolyl, oxazolyl, pyridyl, pyrazinyl, pyrimidyl or pyridazinyl;

ring B is $C_{3-12}$ carbocycle, or 4- to 12-membered heterocycle containing 1-3 heteroatoms selected from N, O or S; in some embodiments, ring B is $C_{9-12}$ fused aryl, $C_{6-12}$ fused cycloalkyl, 8- to 12-membered fused heterocycloalkyl containing 1-3 heteroatoms selected from N, O or S, or 9- to 12-membered fused heteroaryl containing 1-3 heteroatoms selected from N, O or S; in some embodiments, ring B is phenyl, $C_{3-6}$ cycloalkyl, 4- to 7-membered heterocycloalkyl containing 1-3 heteroatoms selected from N, O or S, or 5- to 6-membered aryl containing 1-3 heteroatoms selected from N, O or S; in some embodiments, ring B is thienyl, thiazolyl, pyrrolyl, imidazolyl or pyrazolyl; in some embodiments, ring B is pyrazolyl;

ring C is $C_{3-12}$ carbocycle, or 4- to 12-membered heterocycle containing 1-3 heteroatoms selected from N, O or S; in some embodiments, ring C is phenyl, $C_{3-6}$ cycloalkyl, 4- to 7-membered heterocycloalkyl containing 1-3 heteroatoms selected from N, O or S, or 5- to 6-membered aryl containing 1-3 heteroatoms selected from N, O or S; in some embodiments, ring C is phenyl, thienyl, thiazolyl, pyrrolyl, imidazolyl, oxazolyl, pyrazolyl, pyridyl, pyrazinyl, pyrimidyl or pyridazinyl; in some embodiments, ring C is phenyl;

$R_1$ is H, D, $C_{1-4}$ alkyl, $C_{1-4}$ alkoxy, $C_{2-4}$ alkenyl, $C_{2-4}$ alkynyl, halogen, CN, OH, $NH_2$ or COOH, wherein the alkyl, alkoxy, alkenyl or alkynyl is optionally substituted with 1-3 groups selected from halogen, D, CN, OH and $NH_2$; in some embodiments, $R_1$ is H, D, $C_{1-4}$ alkyl, halogen or CN, wherein the alkyl is optionally substituted with 1-3 groups selected from halogen, D, CN and OH; in some embodiments, $R_1$ is H, D, $C_{1-4}$ alkyl, F, Cl, Br, I or CN, wherein the alkyl is optionally substituted with 1-3 groups selected from F, Cl, Br, I, D, CN and OH;

$R_2$ is OH, $NH_2$, $-CH_2NH_2$ or -NH-OH; in some embodiments, $R_2$ is OH, $NH_2$ or -NH-OH; in some embodiments, $R_2$ is $NH_2$;

$R_3$ and $R_4$ are independently H, D, halogen, $C_{1-4}$ alkyl, CN, OH, $NH_2$, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$; in some embodiments, $R_3$ and $R_4$ are independently H, D, halogen, $C_{1-4}$ alkyl, CN, OH or $NH_2$, wherein the alkyl is optionally substituted with 1-3 groups selected from halogen, D, CN, OH and $NH_2$; in some embodiments, $R_3$ and $R_4$ are independently H, D, F, Cl, Br, I, $C_{1-4}$ alkyl or CN, wherein the alkyl is optionally substituted with 1-3 groups selected from F, Cl, Br, I, D, CN, OH and $NH_2$; in some embodiments, $R_3$ and $R_4$ are independently H or D;

each $R_5$ is independently H, D, =O, OH, $C_{1-4}$ alkyl, CN, halogen, $C_{1-4}$ alkoxy, $C_{2-4}$ alkenyl, $C_{2-4}$ alkynyl, 5- to 6-membered heteroaryl containing 1-3 heteroatoms selected from N, O or S, 4- to 7-membered heterocycloalkyl containing 1-3 heteroatoms selected from N, O or S, or $C_{3-6}$ cycloalkyl, wherein the alkyl, alkoxy, alkenyl, alkynyl, heteroaryl or cycloalkyl is optionally substituted with 1-3 groups selected from halogen, D, CN, OH, $C_{1-4}$ alkyl, $C_{1-4}$ alkoxy and $NH_2$; in some embodiments, each $R_5$ is independently H, D, OH, $C_{1-4}$ alkyl, CN, halogen, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from halogen, D, CN, OH, $C_{1-4}$ alkyl, $C_{1-4}$ alkoxy and $NH_2$; in some embodiments, each $R_5$ is independently H, D, OH, $C_{1-4}$ alkyl, CN, F, Cl, Br, I, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from F, Cl, Br, I, D, CN, OH, methoxy and $NH_2$; in some embodiments, each $R_5$ is independently H, D, =O, OH, $C_{1-4}$ alkyl, CN, halogen, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$; in some embodiments, each $R_5$ is independently H, D, OH, $C_{1-4}$ alkyl, CN, halogen, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy,

alkenyl, alkynyl, heteroaryl, heterocycloalkyl or cycloalkyl is optionally substituted with 1-3 groups selected from halogen, D, CN, OH and $NH_2$; in some embodiments, each $R_5$ is independently H, D, OH, $C_{1-4}$ alkyl, CN, F, Cl, Br, I, $C_{1-4}$ alkoxy, $C_{2-4}$ alkenyl or $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from F, Cl, Br, I, D, CN, OH and $NH_2$; in some embodiments, each $R_5$ is independently H, D, CN, F, Cl, $C_{1-2}$ alkoxy, $C_{2-4}$ alkenyl, $C_{2-4}$ alkynyl, pyrazolyl, azetidinyl, cyclopropyl or cyclobutyl, wherein the alkyl, alkenyl, alkynyl, pyrazolyl, azetidinyl, cyclopropyl or cyclobutyl is optionally substituted with 1, 2 or 3 groups selected from F, Cl, Br, I, D, CN, OH and $NH_2$; in some embodiments, each $R_5$ is independently H, D, OH, $C_{1-4}$ alkyl, CN, halogen, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from halogen, D, CN, OH and $NH_2$; in some embodiments, each $R_5$ is independently H, D, OH, $C_{1-4}$ alkyl, CN, F, Cl, Br, I, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from F, Cl, Br, I, D, CN, OH and $NH_2$;

$R_6$ is H, D, $C_{1-4}$ alkyl or $C_{3-6}$ cycloalkyl, wherein the alkyl or cycloalkyl is optionally substituted with 1-3 groups selected from halogen, D, CN, OH and $NH_2$; in some embodiments, $R_6$ is H;

each $R_7$ is independently H, =O, D, OH, CN, halogen, $C_{1-4}$ alkyl, $C_{1-4}$ alkoxy, $N(R_7')_2$, $C_{2-4}$ alkenyl, $C_{2-4}$ alkynyl, $C_{3-6}$ cycloalkyl or 4- to 7-membered heterocycloalkyl containing 1-3 heteroatoms selected from N, O or S, wherein the alkyl, alkoxy, alkenyl, alkynyl, cycloalkyl or heterocycloalkyl is optionally substituted with 1-3 groups selected from halogen, D, OH, $NH_2$, CN, $C_{1-4}$ alkyl, - $C(O)C_{1-4}$ alkyl, $-C(O)NHC_{1-4}$ alkyl, $-C(O)NH_2$, $-NHC(O)C_{1-4}$ alkyl and $C_{1-4}$ alkoxy; in some embodiments, each $R_7$ is independently H, =O, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl, $-C(O)C_{1-4}$ alkyl, $-C(O)NHC_{1-4}$ alkyl, $-C(O)NH_2$, - $NHC(O)C_{1-4}$ alkyl and $C_{1-4}$ alkoxy; in some embodiments, each $R_7$ is independently H, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl and $C_{1-4}$ alkoxy;

each $R_8$ is independently H, D, nitro, amino, $C_{1-6}$ alkoxy, =O, OH, CN, halogen, $C_{2-6}$ alkenyl, $C_{2-6}$ alkynyl, $-SF_5$, $N_3$, $C_{1-6}$ alkyl, $-COC_{1-4}$ alkyl, $-N(R_7')_2$, - $C(=O)N(R_7')_2$, $-NR_7'C(=O)-R_7'$, $-C(=O)-R_7'$, $-(CH_2)_r-O-(CH_2)_r-R_8'$, -S-C1$_{-6}$ alkyl, - $S(O)-C_{1-6}$ alkyl, $-S(O)_2-C_{1-6}$ alkyl, $-S-(CH_2)_r-R_8'$, $-NR_7'-(CH_2)_r-R_8'$ or $-(CH_2)_r-R_8'$, wherein the alkyl, alkenyl or alkynyl is optionally substituted with 1-5 groups selected from $R_{8''}$; in some embodiments, each $R_8$ is independently H, D, =O, OH, CN, halogen, $C_{2-4}$ alkenyl, $C_{2-4}$ alkynyl, $N_3$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)_r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy; in some embodiments, each $R_8$ is independently D, =O, OH, CN, halogen, $C_{2-4}$ alkenyl, $C_{2-4}$ alkynyl, $N_3$, $C_{1-4}$ alkyl, - $COC_{1-4}$ alkyl, $-CONR_7'C_{1-4}$ alkyl, $-NR_7'COC_{1-4}$ alkyl, $N(R_7')_2$, $-O-(CH_2)r-R_8'$ or - $(CH_2)r-R_8'$, wherein the alkyl, alkenyl or alkynyl is optionally substituted with 1-3 groups selected from halogen, D, OH, $NH_2$, CN, $C_{1-2}$ alkyl and $C_{1-2}$ alkoxy; in some embodiments, each $R_8$ is independently D, CN, F, Cl, Br, $C_{2-4}$ alkenyl, $C_{2-4}$ alkynyl, $N_3$, $C_{1-4}$ alkyl, $-O-(CH_2)r-R_8'$ or $-(CH_2)_r-R_8'$, wherein the alkyl, alkenyl or alkynyl is optionally substituted with 1-3 groups selected from halogen, D, OH, $NH_2$, CN, $C_{1-2}$ alkyl and $C_{1-2}$ alkoxy; or

each $R_8$ is independently H, D, nitro, amino, $C_{1-6}$ alkoxy, =O, OH, CN, halogen, $C_{2-6}$ alkenyl, $C_{2-6}$ alkynyl, $-SF_5$, $C_{1-6}$ alkyl, $-COC_{1-4}$ alkyl, $-N(R_7')_2$, $-C(=O)N(R_7')_2$, $-NR_7'C(=O)-R_7'$, $-C(=O)-R_7'$, $-(CH_2)_r-O-(CH_2)_r-R_8'$, $-S-C_{1-6}$ alkyl, $-S(O)-C_{1-6}$ alkyl or $-S(O)_2-C_{1-6}$ alkyl, $-S-(CH_2)_r-R_8'$, $-NR_7'-(CH_2)_r-R_8'$ or $-(CH_2)_r-R_8'$, wherein the alkyl, alkenyl or alkynyl is optionally substituted with 1-5 groups selected from $R_{8''}$; in some embodiments, each $R_8$ is independently halogen, deuterium, nitro, cyano, amino, hydroxyl, =O, $C_{1-6}$ alkyl, $C_{1-6}$ alkoxy, $C_{2-6}$ alkenyl, $C_{2-6}$ alkynyl, $-SF_5$, - $(CH_2)_r-O-(CH_2)_r-R_8'$, $-(CH_2)_r-C_{3-12}$ cycloalkyl, $-(CH_2)_r$-(3- to 12-membered heterocycloalkyl), $-O-(CH_2)_r-C_{3-12}$ cycloalkyl, $-O-(CH_2)_r$-(3- to 12-membered heterocycloalkyl), $-NR_7'-C_{3-12}$ cycloalkyl, $-NR_7'$-(3- to 12-membered heterocycloalkyl), $-S-C_{3-12}$ cycloalkyl, -S-(3- to 12-membered heterocycloalkyl), - $(CH_2)_r$-(5- to 12-membered heteroaryl), $-(CH_2)_r$-(6- to 12-membered aryl), $-O-(CH_2)_r$-(5- to 12-membered heteroaryl), $-O-(CH_2)_r$-(6- to 12-membered aryl), $-NR_7'-(CH_2)_r$-(5- to 12-membered heteroaryl), $-NR_7'-(CH_2)_r$-(6- to 12-membered aryl), - $N(R_7')_2$, $-C(=O)N(R_7')_2$, $-NR_7'C(=O)-R_7'$, $-C(=O)-R_7'$, $-S-C_{1-6}$ alkyl, $-S(O)-C_{1-6}$ alkyl or $-S(O)_2-C_{1-6}$ alkyl, wherein the alkyl, alkoxy, $CH_2$, heterocycloalkyl, cycloalkyl, heteroaryl or aryl is optionally further substituted with 1-5 $R_{8''}$; or

each $R_8$ is independently H, D, =O, OH, CN, halogen, $C_{2-4}$ alkenyl, $C_{2-4}$ alkynyl, $-SF_5$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)_r-R_8'$, $-S-(CH_2)_r-R_8'$, $-NR_7'-(CH_2)_r-R_8'$ or $-(CH_2)_r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy; in some embodiments, each $R_8$ is independently H, D, =O, OH, CN, halogen, $C_{2-4}$ alkenyl, $C_{2-4}$ alkynyl, $C_{1-4}$ alkyl, - $COC_{1-4}$ alkyl, $-CONR_7'C_{1-4}$ alkyl, $-NR_7'COC_{1-4}$ alkyl, $N(R_7')_2$, $-O-(CH_2)r-R_8'$ or - $(CH_2)_r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy; in some embodiments, each $R_8$ is independently H, D, OH, CN, F, Cl, Br, I, $C_{2-4}$ alkenyl, $C_{2-4}$ alkynyl, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)_r-R_8'$ or $-(CH_2)_r-R_8'$,

wherein the alkyl, alkenyl or alkynyl is optionally substituted with 1-3 groups selected from halogen, D, OH, $NH_2$, CN, $C_{1-4}$ alkyl and $C_{1-4}$ alkoxy; or

each $R_8$ is independently CN, F, Cl, $N_3$, vinyl, fluorovinyl, $-O-CH_2CH_2-OCH_3$, $-N(CH_3)_2$,

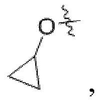

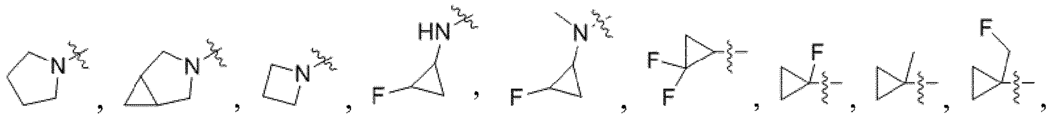

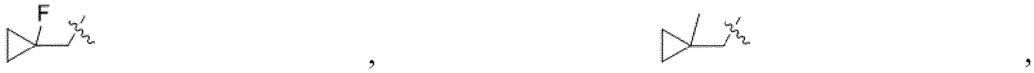

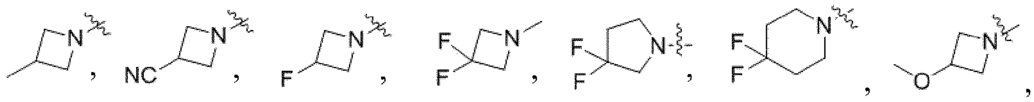

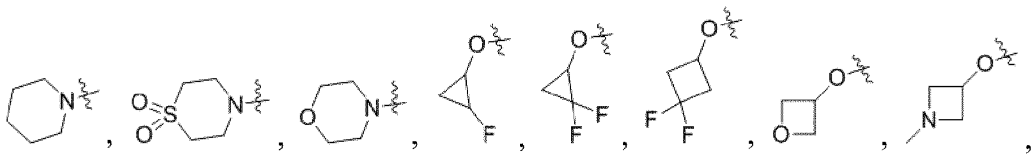

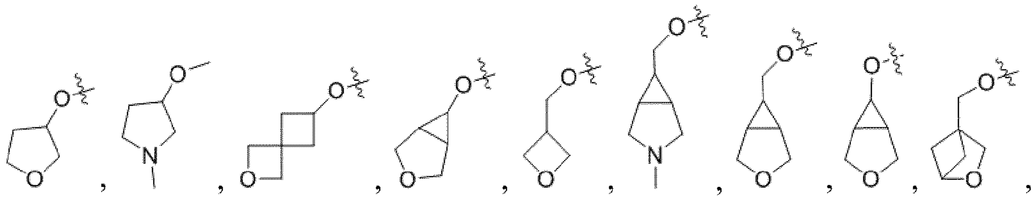

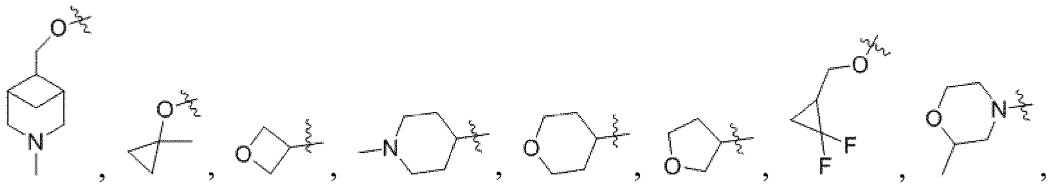

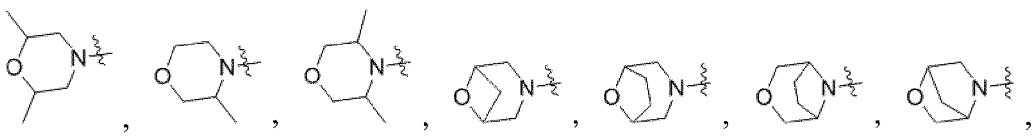

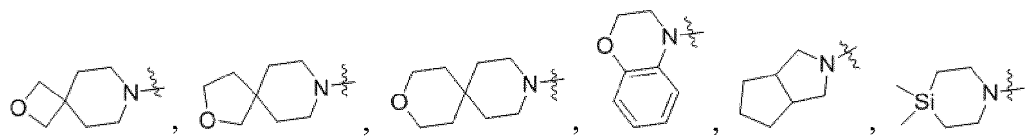

or

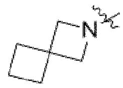
;

each $R_7$' is independently H, D, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $C_{3-6}$ cycloalkyl, $C_{3-12}$ heterocycloalkyl, $-NHC_{1-6}$ alkyl, $-N(C_{1-6}$ alkyl$)_2$ or deuterated $C_{1-6}$ alkoxy, wherein the cycloalkyl or heterocycloalkyl is optionally substituted with 1 to 3 groups selected from deuterium, halogen, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy or deuterated $C_{1-6}$ alkoxy; in some embodiments, each $R_7$' is independently H, D, amino, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, $C_{3-6}$ cycloalkyl, $C_{3-12}$ heterocycloalkyl, $-NHC_{1-4}$ alkyl, $-N(C_{1-4}$ alkyl$)_2$ or deuterated $C_{1-4}$ alkoxy, wherein the cycloalkyl or heterocycloalkyl is optionally substituted with 1 to 3 groups selected from deuterium, halogen, cyano, amino, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy or deuterated $C_{1-4}$ alkoxy; in some embodiments, each $R_7$' is independently H, D, amino, hydroxyl, $C_{1-4}$ alkyl, halo $C_{1-2}$ alkyl, deuterated $C_{1-2}$ alkyl, $C_{1-2}$ alkoxy, halo $C_{1-2}$ alkoxy, $C_{3-6}$ cycloalkyl, $C_{3-12}$ heterocycloalkyl, $-NHC_{1-2}$ alkyl, $-N(C_{1-2}$ alkyl$)_2$ or deuterated $C_{1-2}$ alkoxy, wherein the cycloalkyl or heterocycloalkyl is optionally substituted with 1 to 3 groups selected from deuterium, halogen, cyano, amino, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy or deuterated $C_{1-2}$ alkoxy; in some embodiments, each $R_7$' is independently H, D, amino, hydroxyl or $C_{1-4}$ alkyl; or

each $R_7$' is independently H, $C_{1-4}$ alkyl or halo $C_{1-4}$ alkyl; in some embodiments, each $R_7$' is independently H or $C_{1-4}$ alkyl; in some embodiments, each $R_7$' is independently H;

each $R_8$' is independently H, D, $C_{1-6}$ alkyl, $C_{1-4}$ alkoxy, $C_{3-12}$ carbocycle, or 4-to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-6}$ alkyl, halo $C_{1-6}$ alkyl, deuterated $C_{1-6}$ alkyl, - $C(O)C_{1-4}$ alkyl, $-C(O)NHC_{1-4}$ alkyl, $-C(O)NH_2$, $-NHC(O)C_{1-4}$ alkyl, $C_{1-6}$ alkoxy, halo $C_{1-6}$ alkoxy and deuterated $C_{1-6}$ alkoxy; the carbocycle or heterocycle is optionally substituted with 1-5 groups selected from $R_{8''}$; in some embodiments, each $R_8$' is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy; in some embodiments, each $R_8$' is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy; in some embodiments, $R_8$' is independently $C_{1-4}$ alkoxy, cyclopropyl, cyclobutyl, cyclopentyl, phenyl, tetrahydropyrrolyl, azetidinyl, piperidyl, piperazinyl, morpholinyl, oxetanyl, tetrahydrofuryl, tetrahydropyranyl,

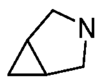
, 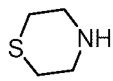
,

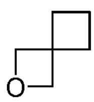
, 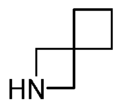
, 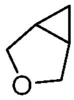
, 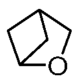
, 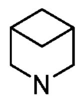
,

thienyl, thiazolyl, pyrrolyl, imidazolyl, oxazolyl, pyridyl, pyrazinyl, pyrimidyl or pyridazinyl optionally substituted with 1-3 groups selected from F, Cl, Br, I, =O, D, OH, $NH_2$, CN, $C_{1-4}$ alkyl, halo $C_{1-2}$ alkyl and $C_{1-2}$ alkoxy; or

each $R_8$' is independently H, D, $C_{1-6}$ alkyl, $C_{1-4}$ alkoxy, $C_{3-12}$ carbocycle, or 4-to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-6}$ alkyl, halo $C_{1-6}$ alkyl, deuterated $C_{1-6}$ alkyl, - $C(O)C_{1-4}$ alkyl, $-C(O)NHC_{1-4}$ alkyl, $-C(O)NH_2$, $-NHC(O)C_{1-4}$ alkyl, $C_{1-6}$ alkoxy, halo $C_{1-6}$ alkoxy and deuterated $C_{1-6}$ alkoxy; the carbocycle or heterocycle is optionally substituted with 1-5 groups selected from $R_{8''}$; in some embodiments, each $R_8$' is independently H, D, $C_{1-4}$ alkyl, $C_{1-4}$ alkoxy, $C_{3-12}$ carbocycle, or 4- to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-4}$ alkyl, halo $C_{1-4}$ alkyl, deuterated $C_{1-4}$ alkyl, $-C(O)C_{1-2}$ alkyl, $-C(O)NHC_{1-2}$ alkyl, $-C(O)NH_2$, $-NHC(O)C_{1-2}$ alkyl, $C_{1-2}$ alkoxy, halo $C_{1-2}$ alkoxy and deuterated $C_{1-2}$ alkoxy; the carbocycle or heterocycle is optionally substituted with 1-5 groups selected from $R_{8''}$; or

each $R_8$' is independently H, $C_{1-4}$ alkoxy, $C_{3-9}$ carbocycle, or 4- to 10-membered heterocycle containing 1-3

heteroatoms selected from N, O or S, wherein the carbocycle or heterocycle is optionally substituted with 1-3 groups selected from halogen, =O, D, OH, $NH_2$, CN, $C_{1-4}$ alkyl, -C(O)$C_{1-4}$ alkyl, -C(O)NH$C_{1-4}$ alkyl, - C(O)$NH_2$, -NHC(O)$C_{1-4}$ alkyl and $C_{1-4}$ alkoxy; in some embodiments, each $R_8$' is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl and $C_{1-4}$ alkoxy; in some embodiments, each $R_8$' is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl and $C_{1-4}$ alkoxy; in some embodiments, $R_8$' is independently $C_{1-4}$ alkoxy, cyclopropyl, cyclobutyl, cyclopentyl, phenyl, tetrahydropyrrolyl, azetidinyl, piperidyl, piperazinyl, morpholinyl, oxetanyl, tetrahydrofuryl, tetrahydropyranyl,

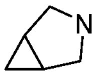, 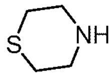, 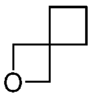, 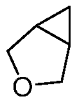, 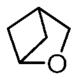, 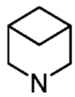,

thienyl, thiazolyl, pyrrolyl, imidazolyl, oxazolyl, pyridyl, pyrazinyl, pyrimidyl or pyridazinyl optionally substituted with 1-3 groups selected from F, Cl, Br, I, =O, D, OH, $NH_2$, CN, $C_{1-4}$ alkyl and $C_{1-4}$ alkoxy;

$R_{8''}$ is selected from halogen, D, OH, $NH_2$, CN, $C_{1-6}$ alkyl and $C_{1-6}$ alkoxy, wherein the alkyl or alkoxy is optionally further substituted with 1 to 5 groups selected from halogen, deuterium, cyano, amino, hydroxyl, $C_{1-6}$ alkyl or $C_{1-6}$ alkoxy; in some embodiments, $R_{8''}$ is selected from halogen, D, OH, $NH_2$, CN, $C_{1-4}$ alkyl and $C_{1-4}$ alkoxy, wherein the alkyl or alkoxy is optionally further substituted with 1 to 3 groups selected from halogen, deuterium, cyano, amino, hydroxyl, $C_{1-2}$ alkyl or $C_{1-2}$ alkoxy; in some specific embodiments, $R_{8''}$ is selected from deuterium, F, Cl, methyl, ethyl, propyl, methoxy, ethoxy or propoxy, wherein the methyl, ethyl, propyl, methoxy, ethoxy or propoxy is optionally further substituted with 1 to 3 groups selected from F, Cl, deuterium, hydroxyl, methyl, ethyl, propyl, methoxy, ethoxy or propoxy;

each r is independently 0, 1, 2 or 3; in some embodiments, each r is independently 0, 1 or 2;

p is 0, 1 or 2; in some embodiments, p is 0 or 1;

n and m are independently an integer of 0-5; in some embodiments, n is 0, 1, 2 or 3; in some embodiments, m is 0, 1, 2, 3 or 4;

In the compound of the present invention:

1. the group

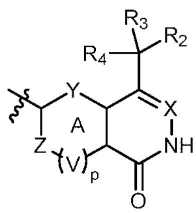

is not

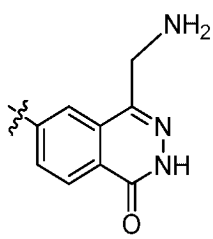

;

2. when

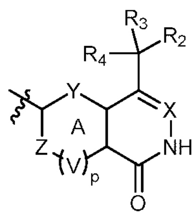 is 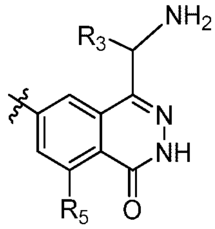 ,

$R_3$ is **H,** $C_{1-4}$ alkyl or $C_{1-4}$ haloalkyl, $R_5$ is H, halogen, $C_{1-4}$ alkyl, $C_{1-4}$ alkoxy or $C_{1-4}$ haloalkyl, and $R_3$ and $R_5$ are not both H, the group

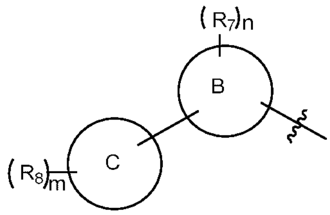 is 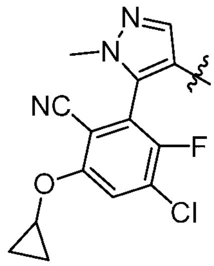 .

More specifically, a first technical solution of the present invention relates to a compound represented by formula (I-a) or (I-b), or a stereoisomer, a deuterated compound, a solvate or a pharmaceutically acceptable salt thereof,

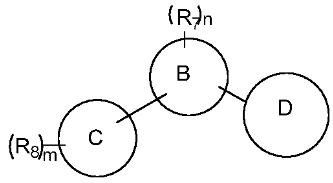

I-a

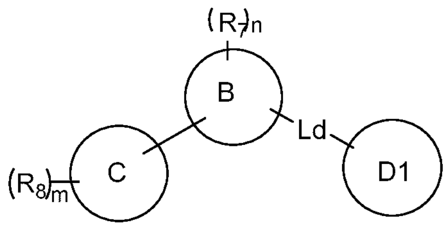

I-b

wherein

D is selected from:

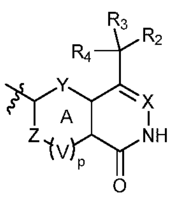 , 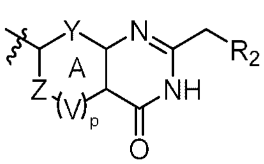 , 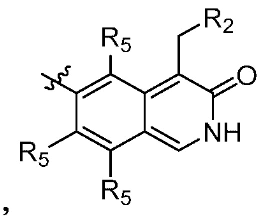 ,

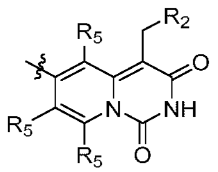, 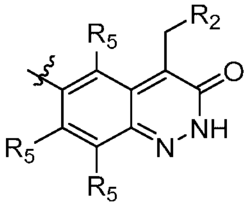, 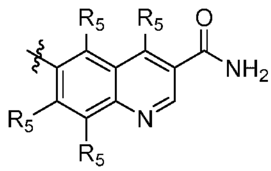, 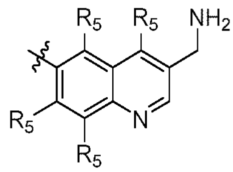,

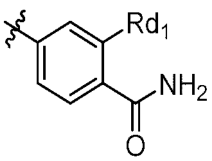, 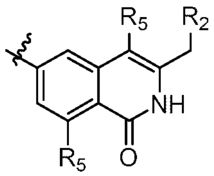, 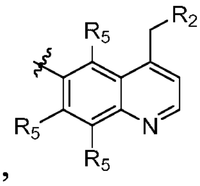, 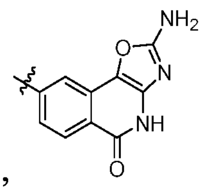, 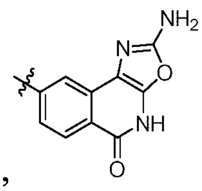

or

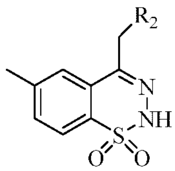;

D1 is selected from:

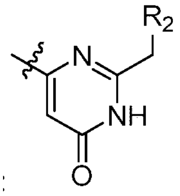;

Ld is selected from $C_{1-4}$ alkyl, $C_{2-4}$ alkenyl or $C_{2-4}$ alkynyl;
Rd1 is selected from 5-membered heteroaryl, wherein the heteroaryl is optionally substituted with 1-3 groups selected from halogen, D, CN, OH, $C_{1-4}$ alkyl, $C_{1-4}$ alkoxy and $NH_2$;
X is selected from $CR_1$ or N;
Y, Z and V are independently $CR_5$, $C(R_5)_2$, $NR_6$, N, O or S, and the total number of O and S is not greater than 1;
ring A is phenyl, 5- to 7-membered heterocycloalkyl containing 1-3 heteroatoms selected from N, O or S, or 5- to 6-membered heteroaryl containing 1-3 heteroatoms selected from N, O or S;
ring B is $C_{3-12}$ carbocycle, or 4- to 12-membered heterocycle containing 1-3 heteroatoms selected from N, O or S;
ring C is $C_{3-12}$ carbocycle, or 4- to 12-membered heterocycle containing 1-3 heteroatoms selected from N, O or S;
$R_1$ is H, D, $C_{1-4}$ alkyl, $C_{1-4}$ alkoxy, $C_{2-4}$ alkenyl, $C_{2-4}$ alkynyl, halogen, CN, OH, $NH_2$ or COOH, wherein the alkyl, alkoxy, alkenyl or alkynyl is optionally substituted with 1-3 groups selected from halogen, D, CN, OH and $NH_2$;
$R_2$ is OH, $NH_2$, $-CH_2NH_2$ or -NH-OH;
$R_3$ and $R_4$ are independently H, D, halogen, $C_{1-4}$ alkyl, CN, OH, $NH_2$, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$;
alternatively, $R_3$ and $R_4$ together form =NH;
each $R_5$ is independently H, D, =O, OH, $C_{1-4}$ alkyl, CN, halogen, $C_{1-4}$ alkoxy, $C_{2-4}$ alkenyl, $C_{2-4}$ alkynyl, 5- to 6-membered heteroaryl containing 1-3 heteroatoms selected from N, O or S, 4- to 7-membered heterocycloalkyl containing 1-3 heteroatoms selected from N, O or S, or $C_{3-6}$ cycloalkyl, wherein the alkyl, alkoxy, alkenyl, alkynyl, heteroaryl or cycloalkyl is optionally substituted with 1-3 groups selected from halogen, D, CN, OH, $C_{1-4}$ alkyl, $C_{1-4}$ alkoxy and $NH_2$;
$R_6$ is H, D, $C_{1-4}$ alkyl or $C_{3-6}$ cycloalkyl, wherein the alkyl or cycloalkyl is optionally substituted with 1-3 groups selected from halogen, D, CN, OH and $NH_2$;
each $R_7$ is independently H, =O, D, OH, CN, halogen, $C_{1-4}$ alkyl, $C_{1-4}$ alkoxy, $N(R_7')_2$, $C_{2-4}$ alkenyl, $C_{2-4}$ alkynyl, $C_{3-6}$

cycloalkyl or 4- to 7-membered heterocycloalkyl containing 1-3 heteroatoms selected from N, O or S, wherein the alkyl, alkoxy, alkenyl, alkynyl, cycloalkyl or heterocycloalkyl is optionally substituted with 1-3 groups selected from halogen, D, OH, $NH_2$, CN, $C_{1-4}$ alkyl, - $C(O)C_{1-4}$ alkyl, $-C(O)NHC_{1-4}$ alkyl, $-C(O)NH_2$, $-NHC(O)C_{1-4}$ alkyl and $C_{1-4}$ alkoxy;

each $R_8$ is independently H, D, nitro, amino, $C_{1-6}$ alkoxy, =O, OH, CN, halogen, $C_{2-6}$ alkenyl, $C_{2-6}$ alkynyl, $-SF_5$, $N_3$, $C_{1-6}$ alkyl, $-COC_{1-4}$ alkyl, $-N(R_7')_2$, - $C(=O)N(R_7')_2$, $-NR_7'C(=O)-R_7'$, $-C(=O)-R_7'$, $-(CH_2)_r-O-(CH_2)_r-R_8'$, $-S-C_{1-6}$ alkyl, - $S(O)-C_{1-6}$ alkyl, $-S(O)_2-C_{1-6}$ alkyl, $-S-(CH_2)_r-R_8'$, $-NR_7'-(CH_2)_r-R_8'$ or $-(CH_2)_r-R_8'$, wherein the alkyl, alkenyl or alkynyl is optionally substituted with 1-5 groups selected from $R_{8''}$;

each $R_7'$ is independently H, D, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $C_{3-6}$ cycloalkyl, $C_{3-12}$ heterocycloalkyl, $-NHC_{1-6}$ alkyl, $-N(C_{1-6}\ alkyl)_2$ or deuterated $C_{1-6}$ alkoxy, wherein the cycloalkyl or heterocycloalkyl is optionally substituted with 1 to 3 groups selected from deuterium, halogen, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy or deuterated $C_{1-6}$ alkoxy;

each $R_8'$ is independently H, D, $C_{1-6}$ alkyl, $C_{1-4}$ alkoxy, $C_{3-12}$ carbocycle, or 4-to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-6}$ alkyl, halo $C_{1-6}$ alkyl, deuterated $C_{1-6}$ alkyl, - $C(O)C_{1-4}$ alkyl, $-C(O)NHC_{1-4}$ alkyl, $-C(O)NH_2$, $-NHC(O)C_{1-4}$ alkyl, $C_{1-6}$ alkoxy, halo $C_{1-6}$ alkoxy and deuterated $C_{1-6}$ alkoxy; the carbocycle or heterocycle is optionally substituted with 1-5 groups selected from $R_{8''}$;

$R_{8''}$ is selected from halogen, D, OH, $NH_2$, CN, $C_{1-6}$ alkyl and $C_{1-6}$ alkoxy, wherein the alkyl or alkoxy is optionally further substituted with 1 to 5 groups selected from halogen, deuterium, cyano, amino, hydroxyl, $C_{1-6}$ alkyl or $C_{1-6}$ alkoxy;

each r is independently 0, 1, 2 or 3;

p is 0, 1 or 2;

n and m are independently an integer of 0-5;

provided that:

1. the group

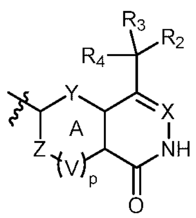

is not

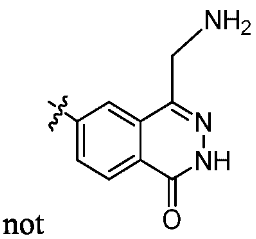

;

2. when

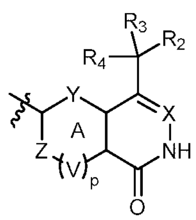

is

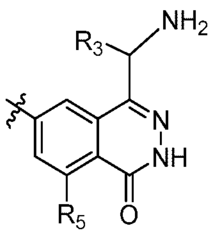

,

$R_3$ is H, $C_{1-4}$ alkyl or $C_{1-4}$ haloalkyl, $R_5$ is H, halogen, $C_{1-4}$ alkyl, $C_{1-4}$ alkoxy or $C_{1-4}$ haloalkyl, and $R_3$ and $R_5$ are not both H, the group

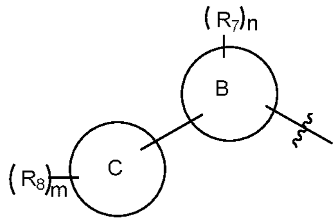 is 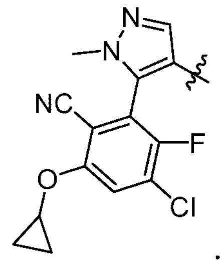 .

More specifically, a second technical solution of the present invention provides a compound represented by formula (I-a), or a stereoisomer, a deuterated compound, a solvate or a pharmaceutically acceptable salt thereof,

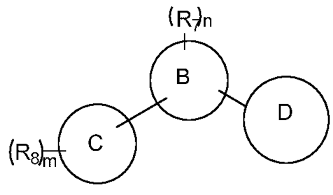

I-a

wherein

D is selected from:

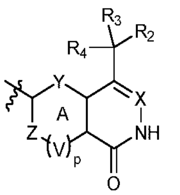 , 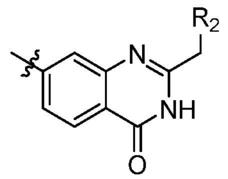 , 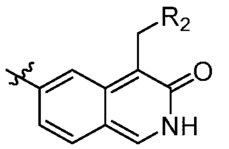 ,

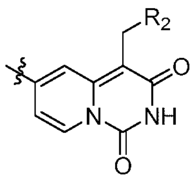 , 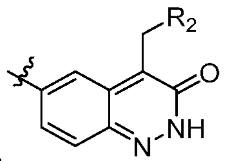 ;

X is selected from $CR_1$ or N;
Y, Z and V are independently $CR_5$, $C(R_5)_2$, $NR_6$, N, O or S, and the total number of O and S is not greater than 1;
ring A is phenyl, 5- to 7-membered heterocycloalkyl containing 1-3 heteroatoms selected from N, O or S, or 5- to 6-membered heteroaryl containing 1-3 heteroatoms selected from N, O or S;
ring B is $C_{3-12}$ carbocycle, or 4- to 12-membered heterocycle containing 1-3 heteroatoms selected from N, O or S;
ring C is $C_{3-12}$ carbocycle, or 4- to 12-membered heterocycle containing 1-3 heteroatoms selected from N, O or S;
$R_1$ is H, D, $C_{1-4}$ alkyl, $C_{1-4}$ alkoxy, $C_{2-4}$ alkenyl, $C_{2-4}$ alkynyl, halogen, CN, OH, $NH_2$ or COOH, wherein the alkyl, alkoxy, alkenyl or alkynyl is optionally substituted with 1-3 groups selected from halogen, D, CN, OH and $NH_2$;
$R_2$ is OH, $NH_2$, $-CH_2NH_2$ or -NH-OH;
$R_3$ and $R_4$ are independently H, D, halogen, $C_{1-4}$ alkyl, CN, OH, $NH_2$, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$;
alternatively, $R_3$ and $R_4$ together form =NH;
each $R_5$ is independently H, D, =O, OH, $C_{1-4}$ alkyl, CN, halogen, $C_{1-4}$ alkoxy, $C_{2-4}$ alkenyl, $C_{2-4}$ alkynyl, 5- to 6-membered heteroaryl containing 1-3 heteroatoms selected from N, O or S, 4- to 7-membered heterocycloalkyl containing 1-3 heteroatoms selected from N, O or S, or $C_{3-6}$ cycloalkyl, wherein the alkyl, alkoxy, alkenyl, alkynyl, heteroaryl or cycloalkyl is optionally substituted with 1-3 groups selected from halogen, D, CN, OH, $C_{1-4}$ alkyl, $C_{1-4}$ alkoxy and $NH_2$;

$R_6$ is H, D, $C_{1-4}$ alkyl or $C_{3-6}$ cycloalkyl, wherein the alkyl or cycloalkyl is optionally substituted with 1-3 groups selected from halogen, D, CN, OH and $NH_2$;

each $R_7$ is independently H, =O, D, OH, CN, halogen, $C_{1-4}$ alkyl, $C_{1-4}$ alkoxy, $N(R_7')_2$, $C_{2-4}$ alkenyl, $C_{2-4}$ alkynyl, $C_{3-6}$ cycloalkyl or 4- to 7-membered heterocycloalkyl containing 1-3 heteroatoms selected from N, O or S, wherein the alkyl, alkoxy, alkenyl, alkynyl, cycloalkyl or heterocycloalkyl is optionally substituted with 1-3 groups selected from halogen, D, OH, $NH_2$, CN, $C_{1-4}$ alkyl, - $C(O)C_{1-4}$ alkyl, $-C(O)NHC_{1-4}$ alkyl, $-C(O)NH_2$, $-NHC(O)C_{1-4}$ alkyl and $C_{1-4}$ alkoxy;

each $R_8$ is independently H, D, nitro, amino, $C_{1-6}$ alkoxy, =O, OH, CN, halogen, $C_{2-6}$ alkenyl, $C_{2-6}$ alkynyl, $-SF_5$, $C_{1-6}$ alkyl, $-COC_{1-4}$ alkyl, $-N(R_7')_2$, $-C(=O)N(R_7')_2$, $-NR_7'C(=O)-R_7'$, $-C(=O)-R_7'$, $-(CH_2)_r-O-(CH_2)_r-R_8'$, $-S-C_{1-6}$ alkyl, $-S(O)-C_{1-6}$ alkyl, $-S(O)_2-C_{1-6}$ alkyl, $-S-(CH_2)_r-R_8'$, $-NR_7'-(CH_2)_r-R_8'$ or $-(CH_2)_r-R_8'$ or $N_3$, wherein the alkyl, alkenyl or alkynyl is optionally substituted with 1-5 groups selected from $R_{8''}$;

each $R_7'$ is independently H, D, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $C_{3-6}$ cycloalkyl, $C_{3-12}$ heterocycloalkyl, $-NHC_{1-6}$ alkyl, $-N(C_{1-6}\ alkyl)_2$ or deuterated $C_{1-6}$ alkoxy, wherein the cycloalkyl or heterocycloalkyl is optionally substituted with 1 to 3 groups selected from deuterium, halogen, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy or deuterated $C_{1-6}$ alkoxy;

each $R_8'$ is independently H, D, $C_{1-6}$ alkyl, $C_{1-4}$ alkoxy, $C_{3-12}$ carbocycle, or 4-to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-6}$ alkyl, halo $C_{1-6}$ alkyl, deuterated $C_{1-6}$ alkyl, - $C(O)C_{1-4}$ alkyl, $-C(O)NHC_{1-4}$ alkyl, $-C(O)NH_2$, $-NHC(O)C_{1-4}$ alkyl, $C_{1-6}$ alkoxy, halo $C_{1-6}$ alkoxy and deuterated $C_{1-6}$ alkoxy; the carbocycle or heterocycle is optionally substituted with 1-5 groups selected from $R_{8''}$;

$R_{8''}$ is selected from halogen, D, OH, $NH_2$, CN, $C_{1-6}$ alkyl and $C_{1-6}$ alkoxy, wherein the alkyl or alkoxy is optionally further substituted with 1 to 5 groups selected from halogen, deuterium, cyano, amino, hydroxyl, $C_{1-6}$ alkyl or $C_{1-6}$ alkoxy;

each r is independently 0, 1, 2 or 3;

p is 0, 1 or 2;

n and m are independently an integer of 0-5;

provided that:

1. the group

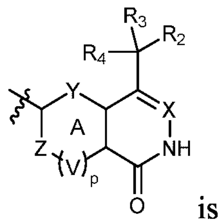 is not 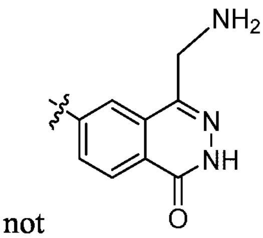 ;

2. when

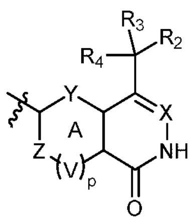 is 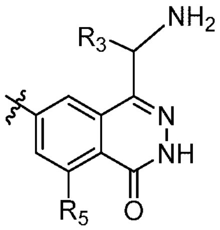 ,

$R_3$ is H, $C_{1-4}$ alkyl or $C_{1-4}$ haloalkyl, $R_5$ is H, halogen, $C_{1-4}$ alkyl, $C_{1-4}$ alkoxy or $C_{1-4}$ haloalkyl, and $R_3$ and $R_5$ are not both H, the group

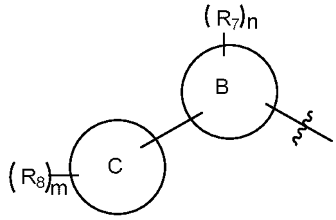 is 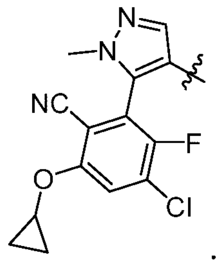 .

More specifically, a third technical solution of the present invention provides a compound represented by formula (I-a), or a stereoisomer, a deuterated compound, a solvate or a pharmaceutically acceptable salt thereof, wherein the compound has a structure of formula (I) below:

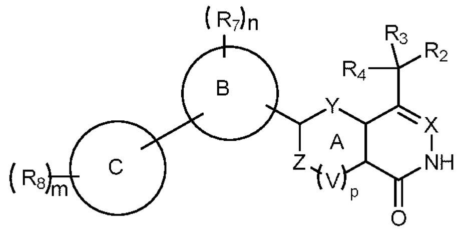

I ;

other groups are as defined in any of the preceding technical solutions.

More specifically, a fourth technical solution of the present invention provides a compound represented by formula (I), or a stereoisomer, a deuterated compound, a solvate or a pharmaceutically acceptable salt thereof,

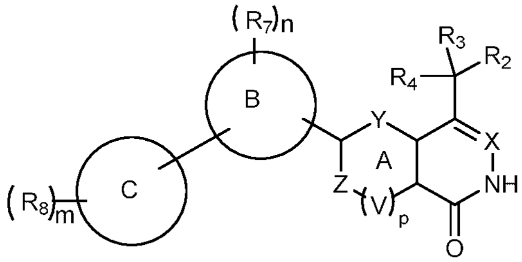

I

wherein X is selected from $CR_1$ or N;

- Y, Z and V are independently $CR_5$, $C(R_5)_2$, $NR_6$, N, O or S, and the total number of O and S is not greater than 1;
- ring A is phenyl, 5- to 7-membered heterocycloalkyl containing 1-3 heteroatoms selected from N, O or S, or 5- to 6-membered heteroaryl containing 1-3 heteroatoms selected from N, O or S;
- ring B is $C_{3-12}$ carbocycle, or 4- to 12-membered heterocycle containing 1-3 heteroatoms selected from N, O or S;
- ring C is $C_{3-12}$ carbocycle, or 4- to 12-membered heterocycle containing 1-3 heteroatoms selected from N, O or S;
- $R_1$ is H, D, $C_{1-4}$ alkyl, $C_{1-4}$ alkoxy, $C_{2-4}$ alkenyl, $C_{2-4}$ alkynyl, halogen, CN, OH, $NH_2$ or COOH, wherein the alkyl, alkoxy, alkenyl or alkynyl is optionally substituted with 1-3 groups selected from halogen, D, CN, OH and $NH_2$;
- $R_2$ is OH, $NH_2$, $-CH_2NH_2$ or -NH-OH; or $R_2$ is OH, $NH_2$ or -NH-OH;
- $R_3$ and $R_4$ are independently H, D, halogen, $C_{1-4}$ alkyl, CN, OH, $NH_2$, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$;
- each $R_5$ is independently H, D, =O, OH, $C_{1-4}$ alkyl, CN, halogen, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$;
- $R_6$ is H, D, $C_{1-4}$ alkyl or $C_{3-6}$ cycloalkyl, wherein the alkyl or cycloalkyl is optionally substituted with 1-3 groups selected from halogen, D, CN, OH and $NH_2$;
- each $R_7$ is independently H, =O, D, OH, CN, halogen, $C_{1-4}$ alkyl, $C_{1-4}$ alkoxy, $N(R_7')_2$, $C_{2-4}$ alkenyl, $C_{2-4}$ alkynyl, $C_{3-6}$

cycloalkyl or 4- to 7-membered heterocycloalkyl containing 1-3 heteroatoms selected from N, O or S, wherein the alkyl, alkoxy, alkenyl, alkynyl, cycloalkyl or heterocycloalkyl is optionally substituted with 1-3 groups selected from halogen, D, OH, $NH_2$, CN, $C_{1-4}$ alkyl, - $C(O)C_{1-4}$ alkyl, -$C(O)NHC_{1-4}$ alkyl, -$C(O)NH_2$, -$NHC(O)C_{1-4}$ alkyl and $C_{1-4}$ alkoxy;

each $R_8$ is independently H, D, =O, OH, CN, halogen, $C_{2-4}$ alkenyl, $C_{2-4}$ alkynyl, -$SF_5$, $C_{1-4}$ alkyl, -$COC_{1-4}$ alkyl, -$CONR_7'C_{1-4}$ alkyl, -$NR_7'COC_{1-4}$ alkyl, $N(R_7')_2$, -O-$(CH_2)$r-$R_8'$, -S-$(CH_2)$r-$R_8'$, -$NR_7'$-$(CH_2)$r-$R_8'$ or -$(CH_2)$r-$R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;

each $R_7'$ is independently H, $C_{1-4}$ alkyl or halo $C_{1-4}$ alkyl;

each $R_8'$ is independently H, $C_{1-4}$ alkoxy, $C_{3-9}$ carbocycle, or 4- to 10-membered heterocycle containing 1-3 heteroatoms selected from N, O or S, wherein the carbocycle or heterocycle is optionally substituted with 1-3 groups selected from halogen, =O, D, OH, $NH_2$, CN, $C_{1-4}$ alkyl, -$C(O)C_{1-4}$ alkyl, -$C(O)NHC_{1-4}$ alkyl, - $C(O)NH_2$, -$NHC(O)C_{1-4}$ alkyl and $C_{1-4}$ alkoxy;

each r is independently 0, 1, 2 or 3;

p is 0, 1 or 2;

n and m are independently an integer of 0-5;

provided that:

1. the group

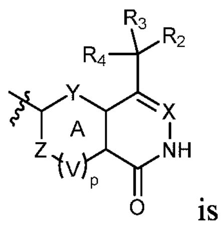 is not 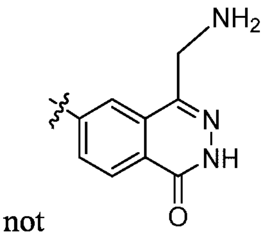 ;

2. when

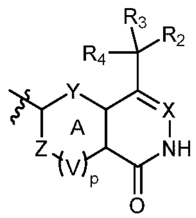 is 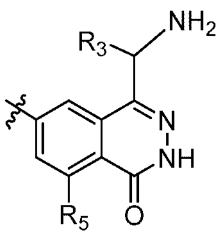 ,

$R_3$ is H, $C_{1-4}$ alkyl or $C_{14}$ haloalkyl, $R_5$ is H, halogen, $C_{1-4}$ alkyl, $C_{1-4}$ alkoxy or $C_{1-4}$ haloalkyl, and $R_3$ and $R_5$ are not both H, the group

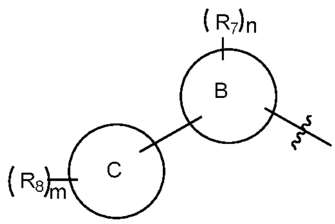 is 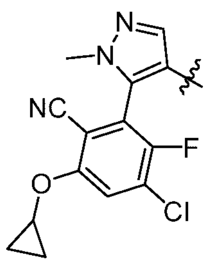 .

More specifically, a fifth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

D is selected from

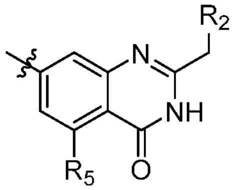 , 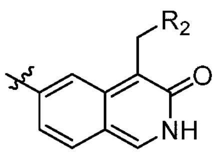 , 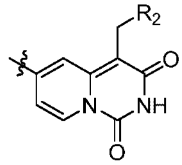 ,

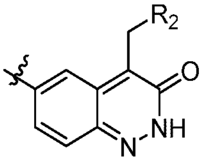 , 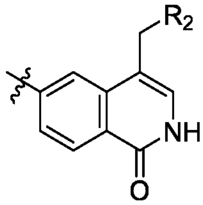 , 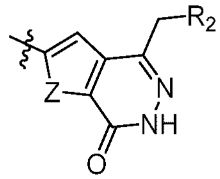 , 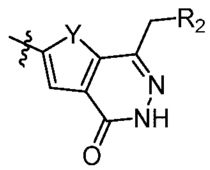 ,

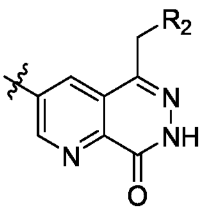 , 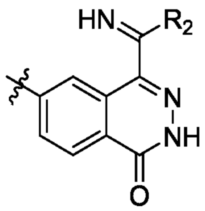 , 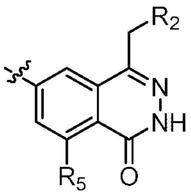 , 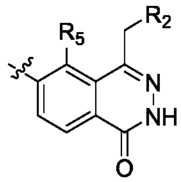 , 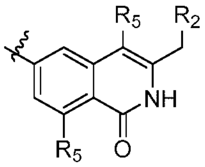 ,

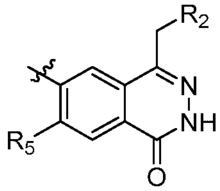 , 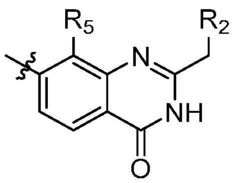 , 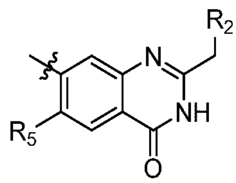 , 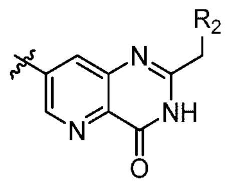 ,

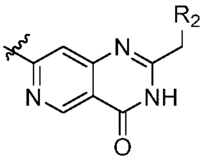 , 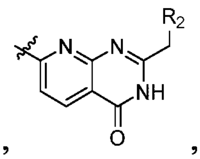 , 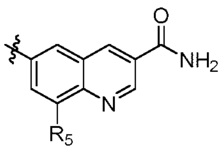 , 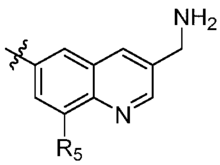 , 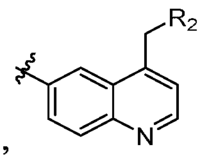 ,

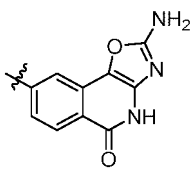 , 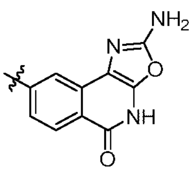 , 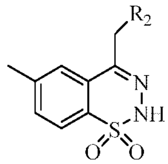 ;

further, D is selected from

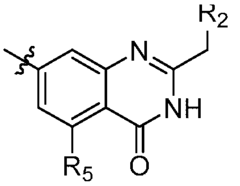 , 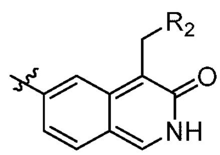 , 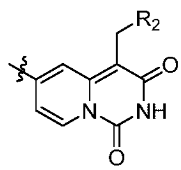 , 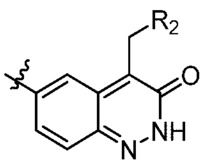 ,

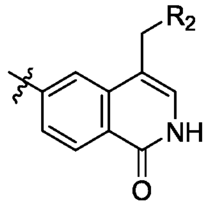, 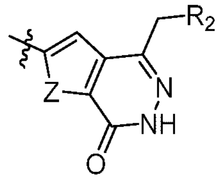, 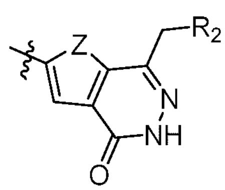, 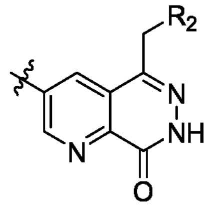,

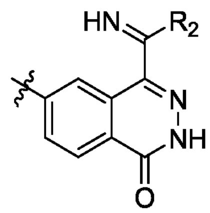, 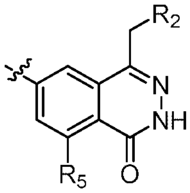, 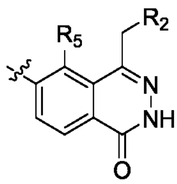, 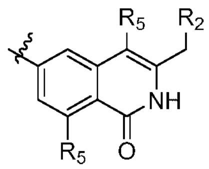, 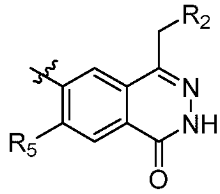,

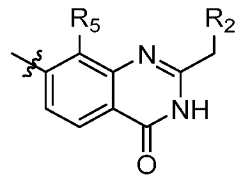, 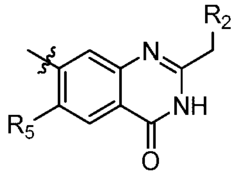, 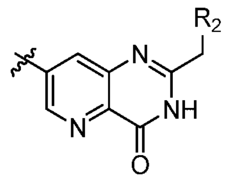, 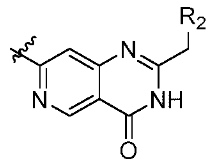,

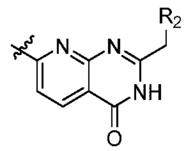, 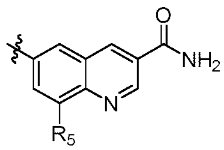, 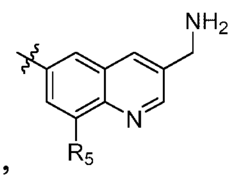, 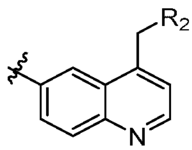, 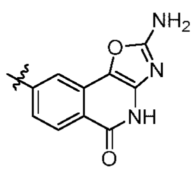,

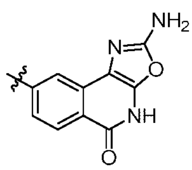;

further, D is selected from

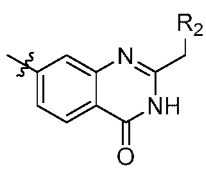, 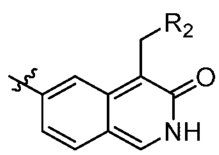,

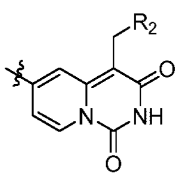, 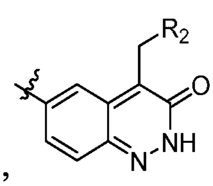, 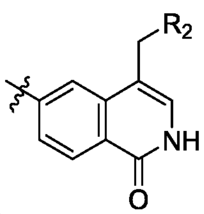, 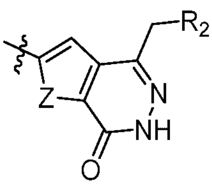, 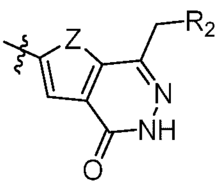,

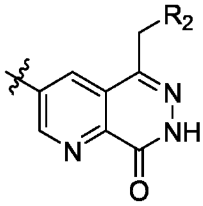 , 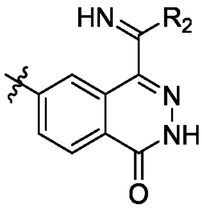 , 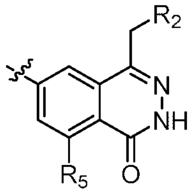 , 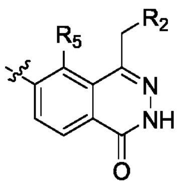 , 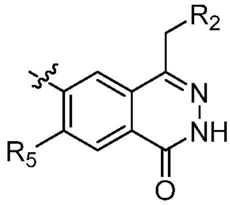 ;

further,
D is selected from

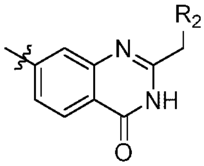 , 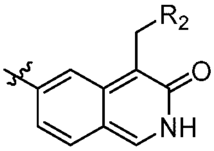 , 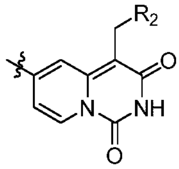 ,

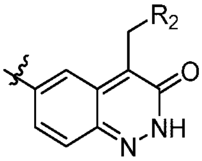 , 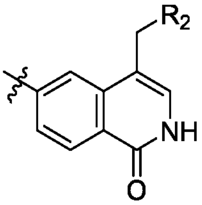 , 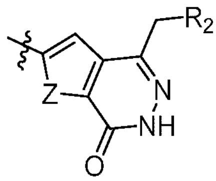 , 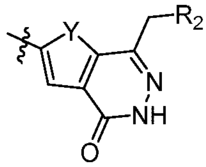 ,

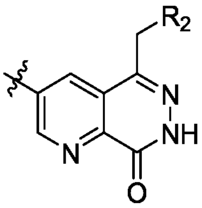 , 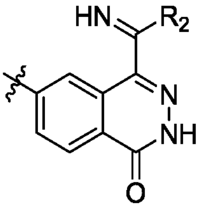 ;

further,
D is selected from

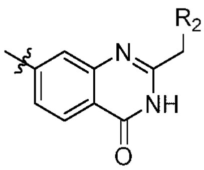 , 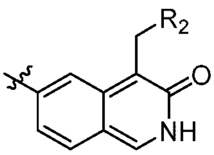 , 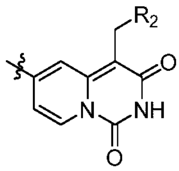 ,

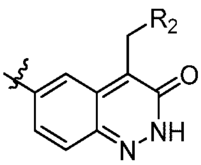 ;

other groups are as defined in any of the preceding technical solutions.

More specifically, a sixth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

D is selected from

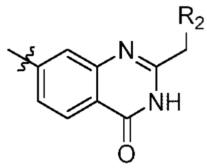, 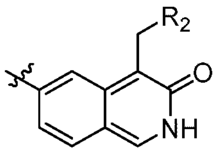, 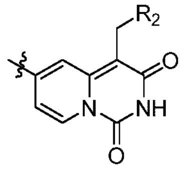,

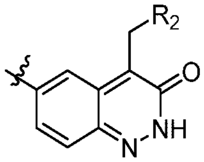, 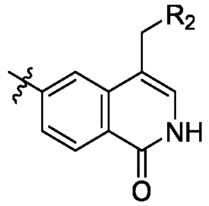, 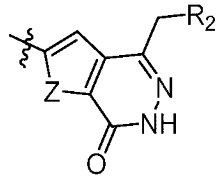, 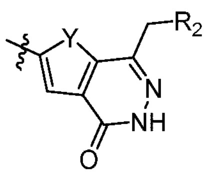,

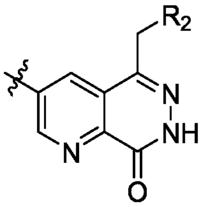, 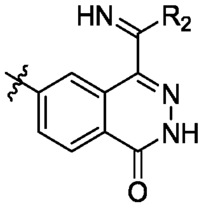;

other groups are as defined in any of the preceding technical solutions.

More specifically, a seventh technical solution of the present invention provides a compound represented by formula (I-a), formula (I-b) or formula (I), or a stereoisomer, a deuterated compound, a solvate or a pharmaceutically acceptable salt thereof,

wherein X is selected from $CR_1$ or N;
Y, Z and V are independently $CR_5$, N, O or S, and the total number of O and S is not greater than 1;
ring A is phenyl, or 5- to 6-membered heteroaryl containing 1-3 heteroatoms selected from N, O or S;
ring B is phenyl, $C_{3-6}$ cycloalkyl, 4- to 7-membered heterocycloalkyl containing 1-3 heteroatoms selected from N, O or S, or 5- to 6-membered aryl containing 1-3 heteroatoms selected from N, O or S;
ring C is phenyl, $C_{3-6}$ cycloalkyl, 4- to 7-membered heterocycloalkyl containing 1-3 heteroatoms selected from N, O or S, or 5- to 6-membered aryl containing 1-3 heteroatoms selected from N, O or S;
$R_1$ is H, D, $C_{1-4}$ alkyl, halogen or CN, wherein the alkyl is optionally substituted with 1-3 groups selected from halogen, D, CN and OH;
$R_2$ is OH, $NH_2$, $-CH_2NH_2$ or -NH-OH; or $R_2$ is OH, $NH_2$ or -NH-OH;
$R_3$ and $R_4$ are independently H, D, halogen, $C_{1-4}$ alkyl, CN, OH or $NH_2$, wherein the alkyl is optionally substituted with 1-3 groups selected from halogen, D, CN, OH and $NH_2$;
each $R_5$ is independently H, D, OH, $C_{1-4}$ alkyl, CN, halogen, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from halogen, D, CN, OH, $C_{1-4}$ alkyl, $C_{1-4}$ alkoxy and $NH_2$; in certain embodiments, each $R_5$ is independently H, D, OH, $C_{1-4}$ alkyl, CN, halogen, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from halogen, D, CN, OH and $NH_2$; or each $R_5$ is independently H, D, OH, $C_{1-4}$ alkyl, CN, halogen, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from halogen, D, CN, OH and $NH_2$;
each $R_7$ is independently H, =O, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl, $-C(O)C_{1-4}$ alkyl, $-C(O)NHC_{1-4}$ alkyl, $-C(O)NH_2$, $-NHC(O)C_{1-4}$ alkyl and $C_{1-4}$ alkoxy;
each $R_8$ is independently H, D, =O, OH, CN, halogen, $C_{2-4}$ alkenyl, $C_{2-4}$ alkynyl, $N_3$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy; or each $R_8$ is

independently H, D, =O, OH, CN, halogen, $C_{2-4}$ alkenyl, $C_{2-4}$ alkynyl, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;
each $R_7'$ is independently H, $C_{1-4}$ alkyl or halo $C_{1-4}$ alkyl;
each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy; or each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl and $C_{1-4}$ alkoxy;
each r is independently 0, 1 or 2;
p is 0 or 1;
n and m are independently 0, 1, 2, 3 or 4;
other groups are as defined in any one of the first, second, third, fourth, fifth and sixth technical solutions.

More specifically, an eighth technical solution of the present invention provides the compound, or the stereoisomer, the deuterated compound, the solvate or the pharmaceutically acceptable salt thereof according to the present invention, wherein ring B is selected from

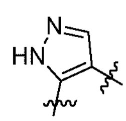 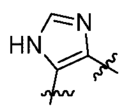 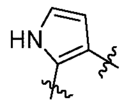 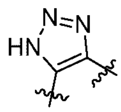 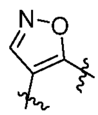 .

More specifically, a ninth technical solution of the present invention provides a compound represented by formula (I-a) or formula (I), or a stereoisomer, a deuterated compound, a solvate or a pharmaceutically acceptable salt thereof, wherein the compound has a structure of formula (II-a) or formula (II):

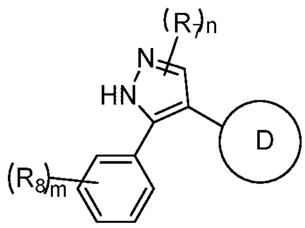
II-a

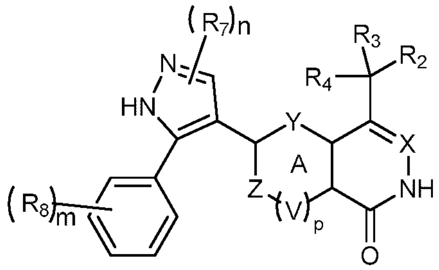
II

;

other groups are as defined in the first, second, third, fourth, fifth, sixth, seventh and eighth technical solutions.

More specifically, a tenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof,
wherein

X is selected from $CR_1$ or N;
Y, Z and V are independently $CR_5$, N, O or S, and the total number of O and S is not greater than 1;
ring A is phenyl, thienyl, thiazolyl, pyrrolyl, imidazolyl, oxazolyl, pyridyl, pyrazinyl, pyrimidyl or pyridazinyl;
D is selected from

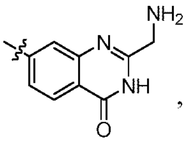, 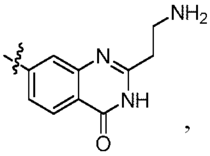, 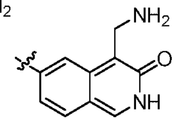, 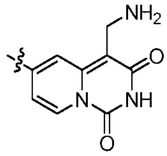, 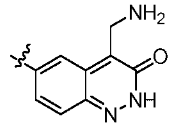,

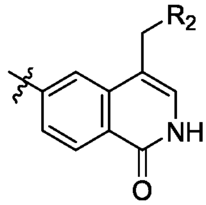, 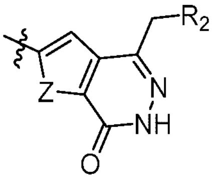, 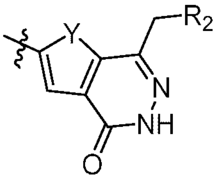, 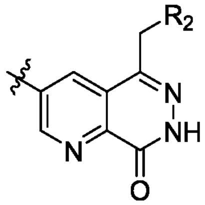,

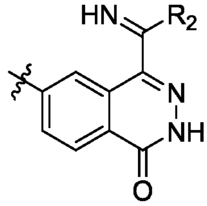, 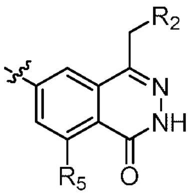, 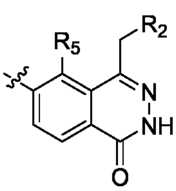, 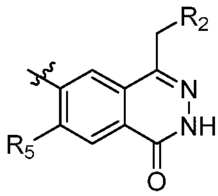, 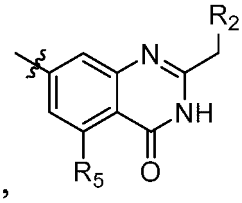,

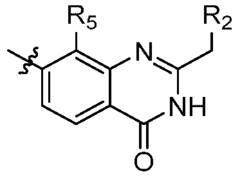, 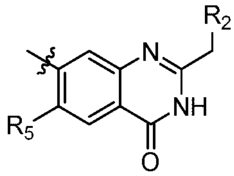, 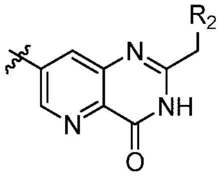, 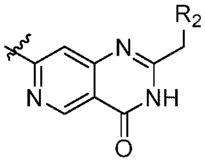,

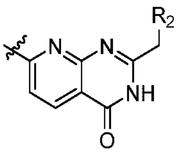, 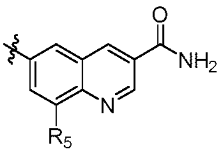, 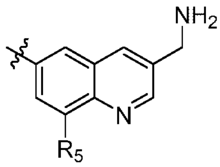, 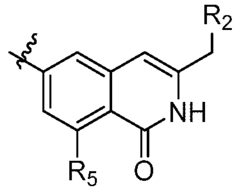,

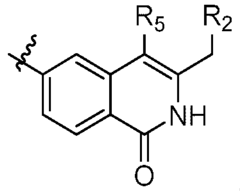, 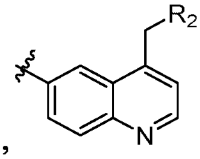, 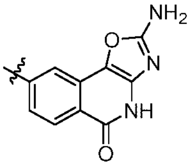, 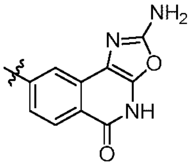 or 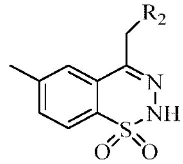;

or D is selected from

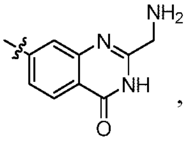, 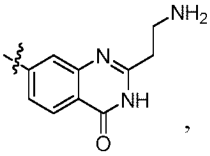, 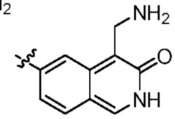, 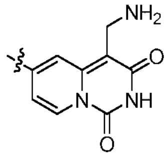, 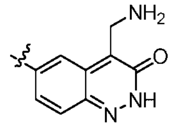,

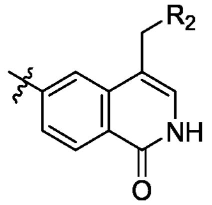,
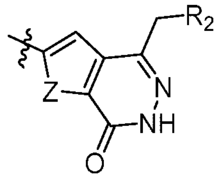,
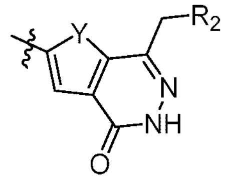,
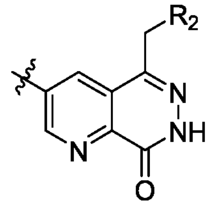,

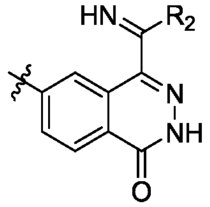,
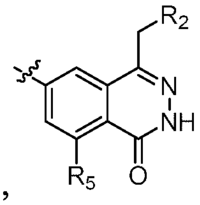,
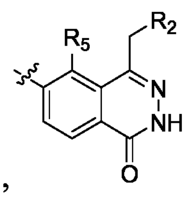;

or D is selected from

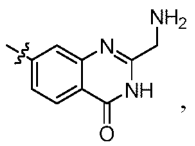,
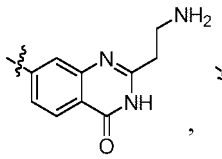,
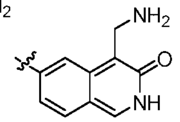,
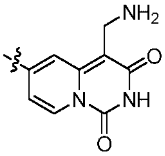,
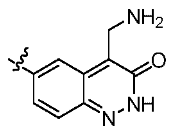;

$R_1$ is H, D, $C_{1-4}$ alkyl, F, Cl, Br, I or CN, wherein the alkyl is optionally substituted with 1-3 groups selected from F, Cl, Br, I, D, CN and OH;

$R_2$ is OH, $NH_2$, $-CH_2NH_2$ or -NH-OH;

$R_3$ and $R_4$ are independently H, D, F, Cl, Br, I, $C_{1-4}$ alkyl or CN, wherein the alkyl is optionally substituted with 1-3 groups selected from F, Cl, Br, I, D, CN, OH and $NH_2$;

each $R_5$ is independently H, D, OH, $C_{1-4}$ alkyl, CN, F, Cl, Br, I, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from F, Cl, Br, I, D, CN, OH, methoxy and $NH_2$; in certain embodiments, each $R_5$ is independently H, D, OH, $C_{1-4}$ alkyl, CN, F, Cl, Br, I, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from F, Cl, Br, I, D, CN, OH and $NH_2$; or each $R_5$ is independently H, D, OH, $C_{1-4}$ alkyl, CN, F, Cl, Br, I, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from F, Cl, Br, I, D, CN, OH and $NH_2$;

each $R_7$ is independently H, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl and $C_{1-4}$ alkoxy;

each $R_8$ is independently H, D, OH, CN, F, Cl, Br, I, $C_{2-4}$ alkenyl, $C_{2-4}$ alkynyl, $N_3$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy; or each $R_8$ is independently H, D, OH, CN, F, Cl, Br, I, $C_{2-4}$ alkenyl, $C_{2-4}$ alkynyl, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;

each $R_7'$ is independently H, $C_{1-4}$ alkyl or halo $C_{1-4}$ alkyl;

each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy; or each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN,

$C_{1-4}$ alkyl and $C_{1-4}$ alkoxy;
each r is independently 0, 1 or 2;
p is 0 or 1;
n is 0, 1, 2 or 3;
m is 0, 1, 2, 3 or 4;
other groups are as defined in the first, second, third, fourth, fifth, sixth, seventh, eighth and ninth technical solutions.

More specifically, an eleventh technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

X is selected from $CR_1$ or N;
Y, Z and V are independently $CR_5$, N, O or S, and the total number of O and S is not greater than 1;
ring A is phenyl, thienyl, thiazolyl, pyrrolyl, imidazolyl, oxazolyl, pyridyl, pyrazinyl, pyrimidyl or pyridazinyl;
D is selected from

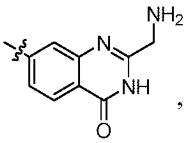 , 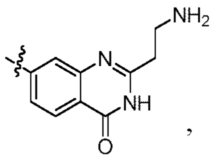 , 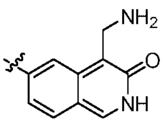 , 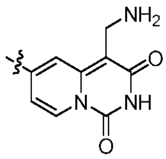 , 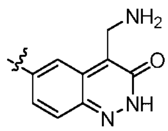 ,

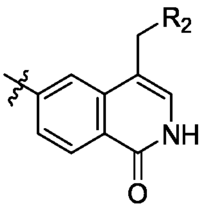 , 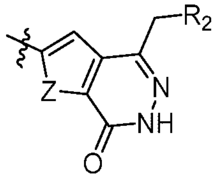 , 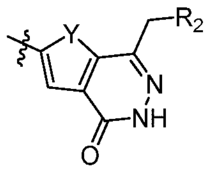 , 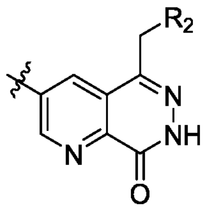 ,

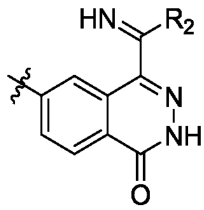 ;

$R_1$ is H, D, $C_{1-4}$ alkyl, F, Cl, Br, I or CN, wherein the alkyl is optionally substituted with 1-3 groups selected from F, Cl, Br, I, D, CN and OH;
$R_2$ is OH, $NH_2$, $-CH_2NH_2$ or -NH-OH;
$R_3$ and $R_4$ are independently H, D, F, Cl, Br, I, $C_{1-4}$ alkyl or CN, wherein the alkyl is optionally substituted with 1-3 groups selected from F, Cl, Br, I, D, CN, OH and $NH_2$;
each $R_5$ is independently H, D, OH, $C_{1-4}$ alkyl, CN, F, Cl, Br, I, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from F, Cl, Br, I, D, CN, OH and $NH_2$;
each $R_7$ is independently H, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl and $C_{1-4}$ alkoxy;
each $R_8$ is independently H, D, OH, CN, F, Cl, Br, I, $C_{2-4}$ alkenyl, $C_{2-4}$ alkynyl, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;
each $R_7'$ is independently H, $C_{1-4}$ alkyl or halo $C_{1-4}$ alkyl;
each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl and $C_{1-4}$ alkoxy;

each r is independently 0, 1 or 2;
p is 0 or 1;
n is 0, 1, 2 or 3;
m is 0, 1, 2, 3 or 4;
other groups are as defined in any of the preceding technical solutions.

More specifically, a twelfth technical solution of the present invention provides a compound represented by formula (I-a), formula (I-b), formula (I), formula (II-a) or formula (II), or a stereoisomer, a deuterated compound, a solvate or a pharmaceutically acceptable salt thereof,

X is selected from $CR_1$ or N;
Y, Z and V are independently $CR_5$, N, O or S, and the total number of O and S is not greater than 1;
ring A is phenyl, thienyl, thiazolyl, pyrrolyl, imidazolyl, oxazolyl, pyridyl, pyrazinyl, pyrimidyl or pyridazinyl;
$R_1$ is H, D, $C_{1-4}$ alkyl, F, Cl, Br, I or CN, wherein the alkyl is optionally substituted with 1-3 groups selected from F, Cl, Br, I, D, CN and OH;
$R_2$ is OH, $NH_2$ or -NH-OH;
$R_3$ and $R_4$ are independently H, D, F, Cl, Br, I, $C_{1-4}$ alkyl or CN, wherein the alkyl is optionally substituted with 1-3 groups selected from F, Cl, Br, I, D, CN, OH and $NH_2$;
each $R_5$ is independently H, D, OH, $C_{1-4}$ alkyl, CN, F, Cl, Br, I, $C_{1-4}$ alkoxy, $C_{2-4}$ alkenyl or $C_{2-4}$ alkynyl, wherein the alkyl, alkoxy, alkenyl or alkynyl is optionally substituted with 1-3 groups selected from F, Cl, Br, I, D, CN, OH and $NH_2$;
each $R_7$ is independently H, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl and $C_{1-4}$ alkoxy;
each $R_8$ is independently H, D, OH, CN, F, Cl, Br, I, $C_{2-4}$ alkenyl, $C_{2-4}$ alkynyl, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;
each $R_7'$ is independently H, $C_{1-4}$ alkyl or halo $C_{1-4}$ alkyl;
each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl and $C_{1-4}$ alkoxy;
each r is independently 0, 1 or 2;
p is 0 or 1;
n is 0, 1, 2 or 3;
m is 0, 1, 2, 3 or 4;
other groups are as defined in any of the preceding technical solutions.

More specifically, a thirteenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof,

each $R_8$ is independently D, CN, F, Cl, Br, $C_{2-4}$ alkenyl, $C_{2-4}$ alkynyl, $N_3$, $C_{1-4}$ alkyl, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-2}$ alkyl and $C_{1-2}$ alkoxy;
other groups are as defined in any of the preceding technical solutions.

More specifically, a fourteenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

$R_8'$ is independently $C_{1-4}$ alkoxy, cyclopropyl, cyclobutyl, cyclopentyl, phenyl, tetrahydropyrrolyl, azetidinyl, piperidyl, piperazinyl, morpholinyl, oxetanyl, tetrahydrofuryl, tetrahydropyranyl,

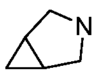 , 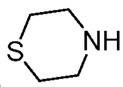 , 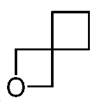 , 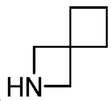 , 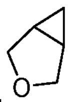 , 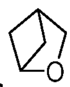 ,

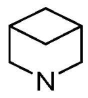,

thienyl, thiazolyl, pyrrolyl, imidazolyl, oxazolyl, pyridyl, pyrazinyl, pyrimidyl or pyridazinyl optionally substituted with 1-3 groups selected from F, Cl, Br, I, =O, D, OH, $NH_2$, CN, $C_{1-4}$ alkyl, halo $C_{1-2}$ alkyl and $C_{1-2}$ alkoxy; or
$R_8$' is independently $C_{1-4}$ alkoxy, cyclopropyl, cyclobutyl, cyclopentyl, phenyl, tetrahydropyrrolyl, azetidinyl, piperidyl, piperazinyl, morpholinyl, oxetanyl, tetrahydrofuryl, tetrahydropyranyl,

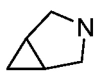, 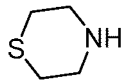, 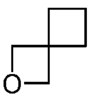, 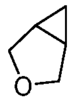, 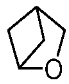, 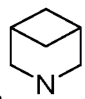,

thienyl, thiazolyl, pyrrolyl, imidazolyl, oxazolyl, pyridyl, pyrazinyl, pyrimidyl or pyridazinyl optionally substituted with 1-3 groups selected from F, Cl, Br, I, =O, D, OH, $NH_2$, CN, $C_{1-4}$ alkyl and $C_{1-4}$ alkoxy;
other groups are as defined in any of the preceding technical solutions.

More specifically, a fifteenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein D is selected from

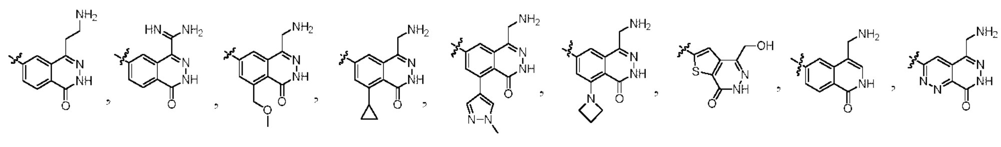,

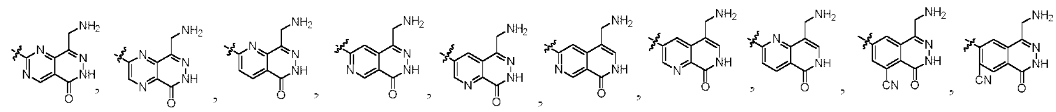,

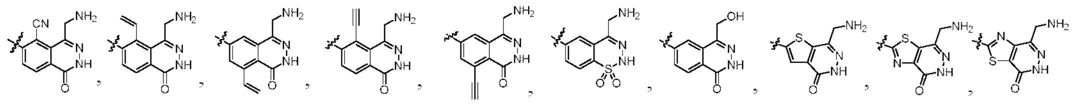,

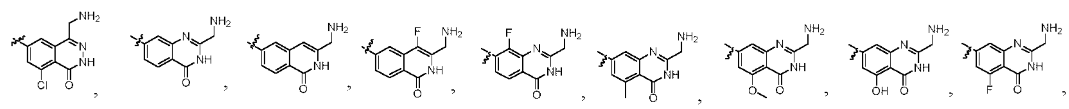,

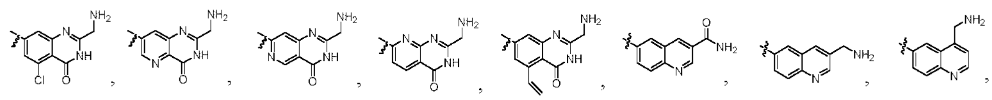,

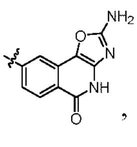, 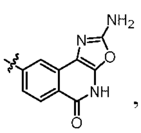, 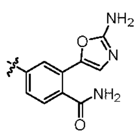 or 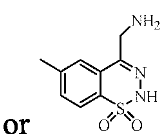;

D is selected from

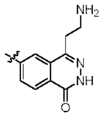, 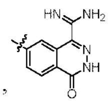, 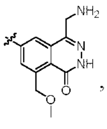, 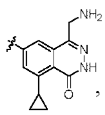, 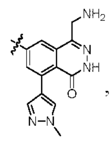, 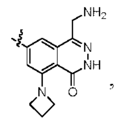, 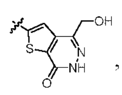, 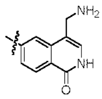, 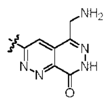,

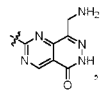, 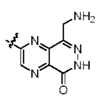, 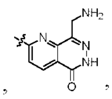, 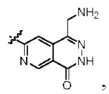, 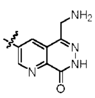, 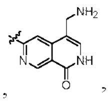, 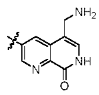, 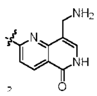, 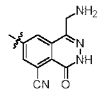, 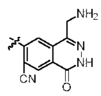,

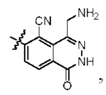, 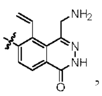, 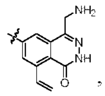, 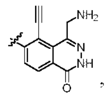, 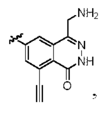, 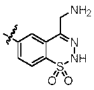, 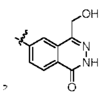, 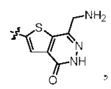, 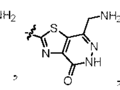, 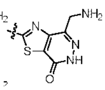;

other groups are as defined in any of the preceding technical solutions.

More specifically, a sixteenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

$R_8$ is selected from -O-$(CH_2)_r$-$R_{8'}$, 3- to 12-membered heterocycloalkyl, $C_{3-12}$ cycloalkyl, -O-$(CH_2)_r$-$C_{3-12}$ cycloalkyl, -O-$(CH_2)_r$-(3- to 12-membered heterocycloalkyl) or -N$(R_{7'})_2$, wherein the $CH_2$, cycloalkyl or heterocycloalkyl is optionally further substituted with 1-3 $R_{8''}$, preferably the cycloalkyl is further substituted with 1-3 $R_{8''}$;

each $R_{7'}$ is independently selected from hydrogen, deuterium, amino, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy or deuterated $C_{1-4}$ alkoxy;
$R_{8'}$ is selected from $C_{1-4}$ alkyl or $C_{1-4}$ alkoxy, wherein the alkyl or alkoxy is optionally further substituted with 1 to 5 groups selected from deuterium, halogen, $C_{1-4}$ alkyl, halo $C_{1-4}$ alkyl, deuterated $C_{1-4}$ alkyl, $C_{1-4}$ alkoxy, halo $C_{1-4}$ alkoxy or deuterated $C_{1-4}$ alkoxy;
each $R_{8''}$ is independently selected from deuterium, =O, halogen, $C_{1-4}$ alkyl or $C_{1-4}$ alkoxy, wherein the alkyl or alkoxy is optionally further substituted with 1 to 3 groups selected from halogen, deuterium, hydroxyl, $C_{1-4}$ alkyl or $C_{1-4}$ alkoxy;
other groups are as defined above.

More specifically, a seventeenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein

$R_8$ is selected from -O-$(CH_2)_r$-O$(CH_2)_r$$CH_3$, 4- to 6-membered monocyclic heterocycloalkyl, 5- to 8-membered fused heterocycloalkyl, 5- to 11-membered spiro heterocycloalkyl, 5- to 8-membered bridged heterocycloalkyl, -O-$(CH_2)_r$-$C_{3-8}$ monocyclic cycloalkyl, -O-$(CH_2)_r$-4- to 6-membered monocyclic heterocycloalkyl, -O-$(CH_2)_r$-5- to 8-membered fused heterocycloalkyl, -O-$(CH_2)_r$-5- to 8-membered spiro heterocycloalkyl, -O-$(CH_2)_r$-5- to 8-membered bridged heterocycloalkyl or - N$(CH_3)_2$, wherein the heterocycloalkyl or cycloalkyl is optionally further substituted with 1-3 $R_{8''}$, preferably the cycloalkyl is further substituted with 1-3 $R_{8''}$;
each $R_{8''}$ is independently selected from deuterium, =O, F, Cl, methyl, ethyl, propyl, methoxy, ethoxy or propoxy, wherein the methyl, ethyl, propyl, methoxy, ethoxy or propoxy is optionally further substituted with 1 to 3 groups selected from F, Cl, deuterium, hydroxyl, methyl, ethyl, propyl, methoxy, ethoxy or propoxy;
other groups are as defined above.

More specifically, an eighteenth technical solution of the present invention provides a compound represented by formula (I-a), formula (I-b), formula (I), formula (II-a) or formula (II), or a stereoisomer, a deuterated compound, a solvate or a pharmaceutically acceptable salt thereof, wherein
each $R_8$ is independently CN, F, Cl, $N_3$, vinyl, fluorovinyl, -O-$CH_2CH_2$-$OCH_3$, -N$(CH_3)_2$

, 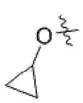,

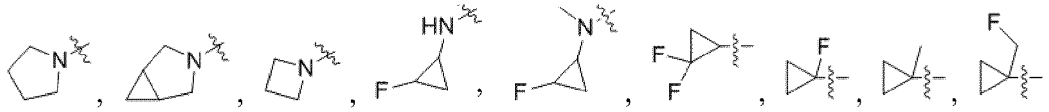
, , , , , , , , ,

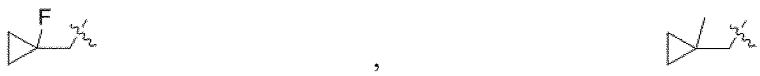
, ,

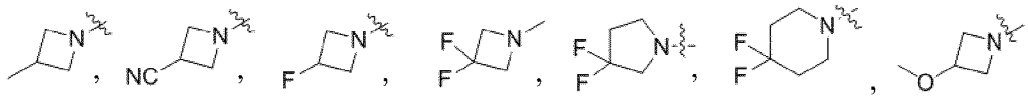
, , , , , , ,

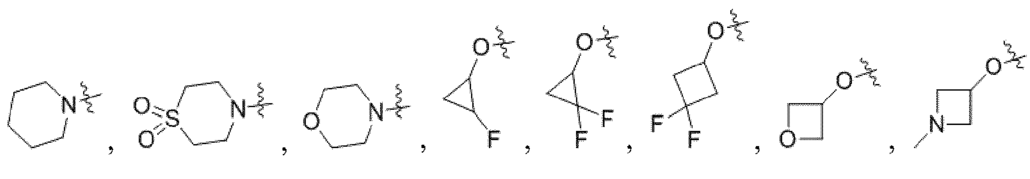
, , , , , , , , ,

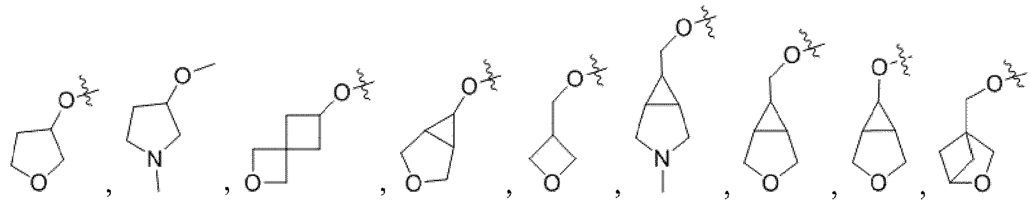
, , , , , , , , , ,

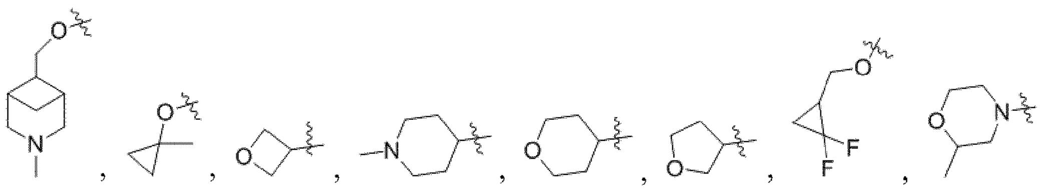
, , , , , , , , ,

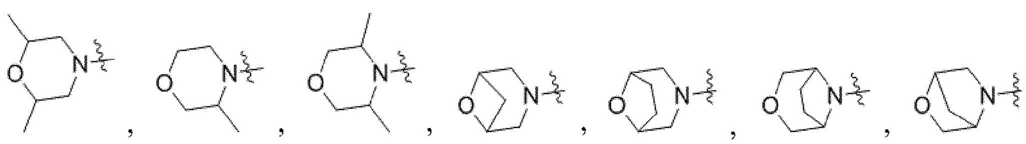
, , , , , , ,

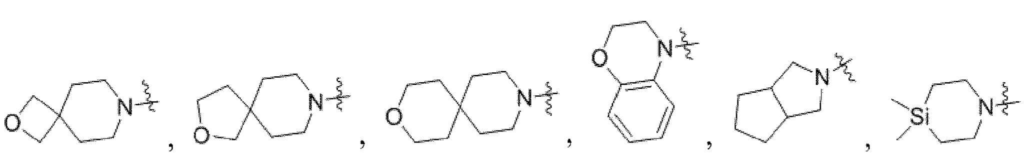
, , , , , or

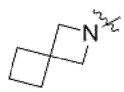
;

further

$R_8$ is selected from $-O-CH_2CH_2-OCH_3$, $-N(CH_3)_2$

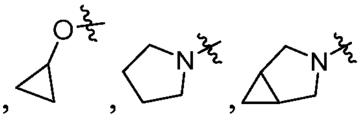
, , , ,

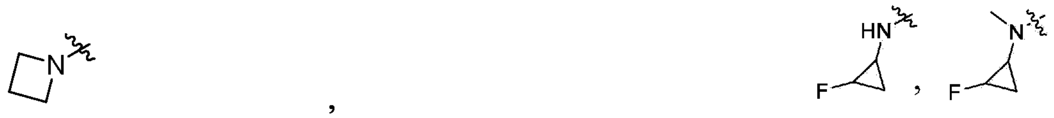
, , ,

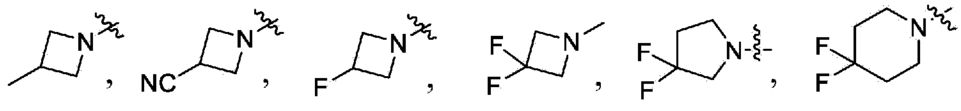
, , , , , ,

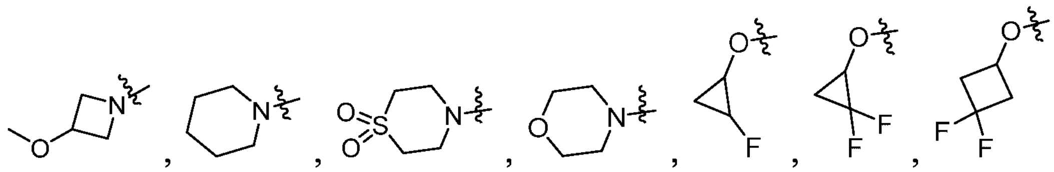
, , , , , , , ,

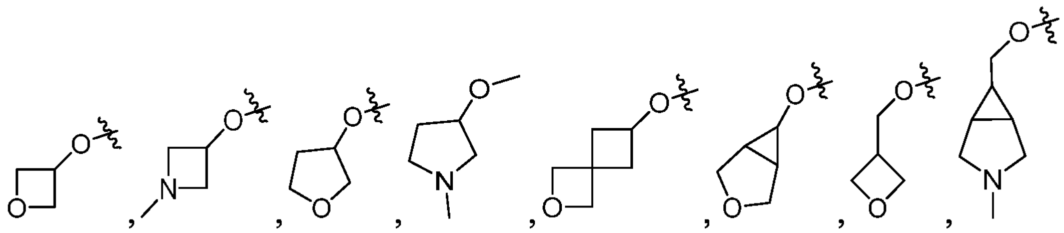
, , , , , , , , ,

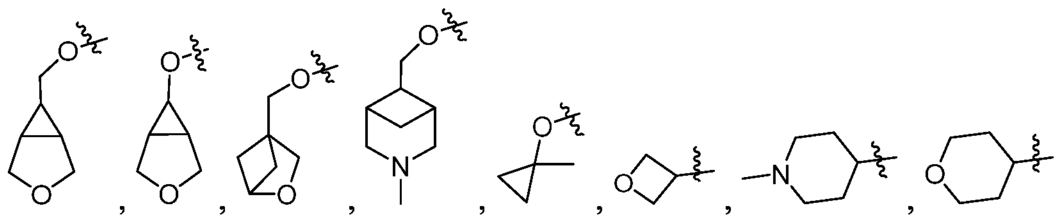
, , , , , , , , ,

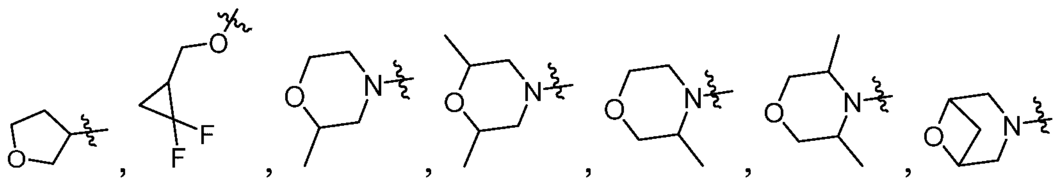
, , , , , , , ,

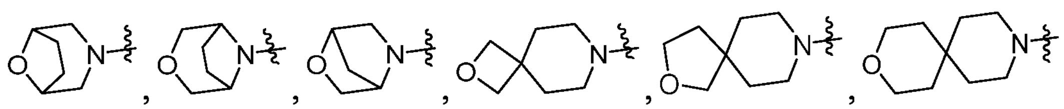
, , , , , ,

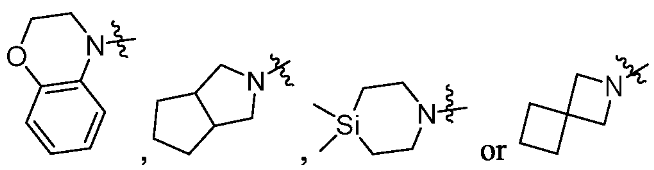
, , or ;

other groups are as defined above.

More specifically, a nineteenth technical solution of the present invention provides a compound represented by formula (I-b), or a stereoisomer, a deuterated compound, a solvate or a pharmaceutically acceptable salt thereof, wherein

D1 is selected from:

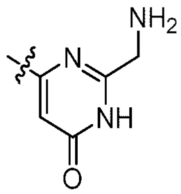

;

Ld is selected from $C_{2-4}$ alkenyl; in certain embodiments, Ld is selected from

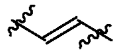

;

Rd1 is selected from 5-membered heteroaryl, wherein the heteroaryl is optionally substituted with 1-2 groups selected from $NH_2$; in certain embodiments, Rd1 is selected from

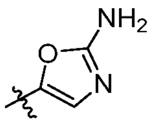

.

More specifically, a twentieth technical solution of the present invention provides the compound, or the stereoisomer, the deuterated compound, the solvate or the pharmaceutically acceptable salt thereof according to the present invention, wherein the compound has a structure selected from those in Table 1 below.

Table 1

| | | | | |
|---|---|---|---|---|
| 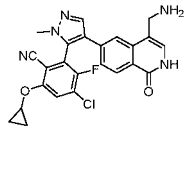 | 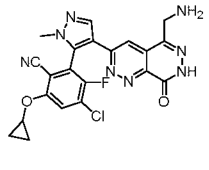 | 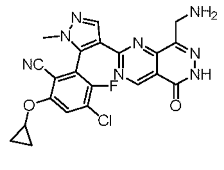 | 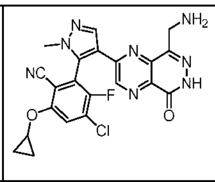 | 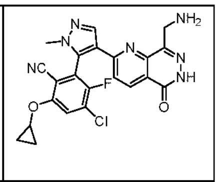 |
| 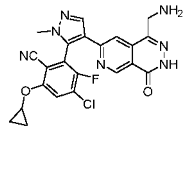 | 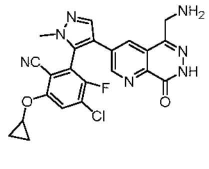 | 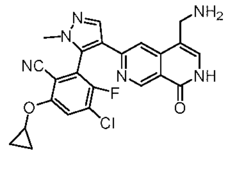 | 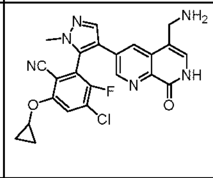 | 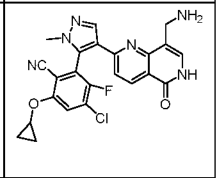 |
| 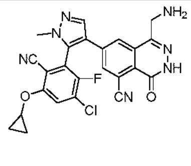 | 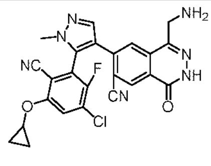 | 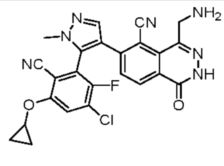 | 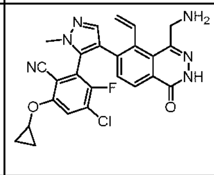 | 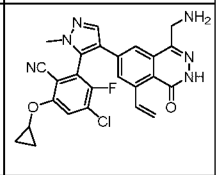 |
| 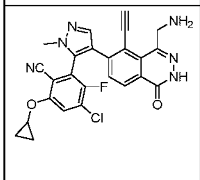 | 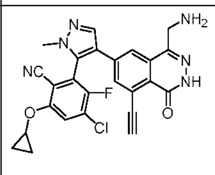 | 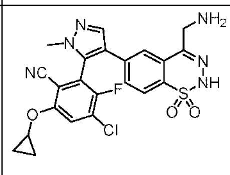 | 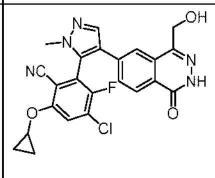 | 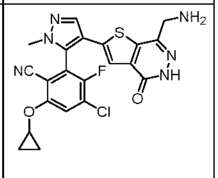 |
| 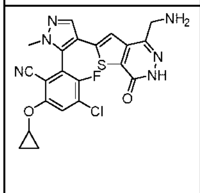 | 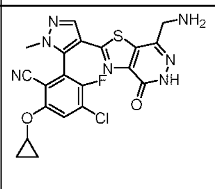 | 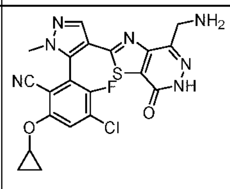 | 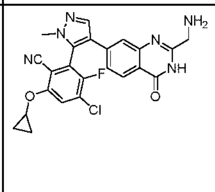 | 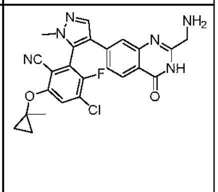 |
| 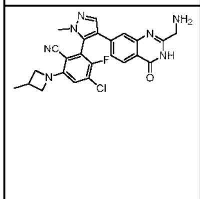 | 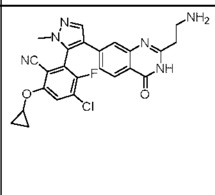 | 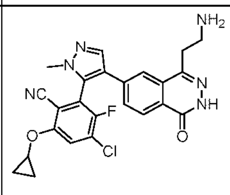 | 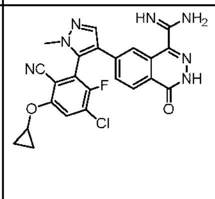 | 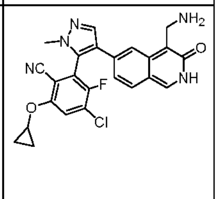 |
| 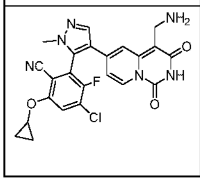 | 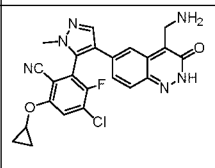 | 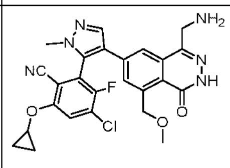 | 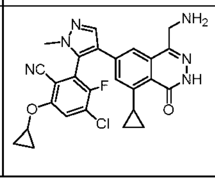 | 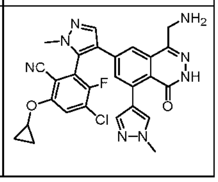 |

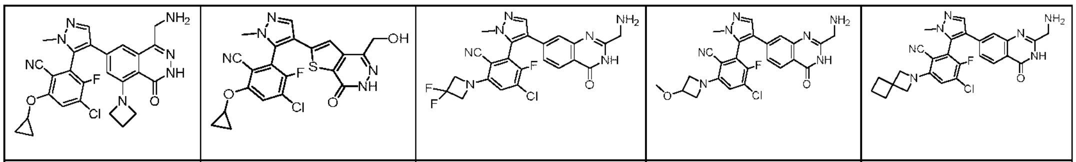

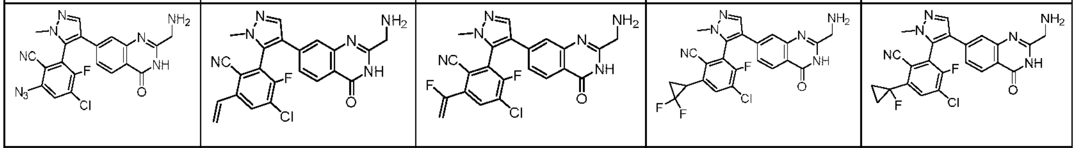

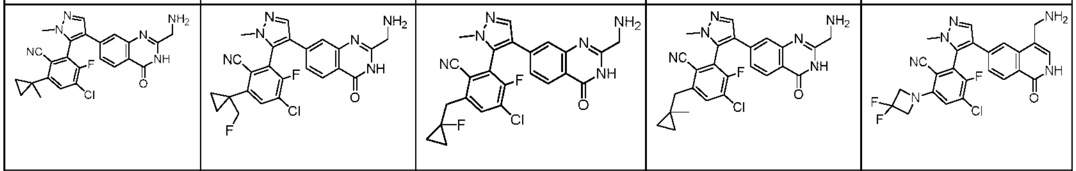

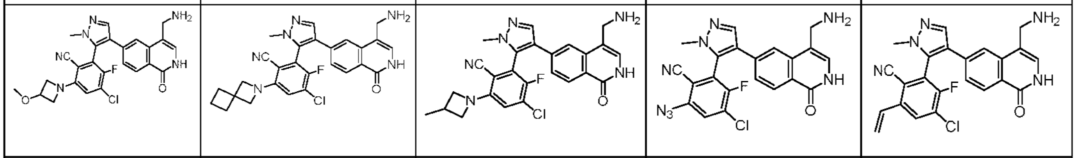

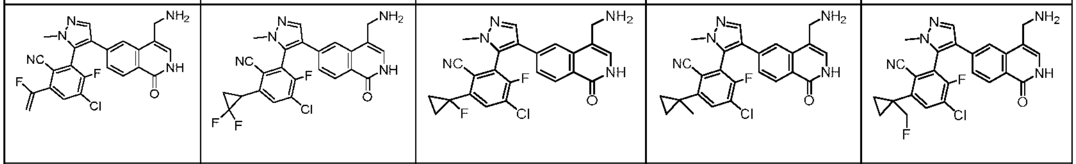

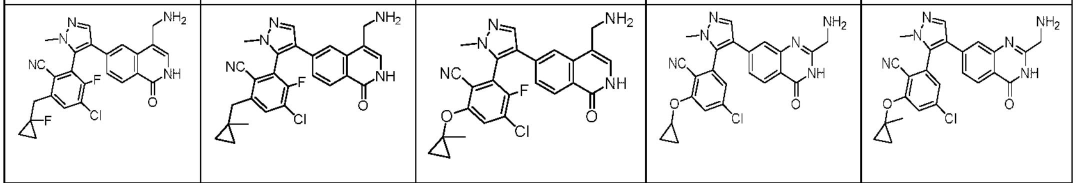

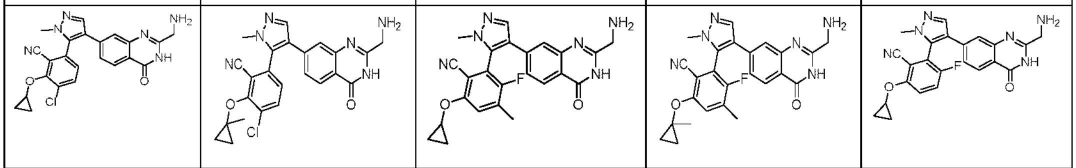

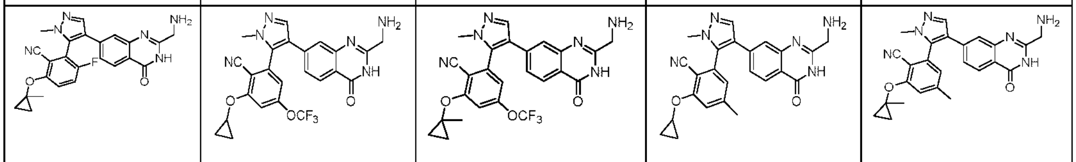

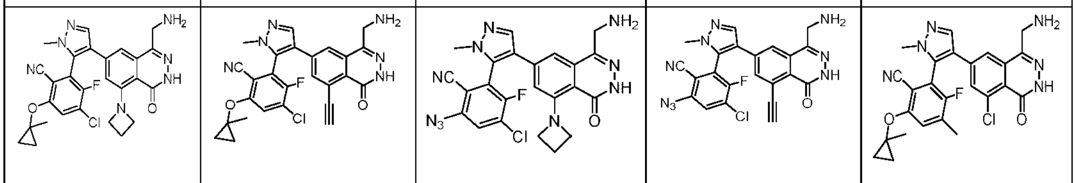

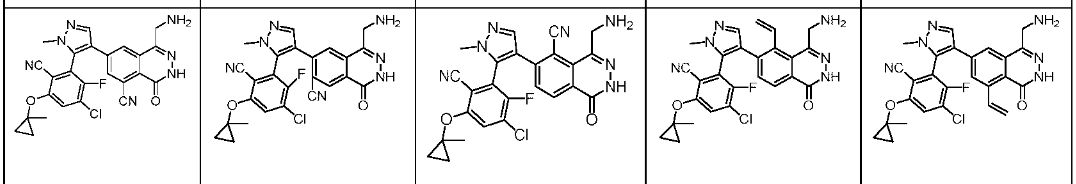

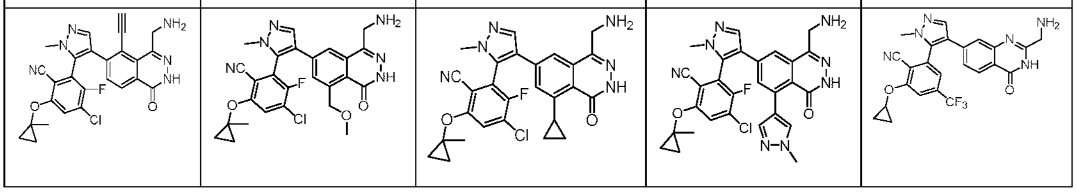

| | | | | |
|---|---|---|---|---|
| 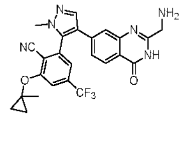 | 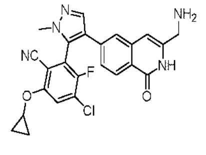 | 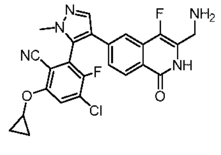 | 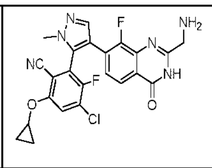 | 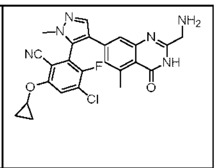 |
| 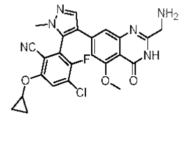 | 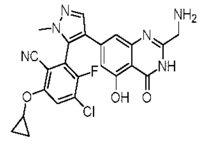 | 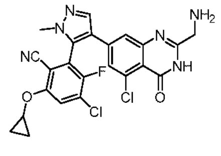 | 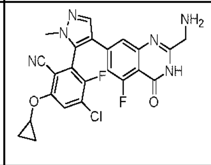 | 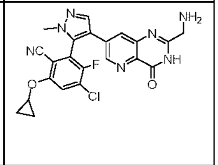 |
| 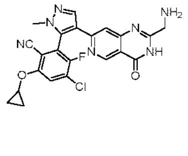 | 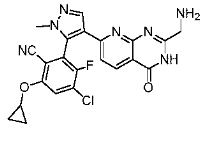 | 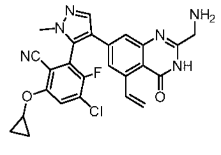 | 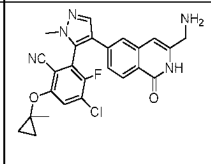 | 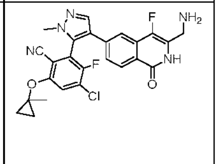 |
| 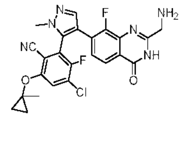 | 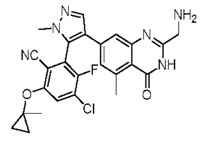 | 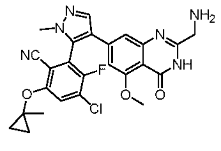 | 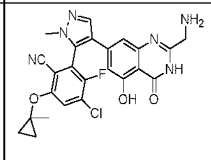 | 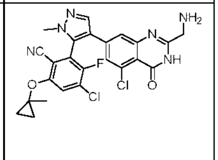 |
| 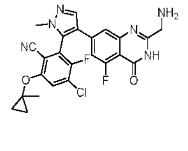 | 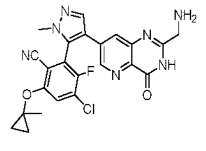 | 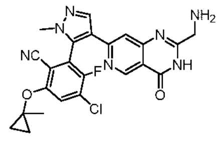 | 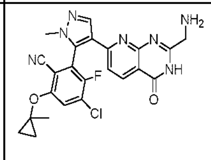 | 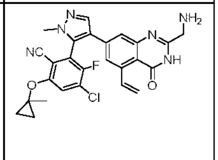 |
| 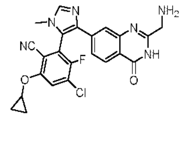 | 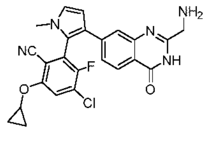 | 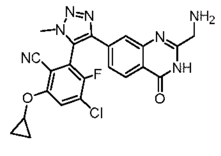 | 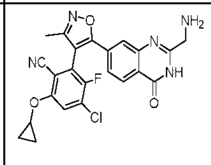 | 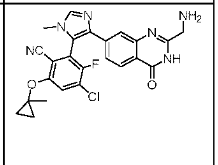 |
| 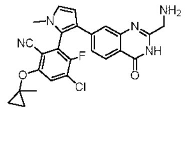 | 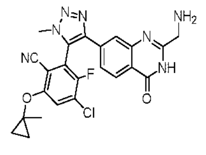 | 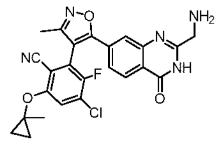 | 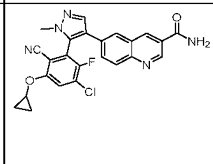 | 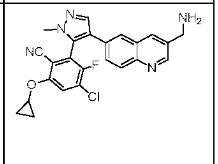 |
| 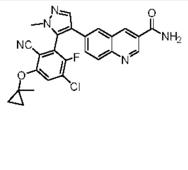 | 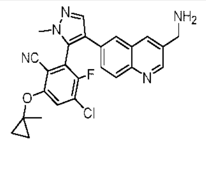 | 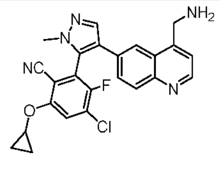 | 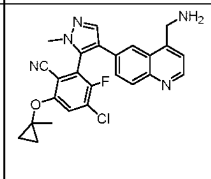 | 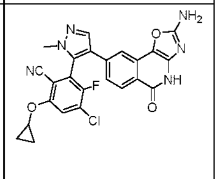 |
| | | | | |
| | | | | |
| | | | | |

| | | | | |
|---|---|---|---|---|
| 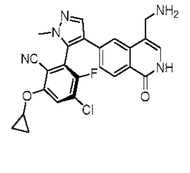 | 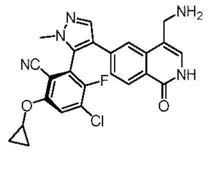 | 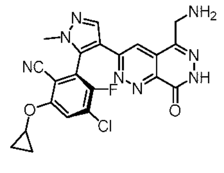 | 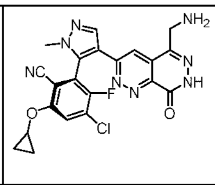 | 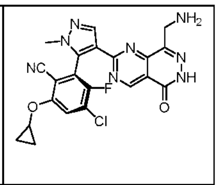 |
| 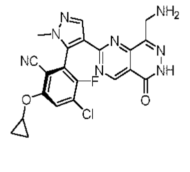 | 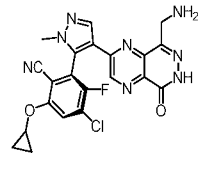 | 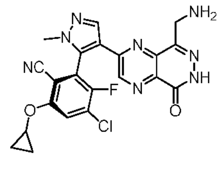 | 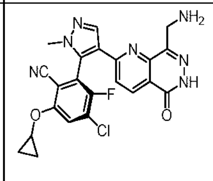 | 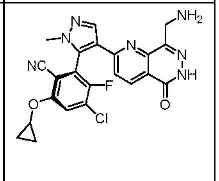 |
| 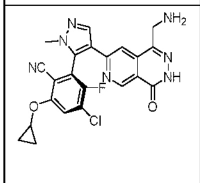 | 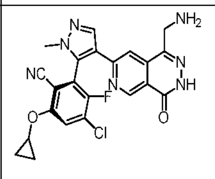 | 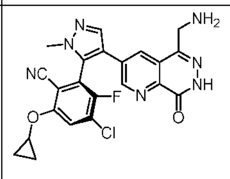 | 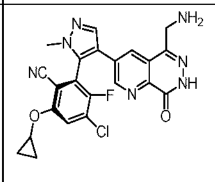 | 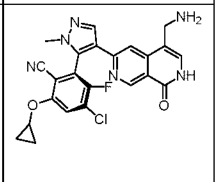 |
| 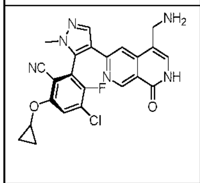 | 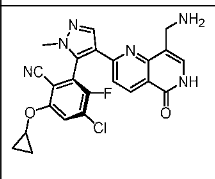 | 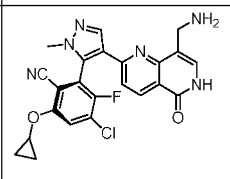 | 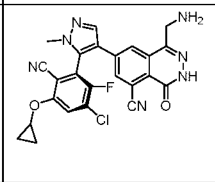 | 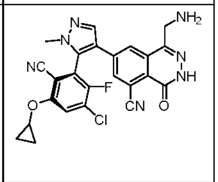 |
| 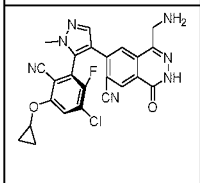 | 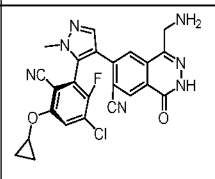 | 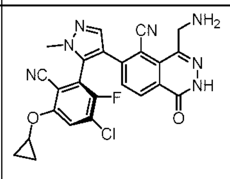 | 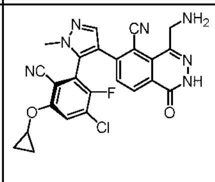 | 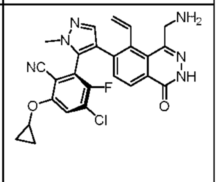 |
| 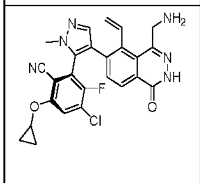 | 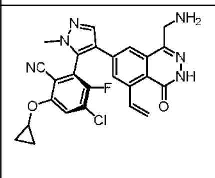 | 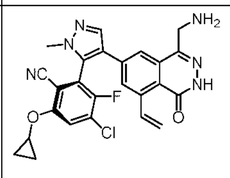 | 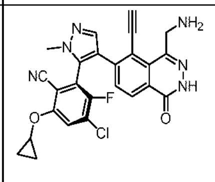 | 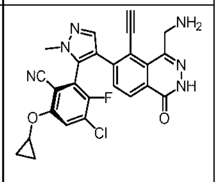 |
| 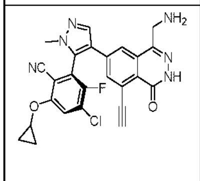 | 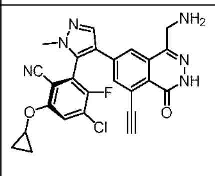 | 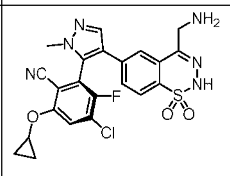 | 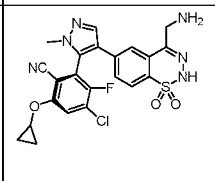 | 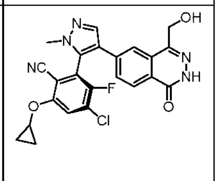 |
| 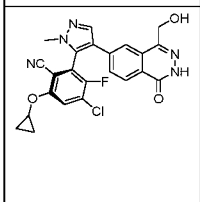 | 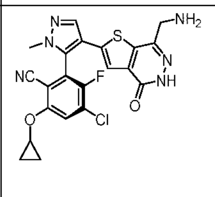 | 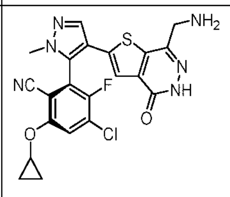 | 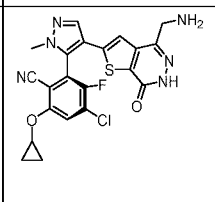 | 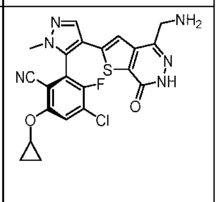 |
| | | | | |
| | | | | |

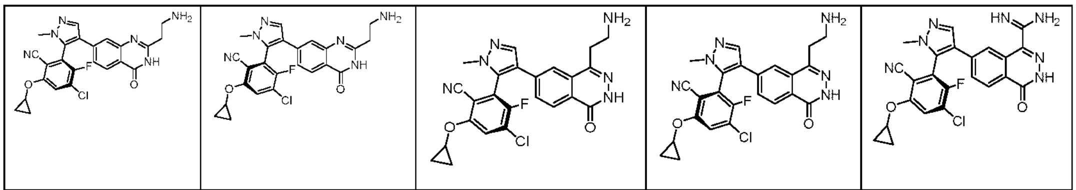

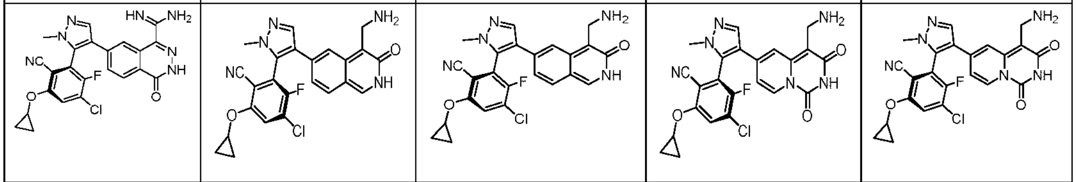

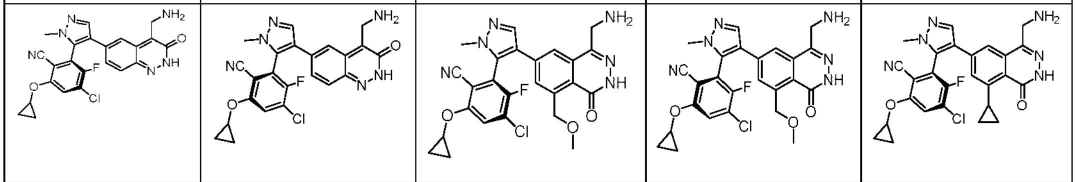

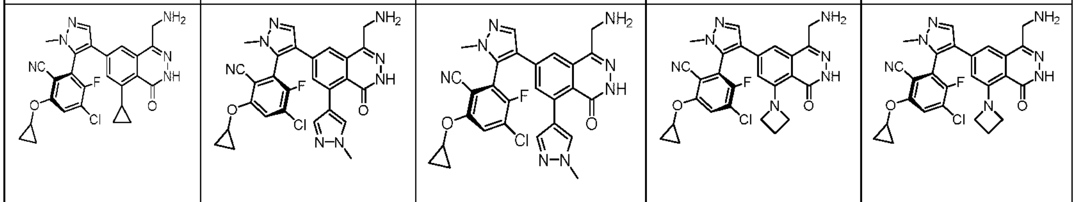

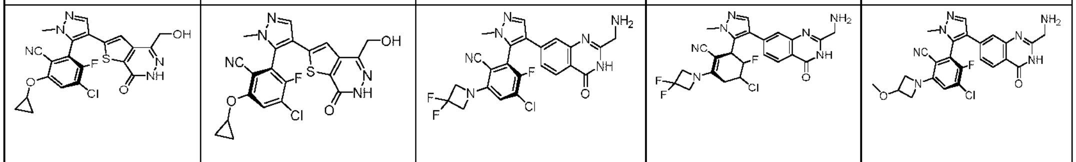

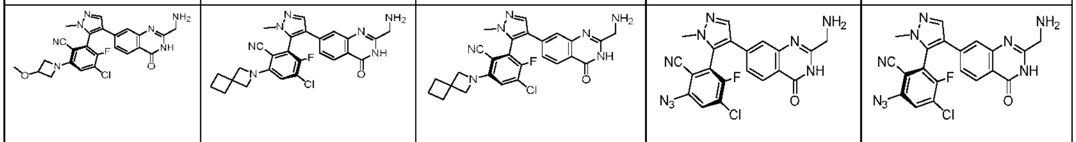

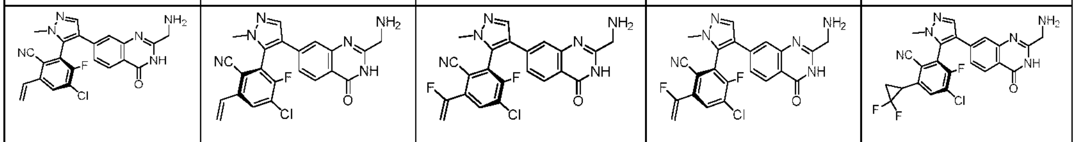

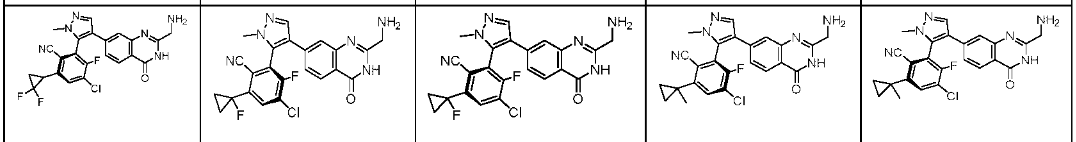

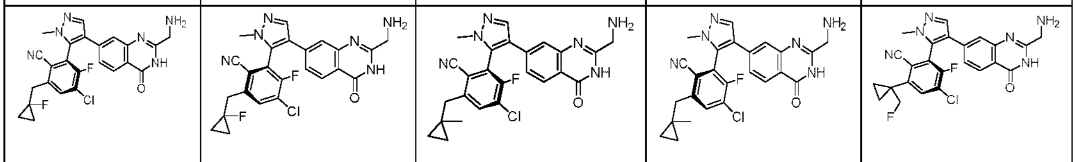

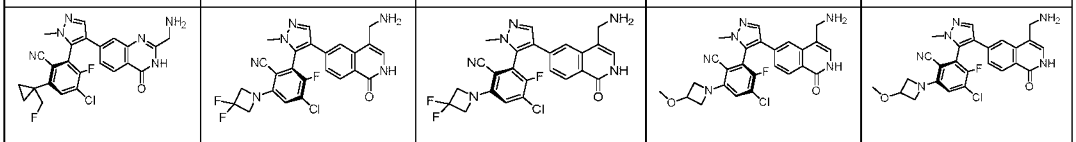

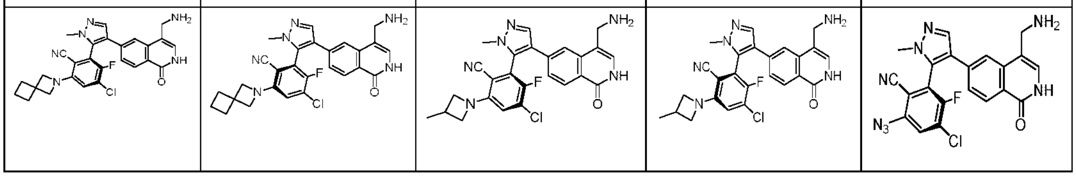

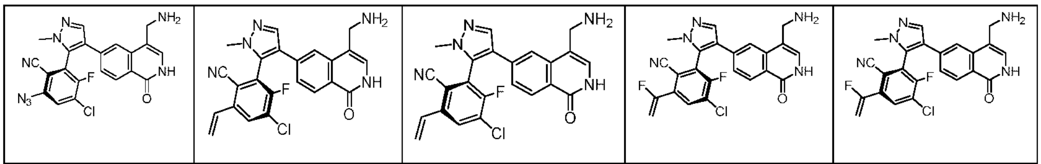

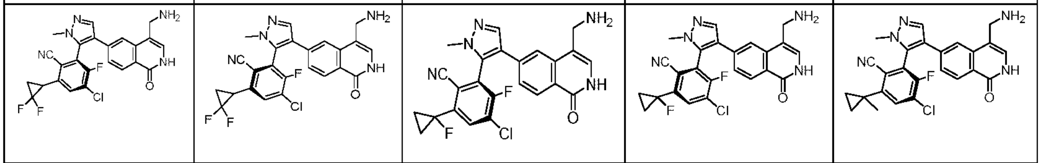

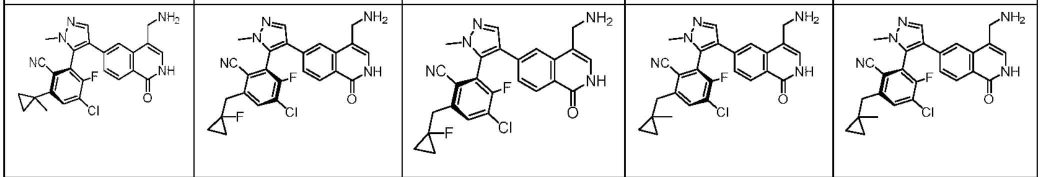

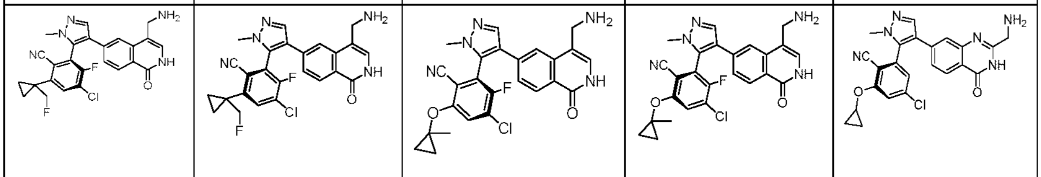

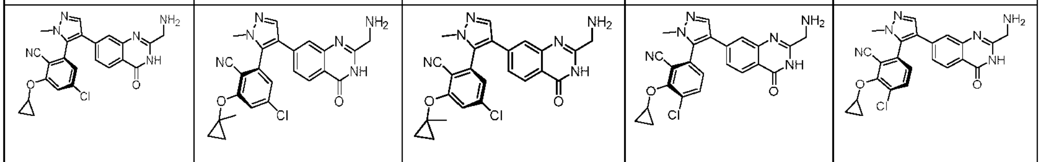

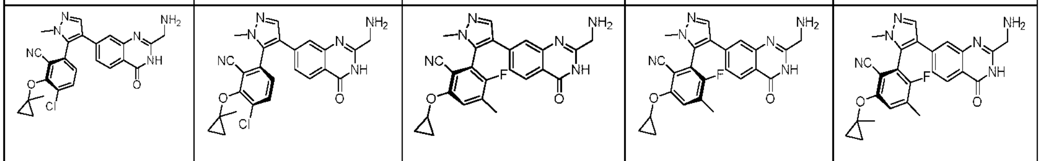

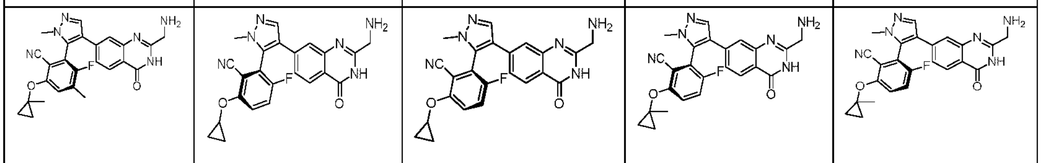

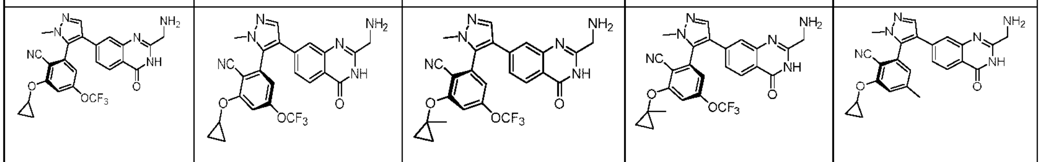

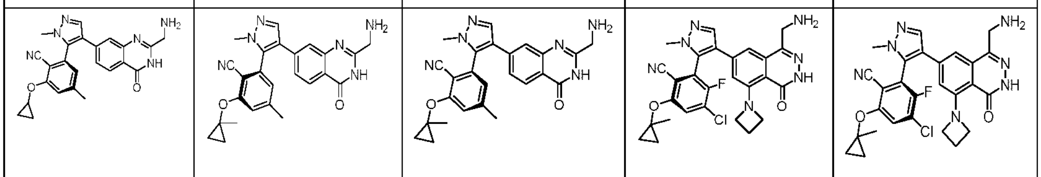

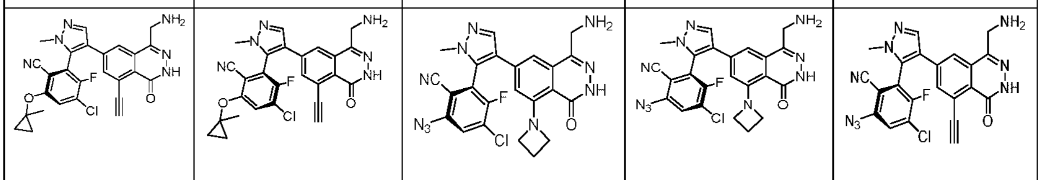

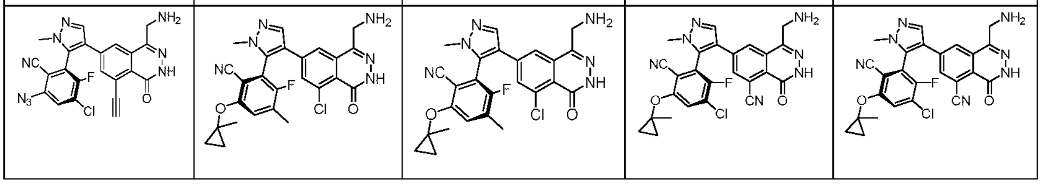

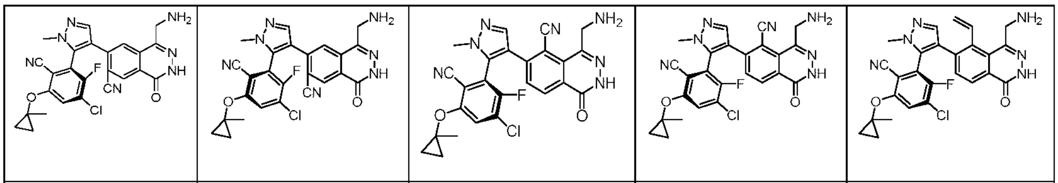

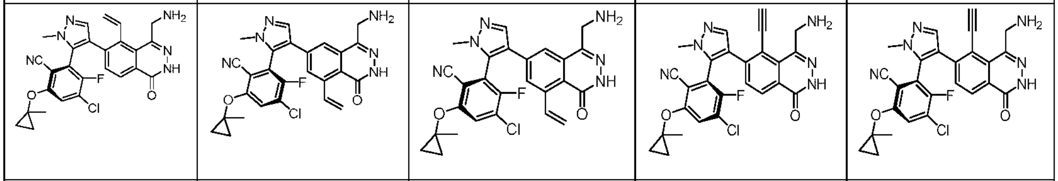

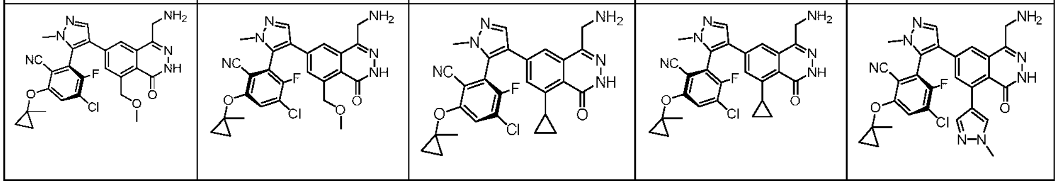

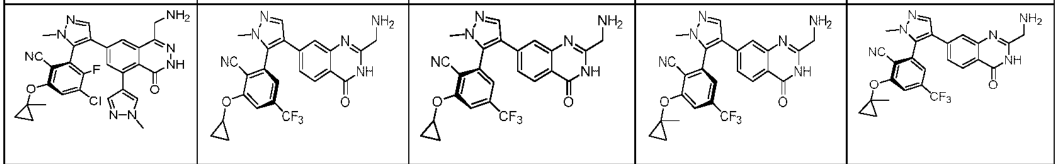

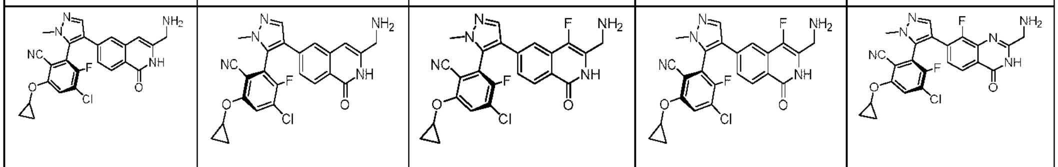

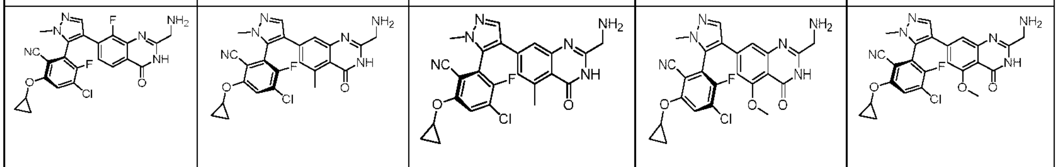

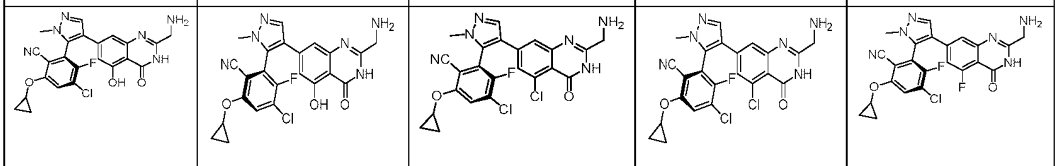

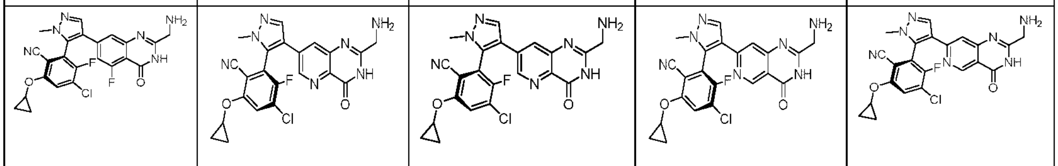

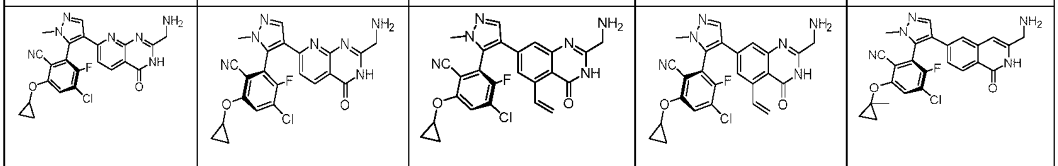

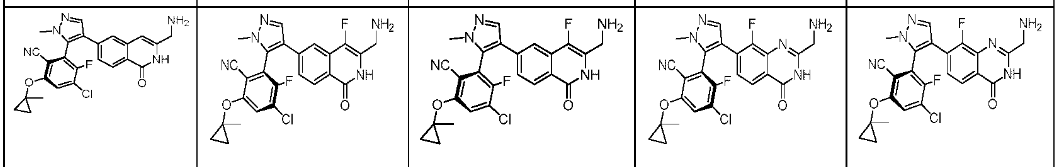

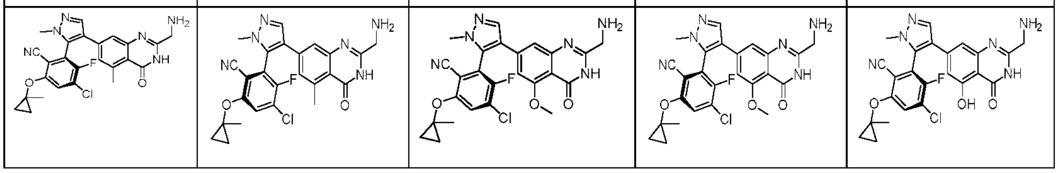

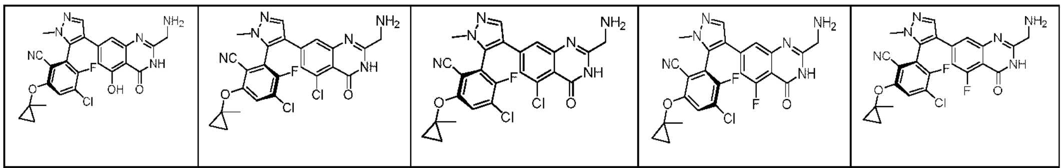

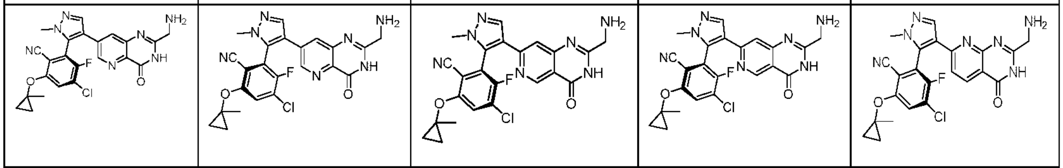

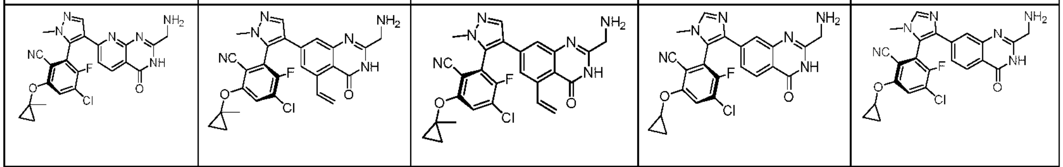

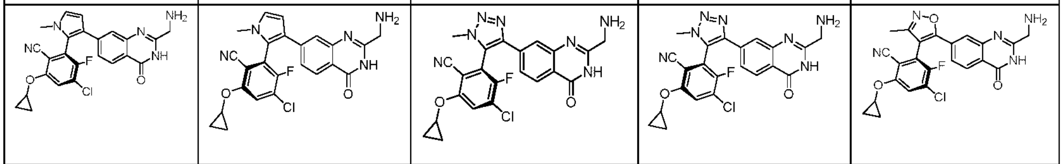

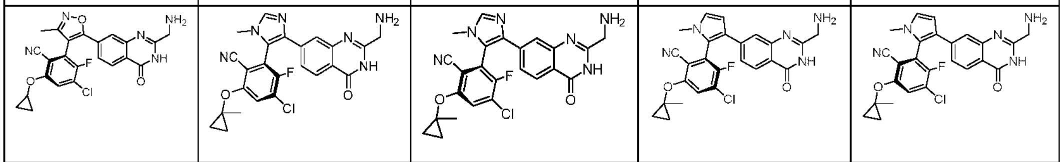

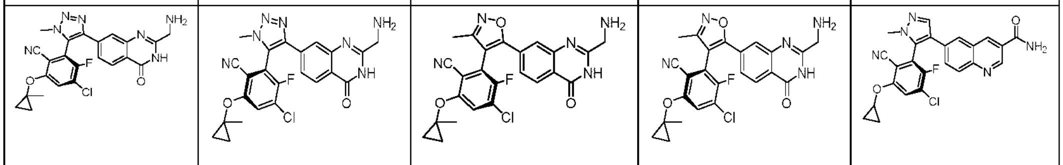

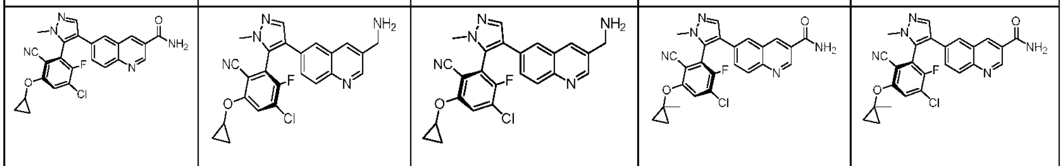

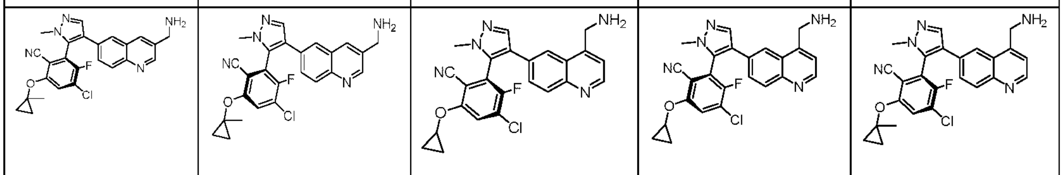

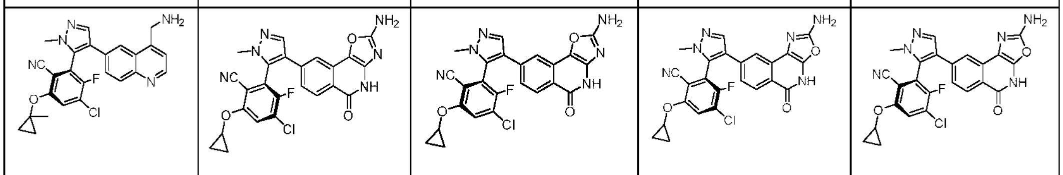

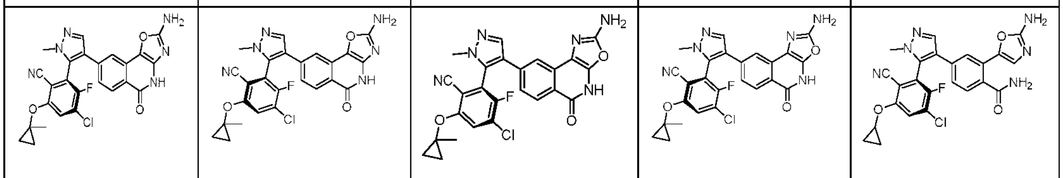

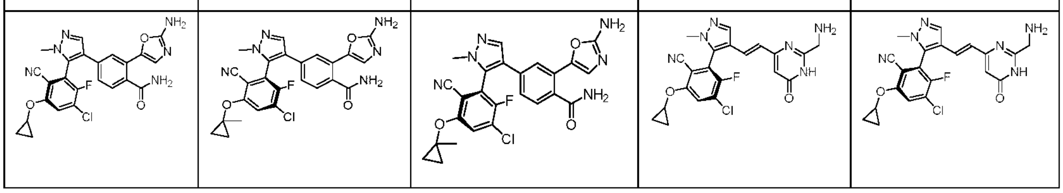

| | | | | |
|---|---|---|---|---|
| 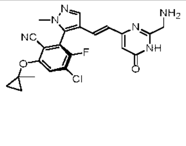 | 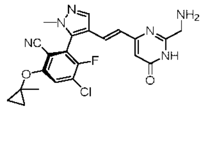 | 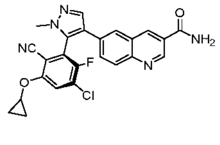 | 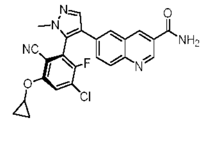 | 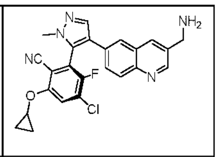 |
| 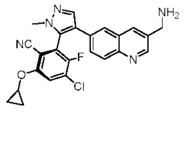 | 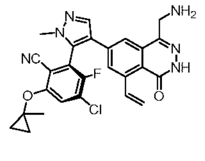 | 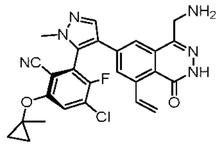 | 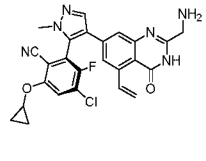 | 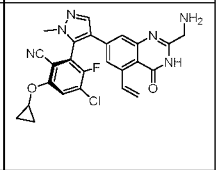 |
| 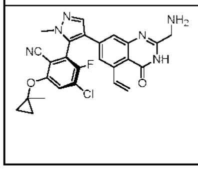 | 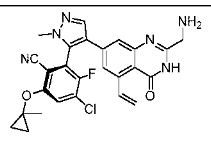 | 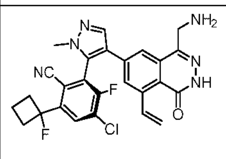 | 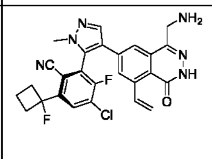 | . |

More specifically, a twenty-first technical solution of the present invention provides a pharmaceutical composition comprising the compound, or the stereoisomer, the deuterated compound, the solvate or the pharmaceutically acceptable salt thereof according to any one of the first to twentieth technical solutions, and a pharmaceutically acceptable carrier and/or excipient.

More specifically, a twenty-second technical solution of the present invention provides the use of the compound, or the stereoisomer, the deuterated compound, the solvate or the pharmaceutically acceptable salt thereof according to any one of the first to twentieth technical solutions, or the composition according to the twenty-first technical solution in the preparation of a drug for treating/preventing PRMT5•MTA-mediated diseases.

Further, the PRMT5•MTA-mediated diseases are selected from liver cancer, breast cancer, skin cancer, bladder cancer, pancreatic cancer, or head and neck cancer.

The present invention further provides a composition or pharmaceutical preparation comprising the compound, or the stereoisomer, the deuterated compound, the solvate or the pharmaceutically acceptable salt thereof according to any one of the preceding solutions, and a pharmaceutically acceptable carrier and/or excipient. The pharmaceutical composition can be in a unit preparation form (the unit preparation is also referred to as "preparation specification").

Further, the composition or pharmaceutical preparation of the present invention comprises 1-1500 mg of the compound, or the stereoisomer, the deuterated compound, the solvate or the pharmaceutically acceptable salt thereof according to any one of the preceding solutions, and a pharmaceutically acceptable carrier and/or excipient.

The present invention further provides the use of the compound, or the stereoisomer, the deuterated compound, the solvate or the pharmaceutically acceptable salt thereof according to any one of the preceding solutions in the preparation of a drug for treating/preventing PRMT5•MTA-mediated diseases. Further, the PRMT5•MTA-mediated disease is liver cancer, breast cancer, skin cancer, bladder cancer, pancreatic cancer, or head and neck cancer.

The present invention further provides a method for treating a disease in a mammal, wherein the method comprises administering to a subject a therapeutically effective amount of the compound, or the stereoisomer, the deuterated compound, the solvate or the pharmaceutically acceptable salt thereof according to any one of the preceding solutions; the disease is preferably liver cancer, breast cancer, skin cancer, bladder cancer, pancreatic cancer, or head and neck cancer; preferably, the therapeutically effective amount is 1-1500 mg. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg,

20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg.

In some embodiments, the pharmaceutical composition or preparation of the present invention contains the above-mentioned therapeutically effective amount of the compound, or the stereoisomer, the deuterated compound, the solvate or the pharmaceutically acceptable salt thereof according to the present invention.

The present invention relates to a pharmaceutical composition or pharmaceutical preparation, wherein the pharmaceutical composition or pharmaceutical preparation comprises a therapeutically effective amount of the compound, or the stereoisomer, the deuterated compound, the solvate or the pharmaceutically acceptable salt thereof according to the present invention, and a carrier and/or excipient. The pharmaceutical composition can be in a unit preparation form (the amount of the active drug in the unit preparation is also referred to as the "preparation specification"). In some embodiments, the pharmaceutical composition comprises, without limitation, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, 1500 mg of the compound, or the stereoisomer, the deuterated compound, the solvate or the pharmaceutically acceptable salt thereof according to the present invention.

Provided is a method for treating a disease in a mammal, wherein the method comprises administering to a subject a therapeutically effective amount of the compound, or the stereoisomer, the deuterated compound, the solvate or the pharmaceutically acceptable salt thereof according to the present invention, and a pharmaceutically acceptable carrier and/or excipient, wherein the therapeutically effective amount is preferably 1-1500 mg, and the disease is preferably liver cancer, breast cancer, skin cancer, bladder cancer, pancreatic cancer, or head and neck cancer.

Provided is a method for treating a disease in a mammal, wherein the method comprises administering to a subject a drug, i.e., the compound, or the stereoisomer, the deuterated compound, the solvate or the pharmaceutically acceptable salt thereof according to the present invention, and a pharmaceutically acceptable carrier and/or excipient at a daily dose of 1-1500 mg/day, wherein the daily dose can be a single dose or divided doses. In some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day and 200-400 mg/day. In some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day and 1500 mg/day.

The present invention relates to a kit, which may include a composition in single-dose or multiple-dose form, wherein the kit comprises the compound, or the stereoisomer, the deuterated compound, the solvate or the pharmaceutically acceptable salt thereof according to the present invention, and the amount of the compound, or the stereoisomer, the deuterated compound, the solvate or the pharmaceutically acceptable salt thereof according to the present invention is the same as that in the above-mentioned pharmaceutical composition.

In the present invention, the amount of the compound, or the stereoisomer, the deuterated compound, the solvate or the pharmaceutically acceptable salt thereof according to the present invention is calculated in the form of a free base in each case.

The term "preparation specification" refers to the weight of the active drug contained in each vial, tablet or other unit preparation.

## Synthetic route

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (such as those listed above) provide custom synthesis services.

**Term**

Unless otherwise specified, the terms of the present invention have the following meanings.

The carbon, hydrogen, oxygen, sulphur, nitrogen and halogen involved in the groups and compounds of the present invention all include isotopes thereof, and are optionally further replaced by one or more of the corresponding isotopes thereof, wherein the isotopes of carbon include $^{12}C$, $^{13}C$ and $^{14}C$; the isotopes of hydrogen include protium (H), deuterium (D, also known as heavy hydrogen) and tritium (T, also known as superheavy hydrogen); the isotopes of oxygen include $^{16}O$, $^{17}O$ and $^{18}O$; the isotopes of sulphur include $^{32}S$, $^{33}S$, $^{34}S$ and $^{36}S$; the isotopes of nitrogen include $^{14}N$ and $^{15}N$; the isotope of fluorine includes $^{19}F$; the isotopes of chlorine include $^{35}Cl$ and $^{37}Cl$; and the isotopes of bromine include $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to $-CF_3$, $- CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally

substituted with a substituent.

The term "alkenylene" refers to a linear or branched divalent unsaturated hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Non-limiting examples of alkenylene include ethynylene and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a linear or branched divalent unsaturated hydrocarbon group containing a carbon-carbon triple bond (C≡C) and generally comprises 2 to 18 carbon atoms, further comprises 2 to 8 carbon atoms, further comprises 2 to 6 carbon atoms, and further comprises 2 to 4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

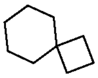, 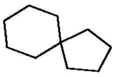, 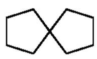, 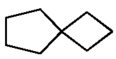, 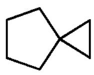, 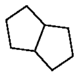,

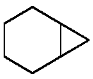, 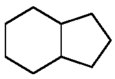, 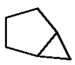, 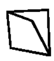, 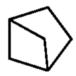, 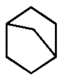, 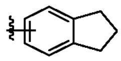,

etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 14 carbon atoms, and further contains 6 to 10 carbon atoms. Non-limiting examples of aryl include phenyl, naphthyl, anthryl, phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes

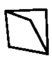, 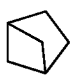, 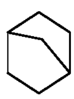,

*etc.,* a bicyclic fused ring includes

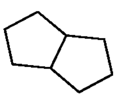, 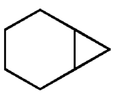, 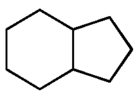, 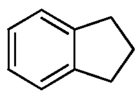, 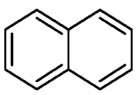, 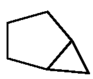,

*etc.,* and a bicyclic spiro ring includes

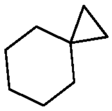, 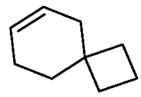, 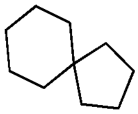, 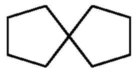,

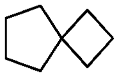, 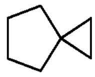,

*etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl,

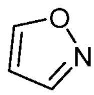, 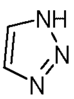, 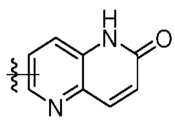,

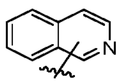, 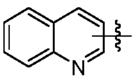, 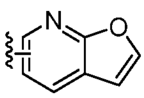, 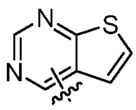, 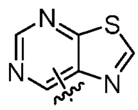, 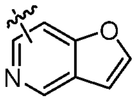,

etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

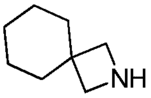, 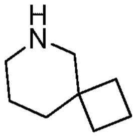, 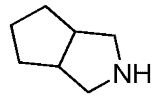,

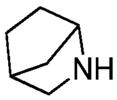, 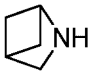, 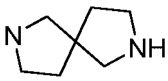, 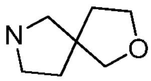, 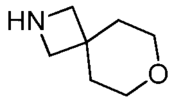, 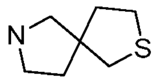,

etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

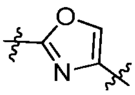, 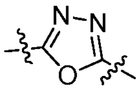, 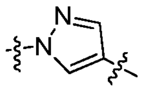, 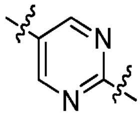,

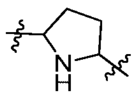,

*etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include:

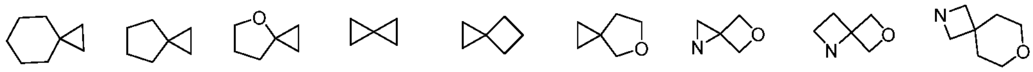

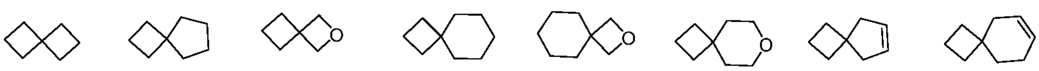

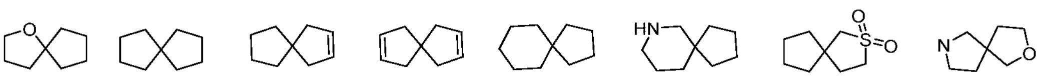

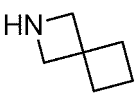, 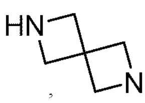, 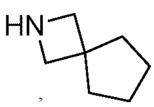, 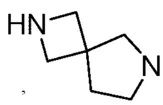, 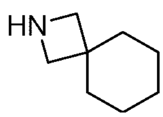, 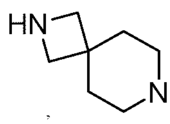

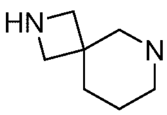, 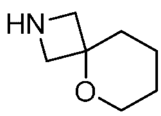, 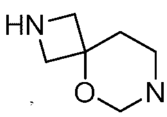, 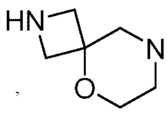, 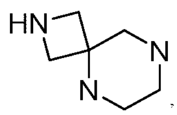,

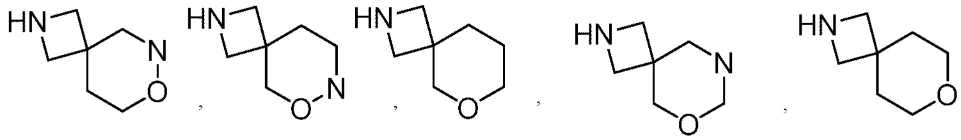,

and the spiro ring may be optionally substituted with a substituent.

The term "fused ring" or "fused" refers to a polycyclic group in which the rings share two adjacent ring atoms and one chemical bond. The fused ring may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and

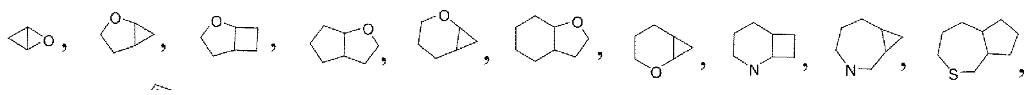,

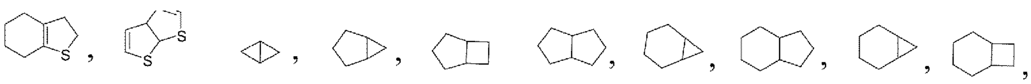,

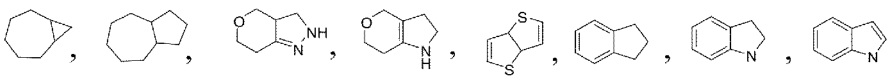;

and the fused ring may be aromatic or non-aromatic and is optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

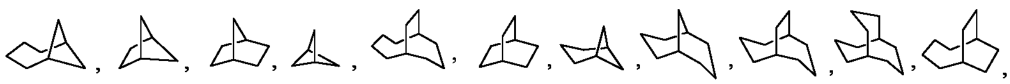,

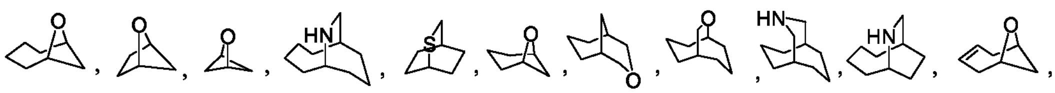,

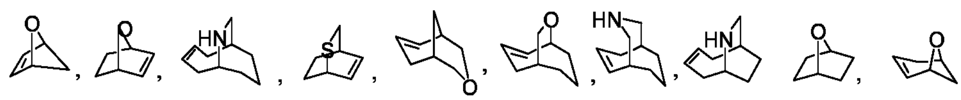

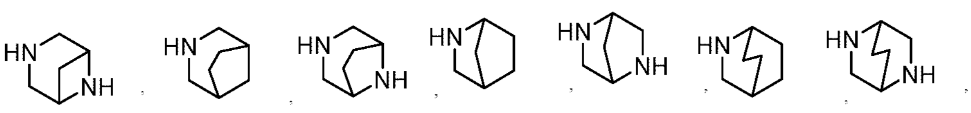

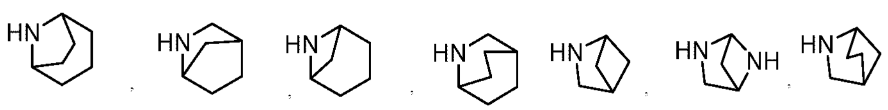

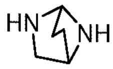, 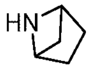, 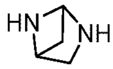, 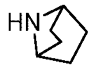

Unless otherwise specified, the term "substitution" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}$ alkyl), $-OC(=O)(C_{1-6}$ alkyl), $-OCO_2(C_{1-6}$ alkyl), - $C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)$C(=O)(C_{1-6}$ alkyl), $-NHCO_2(C_{1-6}$ alkyl), $-NHC(=O)N(C_{1-6}$ alkyl$)_2$, - $HC(=O)NH(C_{1-6}$ alkyl), $-NHC(=O)NH_2$, $-NHSO_2(C_{1-6}$ alkyl), $-SO_2N(C_{1-6}$ alkyl$)_2$, - $SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, deuterated compound, solvates, pharmaceutically acceptable salts or eutectic crystals thereof as described herein and other components including physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, excipient, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrating agent, an absorbent, a preservative, a surfactant, a colorant, a flavouring agent, and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

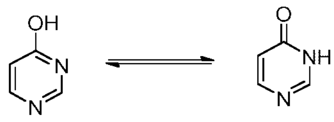

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "eutectic crystal" refers to a crystal formed by the combination of active pharmaceutical ingredient (API)

and co-crystal former (CCF) under the action of hydrogen bonds or other non-covalent bonds. The pure state of API and CCF are both solid at room temperature, and there is a fixed stoichiometric ratio between various components. The eutectic crystal is a multi-component crystal, which includes both a binary eutectic crystal formed between two neutral solids and a multi-element eutectic crystal formed between a neutral solid and a salt or solvate.

Brief Description of the Drawings

FIG. 1 shows the *in-vivo* efficacy results (tumour volume (panel a) and mouse body weight (panel b)) of compound 30.
FIG. 2 shows the *in-vivo* efficacy results (tumour weight) of compound 30.

Detailed Description of Embodiments

The technical solutions of the present invention will be described in detail below in conjunction with the drawings and examples, but the scope of protection of the present invention includes but is not limited thereto.

**Test method**

The structure of the compound is determined by nuclear magnetic resonance (NMR) or (and) mass spectrometry (MS). The NMR shift (δ) is given in the unit of $10^{-6}$ (ppm). NMR is measured with (Bruker Avance III 400 and Bruker Avance 300) NMR instrument, and the solvent for determination is deuterated dimethyl sulfoxide (DMSO-$d_6$), deuterated chloroform ($CDCl_3$), and deuterated methanol ($CD_3OD$), and the internal standard is tetramethylsilane (TMS);

MS is determined with Agilent 6120B (ESI) and Agilent 6120B (APCI); and
HPLC is determined with Agilent 1260DAD high pressure liquid chromatograph (Zorbax SB-$C_{18}$ 100 × 4.6 mm,3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

**Example 1**

2-(4-(4-(aminomethyl)-1-oxo-1,2-dihydroisoquinolin-6-yl)-1-methyl-1H-pyrazol-5-yl)-4-chloro-6-cyclopropoxy-3-fluorobenzonitrile (compound 1)

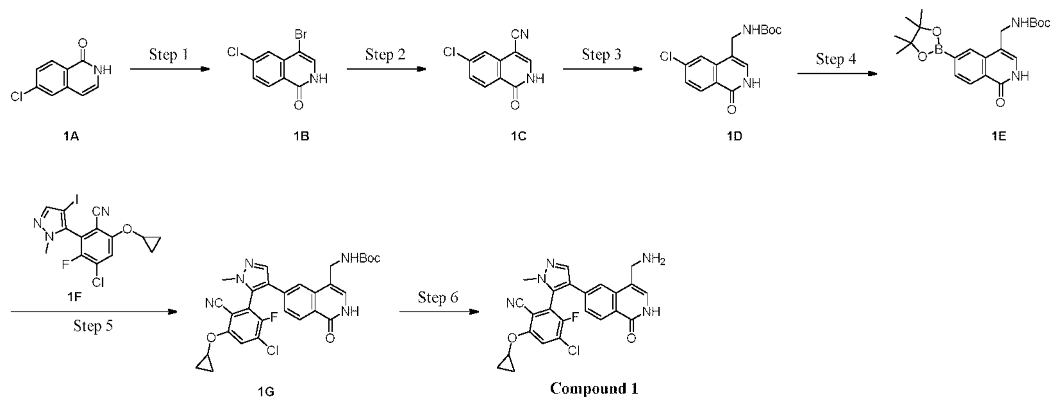


1A 1B 1C 1D 1E 1F 1G Compound 1

Step 1: 1-hydroxyl-6-chloroisoquinoline (compound 1A) (5.00 g, 27.84 mmol, CAS No. 131002-09-0, supplier: Shanghai Titan Scientific Co., Ltd.) was added to a mixed solution of tetrahydrofuran (120 mL) and dichloromethane (80 mL), and the mixture was stirred for 15 min. Then, N-bromosuccinimide (5.45 g, 30.62 mmol) was slowly added at the temperature below 30°C. Upon completion of the addition, the mixture was reacted at room temperature for 8 hours. After

the reaction was complete, the reaction solution was concentrated to dryness. Dichloromethane (50 mL) was added, and the mixture was stirred for 1 hour and then filtered to obtain compound 1B (6.20 g, yield: 86.11%), which was directly used in the next reaction.

LC-MS (ESI): m/z = 260.0 $[M+H]^+$.

Step 2: Compound 1B (5.00 g, 19.34 mmol), zinc cyanide (1.14 g, 9.67 mmol) and 1,1'-bis(diphenylphosphino) ferrocene (1.08 g, 1.93 mmol) were added to N,N-dimethylformamide (150 mL) solvent. The mixture was subjected to nitrogen replacement 3 times, and then tris(dibenzylideneacetone)dipalladium (0.89 g, 0.97 mmol) was added. The resulting mixture was further subjected to nitrogen replacement 3 times, and then warmed to 100°C and reacted for 2 hours. After the reaction was complete, the reaction solution was concentrated to dryness. Dichloromethane (50 mL) was added, and the mixture was concentrated to dryness again. Dichloromethane (30 mL) was added, and the resulting mixture was stirred for 1 hour and then filtered to obtain compound 1C (3.3 g, yield: 83.39%).

LC-MS (ESI): m/z = 203.1 $[M-H]^-$.

Step 3: Compound 1C (1.00 g, 4.84 mmol) was placed in a methanol (100 mL) solution, and then cobalt chloride (0.63 g, 4.84 mmol) was added; and the mixture was stirred for 15 minutes. Then, sodium borohydride (0.46 g, 12.10 mmol) was slowly added, and the resulting mixture was stirred at room temperature for 2 hours. Di-tert-butyl dicarbonate (1.27 g, 5.81 mmol) was added, and the mixture was reacted at room temperature overnight. After the reaction was complete, the reaction mixture was filtered. The filtrate was washed with methanol and concentrated to dryness. The residue was separated by silica gel column chromatography (dichloromethane : methanol (v : v)=100%-90%) to obtain compound 1D (600 mg, yield: 39.89%).

LC-MS (ESI): m/z = 309.1 $[M+H]^+$.

Step 4: Compound 1D (700 mg, 2.27 mmol), bis(pinacolato)diboron (1.15 g, 4.54 mmol) and potassium acetate (1.11 g, 11.35 mmol) were successively added to 1,4-dioxane (150 mL) solvent. The mixture was subjected to nitrogen replacement 3 times. Chloro(2-dicyclohexylphosphino-2',4',6'-triisopropyl-1,1'-biphenyl)[2-(2'-amino-1,1'-biphenyl)]palladium (II) (0.18 g, 0.23 mmol) was added. The resulting mixture was further subjected to nitrogen replacement 3 times, warmed to 105°C and reacted for 1 hour. The reaction solution was cooled and then concentrated to dryness. The residue was separated by silica gel column chromatography (dichloromethane : methanol (v : v)=100%-90%) to obtain compound 1E (800 mg, yield: 88.04%).

LC-MS (ESI): m/z = 309.1 $[M+H]^+$.

Step 5: Compound 1F (100 mg, 0.27 mmol) (synthesized with reference to patent WO 2021050915A1), compound 1E (110 mg, 0.27 mmol) and sodium bicarbonate (45 mg, 0.54 mmol) were successively added to a solution of 1,4-dioxane (9 mL) and water (3 mL). The mixture was subjected to nitrogen replacement 3 times, and then [1,1'-bis(diphenylphosphino)ferrocene]palladium dichloride (22 mg, 0.027 mmol) was added. The resulting mixture was further subjected to nitrogen replacement 3 times, warmed to 60°C and reacted for 1 hour. After the reaction was complete, the reaction solution was concentrated to dryness. The residue was separated by silica gel column chromatography (dichloromethane : methanol (v : v)=100%-90%) to obtain compound 1G (90 mg, yield: 59.10%).

LC-MS (ESI): m/z = 564.2 $[M+H]^+$.

Step 6: Compound 1G (90 mg, 0.16 mmol) was added to a solution of 4 mol/L hydrogen chloride in methanol (10 mL), and the mixture was stirred at room temperature for 2 hours. Then, the reaction solution was concentrated to dryness. The residue was separated by reverse phase column chromatography (instrument: Biotage Isolera One, chromatographic column: Agela C18 reverse phase column, mobile phase: acetonitrile : water (v : v)=98%-30%) to obtain compound 1 (6 mg, yield: 8.08%).

$^1H$ NMR (400 MHz, DMSO-$d_6$) δ 11.06 (s, 1H), 8.18 (s, 1H), 8.13 (d, 1H), 7.99 (d, 1H), 7.45 - 7.38 (m, 2H), 7.04 (s, 1H), 4.21-4.18 (m, 1H), 3.77 (s, 3H), 3.52 (s, 2H), 0.96 - 0.76 (m, 4H);

LC-MS (ESI): m/z = 462.1 $[M-H]^-$.

**Example 2**

4-chloro-6-cyclopropoxy-3-fluoro-2-(4-(4-(hydroxymethyl)-7-oxo-6,7-dihydrothieno[2,3-d]pyridazin-2-yl)-1-methyl-1H-pyrazol-5-yl)benzonitrile (compound 2)

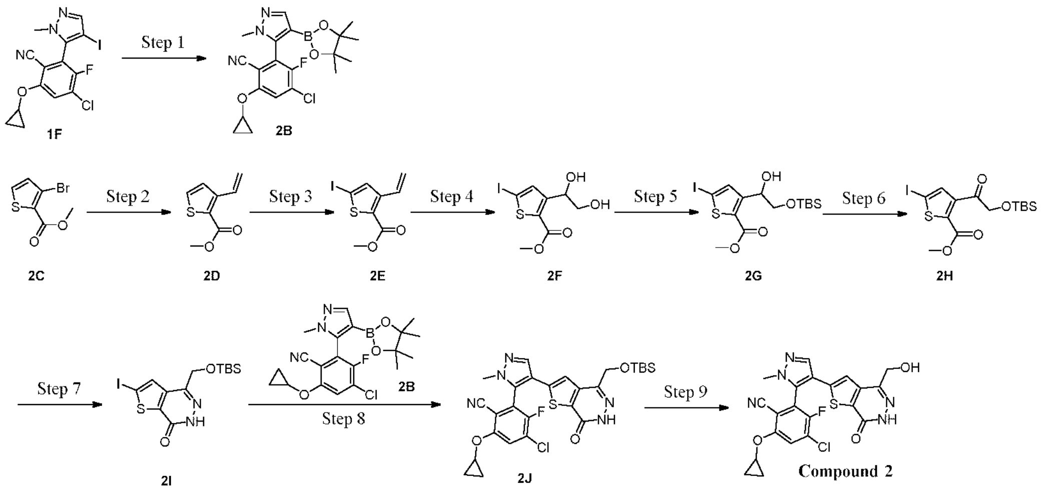


Step 1: Compound 1F (2.0 g, 4.8 mmol), pinacolborane (896.0 mg, 7.0 mmol), tetrakis(triphenylphosphino) palladium (554.0 mg, 0.48 mmol) and triethylamine (960.0 mg, 9.6 mmol) were dissolved in 1,4-dioxane (20.0 mL), and the mixture was subjected to nitrogen replacement three times, heated to 90°C and reacted overnight. The reaction solution was cooled to room temperature, passed through a short silica gel column and rinsed with ethyl acetate (50.0 mL). The filtrate was collected and concentrated. The resulting residue was purified by column chromatography (petroleum ether : ethyl acetate (v : v)=10 : 1) to obtain compound 2B (1.5 g, yield: 75%).

LC-MS (ESI): m/z = 418.1 $[M+H]^+$.

Step 2: Methyl 3-bromothiophene-2-carboxylate (compound 2C) (10.1 g, 45.7 mmol) was dissolved in 1,4-dioxane (150 mL) and water (10 mL). [1,1'-bis(diphenylphosphino)ferrocene]palladium (II) dichloride (1.7 g, 2.3 mmol), caesium carbonate (29.3 g, 90.0 mmol) and potassium vinyl trifluoroborate (6.7 g, 50.0 mmol) were added, and the mixture was subjected to nitrogen replacement three times, heated to 90°C and reacted for 2 h. The reaction solution was cooled to room temperature, passed through a short silica gel column and rinsed with ethyl acetate. The filtrate was collected and concentrated. The resulting residue was purified by column chromatography (petroleum ether : ethyl acetate (v : v)=20 : 1) to obtain compound 2D (6.1 g, yield: 80%).

LC-MS (ESI): m/z = 168.1 $[M+H]^+$.

Step 3: Compound 2D (6.1 g, 36.5 mmol) was dissolved in tetrahydrofuran (250 mL). Under an ice bath, lithium bis(trimethylsilyl)amide (40 mL, 1.0 M in tetrahydrofuran, 40.0 mmol) was slowly added, and further reacted for 0.5 h. Iodine (10.1 g, 40.0 mmol) was added, and the mixture was slowly warmed to room temperature and reacted overnight. The reaction was quenched by adding aqueous sodium thiosulphate solution (100 mL) and extracted with ethyl acetate (100 mL × 3). The organic phase was dried over anhydrous sodium sulphate and concentrated. The resulting residue was purified by column chromatography (petroleum ether : ethyl acetate (v : v)=15 : 1) to obtain compound 2E (8.5 g, yield: 81%).

LC-MS (ESI): m/z = 294.9 $[M+H]^+$.

Step 4: Compound 2E (8.5 g, 29.5 mmol) was dissolved in acetone (50 mL), and then potassium osmate dihydrate (0.9 g, 2.9 mmol) and 4-methylmorpholine-N-oxide (7.0 g, 60.0 mmol) were dissolved in water (10 mL) and added to the above system. The mixture was reacted at room temperature for 2 h. The reaction was quenched by adding saturated aqueous sodium sulphite solution, concentrated to remove acetone and extracted with ethyl acetate (100 mL × 3). The organic phase was dried over anhydrous sodium sulphate and concentrated. The resulting residue was purified by column chromatography (dichloromethane : methanol (v : v)=10 : 1) to obtain compound 2F (7.2 g, yield: 74%).

LC-MS (ESI): m/z = 328.9 $[M+H]^+$.

Step 5: Compound 2F (7.2 g, 21.9 mmol) was dissolved in N,N-dimethylformamide (100 mL). Imidazole (3.0 g, 44.0 mmol) was added, and then tert-butyl dimethylsilyl chloride (3.3 g, 22.0 mmol) was added. The mixture was reacted at room temperature for 2 h. The reaction was quenched by adding water (50 mL) and extracted with ethyl acetate (100 mL × 3). The organic phase was dried over anhydrous sodium sulphate and concentrated to obtain compound 2G (7.4 g, crude), which was directly used in the next reaction.

LC-MS (ESI): m/z = 443.0 $[M+H]^+$.

Step 6: Compound 2G (7.4 g, 16.7 mmol) was dissolved in ethyl acetate (100 mL), and then 2-iodoxybenzoic acid (9.8 g, 35.0 mmol) was added. The mixture was heated to 80°C and reacted overnight. The reaction solution was cooled to room temperature and filtered to remove solids. The filtrate was collected and concentrated to obtain compound 2H (6.4 g, crude), which was directly used in the next reaction.

LC-MS (ESI): m/z = 441.0 $[M+H]^+$.

Step 7: Compound 2H (6.4 g, 14.5 mmol) was dissolved in ethanol (20 mL), and then hydrazine hydrate (6.0 mL) was slowly added. After about 5 min, solids appeared. The mixture was further stirred for 10 min. The reaction solution was filtered. The filter cake was rinsed with ethanol (5.0 mL). The filtrate was collected and concentrated to obtain compound 2I (4.5 g, crude), which was directly used in the next reaction.

LC-MS (ESI): m/z = 423.0 $[M+H]^+$.

Step 8: Compound 2I (590.0 mg, 1.4 mmol), compound 2B (600.0 mg, 1.4 mmol), [1,1'-bis(diphenylphosphino) ferrocene]palladium (II) dichloride (91.0 mg, 0.14 mmol) and sodium bicarbonate (403.2 mg, 4.8 mmol) were dissolved in 1,4-dioxane (10.0 mL) and water (3.0 mL), and the mixture was subjected to nitrogen replacement three times, heated to 80°C and reacted for 2 h. The reaction solution was cooled to room temperature and filtered by a short silica gel column. The filter cake was rinsed with ethyl acetate (50.0 mL). The filtrate was collected and concentrated. The resulting residue was purified by column chromatography (petroleum ether : ethyl acetate (v : v)=1 : 1) to obtain compound 2J (690.0 mg, yield: 84%).

LC-MS (ESI): m/z = 586.1 $[M+H]^+$.

Step 9: Compound 2J (690.0 mg, 1.1 mmol) was dissolved in tetrahydrofuran (20 mL), and then tetrabutylammonium fluoride (1.0 M in tetrahydrofuran, 1.5 mL) was slowly added. The mixture was reacted at room temperature for 1 h. The reaction solution was directly concentrated. The resulting residue was purified by column chromatography (dichloromethane : methanol (v : v)=10 : 1) to obtain compound 2 (480.0 mg, yield: 99%).

$^1$H NMR (400 MHz, DMSO-$d_6$) δ 12.70 (s, 1H), 8.28 (s, 1H), 8.09 (d, 1H), 7.69 (s, 1H), 5.44 (s, 1H), 4.57 (d, 2H), 4.38 - 4.09 (m, 1H), 3.78 (s, 3H), 0.95 - 0.92 (m, 2H), 0.88 - 0.74 (m, 2H);
LC-MS (ESI): m/z = 472.0 $[M+H]^+$.

**Example 3**

2-(4-(4-(aminomethyl)-7-oxo-6,7-dihydrothieno[2,3-d]pyridazin-2-yl)-1-methyl-1H-pyrazol-5-yl)-4-chloro-6-cyclopropoxy-3-fluorobenzonitrile (compound 3)

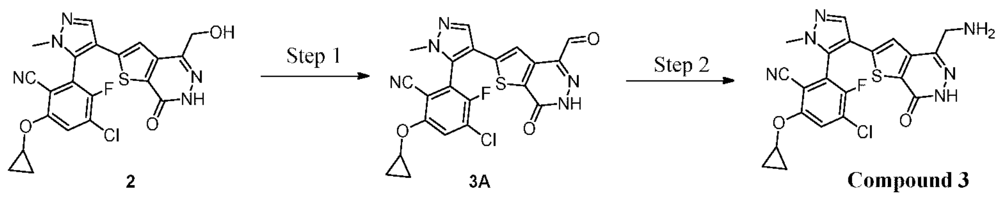


2 3A Compound 3

Step 1: Compound 2 (420.0 mg, 0.9 mmol) was dissolved in ethyl acetate (10 mL), and then 2-iodoxybenzoic acid (504.0 mg, 1.8 mmol) was added. The mixture was heated to reflux and reacted overnight. The reaction solution was cooled to room temperature and filtered. The filter cake was rinsed with ethyl acetate. The filtrate was collected and concentrated. The resulting residue was purified by column chromatography (dichloromethane : methanol (v : v)=10 : 1) to obtain compound 3A (400.0 mg, yield: 95%).

LC-MS (ESI): m/z = 470.0 $[M+H]^+$.

Step 2: Compound 3A (400.0 mg, 0.8 mmol) was dissolved in methanol (10 mL), and then ammonium acetate (246.0 mg, 3.2 mmol) was added. The mixture was heated to reflux and reacted for 1 h. Sodium cyanoborohydride (201.0 mg, 3.2 mmol) was added, and the resulting mixture was reacted for 4 h. The reaction solution was cooled to room temperature and extracted with ethyl acetate (20 mL × 3). The organic phase was dried over anhydrous sodium sulphate, and the resulting crude was concentrated. The residue was purified by column chromatography (dichloromethane : methanol (v : v)=10 : 1) to obtain compound 3 (70.0 mg, yield: 17%).

$^1$H NMR (400 MHz, DMSO-$d_6$) δ 12.66 (s, 1H), 8.30 (s, 1H), 8.09 (d, 1H), 7.85 (s, 1H), 4.42 - 4.15 (m, 3H), 3.87 (s, 2H), 3.77 (s, 3H), 0.95 - 0.93 (m, 2H), 0.90 - 0.73 (m, 2H);
LC-MS (ESI): m/z = 471.0 $[M+H]^+$.

**Example 4**

2-(4-(5-(aminomethyl)-8-oxo-7,8-dihydropyrido[2,3-d]pyridazin-3-yl)-1-methyl-1H-pyrazol-5-yl)-4-chloro-6-cyclopropoxy-3-fluorobenzonitrile (compound 4)

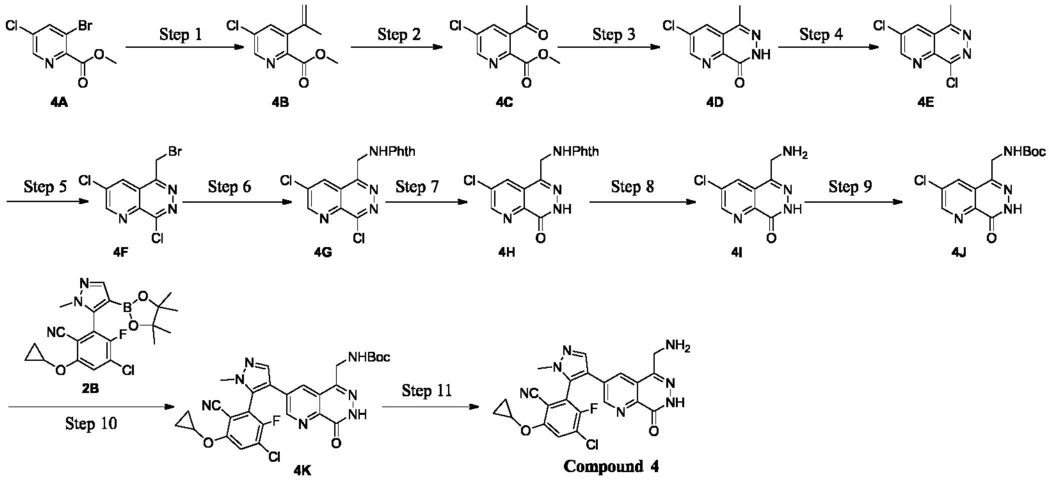


Step 1: Compound 4A (15 g, 59.89 mmol), isopropenylboronic acid pinacol ester (10.57 g, 62.88 mmol), [1,1'-bis(diphenylphosphino)ferrocene]palladium (II) dichloride (2.19 g, 2.99 mmol) and potassium carbonate (20.69 g, 149.72 mmol) were dissolved in 1,4-dioxane (125 mL) and water (25 mL). The mixture was subjected to nitrogen replacement 3 times, and reacted at 80°C for 2 h. The reaction was quenched by adding water (150 mL) and ethyl acetate (100 mL) and filtered. The filtrate was extracted with ethyl acetate (150 mL × 3). The organic phases were combined, dried over anhydrous sodium sulphate, filtered and concentrated. The resulting residue was purified by column chromatography (petroleum ether : ethyl acetate (v : v)=5 : 1) to obtain compound 4B (12.0 g, yield: 94.67%).

LCMS (ESI): m/z = 212.1 $[M+H]^+$.

Step 2: Compound 4B (12 g, 56.70 mmol) was dissolved in tetrahydrofuran (120 mL) and water (120 mL), and then potassium osmate dihydrate (420 mg, 1.13 mmol) and sodium periodate (42.45 g, 198.45 mmol) were added. The mixture was reacted at room temperature overnight. The reaction solution was filtered. The filtrate was quenched with sodium thiosulphate solution (200 mL) and then extracted with ethyl acetate (200 mL × 3). The organic phases were combined, dried over anhydrous sodium sulphate, filtered and concentrated. The resulting crude was purified by column chromatography (petroleum ether : ethyl acetate (v : v)=2 : 1) to obtain compound 4C (8.8 g, yield: 72.65%).

LCMS (ESI): m/z = 214.1 $[M+H]^+$.

Step 3: Compound 4C (8.8 g, 41.19 mmol) was dissolved in ethanol (130 mL), and then hydrazine hydrate (3.75 mL, 61.73 mmol) was added. The mixture was refluxed and reacted at 80°C for 2 hours. The reaction solution was cooled to room temperature and then filtered. The filter cake was rinsed once with ethanol, and the filter cake was collected and dried to obtain compound 4D (6.5 g, yield: 80.67 %).

LCMS (ESI): m/z = 196.0 $[M+H]^+$.

Step 4: Compound 4D (2.0 g, 10.22 mmol) was dissolved in acetonitrile (25 mL), and then phosphorus oxychloride (7.84 g, 51.06 mmol) was added. The mixture was reacted at 80°C for 1.5 hours. The reaction solution was cooled and then concentrated under reduced pressure to remove excess phosphorus oxychloride and acetonitrile. The residue was dissolved in dichloromethane (200 mL), and then washed with saturated sodium bicarbonate (200 mL). The liquid separation was conducted. The organic phase was dried over anhydrous sodium sulphate, filtered and concentrated to obtain compound 4E (2.0 g, crude), which was directly used in the next reaction.

Step 5: Compound 4E (2.0 g, 9.34 mmol) was dissolved in acetonitrile (25 mL), and then azodiisobutyronitrile (0.31 g, 1.89 mmol) and N-bromosuccinimide (2.49 g, 14.04 mmol) were added. The mixture was reacted at 80°C for 16 hours. The reaction solution was cooled and then concentrated under reduced pressure to remove acetonitrile. The residue was dissolved in dichloromethane (20 mL) and then purified by column chromatography to obtain compound 4F (0.73 g, yield: 26.68%).

LCMS (ESI): m/z = 291.9 $[M+H]^+$.

Step 6: Compound 4F (0.73 g, 2.49 mmol) was dissolved in N,N-dimethylformamide (6 mL). At room temperature, phthalimide potassium salt (0.48 g, 2.61 mmol) was added in portions, and the mixture was reacted at room temperature for 1 hour. After the reaction was complete, water (60 mL) was added, and then the mixture was extracted with ethyl acetate (50 mL × 3). The organic phase was washed with water (40 mL × 3), dried over anhydrous sodium sulphate, filtered and concentrated to obtain compound 4G (0.80 g, crude), which was directly used in the next reaction.

LCMS (ESI): m/z = 359.0 $[M+H]^+$.

Step 7: Compound 4G (0.8 g, 2.23 mmol) was dissolved in acetic acid (8 mL), and then potassium acetate hydrate (1.3 g, 11.19 mmol) was added. The mixture was reacted at 100°C for 1 hour. The reaction solution was cooled and then concentrated under reduced pressure to remove acetic acid. Water (50 mL) was added to the residue, and then the mixture was extracted with ethyl acetate (50 mL × 3). The organic phase was dried over anhydrous sodium sulphate, filtered and concentrated to obtain compound 4H (0.80 g, crude), which was directly used in the next reaction.

LCMS (ESI): m/z = 341.0 $[M+H]^+$.

Step 8: Compound 4H (0.8 g) was dissolved in ethanol (15 mL), and then hydrazine hydrate (0.18 g, 3.6 mmol) was added. The mixture was reacted at 80°C for 2 hours. The reaction solution was cooled and then concentrated under reduced pressure to obtain compound 4I (0.6 g, crude), which was directly used in the next reaction.

LCMS (ESI): m/z = 211.1 $[M+H]^+$.

Step 9: Compound 4I (0.8 g, 2.85 mmol) was dissolved in dichloromethane (12 mL), and then triethylamine (0.87 g, 8.61 mmol) and di-tert-butyl dicarbonate (1.24 g, 5.7 mmol) were added. The mixture was reacted at room temperature for 2 hours. The reaction solution was filtered, and the filter cake was collected to obtain compound 4J (0.55 g, crude), which was directly used in the next reaction.

LCMS (ESI): m/z =311.1 $[M+H]^+$.

Step 10: Compound 4J (0.25 g, 0.80 mmol), compound 2B (0.37 g, 0.89 mmol), potassium acetate (0.39 g, 3.98 mmol) and [1,1'-bis(diphenylphosphino)ferrocene]palladium (II) dichloride (0.065 g, 0.079 mmol) were dissolved in acetonitrile (3 mL) and water (1 mL). The mixture was subjected to nitrogen replacement three times and reacted at 130°C for 1 h. The reaction solution was directly concentrated. Dichloromethane (10 mL) was added, and the mixture was filtered. The filtrate was collected and concentrated. The resulting residue was purified by column chromatography (dichloromethane : methanol (v : v)=25 : 1) to obtain compound 4K (0.15 g, yield: 33.13%).

LCMS (ESI): m/z = 566.1 $[M+H]^+$.

Step 11: To a 25 mL single-necked flask, compound 4K (0.15 g, 0.27 mmol) and dichloromethane (4 mL) were added, and trifluoroacetic acid (1 mL) was added dropwise. Upon completion of the addition, the mixture was stirred at room temperature for 1.5 hours. The system was concentrated at 35°C. Dichloromethane (10 mL) and triethylamine (0.3 g) were added, and the mixture was stirred for five minutes. The reaction solution was concentrated, and the resulting crude was purified by preparative HPLC. Preparation method: 1. Instruments: waters 2767 (preparative liquid phase chromatographic instrument); chromatographic column: SunFire@ PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% $NH_3 \cdot H_2O$); b. gradient elution, mobile phase A: 20%-70%; c. flow rate: 20 mL/min; d. elution time: 15 min; retention time: 3.10 min. Compound 4 (0.032 g, yield: 25.44%) was obtained.

$^1$H NMR (400 MHz, DMSO-$d_6$) δ 12.66 (s, 1H), 8.90 (d, 1H), 8.39 (s, 1H), 8.22 (d, 1H), 8.00 (d, 1H), 4.24 - 4.15 (m, 1H), 3.84 (d, 2H), 3.81 (s, 3H), 0.90 (dd, 2H), 0.81 (dd, 2H);
LC-MS (ESI): m/z = 466.1 $[M+H]^+$.

### Example 5

2-(4-(2-(aminomethyl)-4-oxo-3,4-dihydroquinazolin-7-yl)-1-methyl-1H-pyrazol-5-yl)-4-chloro-6-cyclopropoxy-3-fluorobenzonitrile (compound 5)

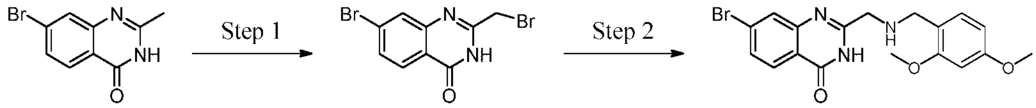


5A 5B 5C

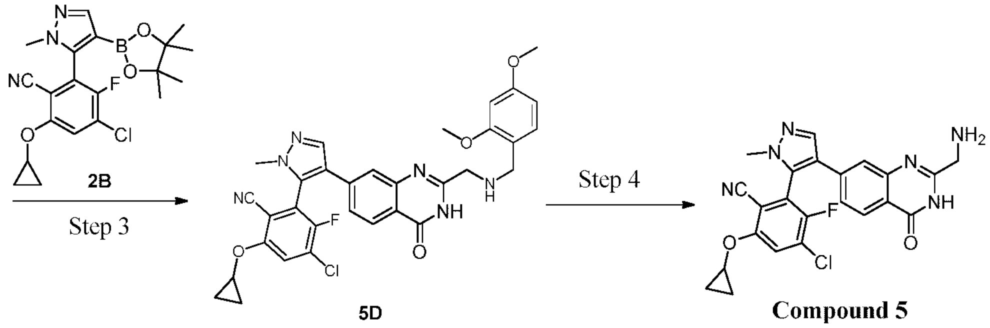


2B

5D Compound 5

Step 1: Compound 5A (4 g, 16.7 mmol, CAS No. 403850-89-5, supplier: Bide Pharmatech Co., Ltd.) and 1-bromopyrrolidine-2,5-dione (2.96 g, 16.7 mmol) were dissolved in N,N-dimethylformamide (60 mL) solution, and the mixture was stirred at 40°C for 12 hours. After the reaction was complete, water (20 mL) was added, and the mixture was extracted with ethyl acetate (20 mL × 3). The organic phase was washed with saturated brine, dried over anhydrous sodium sulphate and concentrated. The resulting residue was purified by column chromatography (dichloromethane : ethyl acetate (v : v)=17 : 100) to obtain compound 5B (1.9 g, yield: 36%).

$^1$H NMR (400 MHz, DMSO-$d_6$) δ 12.72 (s, 1H), 8.03 (d, 1H), 7.91-7.88 (m, 1H), 7.71-7.69 (m, 1H), 4.54 (s, 2H).

Step 2: Compound 5B (0.75 g, 2.36 mmol), (2,4-dimethoxyphenyl)methylamine (0.51 g, 3.05 mmol) and potassium carbonate (0.65 g, 4.72 mmol) were dissolved in N,N-dimethylformamide (20 mL), and the reaction was stirred at room temperature for 1 hour. Water (10 mL) was added, and the mixture was extracted with ethyl acetate (20 mL × 3). The organic phase was washed with saturated brine, dried over anhydrous sodium sulphate and concentrated. The resulting residue was purified by preparative HPLC. Purification methods: 1. Instruments: waters 2767 (preparative liquid phase chromatographic instrument); chromatographic column: SunFire@ PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water; b. gradient elution, mobile phase A: 10%-40%; c. flow rate: 80 mL/min; d. elution time: 20 min; retention time: 10.50 min. Compound 5C (0.27 g, yield: 28%) was obtained.

LC-MS (ESI): m/z = 404.2 $[M+H]^+$.

Step 3: Compound 5C (0.2 g, 0.49 mmol), compound 2B (0.37 g, 0.89 mmol), [1,1'-bis(diphenylphosphino)ferrocene]palladium (II) dichloride (0.036 g, 0.049 mmol) and potassium acetate (0.096 g, 0.98 mmol) were dissolved in a mixed solvent of acetonitrile (9 mL) and water (3 mL). The mixture was subjected to nitrogen replacement 3 times, heated to 130°C under microwave and stirred for 1 hour. Water (10 mL) was added, and the mixture was extracted with ethyl acetate (10 mL × 3). The organic phase was washed with saturated brine, dried over anhydrous sodium sulphate and concentrated. The resulting residue was purified by column chromatography (dichloromethane : methanol (v : v)= 100 : 8) to obtain compound 5D (0.13 g, yield: 43%).

LC-MS (ESI): m/z = 615.1 $[M+H]^+$.

Step 4: Compound 5D (0.1 g, 0.16 mmol) was dissolved in a mixed solvent of trifluoroacetic acid (4 mL) and concentrated hydrochloric acid (1 mL). The mixture was reacted at 50°C for 3 hours. After the reaction was complete, the reaction solution was directly concentrated, and the resulting residue was purified by preparative HPLC. Preparation method: 1. Instruments: waters 2767 (preparative liquid phase chromatographic instrument); chromatographic column: SunFire@ PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% $NH_3.H_2O$); b. gradient elution, mobile phase A: 5%-50%; c. flow rate: 20 mL/min; d. elution time: 20 min; retention time: 15 min. Compound 5 (0.0015 g, yield: 2%) was obtained.

$^1$H NMR (400 MHz, DMSO-$d_6$) δ 12.31 (s, 1H), 8.13 (s, 1H), 8.05 - 7.97 (m, 2H), 7.43 (d, 1H), 7.27-7.24 (m, 1H), 4.20-4.16 (m, 1H), 4.04 (s, 2H), 3.76 (s, 3H), 0.95-0.90 (m, 2H), 0.84 - 0.76 (m, 2H);
LC-MS (ESI): m/z = 465.1 $[M+H]^+$.

**Example 6**

7-(5-(3-chloro-6-cyano-5-cyclopropoxy-2-fluorophenyl)-1-methyl-1H-pyrazol-4-yl)-4-oxo-3,4-dihydrophthalazine-1-carboximidamide (compound 6)

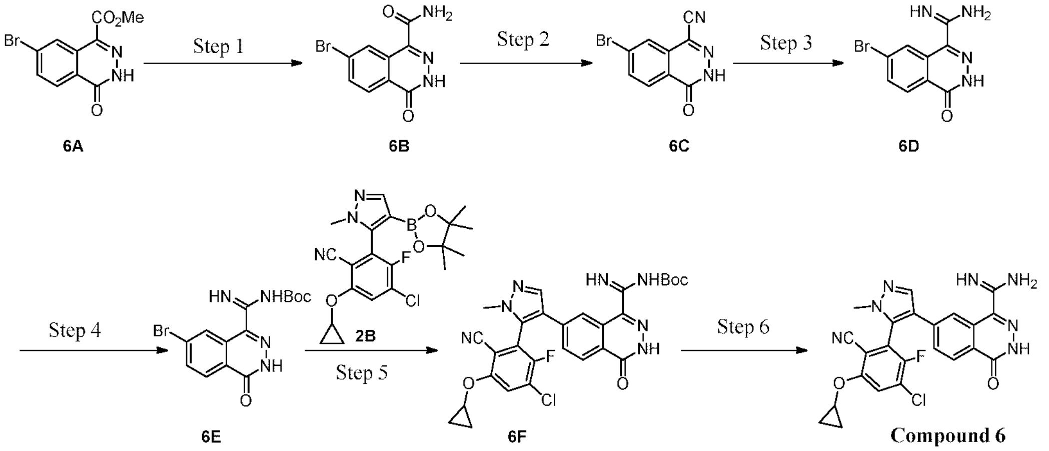


6A 6B 6C 6D 2B 6E 6F Compound 6

Step 1: Compound 6A (1.3 g, 4.59 mmol, synthesized with reference to US 202178994) was added to a sealed tube, and ammonia solution (20 mL, 7 M in methanol) was added. The mixture was heated to 100°C and reacted for 16 hours. The reaction solution was cooled and filtered. The filter cake was collected to obtain compound 6B (1.1 g, crude), which was directly used in the next reaction.

LC-MS (ESI): m/z = 265.9 $[M-H]^-$.

Step 2: Compound 6B (1.1 g, crude, 4.10 mmol) and dioxane (20 mL) were added to a flask, and then phosphorus oxychloride (1.87 mL, 20.46 mmol) was added. The mixture was reacted at 120°C for 6 hours. The reaction solution was concentrated, diluted by adding dichloromethane, adjusted to about pH 8 by adding 30 mL of water and saturated aqueous sodium carbonate solution and extracted with dichloromethane. The organic phase was washed with saturated brine, dried over anhydrous sodium sulphate and concentrated. The resulting residue was separated by column chromatography (petroleum ether : ethyl acetate (v : v)=3 : 1) to obtain compound 6C (690 mg, yield: 67.3%).

LC-MS (ESI): m/z = 248.0 $[M-H]^-$.

Step 3: Ammonium chloride (612 mg, 11.45 mmol) and xylene (10 mL) were added to a flask. Under an ice bath, trimethylaluminium (5.4 mL, 10.80 mmol, 2 M in hexane) was added dropwise, and the mixture was reacted at room temperature for 1 hour. Compound 6C (540 mg, 2.16 mmol) was added, and the mixture was warmed to 120°C and reacted for 24 hours. Then, diethylene glycol dimethyl ether (10 mL) was added, and the mixture was warmed to 160°C and reacted for 8 hours. The reaction solution was cooled. Methanol (5 mL) was added, and the mixture was stirred for 1 hour and filtered. The filter cake was washed with ethyl acetate/methanol (10 mL/10 mL). The filtrate was collected, dried over anhydrous sodium sulphate and then concentrated to obtain compound 6D (3 g, crude), which was directly used in the next reaction.

LC-MS (ESI): m/z = 266.9 $[M+H]^+$.

Step 4: Compound 6D (3 g, crude, 11.23 mmol), methanol (30 mL) and water (10 mL) were added to a flask. Potassium carbonate (4.66 g, 33.69 mmol) and di-tert-butyl dicarbonate (4.9 g, 22.46 mmol) were added. The mixture was reacted at room temperature for 4 hours. Ethyl acetate (50 mL) and saturated sodium chloride solution (50 mL) were added to the system. The mixture was extracted with ethyl acetate. The organic phase was washed with saturated brine, dried over anhydrous sodium sulphate and concentrated. The resulting residue was purified by column chromatography (petroleum ether : ethyl acetate (v : v)=1 : 1) to obtain compound 6E (380 mg, two-step yield: 47.91%).

LCMS (ESI): m/z = 367.0 $[M+H]^+$.

Step 5: Compound 6E (180 mg, 0.49 mmol), compound 2B (250 mg, 0.60 mmol) and sodium bicarbonate (164 mg, 1.96 mmol) were dissolved in 1,4-dioxane (9 mL) and water (3 mL). Under nitrogen protection, [1,1'-bis(diphenylphosphino)ferrocene]palladium (II) dichloride (18 mg, 0.025 mmol) was added. Under nitrogen protection, the mixture was reacted at 80°C for 2 hours. The reaction solution was cooled and then directly concentrated. The resulting residue was separated by column chromatography (petroleum ether : ethyl acetate (v : v)=1 : 4) to obtain compound 6F (80 mg, yield: 28.25%).

LCMS (ESI): m/z = 578.2 $[M+H]^+$.

Step 6: Compound 6F (80 mg, 0.14 mmol) and dichloromethane (6 mL) were added to a flask. Trifluoroacetic acid (1 mL) was added. The mixture was reacted at 35°C for 2 hours. The reaction solution was directly concentrated. The

resulting residue was purified by preparative HPLC. Preparation method: 1. Instruments: waters 2767 (preparative liquid phase chromatographic instrument); chromatographic column: SunFire @ PrepC18 (19 mm × 250 mm). 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% $NH_3.H_2O$); b. gradient elution, mobile phase A: 10%-40%; c. flow rate: 20 mL/min; d. elution time: 10 min; retention time: 3.28 min. Compound 6 (23 mg, yield: 34%) was obtained.

$^1$H NMR (400 MHz, $CD_3OD$) δ 8.33 (d, 1H), 8.10 (s, 1H), 7.89 (d, 1H), 7.85 (s, 1H), 7.80 (t, 1H), 4.09-4.07 (m, 1H), 3.81 (s, 3H), 0.94-0.91 (m, 2H), 0.87-0.84 (m, 2H);
LC-MS (ESI): m/z = 478.4 [M+H]$^+$.

**Example 7**

2-(4-(4-(aminomethyl)-8-(1-methyl-1H-pyrazol-4-yl)-1-oxo-1,2-dihydrophthalazin-6-yl)-1-methyl-1H-pyrazol-5-yl)-4-chloro-6-cyclopropoxy-3-fluorobenzonitrile (compound 7)

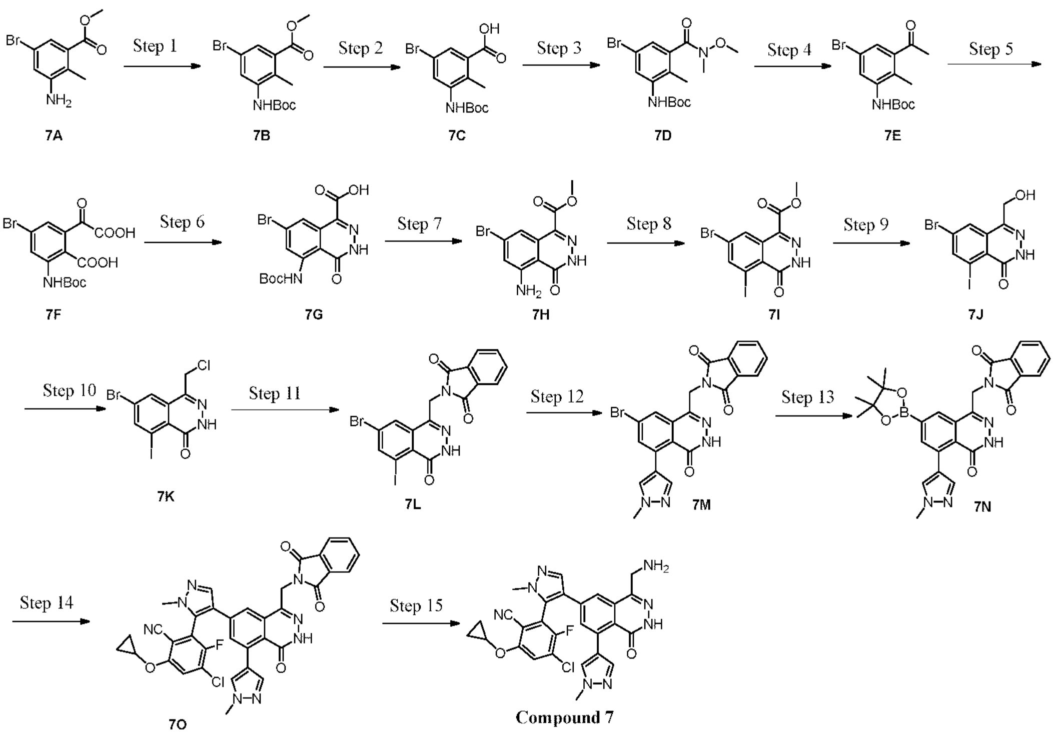

7A 7B 7C 7D 7E 7F 7G 7H 7I 7J 7K 7L 7M 7N 7O Compound 7

Step 1: Compound 7A (100 g, 410 mmol) was dissolved in acetonitrile (1.1 L). Triethylamine (110 mL, 820 mmol) was added, and then di-tert-butyl dicarbonate (119 g, 533 mmol) and 4-dimethylaminopyridine (5.0 g, 41 mmol) were added. The mixture was heated to 50°C and stirred for 2 hours. The reaction solution was cooled and then directly concentrated. Water (1 L) was added, and the mixture was extracted with ethyl acetate (1 L × 3). The organic phases were combined, dried and concentrated to obtain compound 7B (123 g, yield: 87.23%).
$^1$H NMR (400 MHz, DMSO-$d_6$) δ 8.88 (s, 1H), 7.74 (t, 1H), 7.63 (d, 1H), 3.84 (s, 3H), 2.27 (s, 3H), 1.48 (s, 9H).
Step 2: Compound 7B (123 g, 358 mmol) and lithium hydroxide monohydrate (45 g, 1.07 mol) were dissolved in tetrahydrofuran (800 mL) and water (250 mL). The mixture was stirred at room temperature for 18 hours. The reaction solution was directly concentrated. The resulting residue was diluted by adding water (300 mL) and adjusted to pH 2-3 with 2 mol/L dilute hydrochloric acid. The aqueous phase was extracted with ethyl acetate (1 L × 3). The organic phase was collected, dried and concentrated to obtain compound 7C (102 g, yield: 86.45%).
$^1$H NMR (400 MHz, DMSO-$d_6$) δ 8.82 (s, 1H), 7.65 (d, 1H), 7.57 (d, 1H), 2.27 (s, 3H), 1.47 (s, 9H).
Step 3: Compound 7C (102 g, 308.9 mmol) was dissolved in N,N-dimethylformamide (1.0 L). At room temperature, N,N-diisopropylethylamine (102 mL, 620 mmol) and 2-(7-azobenzotriazole)-N,N,N',N'-tetramethyluronium hexafluorophosphate (HATU) (153 g, 403 mmol) were added. At room temperature, the mixture was stirred for 30 min. Finally, dimethylhydroxylamine hydrochloride (45.6 g, 465 mmol) was added, and the mixture was reacted at room

temperature for 2 h. The reaction solution was poured into 2 L of water, and then the mixture was extracted three times with ethyl acetate (1 L × 3). The organic phase was collected, dried and concentrated. The resulting residue was purified by silica gel column chromatography (petroleum ether : ethyl acetate (v : v)=3 : 1) to obtain compound 7D (82 g, yield: 71.12%).

$^1$H NMR (400 MHz, DMSO-$d_6$) δ 8.78 (s, 1H), 7.63 (s, 1H), 7.24 (d, 1H), 3.43 (s, 3H), 3.26 (s, 3H), 2.04 (s, 3H), 1.47 (s, 9H).

Step 4: Compound 7D (82 g, 220 mmol) was dissolved in tetrahydrofuran (800 mL). At 0°C, methylmagnesium bromide (220 mL, 660 mmol, 3 M in ether) was added, and the mixture was slowly warmed to room temperature and reacted for 16 hours. To the reaction system, saturated ammonium chloride solution (1 L) was added, and the mixture was stirred for 10 minutes and then extracted with ethyl acetate (500 mL × 3). The organic phase was collected, dried and concentrated. The resulting residue was purified by silica gel column chromatography (petroleum ether : ethyl acetate (v : v)=3 : 1) to obtain compound 7E (70 g, yield: 97.08%).

$^1$H NMR (400 MHz, DMSO-$d_6$) δ 8.84 (s, 1H), 7.67 (d, 1H), 7.62 (d, 1H), 2.53 (s, 3H), 2.13 (s, 3H), 1.47 (s, 9H).

Step 5: Compound 7E (53 g, 160 mmol) was dissolved in tert-butanol (500 mL) and water (500 mL). Anhydrous potassium carbonate (44 g, 320 mmol) was added. The mixture was heated to 70°C and stirred. Finally, potassium permanganate (179 g, 1.13 mol) was added to the above-mentioned reaction system in six portions with an interval of 30 min. Upon completion of the addition, the mixture was further reacted at 70°C for 1 hour. The reaction solution was cooled to room temperature and filtered over celite. The filtrate was collected, adjusted to pH 2-3 with 2 mol/L dilute hydrochloric acid and then extracted with a mixed solvent of ethyl acetate and tetrahydrofuran (v : v=10 : 1) (700 mL × 3). The organic phase was collected, dried and concentrated to obtain compound 7F (39 g, crude), which was directly used in the next reaction.

LC-MS (ESI): m/z = 388.1 $[M+H]^+$.

Step 6: Compound 7F (39 g, 100 mmol) was dissolved in ethanol (200 mL), and then hydrazine hydrate (7.24 mL, 120 mmol) was added. The mixture was heated to 75°C and reacted for 15 hours. The reaction solution was cooled to room temperature and filtered. The filter cake was washed with ethanol (100 mL), collected and dried to obtain compound 7G (17 g, yield: 43.81%).

$^1$H NMR (400 MHz, DMSO-$d_6$) δ 12.00 (s, 1H), 8.63 (s, 1H), 8.05 (s, 1H), 1.50 (s, 9H).

Step 7: Compound 7G (17 g, 44.2 mmol) was dissolved in methanol (150 mL). Hydrogen chloride in methanol (4 mol/L, 240 mL) was added. The mixture was heated to 70°C and reacted for 2 hours. The reaction solution was cooled and then directly concentrated. To the residue, water (200 mL) was added. The mixture was adjusted to pH 8 with sodium bicarbonate and filtered. The filter cake was washed with water (100 mL), collected and dried to obtain compound 7H (13 g, yield: 98.56%).

$^1$H NMR (400 MHz, DMSO-$d_6$) δ 12.87 (s, 1H), 7.58 (s, 2H), 7.13 (s, 1H), 3.87 (s, 3H);
LC-MS (ESI): m/z = 298.0 $[M+H]^+$.

Step 8: Compound 7H (13 g, 43.6 mmol) and p-toluenesulfonic acid (33 g, 174.4 mmol) were dissolved in acetonitrile (2.0 L). The mixture was subjected to nitrogen replacement twice, and then sodium nitrite (7.5 g, 109 mmol) was dissolved in water (20 mL) and added dropwise to the above-mentioned system. The mixture was reacted at this temperature for 1 h. Finally, potassium iodide (21.7 g, 130.8 mmol) was dissolved in water (20 mL) and added dropwise to the above-mentioned reaction solution. Upon completion of the addition, the mixture was warmed to room temperature and reacted for 2 hours. Saturated sodium thiosulphate solution (200 mL) was added to the system, and the mixture was stirred for ten minutes, concentrated to remove the organic phase and filtered. The filter cake was washed with water (300 mL), collected and dried to obtain compound 7I (8.2 g, yield: 45.98%).

$^1$H NMR (400 MHz, DMSO-$d_6$) δ 13.24 (s, 1H), 8.73 (d, 1H), 8.61 (d, 1H), 3.90 (s, 3H);
LC-MS (ESI): m/z = 409.1 $[M+H]^+$.

Step 9: Compound 7I (8.2 g, 20 mmol) was dissolved in a mixed solvent of tetrahydrofuran (240 mL) and ethanol (240 mL). At 0°C, anhydrous calcium chloride (2.5 g, 2.4 mmol) was added, and then sodium borohydride (1.47 g, 40 mmol) was added to the above-mentioned reaction system in four portions with an interval of 10 min. Upon completion of the addition, the mixture was further reacted for 10 min. Saturated ammonium chloride solution (100 mL) was added to the system, and the mixture was stirred at room temperature for 10 min and filtered. The filter cake was washed with water (100 mL), collected and dried. Then, the solid was mixed evenly with methanol (200 mL). The mixture was stirred for three hours and filtered, and the filter cake was collected and dried to obtain compound 7J (4.8 g, yield: 62.84%).

$^1$H NMR (400 MHz, DMSO-$d_6$) δ 12.61 (s, 1H), 8.55 (d, 1H), 8.28 (d, 1H), 5.55 (t, 1H), 4.61 (d, 2H);
LC-MS (ESI): m/z = 381.1 $[M+H]^+$.

Step 10: Compound 7J (0.95 g, 2.5 mmol) was dissolved in thionyl chloride (10 mL), and the mixture was heated to 70°C and reacted for 2 hours. The reaction solution was cooled and then directly concentrated. The resulting residue was added to petroleum ether (20 mL) and mixed evenly. The mixture was stirred for 20 min and filtered. The filter cake was washed with petroleum ether (30 mL), collected and dried to obtain compound 7K (0.92 g, yield: 89.93%).

$^1$H NMR (400 MHz, DMSO-$d_6$) δ 12.86 (s, 1H), 8.60 (d, 1H), 8.27 (d, 1H), 5.01 (s, 2H).

Step 11: Compound 7K (0.92 g, 2.24 mmol) was dissolved in N,N-dimethylformamide (20 mL). Phthalimide potassium salt (0.42 g, 2.24 mmol) was added, and the mixture was reacted at room temperature for 2 hours. The reaction solution was poured into water (200 mL), and the mixture was stirred for 10 minutes and then filtered. The filter cake was washed with water (50 mL), collected and dried to obtain compound 7L (1.1 g, yield: 96.32%).

$^1$H NMR (400 MHz, DMSO-$d_6$) δ 12.54 (s, 1H), 8.61 (d, 1H), 8.40 (d, 1H), 7.96 - 7.94 (m, 2H), 7.90 - 7.88 (m, 2H), 5.12 (s, 2H);
LC-MS (ESI): m/z = 510.3 [M+H]$^+$.

Step 12: Compound 7L (300 mg, 0.59 mmol), 1-methyl-4-(4,4,5,5-tetramethyl-1,3,2-dioxaborolan-2-yl)-1H-pyrazole (110 mg, 0.53 mmol), [1,1'-bis(diphenylphosphino)ferrocene]palladium (II) dichloride (43 mg, 0.059 mmol) and sodium bicarbonate (200 mg, 2.36 mmol) were dissolved in a mixed solvent of N,N-dimethylformamide and water (v : v=2 : 1, 12 mL). The mixture was subjected to nitrogen replacement three times, heated to 70°C and reacted for 5 hours. The reaction solution was cooled to room temperature and directly concentrated. The resulting residue was purified by silica gel column chromatography (dichloromethane : methanol (v : v)=20 : 1-10 : 1) to obtain compound 7 M (180 mg, yield: 65.72%).

LC-MS (ESI): m/z = 466.1 [M+H]$^+$.

Step 13: Compound 7 M (180 mg, 0.38 mmol), bis(pinacolato)diboron (190 mg, 0.76 mmol), [1,1'-bis(diphenylphosphino)ferrocene]palladium (II) dichloride (27 mg, 0.038 mmol) and potassium acetate (88 mg, 1.8 mmol) were dissolved in 1,4-dioxane (10 mL). The mixture was subjected to nitrogen replacement three times, heated to 80°C and reacted for 16 hours. The reaction solution was cooled and then directly concentrated. The resulting residue was mixed evenly with a mixed solvent of petroleum ether and ethyl acetate (v : v=10 : 1, 20 mL), and the mixture was stirred at room temperature for 1 hour and filtered. The filter cake was washed once with a mixed solvent of petroleum ether and ethyl acetate (v : v=10 : 1, 20 mL), collected and dried to obtain compound 7N (300 mg, crude), which was directly used in the next reaction.

LC-MS (ESI): m/z = 430.1 [M+H]$^+$.

Step 14: Compound 7N (300 mg, 0.58 mmol), compound 1F (242 mg, 0.58 mmol), [1,1'-bis(diphenylphosphino) ferrocene]palladium (II) dichloride (42 mg, 0.058 mmol) and sodium bicarbonate (101 mg, 1.2 mmol) were dissolved in a mixed solvent of 1,4-dioxane and water (v : v=5 : 1, 18 mL). The mixture was subjected to nitrogen replacement three times, warmed to 80°C and reacted for 5 hours. The reaction solution was cooled and then directly concentrated to obtain compound 7O (600 mg, crude), which was directly used in the next reaction.

LC-MS (ESI): m/z = 676.1 [M+H]$^+$.

Step 15: Compound 7O (600 mg) was dissolved in ethanol (30 mL), and then hydrazine hydrate (0.1 mL) was added. The mixture was reacted at 80°C for 1 hour. The reaction solution was cooled and then directly concentrated. The resulting residue was purified by preparative HPLC. Preparation method: 1. Instruments: waters 2767 (preparative liquid phase chromatographic instrument); chromatographic column: SunFire@PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% $NH_3 \cdot H_2O$); b. gradient elution, mobile phase A: 20%-60%; c. flow rate: 20 mL/min; d. elution time: 20 min; retention time: 3.26 min. Compound 7 (26 mg, yield: 12.58%) was obtained.

$^1$H NMR (400 MHz, DMSO-$d_6$) δ 12.18 (s, 1H), 8.35 (s, 1H), 8.05 (d, 1H), 7.84 (s, 1H), 7.59 (s, 1H), 7.50 (s, 1H), 7.40 (s, 1H), 4.21 (s, 1H), 3.86 (s, 3H), 3.79 (s, 3H), 3.75 (s, 2H), 0.92 (d, 2H), 0.84 - 0.74 (m, 2H);
LC-MS (ESI): m/z = 545.1 [M+H]$^+$.

**Example 8 and Example 9**

(2S)-2-(4-(4-(aminomethyl)-1-oxo-1,2-dihydroisoquinolin-6-yl)-1-methyl-1H-pyrazol-5-yl)-4-chloro-3-fluoro-6-(3-methylazetidin-1-yl)benzonitrile and (2R)-2-(4-(4-(aminomethyl)-1-oxo-1,2-dihydroisoquinolin-6-yl)-1-methyl-1H-pyrazol-5-yl)-4-chloro-3-fluoro-6-(3-methylazetidin-1-yl)benzonitrile (compound 8, compound 9)

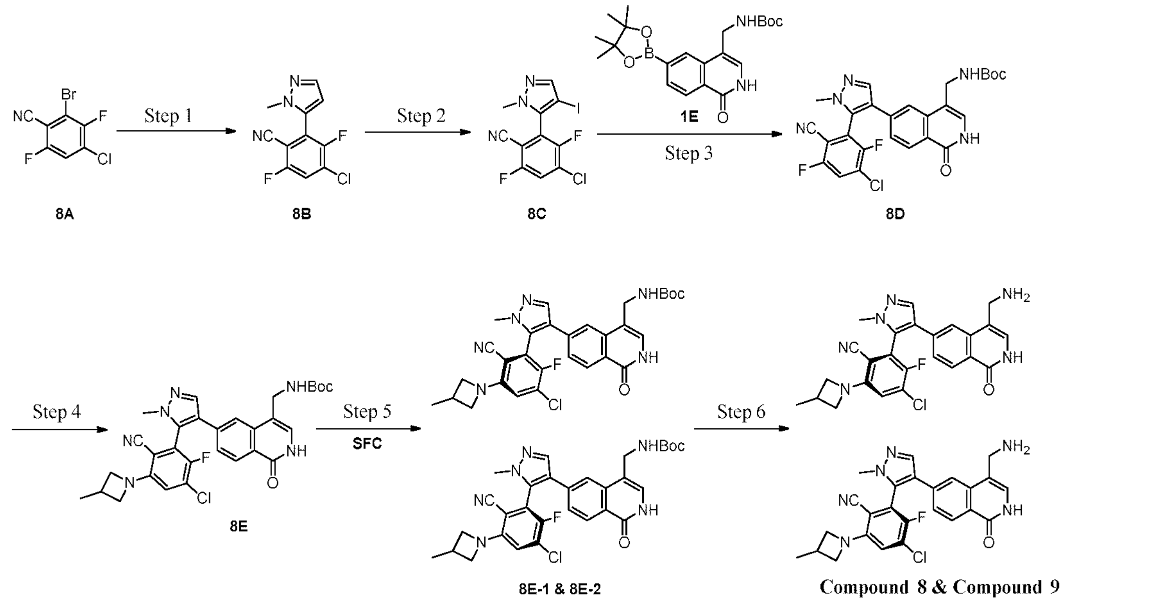


Step 1: Compound 8A (30 g, 118.84 mmol, synthesized with reference to US 202178994), 1-methyl-5-(4,4,5,5-tetramethyl-1,3,2-dioxaborolan-2-yl)-1H-pyrazole (40 g, 190.2 mmol), caesium fluoride (30 g, 202 mmol) and [1,1'-bis(diphenylphosphino)ferrocene]palladium (II) dichloride (8.7 g, 11.9 mmol) were weighed into a three-necked flask. Cyclopentyl methyl ether was added. Under nitrogen protection, the mixture was reacted at 90°C for 3 hours. A mixed solvent of petroleum ether and ethyl acetate (v : v=1 : 1, 250 mL) was added, and the mixture was stirred and filtered. The filtrate was collected. The filter cake was washed twice with a mixed solvent of petroleum ether and ethyl acetate (v : v=1 : 1, 250 mL). The filtrates were combined and concentrated. The resulting residue was separated by column chromatography (petroleum ether:ethyl acetate (v : v)=10 : 1) to obtain compound 8B (18.80 g, yield: 62%).

LC-MS (ESI): m/z = 254.0 $[M+H]^+$.

Step 2: Compound 8B (3.00 g, 11.83 mmol) was dissolved in acetonitrile (80 mL). N-iodosuccinimide (5.32 g, 23.66 mmol) and trifluoroacetic acid (0.67 g, 5.92 mmol) were added, and then the mixture was stirred at 50°C for 4 h. The reaction solution was directly concentrated. The resulting residue was separated by column chromatography (petroleum ether : ethyl acetate (v : v)=1 : 5) to obtain compound 8C (4.30 g, yield: 95%).

LC-MS (ESI): m/z = 379.9 $[M+H]^+$.

Step 3: Compound 8C (1.14 g, 3.00 mmol), compound 1E (1.00 g, 2.50 mmol) and sodium bicarbonate (0.63 g, 7.50 mmol) were successively added to a solution of 1,4-dioxane (75 mL) and water (25 mL). The mixture was subjected to nitrogen replacement 3 times, and then [1,1'-bis(diphenylphosphino)ferrocene]palladium (II) dichloride (0.18 g, 0.25 mmol) was added. The resulting mixture was further subjected to nitrogen replacement 3 times, warmed to 60°C and reacted for 1 hour. The reaction solution was cooled and then directly concentrated. The resulting residue was separated by silica gel column chromatography (dichloromethane : methanol (v : v)=100%-90%) to obtain compound 8D (680 mg, yield: 51.72%).

LC-MS (ESI): m/z = 526.2 $[M+H]^+$.

Step 4: Compound 8D (400 mg, 0.76 mmol), potassium carbonate (0.32 g, 2.32 mmol) and 3-methylazetidine (65 mg, 0.91 mmol) were successively added to N,N-dimethylformamide (20 mL). The mixture was warmed to 80°C and reacted for 1 hour. The reaction solution was cooled and then filtered, and ethyl acetate (50 mL) and water (50 mL) were added to the filtrate. The liquid separation was conducted. The organic phase was collected, dried and concentrated. The resulting residue was purified by silica gel column chromatography (dichloromethane : methanol (v : v)=100%-90%) to obtain compound 8E (79 mg, yield: 18.01%).

LC-MS (ESI): m/z = 575.0 $[M-H]^-$.

Step 5: Compound 8E (79 mg, 0.13 mmol) was resolved by SFC to obtain P1 (retention time: 1.404 min, set as

compound 8E-1) and P2 (retention time: 2.049 min, set as compound 8E-2). Resolution conditions: instrument: WATERS 150 preparative SFC(SFC-26); chromatographic column: ChiralCel OX, 250 × 30 mm I.D., 5 μm; mobile phase: A for $CO_2$, and B for isopropanol and acetonitrile (containing 0.1% aqueous ammonia); gradient: B 50%; flow rate: 120 mL/min; column pressure: 100 bar; column temperature: 25°C; wavelength: 220 nm; cycle time: 4.5 min; sample preparation: the compound was dissolved in acetonitrile; injection: 3 mL/injection; treatment: after the separation, the mixture was concentrated by a rotary evaporator at 35°C, and then the solvent was dried by a lyophilizer at -80°C. Compound 8E-1 (38 mg, 48.1%) and compound 8E-2 (40 mg, 50.6%) were obtained.

Compound 8E-1: LC-MS (ESI): m/z = 577.5 $[M+H]^+$.

Compound 8E-2: LC-MS (ESI): m/z = 577.5 $[M+H]^+$.

Step 6: Compound 8E-1 (38 mg, 0.066 mmol) was dissolved in dichloromethane (2 mL), and then trifluoroacetic acid (0.5 mL) was added. At room temperature, the mixture was stirred and reacted for 1 hour. The reaction solution was directly concentrated. The resulting residue was purified by preparative HPLC. Separation method: 1. Instruments: waters 2767 (preparative liquid phase chromatographic instrument); chromatographic column: SunFire @ PrepC18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.1% $NH_3 \cdot H_2O$); b. gradient elution, mobile phase A: 10%-70%; c. flow rate: 20 mL/min; d. elution time: 15 min; retention time: 11.0 min. Compound 8 (14 mg, yield: 44%) was obtained.

Compound 8: $^1H$ NMR (400 MHz, $CDCl_3$) δ 8.23 (s, 1H), 7.92 (s, 1H), 7.55 (s, 1H), 7.14 (s, 2H), 6.56 (d,1H), 4.31-4.23 (m,3H), 3.81 (s, 4H), 3.77-3.73 (m, 1H), 3.70-3.66 (m, 1H), 2.79 (s, 1H), 1.26 (s, 3H); LC-MS (ESI): m/z = 475.0 $[M-H]^-$.

Compound 8E-2 (40 mg, 0.069 mmol) was dissolved in dichloromethane (2 mL), and then trifluoroacetic acid (0.5 mL) was added. At room temperature, the mixture was stirred and reacted for 1 hour. The reaction solution was directly concentrated. The resulting residue was purified by preparative HPLC. Separation method: 1. Instruments: waters 2767 (preparative liquid phase chromatographic instrument); chromatographic column: SunFire @ PrepC18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.1% $NH_3 \cdot H_2O$); b. gradient elution, mobile phase A: 10%-70%; c. flow rate: 20 mL/min; d. elution time: 15 min; retention time: 12.5 min. Compound 9 (14.6 mg, yield: 44%) was obtained.

Compound 9: $^1H$ NMR (400 MHz, $CDCl_3$) δ 8.23 (s, 1H), 7.92 (s, 1H), 7.54 (s, 1H), 7.15 (s, 2H), 6.56 (d, 1H), 4.30-4.23(m, 3H), 3.82 (s, 4H), 3.77-3.73 (m, 1H), 3.70-3.66 (m, 1H), 2.79 (s, 1H), 1.25 (s, 3H); LC-MS (ESI): m/z=475.0 $[M-H]^-$.

### Example 10

2-(4-(4-(2-aminoethyl)-1-oxo-1,2-dihydrophthalazin-6-yl)-1-methyl-1H-pyrazol-5-yl)-4-chloro-6-cyclopropoxy-3-fluorobenzonitrile (compound 10)

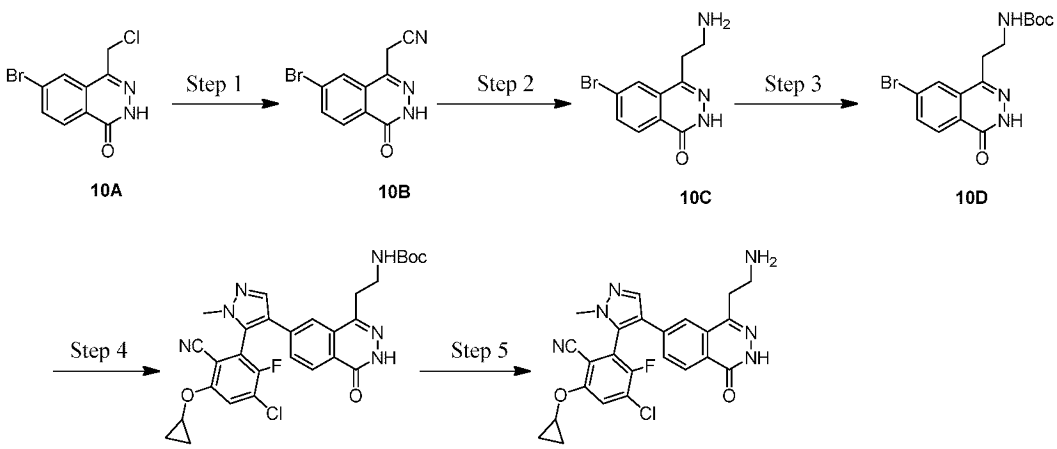


10A 10B 10C 10D

10E Compound 10

Step 1: Compound 10A (2.98 g, 11 mmol, synthesized with reference to Journal of Medicinal Chemistry, 2022, vol. 65, # 3, p. 1749 - 1766) was dissolved in anhydrous acetonitrile (30 mL). Trimethylsilyl cyanide (2.75 mL, 22 mmol) was

added. The mixture was subjected to nitrogen replacement, and then tetrabutylammonium fluoride (11.51 g, 44 mmol) was added. The resulting mixture was heated to 80°C and stirred for 3 h. The reaction was cooled to room temperature, quenched with water (100 mL) and extracted with ethyl acetate (200 mL × 3). The organic phase was dried over anhydrous sodium sulphate and concentrated. The resulting residue was slurried and purified with a mixed solvent of petroleum ether and dichloromethane (v : v=10 : 1) to obtain compound 10B (2.6 g, yield: 90%).

$^1$H NMR (400 MHz, DMSO-$d_6$) δ 12.86 (s, 1H), 8.17 (dd, 2H), 8.06 (dd, 1H), 4.49 (s, 2H);
LC-MS (ESI): m/z = 264.0 $[M+H]^+$.

Step 2: Compound 10B (1.0 g, 3.8 mmol) was dissolved in ethanol (15 mL). Nickel chloride (0.41 g, 3.1 mmol) and sodium borohydride (11.51 g, 30.4 mmol) were added. The mixture was heated to 45°C and stirred for 3 hours. Compound 10C in ethanol was obtained, and the reaction solution was directly used in the next reaction.

LC-MS (ESI): m/z = 268.0 $[M+H]^+$.

Step 3: To compound 10C in ethanol (15 mL), triethylamine (0.16 g, 15.2 mmol) and di-tert-butyl dicarbonate (6.63 g, 30.4 mmol) were slowly added dropwise. The mixture was reacted at room temperature overnight. Water (100 mL) was added, and the mixture was extracted with ethyl acetate (200 mL × 3). The organic phase was dried over anhydrous sodium sulphate and concentrated. The resulting residue was purified by column chromatography (petroleum ether : ethyl acetate (v/v)= 5 : 1) to obtain compound 10D (0.068 g, yield: 4.9%).

LC-MS (ESI): m/z = 368.2 $[M+H]^+$.

Step 4: Compound 10D (68 mg, 0.18 mmol), compound 2B (150 mg, 0.36 mmol), [1,1'-bis(diphenylphosphino) ferrocene]palladium (II) dichloride (29 mg, 0.036 mmol) and sodium bicarbonate (53 mg, 0.63 mmol) were dissolved in 1,4-dioxane (10 mL) and water (2 mL). The mixture was subjected to nitrogen replacement 3 times, heated to 80°C and stirred for 2 hours. The reaction solution was cooled, and then water (50 mL) was added. The mixture was extracted with ethyl acetate (100 mL × 3). The organic phase was dried over anhydrous sodium sulphate and concentrated. The resulting residue was separated by column chromatography (petroleum ether : ethyl acetate (v/v)=1 : 5) to obtain compound 10E (87 mg, yield: 83%).

LC-MS (ESI): m/z = 579.3 $[M+H]^+$.

Step 5: Compound 10E (87 mg, 0.15 mmol) was dissolved in dichloromethane (4 mL). Trifluoroacetic acid (1 mL) was added, and the mixture was stirred at room temperature for 1 hour and concentrated. The resulting residue was purified by preparative HPLC. Preparation method: 1. Instruments: waters 2767 (preparative liquid phase chromatographic instrument); chromatographic column: SunFire@ PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water; b. gradient elution, mobile phase A: 10%-40%; c. flow rate: 20 mL/min; d. elution time: 10 min. Compound 10 (5 mg, yield: 7%) was obtained.

$^1$H NMR (400 MHz, DMSO-$d_6$) δ 12.44 (s, 1H), 8.26 (s, 1H), 8.16 (t, 1H), 8.02 (t, 1H), 7.67 (d, 1H), 7.56 (s, 1H), 4.24 - 4.13 (m, 1H), 3.77 (d, 3H), 2.80 (dd, 4H), 0.90 (t, 2H), 0.83 (d, 2H);
LC-MS (ESI): m/z = 479.50 $[M+H]^+$.

## Example 11 and Example 12

(2S)-2-(4-(2-(aminomethyl)-4-oxo-3,4-dihydroquinazolin-7-yl)-1-methyl-1H-pyrazol-5-yl)-4-chloro-6-cyclopropoxy-3-fluorobenzonitrile and (2R)-2-(4-(2-(aminomethyl)-4-oxo-3,4-dihydroquinazolin-7-yl)-1-methyl-1H-pyrazol-5-yl)-4-chloro-6-cyclopropoxy-3-fluorobenzonitrile (compound 11, compound 12)

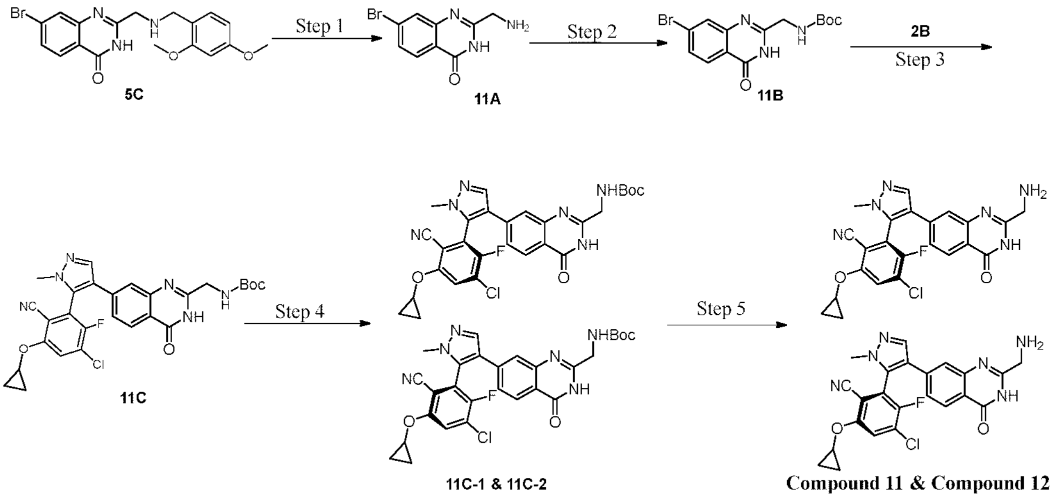


Step 1: Compound 5C (4 g, 9.89 mmol) was dissolved in trifluoroacetic acid (20 mL), and the mixture was heated to 50°C and stirred for 1 hour. The reaction solution was directly concentrated. Saturated aqueous sodium carbonate solution (50 mL) was added to the resulting residue, and the mixture was stirred for half an hour and filtered. The filter cake was collected, washed twice with water and dried to obtain compound 11A (2.4 g, crude), which was directly used in the next reaction.

LC-MS (ESI): m/z = 254.0 $[M+H]^+$.

Step 2: Compound 11A (2.5 g, 9.84 mmol) was dissolved in methanol (20 mL) and dichloromethane (20 mL), and then di-tert-butyl dicarbonate (6.5 g, 29.51 mmol) was added. The mixture was stirred at room temperature for 1 hour. The reaction solution was directly concentrated. Water (50 mL) was added, and the mixture was stirred evenly and filtered. The filter cake was collected, washed 3 times with petroleum ether and dried to obtain compound 11B (1.6 g, crude), which was directly used in the next reaction.

LC-MS (ESI): m/z = 354.0 $[M+H]^+$.

Step 3: Compound 11B (0.1 g, 0.24 mmol), compound 2B (0.16 g, 0.24 mmol), [1,1'-bis(diphenylphosphino) ferrocene]palladium (II) dichloride (0.018 g, 0.024 mmol) and sodium bicarbonate (0.06 g, 0.72 mmol) were dissolved in a mixed solvent of 1,4-dioxane (5 mL) and water (1 mL). The mixture was subjected to nitrogen replacement 3 times, heated to 80°C and stirred for 1 hour. The reaction solution was cooled, and then water (10 mL) was added. The mixture was extracted with ethyl acetate (10 mL × 3). The organic phase was collected, washed with saturated brine, dried over anhydrous sodium sulphate and concentrated. The resulting residue was purified by column chromatography (dichloromethane : methanol (v : v)=100:8) to obtain compound 11C (0.08 g, yield: 59.00%).

LC-MS (ESI): m/z = 565.2 $[M+H]^+$.

Step 4: Compound 11C (320 mg) was resolved by chiral SFC to obtain P1 (retention time: 1.891 min, set as compound 11C-1) and P2 (retention time: 2.139 min, set as compound 11C-2). Resolution method: instrument: Waters 150 SFC; chromatographic column: Chiralcel OD-Column; mobile phase: A for $CO_2$ and B for EtOH (0.1% $NH_3$•$H_2O$); gradient: 35% phase B isocratic elution; flow rate: 80 mL/min; column pressure: 100 bar; column temperature: 25°C; absorption wavelength: 220 nm; cycle time: 3 min; sample preparation: the compound was dissolved in acetonitrile at a concentration of 5 mg/mL; injection: 3 mL/injection; treatment: after the separation, the mixture was concentrated by a rotary evaporator at 35°C, and then the solvent was dried by a lyophilizer at -80°C to obtain compounds 11C- 1 (93.6 mg, 29.25%) and 11C-2 (100 mg, 31.25%).

Step 5: Compound 11C-1 (93.6 mg, 0.17 mmol) was dissolved in hydrogen chloride in dioxane (5 mL, 4 M), and the mixture was stirred at room temperature for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was separated by reverse phase column chromatography (water : acetonitrile (v/v)=67 : 33) to obtain compound 11 (38 mg, yield: 48.08%).

Compound 11: $^1$H NMR (400 MHz, DMSO-$d_6$) δ 8.16 (s, 1H), 8.00 (dd, 2H), 7.37 (d, 1H), 7.21 (dd, 1H), 4.25 - 4.15 (m, 1H), 3.76 (s, 3H), 3.63 (s, 2H), 0.94 - 0.75 (m, 4H);
LC-MS (ESI): m/z = 465.4 $[M+H]^+$.

Compound 11C-2 (100 mg, 0.18 mmol) was dissolved in hydrogen chloride in dioxane (5 mL, 4 M), and the mixture was stirred at room temperature for 2 hours. The reaction system was concentrated and then diluted by adding

dichloromethane. The mixture was adjusted to a basic pH by adding sodium bicarbonate solution and extracted with dichloromethane. The liquid separation was conducted. The organic phase was collected, dried and concentrated. The resulting crude was separated by reverse phase column chromatography (water : acetonitrile (v/v)=67 : 33) to obtain compound 12 (40 mg, yield: 47.80%).

Compound 12: $^1$H NMR (400 MHz, DMSO-$d_6$) δ 8.16 (s, 1H), 8.00 (dd, 2H), 7.37 (d, 1H), 7.21 (dd, 1H), 4.26 - 4.16 (m, 1H), 3.76 (s, 3H), 3.65 (s, 2H), 0.97 - 0.74 (m, 4H);
LC-MS (ESI): m/z = 465.5 [M+H]$^+$.

**Example 13**

1-(aminomethyl)-7-(5-(3-chloro-6-cyano-5-cyclopropoxy-2-fluorophenyl)-1-methyl-1H-pyrazol-4-yl)-4-oxo-3,4-dihydrophthalazine-5-carbonitrile (compound 13)

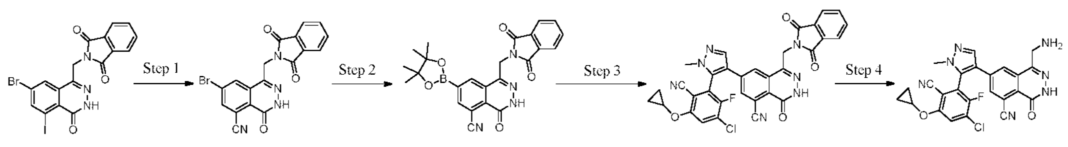


7L 13A 13B 13C Compound 13

Step 1: Compound 7L (300 mg, 0.59 mmol), zinc cyanide (70 mg, 0.53 mmol) and tetrakis(triphenylphosphino) palladium (68 mg, 0.059 mmol) were dissolved in N,N-dimethylformamide (8 mL). The mixture was subjected to nitrogen replacement three times, heated to 80°C and reacted for 4 hours. The reaction solution was cooled and then directly concentrated. The resulting residue was purified by column chromatography (petroleum ether : ethyl acetate (v : v)=1 : 1-1 : 2) to obtain compound 13A (130 mg, yield: 53.85%).

$^1$H NMR (400 MHz, DMSO-$d_6$) δ 12.86 (s, 1H), 8.73 (d, 1H), 8.65 (d, 1H), 7.95 (dd, 2H), 7.90 (dd, 2H), 5.21 (s, 2H);
LC-MS (ESI): m/z = 409.0 [M+H]$^+$.

Step 2: Compound 13A (130 mg, 0.32 mmol), bis(pinacolato)diboron (163 mg, 0.64 mmol), [1,1'-bis(diphenylphosphino)ferrocene]palladium (II) dichloride (23 mg, 0.032 mmol) and potassium acetate (76 mg, 0.8 mmol) were dissolved in 1,4-dioxane (6 mL). The mixture was subjected to nitrogen replacement three times, heated to 80°C and reacted for 16 hours. The reaction solution was cooled and then directly concentrated. The resulting residue was added to a mixed solvent of petroleum ether and ethyl acetate (v : v=10 : 1, 20 mL). The mixture was stirred for 1 h and filtered. The filter cake was washed once with a mixed solvent of petroleum ether and ethyl acetate (v : v=10 : 1, 20 mL). The filter cake was collected and dried to obtain compound 13B (210 mg, crude), which was directly used in the next reaction.

LC-MS (ESI): m/z = 375.30 [M+H]$^+$.

Step 3: Compound 13B (210 mg, 0.46 mmol), compound 1F (191 mg, 0.46 mmol), [1,1'-bis(diphenylphosphino) ferrocene]palladium (II) dichloride (33 mg, 0.046 mmol) and sodium bicarbonate (77 mg, 0.92 mmol) were dissolved in a mixed solvent of 1,4-dioxane and water (v : v=5 : 1, 12 mL). The mixture was subjected to nitrogen replacement three times, heated to 80°C and reacted for 5 hours. The reaction solution was cooled and then directly concentrated to obtain compound 13C (400 mg, crude), which was directly used in the next reaction.

Step 4: Compound 13C (400 mg) was dissolved in ethanol (20 mL), and then hydrazine hydrate (0.1 mL) was added. The mixture was heated to 80°C and reacted for 1 hour. The reaction solution was cooled and then directly concentrated. The resulting residue was purified by preparative HPLC. Preparation method: 1. Instruments: waters 2767 (preparative liquid phase chromatographic instrument); chromatographic column: SunFire@PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% $NH_3 \cdot H_2O$); b. gradient elution, mobile phase A: 50%-90%; c. flow rate: 20 mL/min; d. elution time: 20 min. retention time: 4.91 min. Compound 13 (18 mg, yield: 11.48%) was obtained.

$^1$H NMR (400 MHz, DMSO-$d_6$) δ 12.79 (s, 1H), 8.42 (s, 1H), 8.35 (s, 1H), 8.03 (d, 1H), 7.91 (s, 1H), 4.21 (s, 1H), 3.80 (s, 3H), 3.73 (s, 2H), 0.91 (d, 2H), 0.80 (d, 2H);
LC-MS (ESI): m/z = 490.50 [M+H]$^+$.

**Example 14 and Example 15**

(2S)-2-(4-(2-(aminomethyl)-4-oxo-3,4-dihydroquinazolin-7-yl)-1-methyl-1H-pyrazol-5-yl)-4-chloro-3-fluoro-6-(1-methylcyclopropoxy)benzonitrile and (2R)-2-(4-(2-(aminomethyl)-4-oxo-3,4-dihydroquinazolin-7-yl)-1-methyl-1H-pyrazol-5-yl)-4-chloro-3-fluoro-6-(1-methylcyclopropoxy)benzonitrile (compound 14, compound 15)

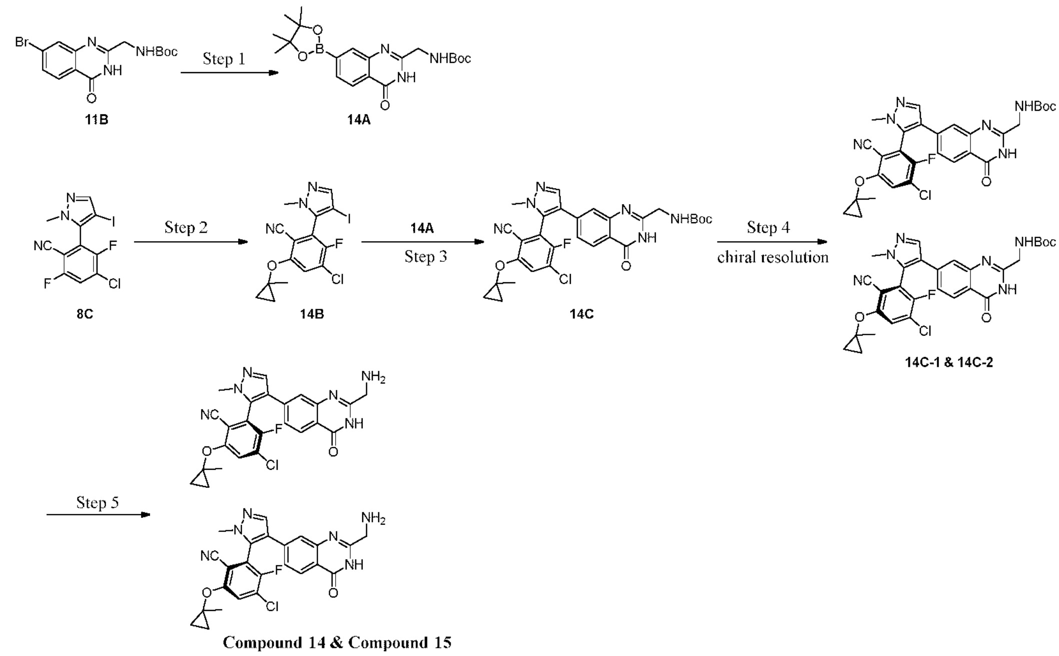

11B
14A
8C
14B
14C
14C-1 & 14C-2

Compound 14 & Compound 15

Step 1: Compound 11B (0.1 g, 0.28 mmol), bis(pinacolato)diboron (0.078 g, 0.31 mmol), [1,1'-bis(diphenylphosphino)ferrocene]palladium (II) dichloride (0.02 g, 0.028 mmol) and potassium acetate (0.082 g, 0.84 mmol) were dissolved in 1,4-dioxane (5 mL). The mixture was subjected to nitrogen replacement three times, heated to 80°C and reacted for 16 hours. The reaction solution was cooled and then directly concentrated. The resulting residue was added to a mixed solvent of petroleum ether and ethyl acetate (v : v=10 : 1, 20 mL). The mixture was stirred for 1 h and filtered. The filter cake was washed once with a mixed solvent of petroleum ether and ethyl acetate (v : v=10 : 1, 20 mL). The filter cake was collected and dried to obtain compound 14A (0.2 g, crude), which was directly used in the next reaction.

LC-MS (ESI): m/z = 402.2 $[M+H]^+$.

Step 2: 1-methylcyclopropanol (5.7 g, 79.05 mmol) was dissolved in tetrahydrofuran (100 mL), and sodium hydride (3.15 g, 79.05 mmol) was added in portions. The mixture was heated to 40°C and stirred for 2 h. The reaction solution was cooled to room temperature. Compound 8C (10 g, 26.35 mmol) was then added, and the mixture was reacted at room temperature for 8 h. The reaction was quenched by adding methanol and directly concentrated. The resulting residue was purified by silica gel column chromatography (ethyl acetate : petroleum ether (v : v)=1 : 5) to obtain compound 14B (2.5 g, yield: 21.98%).

LC-MS (ESI): m/z = 432.3 $[M+H]^+$.

Step 3: Compound 14B (0.5 g, 1.16 mmol), compound 14A (0.47 g, 1.16 mmol), [1,1'-bis(diphenylphosphino)ferrocene]palladium (II) dichloride (0.085 g, 0.12 mmol) and sodium bicarbonate (0.29 g, 3.48 mmol) were dissolved in a mixed solvent of 1,4-dioxane (10 mL) and water (2 mL). The mixture was subjected to nitrogen replacement 3 times, heated to 80°C and stirred for 2 hours. Water (10 mL) was added, and the mixture was extracted with ethyl acetate (10 mL × 3). The organic phase was collected, washed, dried and concentrated. The resulting residue was purified by silica gel column chromatography (dichloromethane : methanol (v : v)=100 : 8) to obtain compound 14C (0.3 g, yield: 44.67%).

LC-MS (ESI): m/z = 579.6 $[M+H]^+$.

Step 4: Compound 14C (100 mg) was resolved by chiral SFC to obtain P1 (retention time: 1.754 min, set as compound 14C-1) and P2 (retention time: 2.035 min, set as compound 14C-2). Resolution method: instrument: Waters 150 SFC; chromatographic column: Chiralcel OD-Column; mobile phase: A for $CO_2$ and B for MeOH (0.1% $NH_3$•$H_2O$); gradient: 35% phase B isocratic elution; flow rate: 100 mL/min; column pressure: 100 bar; column temperature: 25°C; absorption wavelength: 220 nm; cycle time: 2 min; sample preparation: the compound was dissolved in acetonitrile at a concentration of 2 mg/mL; injection: 2.5 mL/injection; treatment: after the separation, the mixture was concentrated by a

rotary evaporator at 35°C, and then the solvent was dried by a lyophilizer at -80°C to obtain compounds 14C-1 (24 mg, 23.51%) and 14C-2 (22 mg, 22.12%).

Step 5: Compound 14C-1 (20 mg, 0.035 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 14 (8 mg, yield: 47.73%).

Compound 14: $^{1}$H NMR (400 MHz, DMSO-$d_6$) δ 8.15 (s, 1H), 7.98 (d, 1H), 7.82 (d, 1H), 7.38 (d, 1H), 7.20 (dd, 1H), 3.76 (s, 3H), 3.71 (s, 2H), 1.58 (s, 3H), 1.05-1.02 (m, 2H), 0.90 (t, 2H); LC-MS (ESI): m/z = 479.4 $[M+H]^+$.

Compound 14C-2 (20 mg, 0.035 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 15 (8 mg, yield: 47.73%).

Compound 15: $^{1}$H NMR (400 MHz, DMSO-$d_6$) δ 8.16 (s, 1H), 7.98 (d, 1H), 7.82 (d, 1H), 7.37 (d, 1H), 7.20 (dd, 1H), 3.76 (s, 3H), 3.66 (s, 2H), 1.58 (s, 3H), 1.05-1.02 (m, 2H), 0.90 (t, 2H); LC-MS (ESI): m/z = 479.5 $[M+H]^+$.

**Example 16 and Example 17**

(2S)-2-(4-(2-(aminomethyl)-4-oxo-3,4-dihydroquinazolin-7-yl)-1-methyl-1H-pyrazol-5-yl)-4-chloro-3-fluoro-6-(3-methylazetidin-1-yl)benzonitrile and (2R)-2-(4-(2-(aminomethyl)-4-oxo-3,4-dihydroquinazolin-7-yl)-1-methyl-1H-pyrazol-5-yl)-4-chloro-3-fluoro-6-(3-methylazetidin-1-yl)benzonitrile (compound 16, compound 17)

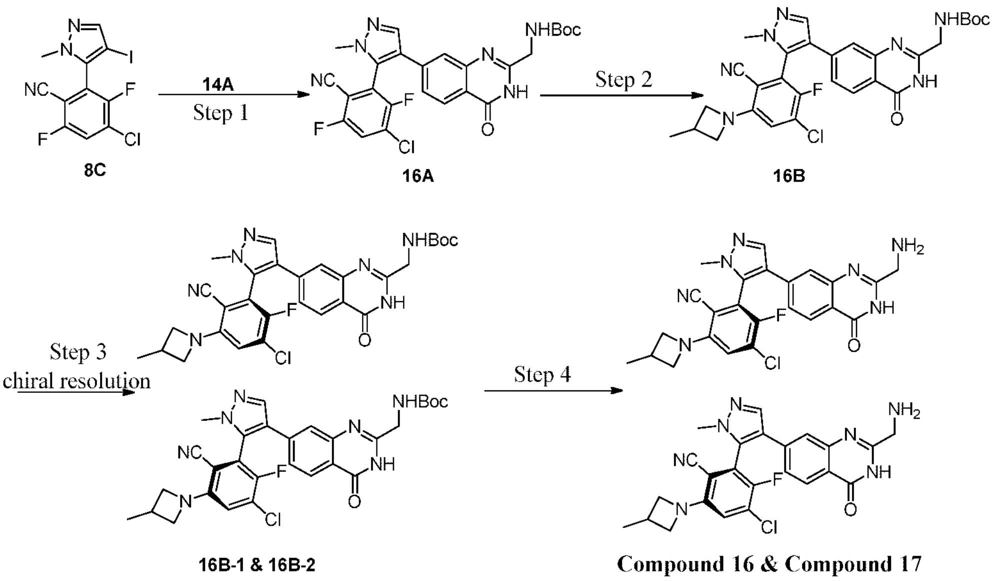


8C 16A 16B

16B-1 & 16B-2

Compound 16 & Compound 17

Step 1: Compound 8C (10 g, 26.35 mmol), 14A (12.7 g, 31.62 mmol), [1,1'-bis(diphenylphosphino)ferrocene] palladium (II) dichloride (1.93 g, 2.64 mmol) and potassium acetate (6.64 g, 79.05 mmol) were dissolved in 1,4-dioxane (100 mL) and water (20 mL). The mixture was subjected to nitrogen replacement three times, heated to 80°C and reacted for 4 hours. The reaction solution was cooled and then directly concentrated. The resulting residue was added to a mixed solvent of petroleum ether and ethyl acetate (v : v=10 : 1, 20 mL). The mixture was stirred for 1 h and filtered. The filter cake was collected and purified by column chromatography (methanol : dichloromethane (v : v)=1 : 20) to obtain compound 16A (9 g, yield: 64.82%).

LC-MS (ESI): m/z = 527.1 $[M+H]^+$.

Step 2: 3-(methoxymethyl)azetidine hydrochloride (0.31 g, 2.89 mmol), compound 16A (1 g, 1.90 mmol) and potassium carbonate (0.78 g, 5.63 mmol) were dissolved in N,N-dimethylformamide (20 mL), and the mixture was heated to 80°C and stirred for 2 h. The reaction solution was cooled to room temperature and directly concentrated. The resulting residue was purified by silica gel column chromatography (methanol : dichloromethane (v : v)=1 : 20) to obtain compound

16B (0.8 g, yield: 72.84%).

LC-MS (ESI): m/z = 578.6 $[M+H]^+$.

Step 3: Compound 16B (600 mg) was resolved by chiral SFC to obtain P1 (retention time: 2.025 min, set as compound 16B-1) and P2 (retention time: 2.288 min, set as compound 16B-2). Resolution method: instrument: Waters 150 SFC; chromatographic column: Chiralcel OJ-Column; mobile phase: A for $CO_2$ and B for MeOH (0.1% $NH_3 \cdot H_2O$); gradient: 45% phase B isocratic elution; flow rate: 120 mL/min; column pressure: 100 bar; column temperature: 25°C; absorption wavelength: 220 nm; cycle time: 3.5 min; sample preparation: the compound was dissolved in methanol at a concentration of 10 mg/mL; injection: 3 mL/injection; treatment: after the separation, the mixture was concentrated by a rotary evaporator at 35°C, and then the solvent was dried by a lyophilizer at -80°C to obtain compounds 16B-1 (80 mg, 13.33%) and 16B-2 (80 mg, 13.33%).

Step 5: 16B-1 (80 mg, 0.14 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 16 (40 mg, yield: 59.78%).

Compound 16: $^1$H NMR (400 MHz, DMSO-$d_6$) δ 8.14 (d, 1H), 7.99 (d, 1H), 7.38- 7.35 (m, 1H), 7.34 - 7.25 (m, 2H), 6.64-6.61 (m, 1H), 3.75 (s, 3H), 3.72 - 3.57 (m, 4H), 3.15 - 3.12 (m, 2H), 1.10-0.95 (m, 4H);
LC-MS (ESI): m/z = 514.5 $[M+HCl+H]^+$.

16B-2 (80 mg, 0.14 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 17 (41 mg, yield: 60.05%).

Compound 17: $^1$H NMR (400 MHz, DMSO-$d_6$) δ 8.14 (d, 1H), 7.99 (d, 1H), 7.37 - 7.33 (m, 1H), 7.33 - 7.27 (m, 2H), 6.64-6.61 (m, 1H), 3.75 (s, 3H), 3.72 - 3.57 (m, 4H), 3.18 - 3.09 (m, 2H), 1.10-0.95 (m, 4H);
LC-MS (ESI): m/z = 514.5 $[M+HCl+H]^+$.

**Example 18 and Example 19**

(2S)-2-(4-(2-(aminomethyl)-4-oxo-3,4-dihydroquinazolin-7-yl)-1-methyl-1H-pyrazol-5-yl)-6-azido-4-chloro-3-fluorobenzonitrile and (2R)-2-(4-(2-(aminomethyl)-4-oxo-3,4-dihydroquinazolin-7-yl)-1-methyl-1H-pyrazol-5-yl)-6-azido-4-chloro-3-fluorobenzonitrile (compound 18, compound 19)

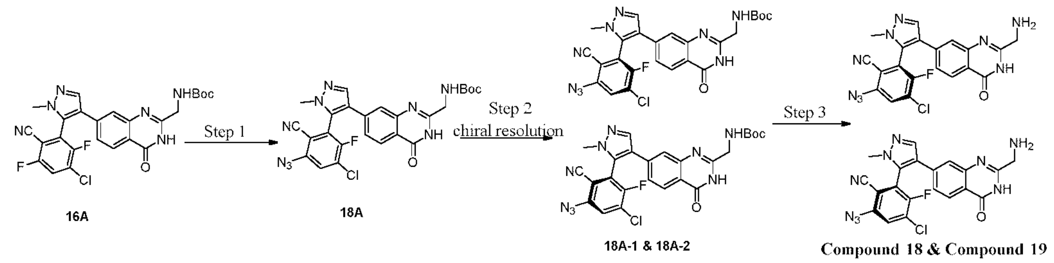


16A 18A 18A-1 & 18A-2 Compound 18 & Compound 19

Step 1: Compound 16A (0.5 g, 0.95 mmol) was dissolved in N,N-dimethylformamide (10 mL), and the mixture was cooled to-20°C. Sodium azide (0.068 g, 1.04 mmol) was then added, and the mixture was slowly warmed to room temperature and reacted for 5 hours. The reaction solution was directly concentrated. The resulting residue was purified by silica gel column chromatography (methanol : dichloromethane (v : v)=1 : 20) to obtain compound 18A (0.4 g, yield: 76.56%).

LC-MS (ESI): m/z = 550.1 $[M+H]^+$.

Step 2: Compound 18A (400 mg) was resolved by chiral SFC to obtain P1 (retention time: 1.156 min, set as compound 18A-1) and P2 (retention time: 1.479 min, set as compound 18A-2). Resolution method: instrument: Waters 150 SFC; chromatographic column: Chiralcel IC-Column; mobile phase: A for $CO_2$ and B for EtOH (0.1% $NH_3 \cdot H_2O$);

gradient: 45% phase B isocratic elution; flow rate: 70 mL/min; column pressure: 100 bar; column temperature: 25°C; absorption wavelength: 220 nm; cycle time: 9 min; sample preparation: the compound was dissolved in acetonitrile at a concentration of 2 mg/mL; injection: 2.5 mL/injection; treatment: after the separation, the mixture was concentrated by a rotary evaporator at 35°C, and then the solvent was dried by a lyophilizer at -80°C to obtain compounds 18A-1 (128 mg, 32.00%) and 18A-2 (126 mg, 31.5%).

Step 3: Compound 18A-1 (128 mg, 0.23 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 18 (50 mg, yield: 48.33%).

Compound 18: $^1H$ NMR (400 MHz, DMSO-$d_6$) $\delta$ 8.21 (d, 1H), 8.16 (s, 1H), 7.97 (d, 1H), 7.39 (d, 1H), 7.23 - 7.16 (m, 1H), 3.76 (s, 3H), 3.61 (s, 2H);
LC-MS (ESI): m/z = 450.5 $[M+H]^+$.

Compound 18A-2 (126 mg, 0.32 mmol) was dissolved in 4 M hydrogen chloride in dioxane (2 mL), and the mixture was reacted at 25°C for 2 hours. The reaction system was concentrated and then diluted by adding dichloromethane. The mixture was adjusted to a basic pH by adding sodium bicarbonate solution and extracted with dichloromethane. The liquid separation was conducted. The organic phase was collected, dried and concentrated. The resulting residue was purified by reverse phase column chromatography (water : acetonitrile (v/v)=67 : 33) to obtain compound 19 (50 mg, yield: 48.32%).

Compound 19: $^1H$ NMR (400 MHz, DMSO-$d_6$) $\delta$ 8.20 (d, 1H), 8.16 (s, 1H), 7.97 (d, 1H), 7.39 (d, 1H), 7.23 - 7.16 (m, 1H), 3.76 (s, 3H), 3.60 (s, 2H);
LC-MS (ESI): m/z = 450.5 $[M+H]^+$.

**Example 20**

2-(4-(4-(aminomethyl)-1-oxo-8-vinyl-1,2-dihydrophthalazin-6-yl)-1-methyl-1H-pyrazol-5-yl)-4-chloro-6-cyclopropoxy-3-fluorobenzonitrile (compound 20)

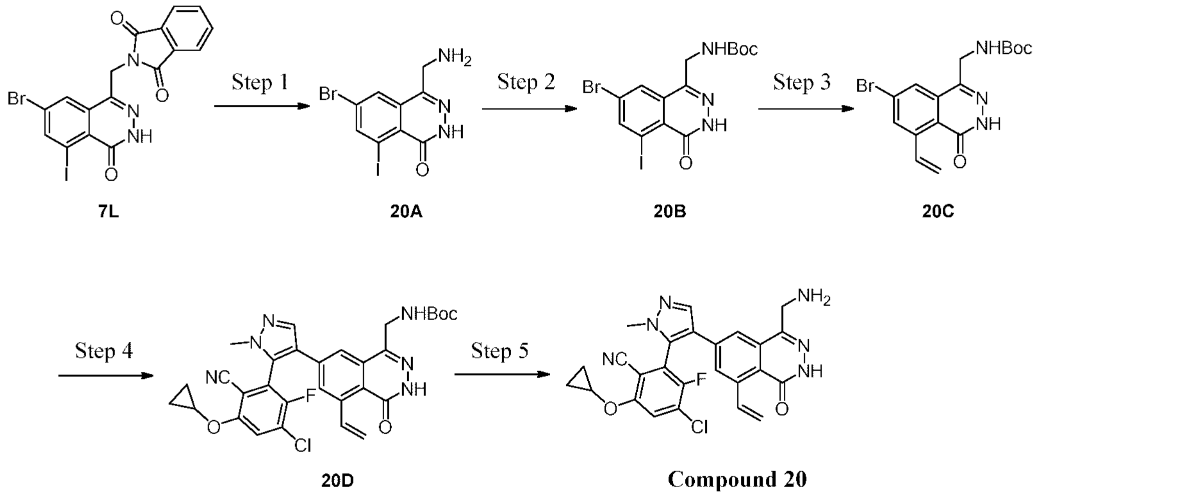

7L 20A 20B 20C
20D Compound 20

Step 1: Compound 7L (5.0 g, 9.8 mmol) was added to a reaction flask and mixed evenly with anhydrous ethanol (25 mL). Then, hydrazine hydrate (3 mL, 39.2 mmol) was added, and the mixture was reacted at 80°C for 1 hour. After the reaction was complete, the reaction solution was cooled to room temperature and filtered. The filter cake was washed once with anhydrous ethanol (25 mL), and then washed once with water (50 mL). The filter cake was collected and dried to obtain compound 20A (3.5 g, yield: 94.59%).

LC-MS (ESI): m/z = 382.0 $[M+H]^+$.

Step 2: Compound 20A (3.5 g, 9.2 mmol) and triethylamine (3.8 mL, 27.6 mmol) were added to a reaction flask

and dissolved in dichloromethane (60 mL). Di-tert-butyl dicarbonate (4.0 g, 18.4 mmol) was then added, and the mixture was reacted at room temperature for 3 hours. After the reaction was complete, the reaction solution was concentrated under reduced pressure. The resulting residue was purified by silica gel column chromatography (petroleum ether : ethyl acetate (v : v)=2 : 1-1 : 2) to obtain compound 20B (1.0 g, yield: 22.73%).

$^1$H NMR (400 MHz, DMSO-$d_6$) δ 12.64 (s, 1H), 8.56 (d, 1H), 8.23 (s, 1H), 7.40 (s, 1H), 4.35 (d, 2H), 1.40 (s, 9H);
LC-MS (ESI): m/z = 480.3 [M+H]$^+$.

Step 3: Compound 20B (400 mg, 0.83 mmol), bistriphenylphosphine palladium dichloride (57 mg, 0.083 mmol) and tetraethylammonium chloride (270 mg, 1.63 mmol) were added to a reaction flask, and dissolved in N,N-dimethylformamide (15 mL). The mixture was subjected to nitrogen replacement three times, and then tributylvinyltin (260 mg, 0.83 mmol) was added. The mixture was reacted at 110°C for 2 hours. The reaction solution was cooled and then poured into water (100 mL). The mixture was extracted with ethyl acetate (50 mL × 3). The organic phases were combined, washed once with saturated brine, dried over anhydrous sodium sulphate and filtered. The filtrate was concentrated. The residue was purified by silica gel column chromatography (petroleum ether : ethyl acetate (v : v)=2 : 1-0 : 1) to obtain compound 20C (200 mg, yield: 63.49%).

$^1$H NMR (400 MHz, DMSO-$d_6$) δ 12.57 (s, 1H), 8.15 (s, 1H), 8.13 - 8.07 (m, 1H), 8.06 (d, 1H), 7.36 - 7.30 (m, 1H), 5.83 (d, 1H), 5.45 (d, 1H), 4.38 (t, 2H), 1.40 (s, 9H);
LC-MS (ESI): m/z = 382.1 [M+H]$^+$.

Step 4: Compound 20C (100 mg, 0.26 mmol), compound 2B (109 mg, 0.26 mmol), chloro(2-dicyclohexylphosphino-2',4',6'-triisopropyl-1,1'-biphenyl)[2-(2'-amino-1,1'-biphenyl)]palladium (II) (21 mg, 0.026 mmol) and sodium bicarbonate (45 mg, 0.53 mmol) were added to a reaction flask, and then 1,4-dioxane and water (v : v=10 : 1, 10 mL) were added. The mixture was subjected to nitrogen replacement three times and reacted at 90°C for 2 hours. The reaction solution was cooled and then concentrated under reduced pressure. The resulting residue was purified by silica gel column chromatography (petroleum ether : ethyl acetate (v : v)=4 : 1-1 : 1) to obtain compound 20D (56 mg, yield: 36.51%).
LC-MS (ESI): m/z = 591.3 [M+H]$^+$.
Step 5: Compound 20D (56 mg, 0.095 mmol) was added to a reaction flask and dissolved in dichloromethane (2 mL). Trifluoroacetic acid (0.6 mL) was then added, and the mixture was reacted at room temperature for 30 min. The reaction solution was directly concentrated under reduced pressure to remove the organic solvent. The residue was purified by preparative HPLC. Method: 1. Instruments: waters 2767 (preparative liquid phase chromatographic instrument); chromatographic column: SunFire@PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% TFA); b. gradient elution, mobile phase A: 20%-60%; c. flow rate: 20 mL/min; d. elution time: 20 min; retention time: 3.45 min. Compound 20 (19 mg, yield: 33.04%) was obtained.

$^1$H NMR (400 MHz, DMSO-$d_6$) δ 12.81 (s, 1H), 8.43 (s, 1H), 8.14 - 8.04 (m,2H), 7.79 (s, 1H), 7.38 (s, 1H), 5.30 (d, 1H), 5.11 (d, 1H), 4.38 (d, 2H), 4.26 - 4.20 (m, 1H), 3.81 (s, 3H), 0.94 - 0.93 (m, 2H), 0.85 - 0.72 (m, 2H);
LC-MS (ESI): m/z = 491.5 [M+H]$^+$.

**Example 21**

2-(4-(4-(aminomethyl)-8-ethynyl-1-oxo-1,2-dihydrophthalazin-6-yl)-1-methyl-1H-pyrazol-5-yl)-4-chloro-6-cyclopropoxy-3-fluorobenzonitrile (compound 21)

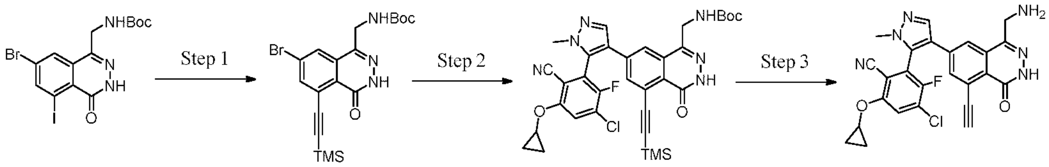


20B 21A 21B Compound 21

Step 1: Compound 20B (0.1 g, 0.21 mmol), trimethylethynylsilane (41 mg, 0.42 mmol), cuprous iodide (8 mg, 0.42

mmol) and triethylamine (42 mg, 0.42 mmol) were dissolved in N,N-dimethylformamide (5 mL). The mixture was bubbled with nitrogen for 1 minute, and then bistriphenylphosphine palladium dichloride (15 mg, 0.021 mmol) was added. The mixture was further bubbled with nitrogen for half a minute, stirred under an ice bath for 1 hour and filtered. The filtrate was diluted by adding water and extracted with ethyl acetate. The organic phase was washed successively with sodium bicarbonate solution and saturated brine, dried over anhydrous sodium sulphate and filtered. The filtrate was concentrated. The resulting residue was purified by silica gel column chromatography (petroleum ether : ethyl acetate (v : v)=3 : 2) to obtain compound 21A (30 mg, yield: 32%).

LCMS (ESI): m/z = 450.1 $[M+H]^+$.

Step 2: Compound 21A (80 mg, 0.18 mmol), compound 2B (110 mg, 0.27 mmol), sodium bicarbonate (30 mg, 0.36 mmol) and chloro(2-dicyclohexylphosphino-2',4',6'-triisopropyl-1,1'-biphenyl)[2-(2'-amino-1,1'-biphenyl)]palladium (II) (14 mg, 0.018 mmol) were dissolved in 1,4-dioxane solvent (5 mL) and water (0.5 mL). Under nitrogen protection, the mixture was heated to 90°C and stirred for 2 h. The reaction solution was cooled and then filtered. The filtrate was concentrated. The resulting residue was purified by silica gel column chromatography (petroleum ether : ethyl acetate (v/v) =1 : 0-1 : 1) to obtain compound 21B (40 mg, yield: 34%).

LCMS (ESI): m/z = 661.2 $[M+H]^+$.

Step 3: Compound 21B (40 mg, 0.06 mmol) was dissolved in dichloromethane (2 mL), and then trifluoroacetic acid (0.6 mL) was added. The mixture was stirred at room temperature for 1 hour. The reaction solution was directly concentrated to dryness. The crude solid was added to anhydrous methanol (3 mL), and then potassium carbonate (17 mg, 0.12 mmol) was added. The mixture was stirred at room temperature for 2 hours. The reaction solution was filtered. The filtrate was concentrated to dryness. The resulting residue was purified by silica gel column chromatography (dichloromethane : methanol (v/v)=1 : 0-9 : 1) to obtain compound 21 (8 mg, yield: 27%).

$^1H$ NMR (400 MHz, $CD_3OD$) δ 8.18 (s, 1H), 7.95 (d, 1H),7.72 (d, 1H), 7.68 (dd, 1H), 4.15-4.11 (m, 1H), 3.95 (s, 1H), 3.93 (d, 2H), 3.85 (s, 3H), 0.97-0.93 (m, 2H), 0.89-0.85 (m, 2H);
LCMS (ESI): m/z = 489.1 $[M+H]^+$.

**Example 22**

2-(4-(4-(aminomethyl)-8-(azetidin-1-yl)-1-oxo-1,2-dihydrophthalazin-6-yl)-1-methyl-1H-pyrazol-5-yl)-4-chloro-6-cyclopropoxy-3-fluorobenzonitrile (compound 22)

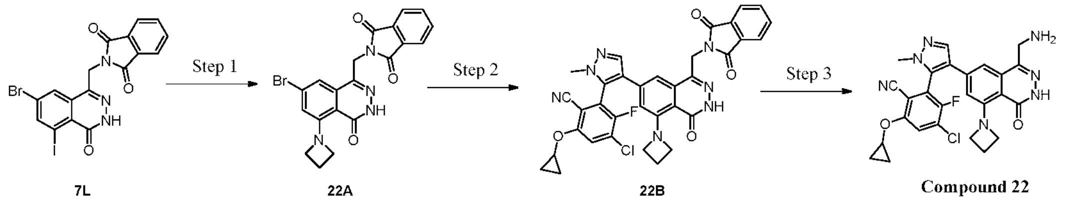


7L 22A 22B Compound 22

Step 1: Compound 7L (0.25 g, 0.49 mmol), anhydrous potassium phosphate (310 mg, 1.47 mmol), cuprous iodide (47 mg, 0.24 mmol) and azetidine (56 mg, 0.98 mmol) were dissolved in N,N-dimethylformamide (5 mL). The mixture was bubbled with nitrogen for 1 minute, heated to 90°C and stirred for 2 h. The reaction solution was cooled and then filtered. The filtrate was diluted by adding water and then extracted with ethyl acetate. The organic phase was collected, washed successively with sodium bicarbonate solution and saturated brine, dried over anhydrous sodium sulphate and filtered. The filtrate was concentrated. The resulting residue was purified by silica gel column chromatography (petroleum ether : ethyl acetate (v : v)=1 : 1) to obtain compound 22A (30 mg, yield: 14%).

LCMS (ESI): m/z = 439.0 $[M+H]^+$.

Step 2: Compound 22A (30 mg, 0.068 mmol), compound 2B (43 mg, 0.10 mmol), sodium bicarbonate (17 mg, 0.20 mmol) and [1,1'-bis(diphenylphosphino)ferrocene]palladium (II) dichloride (5 mg, 0.0068 mmol) were dissolved in 1,4-dioxane (3 mL) and water (0.3 mL). Under nitrogen protection, the mixture was heated to 90°C and stirred for 2 h. The reaction solution was cooled and then filtered. The filtrate was collected and concentrated. The resulting residue was purified by silica gel column chromatography (dichloromethane : methanol (v/v)=10 : 1) to obtain compound 22B (40 mg, yield: 94%).

LCMS (ESI): m/z = 650.2 $[M+H]^+$.

Step 3: Compound 22B (40 mg, 0.062 mmol) was dissolved in anhydrous methanol (3 mL), and then methylamine aqueous solution (0.3 mL) was added. The mixture was heated to 45°C and reacted for 3 hours. The

reaction solution was cooled and then directly concentrated to dryness. The resulting residue was purified by silica gel column chromatography (dichloromethane : methanol (v/v)=1 : 0-9 : 1) to obtain compound 22 (10 mg, yield: 31%).

$^1$H NMR (400 MHz, $CD_3OD$) δ 8.01 (s, 1H), 7.82 (d, 1H),6.92 (s, 1H), 6.27 (s, 1H), 4.02 -3.97 (m, 3H), 3.84 - 3.78 (m, 4H), 3.71 (s, 3H), 2.21 - 2.15 (m, 2H), 0.85 - 0.80 (m, 2H), 0.79 - 0.73 (m, 2H);
LCMS (ESI): m/z = 520.2 $[M+H]^+$.

**Examples 23 and 24**

(2S)-2-(4-(2-(aminomethyl)-4-oxo-3,4-dihydroquinazolin-7-yl)-1-methyl-1H-pyrazol-5-yl)-4-chloro-3-fluoro-6-vinylbenzonitrile and (2R)-2-(4-(2-(aminomethyl)-4-oxo-3,4-dihydroquinazolin-7-yl)-1-methyl-1H-pyrazol-5-yl)-4-chloro-3-fluoro-6-vinylbenzonitrile (compound 23, compound 24)

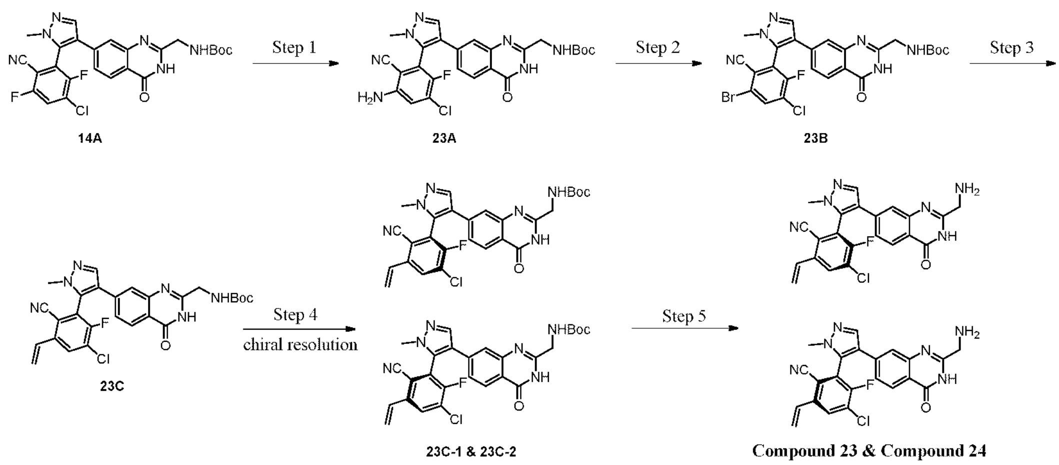


Step 1: Compound 14A (2.00 g, 3.80 mmol) was dissolved in tetrahydrofuran (10 mL). Aqueous ammonia (40%, 50 mL) was added. The mixture was heated to 100°C under a sealed condition and stirred for 6 hours. The reaction solution was cooled, and then ethyl acetate (200 mL) and water (200 mL) were added. The liquid separation was conducted. The organic phase was collected, washed with saturated brine (200 mL × 3), dried over anhydrous sodium sulphate and concentrated to obtain compound 23A (1.86 g, crude), which was directly used in the next reaction.
LC-MS (ESI): m/z = 525.2 $[M+H]^+$.
Step 2: Compound 23A (1.86 g, 3.55 mmol) was dissolved in acetonitrile (50 mL). Copper bromide (0.87 g, 3.91 mmol) was added. Under nitrogen protection, the mixture was cooled to -15°C to -10°C, and tert-butyl nitrite (0.50 g, 4.85 mmol) was slowly added. The mixture was naturally warmed to 30°C and stirred for 1 h. Ethyl acetate (200 mL) and water (200 mL) were added. The liquid separation was conducted. The organic phase was washed with saturated brine (200 mL × 3), dried over anhydrous sodium sulphate and concentrated to obtain compound 23B (2.00 g, crude), which was directly used in the next reaction.
LC-MS (ESI): m/z = 587.2 $[M+H]^+$.
Step 3: Compound 23B (2.00 g, 3.40 mmol), potassium vinyl trifluoroborate (0.68 g, 5.10 mmol) and potassium carbonate (2.82 g, 20.40 mmol) were added to a mixed solution of 1,4-dioxane (100 mL) and water (20 mL). The mixture was subjected to nitrogen replacement, and then [1,1'-bis(diphenylphosphino)ferrocene]palladium (II) dichloride (0.12 g, 0.17 mmol) was added. The mixture was further subjected to nitrogen replacement, warmed to 90°C and reacted for 2 hours. The reaction solution was cooled and then filtered. Ethyl acetate (200 mL) and water (200 mL) were added to the filtrate. The liquid separation was conducted. The organic phase was washed with saturated brine (200 mL × 3), dried over anhydrous sodium sulphate and concentrated to obtain compound 23C (1.50 g, crude), which was directly used in the next reaction.
LC-MS (ESI): m/z = 535.1 $[M+H]^+$.
Step 4: Compound 23C (1.50 g) was resolved by chiral SFC to obtain P1 (retention time: 1.927 min, set as compound 23C-1) and P2 (retention time: 2.093 min, set as compound 23C-2). Resolution method: instrument: Waters 150 SFC; chromatographic column: Chiralcel OD-Column; mobile phase: A for CO2 and B for MeOH (0.1% $NH_3 \cdot H_2O$); gradient: 35% phase B isocratic elution; flow rate: 100 mL/min; column pressure: 100 bar; column temperature: 25°C; absorption wavelength: 220 nm; cycle time: 5 min; sample preparation: the compound was dissolved in methanol at a

concentration of 10 mg/mL; injection: 3.5 mL/injection; treatment: after the separation, the mixture was concentrated by a rotary evaporator at 35°C, and then the solvent was dried by a lyophilizer at -80°C to obtain compound 23C-1 (211 mg, 14.1%) and compound 23C-2 (121 mg, 8.1%).

Step 5: Compound 23C-1 (211 mg) was dissolved in 20% trifluoroacetic acid in dichloromethane (5 mL), and the mixture was stirred at room temperature for 2 hours. The reaction solution was directly concentrated to dryness and adjusted to a basic pH by adding methanol solution (15 mL) and solid sodium bicarbonate. The reaction solution was directly separated by reverse phase column chromatography (acetonitrile : water (v/v)=5%-50%) to obtain compound 23 (123 mg, yield: 76.5%).

Compound 23: $^1$H NMR (400 MHz, $CDCl_3$) δ 8.14 (d, 1H), 7.90 (s, 1H), 7.85 (d, 1H), 7.46 (s, 1H), 7.15 (d, 1H), 6.98-6.88 (m, 1H), 5.94 (d, 1H), 5.63 (d, 1H), 3.93(s, 2H), 3.82 (s, 3H); LC-MS (ESI): m/z = 435.5 $[M+H]^+$.

Compound 23C-2 (121 mg) was dissolved in 20% trifluoroacetic acid in dichloromethane (5 mL), and the mixture was stirred at room temperature for 2 hours. The reaction solution was directly concentrated to dryness and adjusted to a basic pH by adding methanol solution (15 mL) and solid sodium bicarbonate. The reaction solution was directly separated by reverse phase column chromatography (acetonitrile : water (v/v)=5%-50%) to obtain compound 24 (19 mg, yield: 19.9%).

Compound 24: $^1$H NMR (400 MHz, $CDCl_3$) δ 8.14 (d, 1H), 7.91 (s, 1H), 7.85 (d, 1H), 7.46 (s, 1H), 7.15 (d, 1H), 6.96-6.92 (m, 1H), 5.94 (d, 1H), 5.63 (d, 1H), 3.94(s, 2H), 3.83 (s, 3H); LC-MS (ESI): m/z = 435.5 $[M+H]^+$.

### Examples 25 and 26

(2S)-2-(4-(2-(aminomethyl)-4-oxo-3,4-dihydroquinazolin-7-yl)-1-methyl-1H-pyrazol-5-yl)-4-chloro-6-(3,3-difluoroazetidin-1-yl)-3-fluorobenzonitrile and (2R)-2-(4-(2-(aminomethyl)-4-oxo-3,4-dihydroquinazolin-7-yl)-1-methyl-1H-pyrazol-5-yl)-4-chloro-6-(3,3-difluoroazetidin-1-yl)-3-fluorobenzonitrile (compound 25, compound 26)

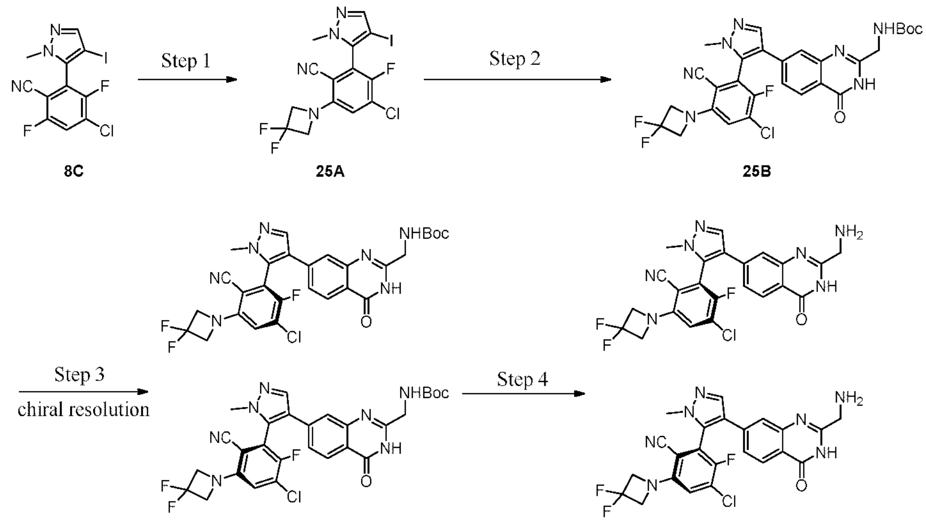


8C 25A 25B

25B-1 & 25B-2 Compound 25 & Compound 26

Step 1: Compound 8C (3.8 g, 10.01 mmol), 3,3-difluoroazetidine hydrochloride (1.56 g, 12 mmol) and potassium carbonate (2.77 g, 20 mmol) were dissolved in dimethyl sulfoxide (40 mL). At 80°C, the mixture was stirred and reacted for 4 hours. The reaction solution was cooled and then extracted by adding ethyl acetate (100 mL) and water (100 mL). The organic phase was collected and concentrated to dryness. The residue was separated by silica gel column chromatography (ethyl acetate : petroleum ether (v : v)=0-25%) to obtain compound 25A (2.5 g, yield: 55.2%).

LC-MS (ESI): m/z= 453.2 $[M+H]^+$.

Step 2: Compound 14A (0.64 g, 1.77 mmol), compound 25A (800 mg, 1.77 mmol) and sodium bicarbonate (0.45 g, 5.31 mmol) were added to a mixed solution of 1,4-dioxane (90 mL) and water (30 mL). The mixture was subjected to nitrogen replacement 3 times, and then [1,1'-bis(diphenylphosphino)ferrocene]palladium (II) dichloride (0.13 g, 0.18 mmol) was added. The resulting mixture was further subjected to nitrogen replacement, warmed to 60°C and reacted for 2 hours. The reaction solution was cooled and then directly filtered. Ethyl acetate (300 mL) and water (300 mL) were added to the filtrate. The liquid separation was conducted. The organic phase was washed with saturated brine (300 mL × 3), dried over anhydrous sodium sulphate and concentrated to dryness to obtain compound 25B (389 mg, crude), which was

directly used in the next reaction.

LC-MS (ESI): m/z = 600.2 $[M+H]^+$.

Step 3: Compound 25B (380 mg) was resolved by chiral SFC to obtain P1 (retention time: 1.284 min, set as compound 25B-1) and P2 (retention time: 1.335 min, set as compound 25B-2). Resolution method: instrument: Waters 150 SFC; chromatographic column: Chiralpak AS Column; mobile phase: A for CO2 and B for EtOH (0.1% $NH_3 \cdot H_2O$); gradient: 15% phase B isocratic elution; flow rate: 100 mL/min; column pressure: 100 bar; column temperature: 25°C; absorption wavelength: 220 nm; cycle time: 2.5 min; sample preparation: the compound was dissolved in ethanol at a concentration of 8.0 mg/mL; injection: 2 mL/injection; treatment: after the separation, the mixture was concentrated by a rotary evaporator at 35°C, and then the solvent was dried by a lyophilizer at -80°C to obtain compound 25B-1 (49 mg, 12.9%) and compound 25B-2 (41 mg, 10.8%).

Step 4: Compound 25B-1 (49 mg) was dissolved in 20% trifluoroacetic acid in dichloromethane (3 mL), and the mixture was stirred at room temperature for 2 hours. The reaction solution was directly concentrated to dryness and adjusted to a basic pH by adding methanol solution (15 mL) and solid sodium bicarbonate. The reaction solution was directly separated by reverse phase column chromatography (acetonitrile : water (v/v)=5%-50%) to obtain compound 25 (11 mg, yield: 26.9%).

Compound 25: $^1H$ NMR (400 MHz, DMSO-$d_6$) δ 8.16 (s, 1H), 7.99 (d, 1H), 7.40 (d, 1H), 7.32 - 7.23 (m, 2H), 4.77-4.55 (m, 4H), 3.76 (s, 3H), 3.60 (s, 2H); LC-MS (ESI): m/z = 500.5 $[M+H]^+$.

Compound 25B-2 (41 mg) was dissolved in 20% trifluoroacetic acid in dichloromethane (3 mL), and the mixture was stirred at room temperature for 2 hours. The reaction solution was directly concentrated to dryness and adjusted to a basic pH by adding methanol solution (15 mL) and solid sodium bicarbonate. The reaction solution was directly separated by reverse phase column chromatography (acetonitrile : water (v/v)=5%-50%) to obtain compound 26 (13 mg, yield: 19.9%).

Compound 26: $^1H$ NMR (400 MHz, DMSO-$d_6$) δ 8.16 (s, 1H), 7.99 (d, 1H), 7.40 (d, 1H), 7.32 - 7.25 (m, 2H), 4.71-4.55 (m, 5H), 3.76 (s, 4H), 3.60 (s, 2H); LC-MS (ESI): m/z = 500.5 $[M+H]^+$.

**Example 27**

6-(5-(3-chloro-6-cyano-5-cyclopropoxy-2-fluorophenyl)-1-methyl-1H-pyrazol-4-yl)quinoline-3-carboxamide (compound 27)

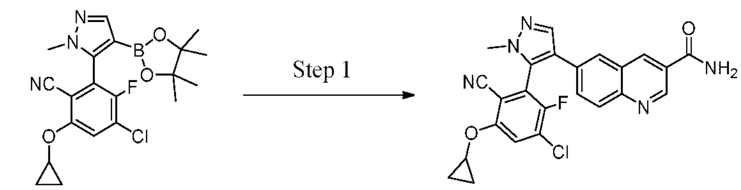


2B

Compound 27

Step 1: 6-bromoquinoline-3-carboxamide (250.0 mg, 1.0 mmol), compound 2B (583.0 mg, 1.4 mmol), [1,1'-bis(diphenylphosphino)ferrocene]palladium (II) dichloride (73.0 mg, 0.1 mmol) and sodium bicarbonate (385.2 mg, 4.0 mmol) were dissolved in 1,4-dioxane (10.0 mL) and water (3.0 mL), and the mixture was subjected to nitrogen replacement three times, heated to 80°C and reacted for 2 h. The reaction solution was cooled to room temperature and filtered through a funnel filled with silica gel. The filter cake was rinsed with ethyl acetate (50.0 mL). The filtrate was collected and concentrated. The resulting residue was purified by silica gel column chromatography (petroleum ether : ethyl acetate (v : v)=1 : 1) to obtain compound 27 (690.0 mg, yield: 84%).

$^1H$ NMR (400 MHz, DMSO-$d_6$) δ 9.23 (d, 1H), 8.70 (d, 1H), 8.27 (s, 1H), 8.15 (s, 1H), 8.07 - 7.94 (m, 2H), 7.81 (d, 1H), 7.75 - 7.59 (m, 2H), 4.26 - 4.11 (m, 1H), 3.78 (s, 3H), 0.91 - 0.89 (m, 2H), 0.85 - 0.74 (m, 2H);
LC-MS (ESI): m/z = 462.1 $[M+H]^+$.

**Example 28**

2-(4-(2-(aminomethyl)-4-oxo-3,4-dihydropyrido[2,3-d]pyrimidin-7-yl)-1-methyl-1H-pyrazol-5-yl)-4-chloro-3-fluoro-6-(1-methylcyclopropoxy)benzonitrile (compound 28)

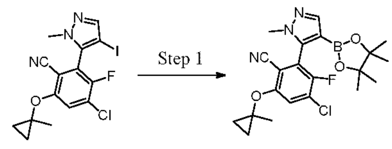


14B 28A

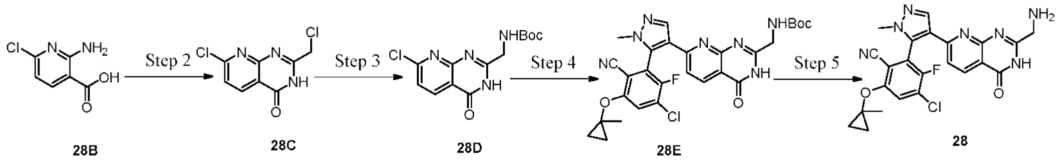


28B 28C 28D 28E 28

Step 1: Compound 14B (2 g, 4.63 mmol), bis(pinacolato)diboron (2.96 g, 23.17 mmol), triethylamine (2.81 g, 27.77 mmol) and tetrakis(triphenylphosphino)palladium (0.54 g, 0.46 mmol) were dissolved in 1,4-dioxane (25 mL). The mixture was subjected to nitrogen replacement 3 times. Under nitrogen atmosphere, the mixture was heated to 90°C and stirred for 12 hours. The reaction solution was cooled, diluted by adding water and extracted 3 times with ethyl acetate. The organic phase was collected, dried and concentrated. The resulting residue was purified by silica gel column chromatography (ethyl acetate : petroleum ether (v : v)=10-13%) to obtain compound 28A (1.25 g, yield: 63%).

LC-MS (ESI): m/z = 432.20 $[M+H]^+$.

Step 2: Sodium metal (0.27 g, 11.7 mmol) was added in portions to methanol (50 mL) and completely dissolved. 2-chloroacetonitrile (13.1 g, 173.5 mmol) was added. At room temperature, the mixture was stirred for 40 minutes, and then 2-amino-6-chloropyridine-3-carboxylic acid (compound 28B) (10 g, 57.9 mmol) was dissolved in methanol (50 mL) and added to the above-mentioned reaction system. The system was reacted at room temperature for 2 hours. The reaction solution was directly concentrated. The resulting residue was purified by silica gel column chromatography (methanol : dichloromethane (v : v)=3%) to obtain compound 28C (3.6 g, yield: 27%).

LC-MS (ESI): m/z = 230.1 $[M+H]^+$.

Step 3: Compound 28C (2.2 g, 9.56 mmol) was dissolved in aqueous ammonia (20 mL), and the mixture was stirred at room temperature for 2 hours until dissolved. The reaction solution was directly concentrated, and the resulting crude was dissolved in methanol (15 mL). Triethylamine (3.87 g, 38.24 mmol) and di-tert-butyl dicarbonate (3.13 g, 14.3 mmol) were added, and the mixture was further stirred at room temperature for 3 hours. The reaction solution was directly concentrated. The resulting residue was purified by silica gel column chromatography (methanol : dichloromethane (v : v) =3-6%) to obtain compound 28D (2.8 g, yield: 94%).

LC-MS (ESI): m/z = 311.3 $[M+H]^+$.

Step 4: Compound 28A (0.4 g, 0.93 mmol), compound 28D (0.40 g, 1.30 mmol), potassium carbonate (0.32 g, 2.33 mmol) and [1,1-bis(diphenylphosphino)ferrocene]palladium (II) dichloride (0.068 g, 0.093 mmol) were dissolved in 1,4-dioxane (10 mL) and water (2.5 mL). The mixture was subjected to nitrogen replacement 3 times. Under nitrogen atmosphere, the mixture was heated to 100°C and stirred for 2 hours. The reaction solution was cooled, diluted by adding water and extracted 3 times with ethyl acetate. The organic phase was collected, dried and concentrated. The resulting residue was purified by silica gel column chromatography (methanol : dichloromethane (v : v)=10-12%) to obtain compound 28E (0.52 g, yield: 96%).

LC-MS (ESI): m/z = 580.2 $[M+H]^+$.

Step 5: Compound 28E (0.15 g, 0.26 mmol) was dissolved in dichloromethane (4 mL), and then trifluoroacetic acid (1 mL) was added. The mixture was stirred at room temperature for 2 hours. The reaction solution was directly concentrated. The resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate); b. gradient elution, mobile phase A: 30%-80%; c. flow rate: 15 mL/min; d. elution time: 40 min; retention time: 30 min. Compound 28 (14 mg, yield: 11%) was obtained.

$^1$H NMR (400 MHz, DMSO-$d_6$) δ 8.43 (s, 1H), 8.36 (d, 1H), 7.78 (d, 1H), 7.67 (d, 1H), 3.78 (s, 3H), 3.58 (s, 2H), 1.62 (s, 3H), 1.03 (dd, 2H), 0.92 (s, 2H).

LC-MS (ESI): m/z = 480.1 $[M+H]^+$.

**Example 29 and Example 30**

(2S)-2-(4-(4-(aminomethyl)-1-oxo-8-vinyl-1,2-dihydrophthalazin-6-yl)-1-methyl-1H-pyrazol-5-yl)-4-chloro-3-fluoro-6-(1-methylcyclopropoxy)benzonitrile and (2R)-2-(4-(4-(aminomethyl)-1-oxo-8-vinyl-1,2-dihydrophthalazin-6-yl)-1-methyl-1H-pyrazol-5-yl)-4-chloro-3-fluoro-6-(1-methylcyclopropoxy)benzonitrile (compound 29, compound 30)

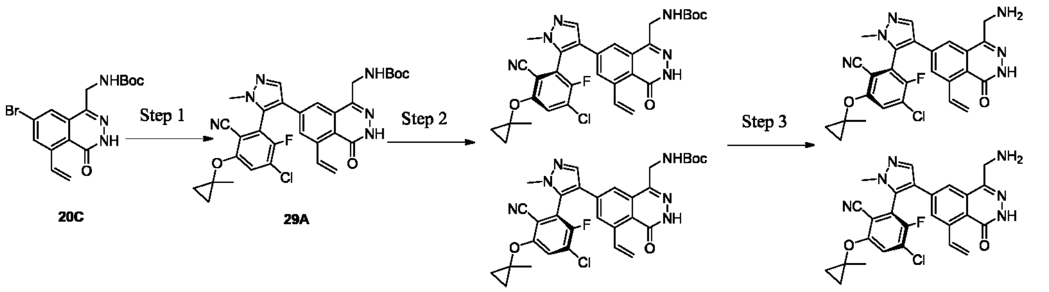


20C

29A

29A-1 & 29A-2

Compound 29 & Compound 30

Step 1: Compound 20C (1.0 g, 2.63 mmol), compound 28A (1.13 g, 2.63 mmol), [1,1'-bis(diphenylphosphino) ferrocene]palladium (II) dichloride (190 mg, 0.26 mmol) and potassium carbonate (1.09 g, 7.89 mmol) were dissolved in a mixed solvent of 1,4-dioxane and water (v : v=6 : 1, 70 mL). The mixture was subjected to nitrogen replacement three times and reacted at 85°C for 6 hours. The reaction solution was cooled and then directly concentrated under reduced pressure. The resulting residue was purified by silica gel column chromatography (petroleum ether : ethyl acetate (v : v)=4 : 1-1 : 1) to obtain compound 29A (690 mg, yield: 43.40%).

LC-MS (ESI): m/z = 605.7 $[M+H]^+$.

Step 2: Compound 29A (0.69 g, 1.14 mmol) was resolved by SFC to obtain P1 (retention time: 1.142 min, set as compound 29A-1) and P2 (retention time: 1.195 min, set as compound 29A-2). Method: instrument: Waters 150 SFC, chromatographic column: Chiralcel AS Column (250 × 30 mm × 10 μm), mobile phase: A for $CO_2$, B for 0.1% $NH_3$•$H_2O$ in MeOH; gradient: B 15 %; flow rate: 100 mL /min; column pressure: 100 bar; column temperature: 25°C; absorption wavelength: 220 nm; cycle time: 6.3 min; sample preparation: the compound was dissolved in acetonitrile at a concentration of 10 mg/mL; injection: 3 mL/injection; treatment: after the separation, the mixture was concentrated by a rotary evaporator at 35°C, and then the solvent was dried by a lyophilizer at -80°C to obtain compounds 29A-1 (92.8 mg, 13.45%) and 29A-2 (66.9 mg, 9.96%).

Compound 29A-1: LC-MS (ESI): m/z = 605.7 $[M+H]^+$.

Compound 29A-2: LC-MS (ESI): m/z = 605.7 $[M+H]^+$.

Step 3: Compound 29A-1 (92.8 mg, 0.15 mmol) was dissolved in dichloromethane (8 mL), and then trifluoroacetic acid (1.6 mL) was added. The mixture was reacted at room temperature for 2 hours. The reaction system was concentrated. The residue was dissolved in dichloromethane (50 mL). The mixture was adjusted to a basic pH by adding saturated sodium bicarbonate solution (50 mL) and allowed to stand for liquid separation. The dichloromethane phase was separated, and the aqueous phase was extracted with dichloromethane (50 mL × 3). The organic phases were combined, dried over anhydrous sodium sulphate and filtered. The filtrate was collected and concentrated. The resulting residue was purified by silica gel column chromatography (dichloromethane : methanol (v : v)=20 : 1) to obtain compound 29 (75 mg, yield: 96.87%).

Compound 29: $^1H$ NMR (400 MHz, DMSO-$d_6$) δ 12.36 (s, 1H), 8.39 (s, 1H), 8.13 (dd, 1H), 7.85 (d, 1H), 7.71 (s, 1H), 7.61 (s, 1H), 5.38 (d, 1H), 5.31 (d, 1H), 3.82 - 3.77 (m, 5H), 1.59 (s, 3H), 1.05 - 1.00 (m, 2H), 0.95 - 0.90 (m, 2H); LC-MS (ESI): m/z = 505.9 $[M+H]^+$.

Compound 29A-2 (66.9 mg, 0.11 mmol) was dissolved in dichloromethane (6 mL), and then trifluoroacetic acid (1.2 mL) was added. The mixture was reacted at room temperature for 2 hours. The reaction system was concentrated. The residue was dissolved in dichloromethane (50 mL). The mixture was adjusted to a basic pH by adding saturated sodium bicarbonate solution (50 mL) and allowed to stand for liquid separation. The dichloromethane phase was separated, and the aqueous phase was extracted with dichloromethane (50 mL × 3). The organic phases were combined, dried over anhydrous sodium sulphate and filtered. The filtrate was collected and concentrated. The resulting residue was purified by silica gel column chromatography (dichloromethane : methanol (v : v)=20 : 1) to obtain compound 30 (43 mg, yield: 77.25%).

Compound 30: $^1H$ NMR (400 MHz, DMSO-$d_6$) δ 12.35 (s, 1H), 8.39 (s, 1H), 8.13 (dd, 1H), 7.85 (d, 1H), 7.71 (s, 1H), 7.61 (s, 1H), 5.38 (d, 1H), 5.31 (d, 1H), 3.81 - 3.77 (m, 5H), 1.59 (s, 3H), 0.94 - 0.91 (m, , 2H), 0.87 - 0.83 (m, 2H);

LC-MS (ESI): m/z = 505.9 $[M+H]^+$.

**Example 31 and Example 32**

(2S)-2-(4-(2-(aminomethyl)-4-oxo-3,4-dihydropyrido[2,3-d]pyrimidin-7-yl)-1-methyl-1H-pyrazol-5-yl)-4-chloro-3-fluoro-6-(1-methylcyclopropoxy)benzonitrile and (2R)-2-(4-(2-(aminomethyl)-4-oxo-3,4-dihydropyrido[2,3-d]pyrimidin-7-yl)-1-methyl-1H-pyrazol-5-yl)-4-chloro-3-fluoro-6-(1-methylcyclopropoxy)benzonitrile (compound 31, compound 32)

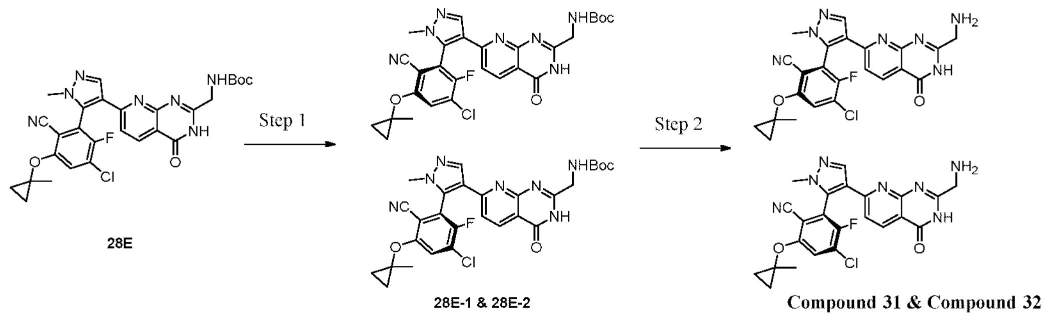


28E

28E-1 & 28E-2

Compound 31 & Compound 32

Step 1: Compound 28E (1.6 g, 2.76 mmol) was resolved by chiral SFC to obtain P1 (retention time: 2.590 min, set as compound 28E-1) and P2 (retention time: 3.949 min, set as compound 28E-2). Resolution method: instrument: MG II preparative SFC (SFC-14); chromatographic column: ChiralPak IE, 250 × 30 mm I.D., 10 μm; mobile phase: A for $CO_2$ and B for EtOH; gradient: 30% phase B; flow rate: 80 mL/min; column pressure: 100 bar; column temperature: 38°C; absorption wavelength: 220 nm; cycle time: 2.5 min; sample preparation: compound was dissolved in ethanol; injection: 2 mL/injection; treatment: after the separation, the mixture was concentrated by a rotary evaporator at 35°C, and then the solvent was dried by a lyophilizer at -80°C to obtain compound 28E-1 (0.45 g, 28%) and compound 28E-2 (0.53 g, 33%).

Step 2: Compound 28E-1 (0.45 g, 0.78 mmol) was dissolved in dichloromethane (8 mL), and then trifluoroacetic acid (2 mL) was added. The mixture was stirred at room temperature for 2 hours. The reaction solution was directly concentrated. The resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.5% $NH_3.H_2O$); b. gradient elution, mobile phase A: 5%-60%; c. flow rate: 20 mL/min; d. elution time: 15 min; retention time: 10.0 min. Compound 31 (19 mg, yield: 5%) was obtained.

Compound 31: $^1H$ NMR (400 MHz, DMSO-$d_6$) δ 8.41 (s, 1H), 8.36 (d,1H), 7.78 (d,1H), 7.64 (d,1H), 3.78 (s, 3H), 3.64 (s, 2H), 1.62 (s, 3H), 1.11-1.00 (m, 2H), 0.98-0.89 (m, 2H);
LC-MS (ESI): m/z = 480.5 $[M+H]^+$.

Compound 28E-2 (0.53 g, 0.91 mmol) was dissolved in dichloromethane (8 mL), and then trifluoroacetic acid (2 mL) was added. The mixture was stirred at room temperature for 2 hours. The reaction solution was directly concentrated. The resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.5% $NH_3.H_2O$); b. gradient elution, mobile phase A: 5%-60%; c. flow rate: 20 mL/min; d. elution time: 15 min; retention time: 10.0 min. Compound 32 (51 mg, yield: 11%) was obtained.

Compound 32: $^1H$ NMR (400 MHz, DMSO-$d_6$) δ 8.42 (s, 1H), 8.36 (d, 1H), 7.78 (d, 1H), 7.65 (d, 1H), 3.78 (s, 3H), 3.63 (s, 2H), 1.62 (s, 3H), 1.11-1.01 (m, 2H), 0.96-0.90 (m, 2H);
LC-MS (ESI): m/z = 480.5 $[M+H]^+$.

**Example 33**

2-(4-(4-(aminomethyl)-8-vinyl-1-oxo-1,2-dihydrophthalazin-6-yl)-1-methyl-1H-pyrazol-5-yl)-4-chloro-3-fluoro-6-(1-fluorocyclobutyl)benzonitrile (compound 33)

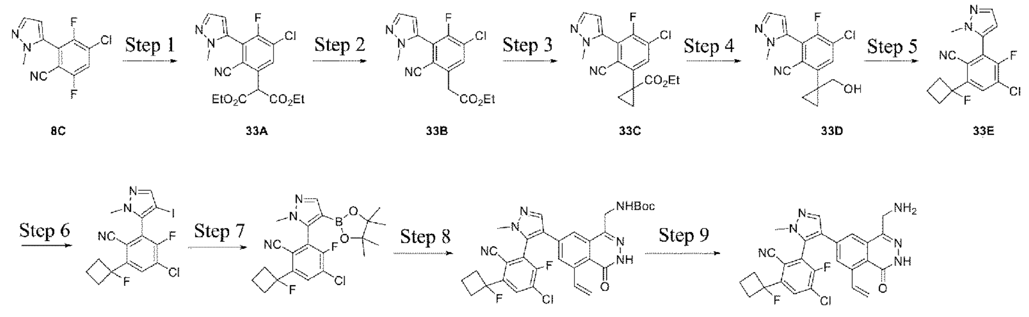


8C 33A 33B 33C 33D 33E

33F 33G 33H Compound 33

Step 1: Compound 8C (7 g, 27.6 mmol) was weighed into a flask. N,N-dimethylformamide (70 mL) and diethyl malonate (8.84 g, 55.20 mmol) were added, and then sodium hydride (3.31 g, 82.81 mmol) was slowly added. The mixture was heated to 80°C and reacted for 15 hours. The reaction solution was extracted with ethyl acetate. The organic phase was collected, washed with saturated brine, dried over anhydrous sodium sulphate and filtered. The filtrate was collected and concentrated. The resulting residue was purified by column chromatography (petroleum ether:ethyl acetate (v/v)=10 : 1) to obtain compound 33A (4.1 g, yield: 37.7%).

LC-MS (ESI): m/z = 394.2 $[M+H]^+$.

Step 2: Compound 33A (4.1 g, 10.43 mmol) was dissolved in dimethyl sulfoxide (40 mL). Lithium chloride (1.33 g, 31.28 mmol) was added, and the mixture was heated to 100°C and reacted for 15 hours. The reaction solution was extracted with ethyl acetate. The organic phase was collected, washed with saturated brine, dried over anhydrous sodium sulphate and filtered. The filtrate was collected and concentrated. The resulting residue was purified by column chromatography (petroleum ether : ethyl acetate (v/v)=5 : 1) to obtain compound 33B (2 g, yield: 59.6%).

LC-MS (ESI): m/z = 322.3 $[M+H]^+$.

Step 3: Compound 33B (1.7 g, 5.28 mmol) was dissolved in dimethyl sulfoxide (10 mL). Diphenyl(vinyl)sulfonium trifluoromethanesulfonate (2.3 g, 6.34 mmol) was added, and the mixture was reacted at 25°C for 5 minutes. Then, 1,8-diazabicyclo[5.4.0]undec-7-ene (2.4 g, 15.84 mmol) was added, and the mixture was reacted at 25°C for 2 hours. The reaction solution was extracted with ethyl acetate. The organic phase was collected, washed with saturated brine, dried over anhydrous sodium sulphate and filtered. The filtrate was collected and concentrated. The resulting residue was purified by column chromatography (petroleum ether : ethyl acetate (v/v)=5 : 1) to obtain compound 33C (1.6 g, yield: 87%).

LC-MS (ESI): m/z = 348.2 $[M+H]^+$.

Step 4: Compound 33C (1.1 g, 3.16 mmol) was weighed into a flask. Tetrahydrofuran (10 mL) and lithium borohydride (137.8 mg, 6.33 mmol) were added, and the mixture was stirred at 25°C. Then, water (1 mL) was slowly added. The system was reacted at 25°C for 15 hours. The reaction solution was extracted with ethyl acetate. The organic phase was collected, washed with saturated brine, dried over anhydrous sodium sulphate and filtered. The filtrate was collected and concentrated. The resulting residue was purified by column chromatography (petroleum ether : ethyl acetate (v/v)=5 : 1) to obtain compound 33D (890 mg, yield: 92%).

LC-MS (ESI): m/z = 306.1 $[M+H]^+$.

Step 5: Compound 33D (300 mg, 0.98 mmol) was dissolved in dichloromethane (10 mL), and the mixture was cooled to -78°C. Diethylaminosulphur trifluoride (315.9 mg, 1.96 mmol) was added with stirring, and the mixture was slowly warmed to room temperature and reacted for 2 hours. The reaction solution was slowly added to saturated aqueous sodium bicarbonate solution (20 mL) and extracted with dichloromethane. The organic phase was collected, washed with saturated brine, dried over anhydrous sodium sulphate and filtered. The filtrate was collected and concentrated. The resulting residue was purified by column chromatography (petroleum ether : ethyl acetate (v/v)=8 : 1) to obtain compound 33E (240 mg, yield: 79.7%).

LC-MS (ESI): m/z = 308.2 $[M+H]^+$.

Step 6: Compound 33E (0.22 g, 0.71 mmol), N-iodosuccinimide (0.24 g, 1.06 mmol) and acetonitrile (5 mL) were added to a single-necked flask. Trifluoroacetic acid (16 mg, 0.14 mmol) was added, and the mixture was heated to 50°C and stirred for 3 hours. The reaction solution was concentrated under reduced pressure. The residue was purified by column chromatography (petroleum ether : ethyl acetate (v : v)=5 : 1) to obtain compound 33F (0.24 g, yield: 78.0%).

LC-MS (ESI): m/z = 434.0 [M+H]+.

Step 7: Compound 33F (0.2 g, 0.46 mmol), pinacolborane (0.35 g, 2.77 mmol), triethylamine (0.28 g, 2.77 mmol) and tetrakis(triphenylphosphino)palladium (0.05 g, 0.05 mmol) were weighed into a flask. 1,4-dioxane (3 mL) was added. Under nitrogen protection, the mixture was heated to 90°C and reacted for 16 hours. The reaction solution was extracted with ethyl acetate. The organic phase was collected, washed with saturated brine, dried over anhydrous sodium sulphate and filtered. The filtrate was collected and concentrated. The resulting residue was purified by column chromatography (petroleum ether : ethyl acetate (v : v)=5 : 1) to obtain compound 33G (130 mg, yield: 65.0%).
LC-MS (ESI): m/z = 434.1 $[M+H]^+$.
Step 8: Compound 33G (0.13 g, 0.30 mmol), compound 20C (0.11 g, 0.30 mmol), sodium bicarbonate (0.06 g, 0.75 mmol) and chloro(2-dicyclohexylphosphino-2',4',6'-triisopropyl-1,1'-biphenyl)[2-(2'-amino-1,1'-biphenyl)]palladium (II) (0.02 g, 0.03 mmol) were dissolved in 1,4-dioxane (3 mL) and water (0.5 mL). Under nitrogen protection, the mixture was heated to 90°C and stirred for 12 hours. The reaction solution was filtered and concentrated under reduced pressure. The residue was purified by column chromatography (petroleum ether : ethyl acetate (v : v)=1 : 1) to obtain compound 33H (0.07 g, yield: 38.5%).
LC-MS (ESI): m/z = 607.6 $[M+H]^+$.
Step 9: Compound 33H (0.03, 0.05 mmol) was dissolved in dichloromethane (3 mL). Trifluoroacetic acid (1 mL) was added. The mixture was reacted at room temperature for 1 hour. The reaction solution was directly concentrated, and the residue was purified by preparative HPLC. Method: 1. Instruments: waters 2767 (preparative liquid phase chromatographic instrument); chromatographic column: SunFire@PrepC18 (19 mm × 250 mm). 2. The sample was dissolved in methanol and filtered with a 0.45 μm filter to prepare a sample solution. 3. Preparative chromatography conditions: a. composition of mobile phases A and B: mobile phase A: acetonitrile; mobile phase B: water (containing 0.1% TFA); b. gradient elution, mobile phase A: 20%-60%; c. flow rate: 20 mL/min; d. elution time: 20 min; retention time: 6.94 min. Compound 33 (8 mg, yield: 31.9%) was obtained.
LC-MS (ESI): m/z = 507.5 $[M+H]^+$.

## Biological test

### 1. Binding experiment for PRMT5 and PRMT5•MTA

1.1 Test method for PRMT5 enzyme activity

1× test buffer (10 mM Tris, 1 mM DTT, 0.01% BSA, 0.01% Tween-20, adjusted to pH 8.0) was prepared. The test compound was dissolved in DMSO to prepare a 10 mM mother liquor, and the mother liquor was diluted to a 100-fold final concentration. 100 nL of the compound was added to each well of the administration group, and 100 nL of DMSO solution was added to blank wells and negative control wells. Then, 5 μL of a PRMT5/MEP50 (BPS, Cat# 31921) enzyme solution (final concentration: 1 nM) was added to the wells of the administration group and negative control wells, and 5 μL of 1× test buffer was added to the blank wells. Incubation was performed at room temperature for 15 minutes. 5 μL of a mixed solution of H4(1-21)S1ac (0.035 μM; GL Biochem, customized) and SAM (0.434 μM; Sigma, Cat# A7007) was added to each well and incubated at room temperature for 1 hour. 1× Epigenetics buffer (PerkinElmer, Cat# AL008F) was prepared and used for diluting acceptor microbeads and donor microbeads. 15 μL of acceptor microbeads (final concentration: 10 μg/mL; PerkinElmer, Cat# AL150C) and donor microbeads (final concentration: 10 μg/mL; PerkinElmer, Cat# AS106M) were added and incubated at room temperature for 1 hour in the dark. Then, detection was performed using the Alpha module of the reader Enspire microplate. Based on the obtained data, the inhibition rate was calculated in Excel according to: inhibition rate (%)=(maximum value negative control-detection signal value)/(maximum value negative control-minimum value blank control) × 100, and $IC_{50}$ values were fitted by using XL-Fit.

1.2 Test method for PRMT5·MTA enzyme activity

1× test buffer (10 mM Tris, 1 mM DTT, 0.01% BSA, 0.01% Tween-20, adjusted to pH 8.0) was prepared. The test compound was dissolved in DMSO to prepare a 10 mM mother liquor, and the mother liquor was diluted to a 100-fold final concentration. 100 nL of the compound was added to each well of the administration group, and 100 nL of DMSO solution was added to blank wells and negative control wells. Then, 5 μL of a solution of PRMT5/MEP50 (BPS, Cat# 31921) (final concentration: 1 nM) and MTA (final concentration: 1 μM) was added to the wells of the administration group and negative control wells, and 5 μL of 1× test buffer was added to the blank wells. Incubation was performed at room temperature for 15 minutes. 5 μL of a mixed solution of H4(1-21)Slac (0.035 μM; GL Biochem, customized) and SAM (0.434 μM; Sigma, Cat# A7007) was added to each well and incubated at room temperature for 1 hour. 1× Epigenetics buffer (PerkinElmer, Cat# AL008F) was prepared and used for diluting acceptor microbeads and donor microbeads. 15 μL of acceptor microbeads (final concentration: 10 μg/mL; PerkinElmer,#AL150C) and donor microbeads (final concentration: 10 μg/mL; PerkinElmer, Cat# AS 106M) were added and incubated at room temperature for 1 hour in the dark. Then, detection was performed

using the Alpha module of the reader Enspire microplate.

Based on the obtained data, the inhibition rate was calculated in Excel according to: inhibition rate (%) = (maximum value negative control-detection signal value)/(maximum value negative control-minimum value blank control) × 100, and $IC_{50}$ values were fitted by using XL-Fit.

The compounds of the present invention have an $IC_{50}$ value of < 100 nM for PRMT5·MTA, and $IC_{50}$ values of some compounds are shown in Table 2, where AA represents $IC_{50}$ < 1 nM, A represents 1 nM ≤ $IC_{50}$ < 10 nM, B represents 10 nM ≤ $IC_{50}$ < 50 nM, and C represents 50 nM ≤ $IC_{50}$ < 100 nM.

Table 2 Inhibitory activity of compounds on PRMT5•MTA

| Compound No. | PRMT5•MTA (nM) | PRMT5 ($IC_{50}$, nM) |
|---|---|---|
| 1 | A | B |
| 30 | AA | A |

Conclusion: The compounds of the present invention, such as the compounds in the examples, have a significant inhibitory activity on PRMT5·MTA. Some preferred compounds, such as compound 30, have an $IC_{50}$ value of 0.80 nM. In addition, in the absence of MTA, the inhibitory activity of the compounds of the present invention on PRMT5 is relatively weak, that is, the compounds of the present invention are selective for inhibiting PRMT5•MTA.

**2. MIAPACA-2 cell proliferation inhibition experiment**

MIAPACA-2 cells were cultured in a DMEM culture medium (ATCC, Cat# 30-2002; supplemented with 10% FBS and 1% double antibody) until the confluence reached about 85%, and then the cells were plated. The culture medium was discarded, and the cells were rinse with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured into a loading slot. The cell suspension was added to a first 96-well plate (Corning, Cat# 3903), and added to 10 wells (as Day 0 wells) in a second 96-well plate. The compound was dissolved in DMSO to 10 mM and stored for later use. During the experiment, the mother liquor of the compound was subjected to 5-fold serial dilution. After 24 hours of plating, the diluted compound was added to the first 96-well plate, and a DMSO vehicle control group was also set up. Incubation was performed at 37°C, 5% $CO_2$ for another 5 days. After 5 days of the incubation, the supernatant was slowly and carefully aspirated from the first 96-well plate using a multichannel pipette, and then TrypLE (Gibco, Cat# 12604-013) was added to each well. The plate was placed in a 37°C incubator for 5 minutes until the cells were completely detached. Then, the digestion was terminated by adding the culture medium, and the mixture was evenly mixed with a pipette. To a third 96-well plate (Corning, Cat# 3903), the DMEM culture medium and the cell suspension that was already digested and mixed evenly with a pipette were added to each well, and were mixed evenly with a multichannel pipette. The diluted compound was added, and a DMSO vehicle control group was also set up. Incubation was performed at 37°C, 5% $CO_2$ for another 5 days. CELL VIABILITY reagent (Vazyme, Cat# DD1101-03) was added to each well of the second 96-well plate 24 hours after plating; CELL VIABILITY reagent was added to each well of the first 96-well plate on the 5th day after administration; and CELL VIABILITY reagent was added to each well of the third 96-well plate on the 10th day after the first administration. The plates were incubated at room temperature for 10 minutes and then gently shaken 5 times. The chemiluminescence readings were detected by a microplate reader. The cell proliferation inhibition rate was calculated according to the following formula: [1-(25×T test-T0/25×T control-T0)]×100, and then the $GI_{50}$ value of the compound for inhibiting cell proliferation was calculated using the DoseResp function and the Origin 9.2 software.

The $GI_{50}$ of the compounds of the present invention for MIAPACA-2 cells was less than 1000 nM, the $GI_{50}$ of some compounds was less than 100 nM, and the $GI_{50}$ of some compounds was less than 50 nM.

The test results of some examples are shown in Table 3, where A < 50 nM, 50 nM ≤ B < 100 nM and 100 nM ≤ C < 500 nM.

Table 3 Proliferation inhibitory activity of compounds on MIAPACA-2 cells

| Compound No. | MIAPACA-2 (day 5 $GI_{50}$, nM) | MIAPACA-2 (day 10 $GI_{50}$, nM) |
|---|---|---|
| 5 | A | A |
| 11 | A | A |

(continued)

| Compound No. | MIAPACA-2 (day 5 $GI_{50}$, nM) | MIAPACA-2 (day 10 $GI_{50}$, nM) |
|---|---|---|
| 14 | A | - |
| 22 | A | - |
| 30 | A | A |

Conclusion: The compounds of the present invention, such as the compounds in the examples, have good inhibitory activity on MIAPACA-2 cells.

### 3. HCT-116 cell proliferation inhibition experiment

HCT-116 cells were cultured in an RPMI 1640 culture medium (ATCC, Cat# 30-2001; supplemented with 10% FBS and 1% double antibody) until the confluence reached about 85%, and then the cells were plated. The culture medium was discarded, and the cells were washed with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured into a loading slot. The cell suspension was added to a first 96-well plate (Corning, Cat# 3903), and added to 10 wells (as Day 0 wells) in a second 96-well plate. The compound was dissolved in DMSO to 10 mM and stored for later use. During the experiment, the mother liquor of the compound was subjected to 5-fold serial dilution. After 24 hours of plating, the diluted compound was added to the first 96-well plate, and a DMSO vehicle control group was also set up. Incubation was performed at 37°C, 5% $CO_2$ for another 5 days. After 5 days of the incubation, the supernatant was slowly and carefully aspirated from the first 96-well plate using a multichannel pipette, and then TrypLE (Gibco, Cat# 12604-013) was added to each well. The plate was placed in a 37°C incubator for 5 minutes until the cells were completely detached. Then, the digestion was terminated by adding the culture medium, and the mixture was evenly mixed with a pipette. To a third 96-well plate (Corning, Cat# 3903), the RPMI-1640 culture medium and the cell suspension that was already digested and mixed evenly with a pipette were added to each well, and were mixed evenly with a multichannel pipette. The diluted compound was added, and a DMSO vehicle control group was also set up. Incubation was performed at 37°C, 5% $CO_2$ for another 5 days. CELL VIABILITY reagent (Vazyme, Cat# DD1101-03) was added to each well of the second 96-well plate 24 hours after plating; CELL VIABILITY reagent was added to each well of the first 96-well plate on the 5th day after administration; and CELL VIABILITY reagent was added to each well of the third 96-well plate on the 10th day after the first administration. The plates were incubated at room temperature for 10 minutes and then gently shaken 5 times. The chemiluminescence readings were detected by a microplate reader. The cell proliferation inhibition rate was calculated according to the following formula: [1-(25×T test-T0/25×T control-T0)] × 100, and then the $GI_{50}$ value of the compound for inhibiting cell proliferation was calculated using the DoseResp function and the Origin 9.2 software.

The test results of some examples are shown in Table 4, where A > 500 nM, 100 nM ≤ B < 500 nM and C < 100 nM.

Table 4 Proliferation inhibitory activity of compounds on HCT-116 cells

| Compound No. | HCT-116 (day 5 $GI_{50}$, nM) | HCT-116 (day 10 $GI_{50}$, nM) |
|---|---|---|
| 30 | A | A |

Conclusion: The compounds of the present invention, such as the compounds in the examples, have weak inhibitory activity on HCT-116 cells.

### 4. HCT-116 MTAP-/- cell proliferation inhibition experiment

HCT-116 $MTAP^{-/-}$ cells were cultured in an RPMI 1640 culture medium (ATCC, Cat# 30-2001; supplemented with 10% FBS and 1% double antibody) until the confluence reached about 85%, and then the cells were plated. The culture medium was discarded, and the cells were washed with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured

into a loading slot. The cell suspension was added to a first 96-well plate (Corning, Cat# 3903), and added to 10 wells (as Day 0 wells) in a second 96-well plate. The compound was dissolved in DMSO to 10 mM and stored for later use. During the experiment, the mother liquor of the compound was subjected to 5-fold serial dilution. After 24 hours of plating, the diluted compound was added to the first 96-well plate, and a DMSO vehicle control group was also set up. Incubation was performed at 37°C, 5% $CO_2$ for another 5 days. After 5 days of the incubation, the supernatant was slowly and carefully aspirated from the first 96-well plate using a multichannel pipette, and then TrypLE (Gibco, Cat# 12604-013) was added to each well. The plate was placed in a 37°C incubator for 5 minutes until the cells were completely detached. Then, the digestion was terminated by adding the culture medium, and the mixture was evenly mixed with a pipette. To a third 96-well plate (Corning, Cat# 3903), the RPMI-1640 culture medium and the cell suspension that was already digested and mixed evenly with a pipette were added to each well, and were mixed evenly with a multichannel pipette. The diluted compound was added, and a DMSO vehicle control group was also set up. Incubation was performed at 37°C, 5% $CO_2$ for another 5 days. CELL VIABILITY reagent (Vazyme, Cat# DD1101-03) was added to each well of the second 96-well plate 24 hours after plating; CELL VIABILITY reagent was added to each well of the first 96-well plate on the 5th day after administration; and CELL VIABILITY reagent was added to each well of the third 96-well plate on the 10th day after the first administration. The plates were incubated at room temperature for 10 minutes and then gently shaken 5 times. The chemiluminescence readings were detected by a microplate reader. The cell proliferation inhibition rate was calculated according to the following formula: [1-(25×T test-T0/25×T control-T0)]×100, and then the $GI_{50}$ value of the compound for inhibiting cell proliferation was calculated using the DoseResp function and the Origin 9.2 software.

Table 5 Proliferation inhibitory activity of compounds on HCT-116 MTAP-/- cells

| Compound No. | HCT-116 $MTAP^{-/-}$ (day 5 $GI_{50}$, nM) | HCT-116 $MTAP^{-/-}$ (day 10 $GI_{50}$, nM) |
|---|---|---|
| 30 | 38.2 | 19.9 |

Conclusion: The compounds of the present invention, such as the compounds in the examples, have strong inhibitory activity on HCT-116$MTAP^{-/-}$ cells.

**5. Experiment for sDMA detection in cells**

5.1 sDMA detection in MIAPACA-2 cells

MIAPACA-2 cells were cultured in a DMEM culture medium (ATCC, Cat# 30-2002; supplemented with 10% FBS and 1% double antibody) until the confluence reached about 85%, and then the cells were plated. The culture medium was discarded, and the cells were rinse with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured into a loading slot. 100 μL of the cell suspension was added to each well of the 96-well plate. The compound was dissolved in DMSO to 10 mM and stored for later use. During the experiment, the compound was subjected to 5-fold serial dilution by using the culture medium. 100 μL of the diluted compound was added to the 96-well plate in which the cells were already plated, and a DMSO vehicle control group was also set up. Incubation was performed at 37°C, 5% $CO_2$ for 3 days. After 3 days, each well was washed twice with cold PBS. The protease and phosphatase inhibitor cocktail (CST, Cat# 5872S) and RIPA lysate were prepared in the required volume according to the proportion, and added to the 96-well plate at 50 μL/well. The plate was placed on ice for 10 minutes, during which the cells were repeatedly pipetted until completely lysed. The cells were then centrifuged at 2500 rpm at 4°C for 35 minutes to collect the protein sample of the supernatant. Protein detection was performed using a BCA kit (Beyotime, Cat# P0011). According to the detection results, the protein concentration of the lysate was adjusted to the required concentration using PBS carbonate-bicarbonate buffer pH 9.6 (Sigma, Cat# C3041), and 100 μL was added to each well of a black high-binding 96-well plate (Nunc Maxisorp, Cat# 437111) and incubated at room temperature for 2 h. After removing the lysate, the plate was washed 4 times with PBST (Cell Signalling, Cat# 9809S), and blocked with 5% BSA blocking solution (solarbio, Cat# SW3015) for 1 hour. After removing the solution, the plate was washed 4 times with PBST. 100 μL of the sDMA antibody (CST, Cat# 13222S) or SMD3 antibody (Abgent, Cat# AP12451A) was added to each well and shaken overnight at 4°C. After removing the antibody, the plate was washed 4 times with PBST. 100 μL of the anti-rabbit secondary antibody (Cell Signalling Technology, Cat# 7074) was added to each well and incubated at room temperature for 1 hour. After removing the secondary antibody, the plate was washed 4 times with PBST. 100 μL of the Immobilon Forte Western HRP substrate (Millipore, Cat# WBLUF0500) was added to each well and incubated with shaking for 15 minutes, followed by chemiluminescence detection using the BMG microplate

reader. After data normalization (SDMA reading/SMD3 reading), Graphpad Prism 8.3.0 software was used to plot the data, and the $IC_{50}$ value was calculated.

5.2 SDMA detection in HCT-116 cells

HCT-116 cells were cultured in an RPMI 1640 culture medium (ATCC, Cat# 30-2001; supplemented with 10% FBS and 1% double antibody) until the confluence reached about 85%, and then the cells were plated. The culture medium was discarded, and the cells were rinse with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured into a loading slot. 100 μL of the cell suspension was added to each well of the 96-well plate. The compound was dissolved in DMSO to 10 mM and stored for later use. During the experiment, the compound was subjected to 5-fold serial dilution by using the culture medium. 100 μL of the diluted compound was added to the 96-well plate in which the cells were already plated, and a DMSO vehicle control group was also set up. Incubation was performed at 37°C, 5% $CO_2$ for 3 days. After 3 days, each well was washed twice with cold PBS. The protease and phosphatase inhibitor cocktail (CST, Cat# 5872S) and RIPA lysate were prepared in the required volume according to the proportion, and added to the 96-well plate at 50 μL/well. The plate was placed on ice for 10 minutes, during which the cells were repeatedly pipetted until completely lysed. The cells were then centrifuged at 2500 rpm at 4°C for 35 minutes to collect the protein sample of the supernatant. Protein detection was performed using a BCA kit (Beyotime, Cat# P0011). According to the detection results, the protein concentration of the lysate was adjusted to the required concentration using PBS carbonate-bicarbonate buffer pH 9.6 (Sigma, Cat# C3041), and 100 μL was added to each well of a black high-binding 96-well plate (Nunc Maxisorp, Cat# 437111) and incubated at room temperature for 2 h. After removing the lysate, the plate was washed 4 times with PBST (Cell Signalling, Cat# 9809S), and blocked with 5% BSA blocking solution (solarbio, Cat# SW3015) for 1 hour. After removing the solution, the plate was washed 4 times with PBST. 100 μL of the sDMA antibody (CST, Cat# 13222S) or SMD3 antibody (Abgent, Cat# AP12451A) was added to each well and shaken overnight at 4°C. After removing the antibody, the plate was washed 4 times with PBST. 100 μL of the anti-rabbit secondary antibody (Cell Signalling Technology, Cat# 7074) was added to each well and incubated at room temperature for 1 hour. After removing the secondary antibody, the plate was washed 4 times with PBST. 100 μL of the Immobilon Forte Western HRP substrate (Millipore, Cat# WBLUF0500) was added to each well and incubated with shaking for 15 minutes, followed by chemiluminescence detection using the BMG microplate reader. After data normalization (SDMA reading/SMD3 reading), Graphpad Prism 8.3.0 software was used to plot the data, and the $IC_{50}$ value was calculated.

Table 6 Inhibitory activity of compounds on SDMA production in HCT-116 cells

| Compound No. | HCT-116 SDMA ($IC_{50}$, nM) |
|---|---|
| 30 | 499 |

Conclusion: The compounds of the present invention, such as the compounds of the examples, have weak inhibitory activity on the production of SDMA in HCT-116 cells.

5.3 SDMA detection in HCT-116 $MTAP^{-/-}$ cells

HCT-116 MTAP-/- cells were cultured in an RPMI 1640 culture medium (ATCC, #30-2001; supplemented with 10% FBS and 1% double antibody) until the confluence reached about 85%, and then the cells were plated. The culture medium was discarded, and the cells were rinse with 1× PBS and digested by trypsin (Gibco, Cat# 15400-054). When the cells became round and began to fall off, the digestion was terminated by adding the culture medium. The cells were pipetted, transferred to a sterile centrifuge tube and centrifuged at 1000 rpm for 3 minutes, and after the centrifugation, the supernatant was discarded. The cells were resuspended by adding the culture medium to the centrifuge tube and then counted. According to the counting results, the cell suspension was adjusted to an appropriate concentration and poured into a loading slot. 100 μL of the cell suspension was added to each well of the 96-well plate. The compound was dissolved in DMSO to 10 mM and stored for later use. During the experiment, the compound was subjected to 5-fold serial dilution by using the culture medium. 100 μL of the diluted compound was added to the 96-well plate in which the cells were already plated, and a DMSO vehicle control group was also set up. Incubation was performed at 37°C, 5% $CO_2$ for 3 days. After 3 days, each well was washed twice with cold PBS. The protease and phosphatase inhibitor cocktail (CST, Cat# 5872S) and RIPA lysate were prepared in the required volume according to the proportion, and added to the 96-well plate at 50 μL/well.

The plate was placed on ice for 10 minutes, during which the cells were repeatedly pipetted until completely lysed. The cells were then centrifuged at 2500 rpm at 4°C for 35 minutes to collect the protein sample of the supernatant. Protein detection was performed using a BCA kit (Beyotime, Cat# P0011). According to the detection results, the protein concentration of the lysate was adjusted to the required concentration using PBS carbonate-bicarbonate buffer pH 9.6 (Sigma, Cat# C3041), and 100 μL was added to each well of a black high-binding 96-well plate (Nunc Maxisorp, Cat# 437111) and incubated at room temperature for 2 h. After removing the lysate, the plate was washed 4 times with PBST (Cell Signalling, Cat# 9809S), and blocked with 5% BSA blocking solution (solarbio, Cat# SW3015) for 1 hour. After removing the solution, the plate was washed 4 times with PBST. 100 μL of the sDMA antibody (CST, Cat# 13222S) or SMD3 antibody (Abgent, Cat# AP12451A) was added to each well and shaken overnight at 4°C. After removing the antibody, the plate was washed 4 times with PBST. 100 μL of the anti-rabbit secondary antibody (Cell Signalling Technology, Cat# 7074) was added to each well and incubated at room temperature for 1 hour. After removing the secondary antibody, the plate was washed 4 times with PBST. 100 μL of the Immobilon Forte Western HRP substrate (Millipore, Cat# WBLUF0500) was added to each well and incubated with shaking for 15 minutes, followed by chemiluminescence detection using the BMG microplate reader. After data normalization (SDMA reading/SMD3 reading), Graphpad Prism 8.3.0 software was used to plot the data, and the $IC_{50}$ value was calculated.

Table 7 Inhibitory activity of compounds on SDMA production in HCT-116 MTAP-/- cells

| Compound No. | HCT-116 $MTAP^{-/-}$SDMA ($IC_{50}$, nM) |
|---|---|
| 30 | 3.39 |

Conclusion: The compounds of the present invention, such as the compounds of the examples, have strong inhibitory activity on the production of SDMA in HCT-116 $MTAP^{-/-}$ cells.

**6. Test for hERG potassium ion channel**

Experimental platform: electrophysiological manual patch-clamp system

Cell line: Chinese hamster ovary (CHO) cell line stably expressing hERG potassium ion channel

Experimental method: in CHO (Chinese Hamster Ovary) cells stably expressing hERG potassium channel, the whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

Data processing: data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition \%} = [1 - (I / Io)] \times 100\%$$

where Inhibition% represents the percentage of inhibition of hERG potassium current by the compound, and *I* and *Io* represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1+10\^{}((\text{LogIC50-X}) \times \text{HillSlope}))$$

where X represents the Log value of the tested concentration of the test sample, Y represents the percentage inhibition at the corresponding concentration, and Bottom and Top represent the minimum and maximum percentage inhibitions, respectively.

### 7. CYP3A4 enzyme inhibition test

The purpose of this study was to evaluate the effect of the test compound on the activity of CYP3A4 enzymes of human liver microsomal cytochrome P450 (CYP) by using an *in vitro* testing system. The specific probe substrates of CYP3A4 enzymes were co-incubated with human liver microsomes and test compounds at different concentrations, and the reaction was initiated by adding reduced nicotinamide adenine dinucleotide phosphate (NADPH). After the completion of the reaction, the samples were treated and subjected to the liquid chromatography-tandem mass spectrometry (LC-MS/MS) to quantitatively detect metabolites produced by specific substrates. The changes in CYP enzyme activity were determined, and $IC_{50}$ values were calculated to evaluate the inhibitory potential of the test compounds on each CYP3A4 enzyme. Under the test conditions, the incubation concentration was 0-30 μM.

Conclusion: The compounds of the present invention, such as the compounds in the examples, have weak inhibitory effects on CYP3A4 enzymes.

### 8. Pharmacokinetic test in mice

8.1 Experimental animals: Male C57 mice, 22-25 g, 6 mice/compound, purchased from Chengdu Ddossy Experimental Animals Co., Ltd.

8.2 Experimental design: On the day of the experiment, (6×number of test compounds (n)) C57 mice were randomly divided into groups according to their body weights. The animals were fasted but with water available for 12 to 14 h one day before the administration and were fed 4 h after the administration.

Before and after the administration, 0.06 mL of blood was taken from the orbit under isoflurane anaesthesia and placed in an EDTAK2 centrifuge tube. Centrifugation was performed at 5000 rpm at 4°C for 10 min, and the plasma was collected. The blood collection time points for the intravenous administration group and intragastric administration group were 0 min, 5 min, 15 min, 30 min, 1 h, 2 h, 4 h, 7 h and 24 h. Before analysis and detection, all samples were stored at -80°C, and a quantitative analysis of samples was performed using LC-MS/MS.

Conclusion: The compounds of the present invention, such as the compounds in the examples, have good pharmacokinetic profiles in mice.

### 9. Pharmacokinetic test in rats

9.1 Experimental animals: male SD rats, about 220 g, 6-8 weeks old, 6 rats/compound, purchased from Chengdu Ddossy Experimental Animals Co., Ltd.

9.2 Experimental design: On the day of the experiment, (6×number of test compounds (n)) SD rats were randomly divided into groups according to their body weights. The animals were fasted but with water available for 12 to 14 h one day before the administration and were fed 4 h after the administration.

Before and after the administration, 0.15 mL of blood was taken from the orbit under isoflurane anaesthesia and placed in an EDTAK2 centrifuge tube. Centrifugation was performed at 5000 rpm at 4°C for 10 min, and the plasma was collected. The blood collection time points for the intravenous administration group and intragastric administration group were 0, 5 min, 15 min, 30 min, 1 h, 2 h, 4 h, 6 h, 8 h and 24 h. Before analysis and detection, all samples were stored at -80°C, and a quantitative analysis of samples was performed using LC-MS/MS.

Conclusion: The compounds of the present invention, such as the compounds in the examples, have good pharmacokinetic profiles in rats.

### 10. Pharmacokinetic test in beagle dogs

10.1 Experimental animals: male beagle dogs, about 8-11 kg, 6 beagle dogs/compound, purchased from Beijing Marshall Biotechnology Co., Ltd.

10.2 Experimental method: On the day of the experiment, (6×number of test compounds (n)) beagle dogs were randomly divided into groups according to their body weights. The animals were fasted but with water available for 12 to 14 h one day before the administration and were fed 4 h after the administration. Administration was carried out according to Table 8.

Table 8 Administration information

| Group | Number | Administration information | | | | | |
|---|---|---|---|---|---|---|---|
| | Male | Test compound | Administra tion dosage (mg/kg) | Administrati on concentratio n (mg/mL) | Administra tion volume (mL/kg) | Collected samples | Mode of administration |
| G1 | 3 | Compound 30 | 1 | 1 | 1 | Plasma | Intravenous administration |
| G2 | 3 | | 5 | 1 | 5 | Plasma | Intragastric administration |
| Note: Vehicle for intravenous administration: 5% DMA+5% Solutol+90% Saline; vehicle for intragastric administration: 0.5% MC | | | | | | | |

(DMA: dimethylacetamide; Solutol: polyethylene glycol-15-hydroxystearate; Saline: physiological saline; MC: methylcellulose solution)

Before and after the administration, 1 mL of blood was taken from the jugular veins or limb veins, and placed in an EDTAK2 centrifuge tube. Centrifugation was performed at 5000 rpm at 4°C for 10 min, and the plasma was collected. The blood collection time points for the intravenous administration group and intragastric administration group were 0 min, 5 min, 15 min, 30 min, 1 h, 2 h, 4 h, 6 h, 8 h, 10 h, 12 h and 24 h. Before analysis and detection, all samples were stored at -80°C, and a quantitative analysis of samples was performed using LC-MS/MS.

Table 9. Pharmacokinetic parameters of test compounds in plasma of beagle dogs

| Test compound | Mode of administration | CL (mL/min/kg) | $T_{1/2}$ (h) | $AUC_{0-t}$ (hr × ng/mL) |
|---|---|---|---|---|
| Compound 30 | i.v. (5 mg/kg) | 0.313 | - | - |
| | i.g. (10 mg/kg) | - | 16.1 | 178458 |
| -: not applicable. | | | | |

Conclusion: The compounds of the present invention, such as the compounds in the examples, have good pharmacokinetic profiles in beagle dogs.

**11. Pharmacokinetic test in monkeys**

11.1 Experimental animals: Male cynomolgus monkeys, 3-5 kg, 3-6 years old, 4 monkeys/compound, purchased from Suzhou Xishan Biotechnology Inc.

11.2 Experimental method: On the day of the experiment, (4×number of test compounds (n)) monkeys were randomly divided into groups according to their body weights. The animals were fasted but with water available for 14 to 18 h one day before the administration and were fed 4 h after the administration.

Before and after the administration, 1.0 mL of blood was taken from the limb veins and placed in an EDTAK2 centrifuge tube. Centrifugation was performed at 5000 rpm at 4°C for 10 min, and the plasma was collected. The blood collection time points for the intravenous administration group and intragastric administration group were 0 min, 5 min, 15 min, 30 min, 1 h, 2 h, 4 h, 6 h, 8 h, 10 h, 12 h, 24 h, 48 h and 72 h. Before analysis and detection, all samples were stored at -80°C, and a quantitative analysis of samples was performed using LC-MS/MS.

Conclusion: The compounds of the present invention, such as the compounds in the examples, have good pharmacokinetic profiles in monkeys.

***12. In-vivo* efficacy experiment**

HCT-116 MTAP-/- cells were cultured in an RPMI-1640 culture medium in a 37°C incubator containing 5% CO2. The cells were passaged every 2 to 3 days. When the number reached the requirement, the cells were collected and counted. The cell concentration was adjusted to an appropriate concentration with PBS and then diluted to 2.5 × 107 cells/ml with Matrigel at a ratio of 1 : 1. According to 5 × 106 cells/200 μL/mouse (purchased from Vital River Laboratory Animal Technology Co., Ltd., Balb/c nude, female), the cells were inoculated into the axilla of the right forelimb. When the tumour grew to 80-100 mm3 (Day 5-7 after the inoculation), the animals were sorted by tumour size and grouped for

administration (the day of grouping for administration was recorded as Day 0). During the administration, the tumour and animal body weight were measured 3 times a week. After the experiment, multi-point PK collection and tumour sample collection were performed, and the tumour weight was recorded and photographed. The *in-vivo* efficacy results (tumour volume (panel a) and mouse body weight (panel b)) of compound 30 are shown in FIG. 1, and the *in-vivo* efficacy results (tumour weight) of compound 30 are shown in FIG. 2.

Experimental data were expressed as mean ± standard error (mean ± S.E.M.). Data were statistically analysed by Graphpad Prism using one-way ANOVA plus Dunnett's multiple comparison test. $P < 0.05$ indicated the significant difference.

(MRTX1719 structure: 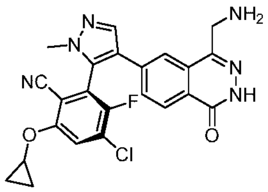 )

Conclusion: The compounds of the present invention, such as the compounds in the examples, have good tumour-suppressing effects in the mouse CDX (HCT-116 MTAP$^{-/-}$ cell) model.

## Claims

1. A compound represented by formula (I-a) or (I-b), or a stereoisomer, a deuterated compound, a solvate or a pharmaceutically acceptable salt thereof,

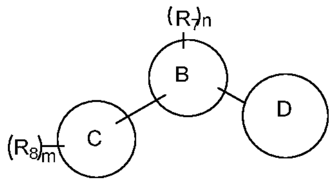

I-a

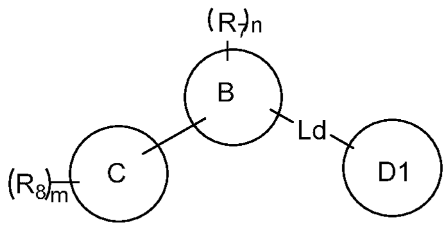

I-b

**characterized in that**

D is selected from:

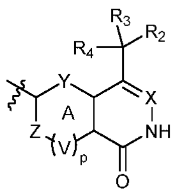 , 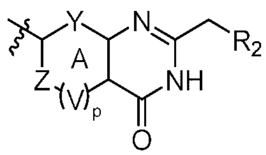 , 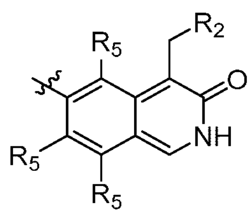 ,

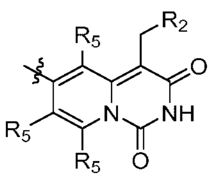 , 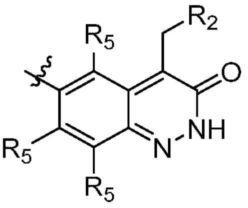 , 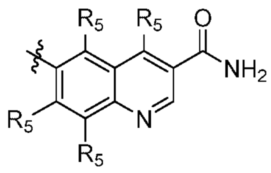 , 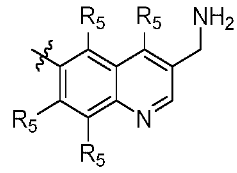 ,

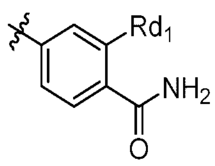 , 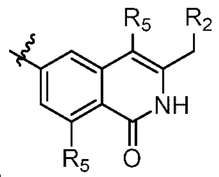 , 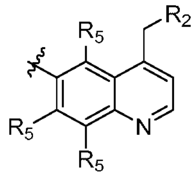 , 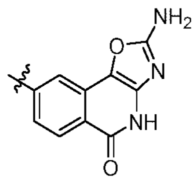 , 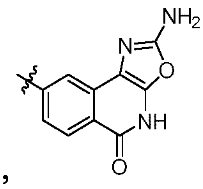 ,

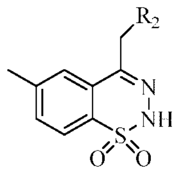 ;

D1 is selected from:

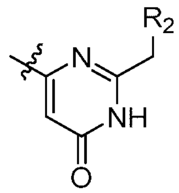 ;

Ld is selected from $C_{1-4}$ alkyl, $C_{2-4}$ alkenyl or $C_{2-4}$ alkynyl;

Rd1 is selected from 5-membered heteroaryl, wherein the heteroaryl is optionally substituted with 1-3 groups selected from halogen, D, CN, OH, $C_{1-4}$ alkyl, $C_{1-4}$ alkoxy and $NH_2$;

X is selected from $CR_1$ or N;

Y, Z and V are independently $CR_5$, $C(R_5)_2$, $NR_6$, N, O or S, and the total number of O and S is not greater than 1;

ring A is phenyl, 5- to 7-membered heterocycloalkyl containing 1-3 heteroatoms selected from N, O or S, or 5- to 6-membered heteroaryl containing 1-3 heteroatoms selected from N, O or S;

ring B is $C_{3-12}$ carbocycle, or 4- to 12-membered heterocycle containing 1-3 heteroatoms selected from N, O or S;

ring C is $C_{3-12}$ carbocycle, or 4- to 12-membered heterocycle containing 1-3 heteroatoms selected from N, O or S;

$R_1$ is H, D, $C_{1-4}$ alkyl, $C_{1-4}$ alkoxy, $C_{2-4}$ alkenyl, $C_{2-4}$ alkynyl, halogen, CN, OH, $NH_2$ or COOH, wherein the alkyl, alkoxy, alkenyl or alkynyl is optionally substituted with 1-3 groups selected from halogen, D, CN, OH and $NH_2$;

$R_2$ is OH, $NH_2$, $-CH_2NH_2$ or -NH-OH;

$R_3$ and $R_4$ are independently H, D, halogen, $C_{1-4}$ alkyl, CN, OH, $NH_2$, $C_{1-4}$ alkoxy, $C_{2-4}$ alkenyl, $C_{2-4}$ alkynyl or $C_{3-6}$ cycloalkyl, wherein the alkyl, alkoxy, alkenyl, alkynyl or cycloalkyl is optionally substituted with 1-3 groups selected from halogen, D, CN, OH and $NH_2$;

alternatively, $R_3$ and $R_4$ together form =NH;

each $R_5$ is independently H, D, =O, OH, $C_{1-4}$ alkyl, CN, halogen, $C_{1-4}$ alkoxy, $C_{2-4}$ alkenyl, $C_{2-4}$ alkynyl, 5- to 6-membered heteroaryl containing 1-3 heteroatoms selected from N, O or S, 4- to 7-membered heterocycloalkyl containing 1-3 heteroatoms selected from N, O or S, or $C_{3-6}$ cycloalkyl, wherein the alkyl, alkoxy, alkenyl, alkynyl, heteroaryl or cycloalkyl is optionally substituted with 1-3 groups selected from halogen, D, CN, OH, $C_{1-4}$ alkyl, $C_{1-4}$ alkoxy and $NH_2$;

$R_6$ is H, D, $C_{1-4}$ alkyl or $C_{3-6}$ cycloalkyl, wherein the alkyl or cycloalkyl is optionally substituted with 1-3 groups selected from halogen, D, CN, OH and $NH_2$;

each $R_7$ is independently H, =O, D, OH, CN, halogen, $C_{1-4}$ alkyl, $C_{1-4}$ alkoxy, $N(R_7')_2$, $C_{2-4}$ alkenyl, $C_{2-4}$ alkynyl, $C_{3-6}$ cycloalkyl or 4- to 7-membered heterocycloalkyl containing 1-3 heteroatoms selected from N, O or S, wherein the alkyl, alkoxy, alkenyl, alkynyl, cycloalkyl or heterocycloalkyl is optionally substituted with 1-3 groups selected

from halogen, D, OH, $NH_2$, CN, $C_{1-4}$ alkyl, - $C(O)C_{1-4}$ alkyl, -$C(O)NHC_{1-4}$ alkyl, -$C(O)NH_2$, -$NHC(O)C_{1-4}$ alkyl and $C_{1-4}$ alkoxy;

each $R_8$ is independently H, D, nitro, amino, $C_{1-6}$ alkoxy, =O, OH, CN, halogen, $C_{2-6}$ alkenyl, $C_{2-6}$ alkynyl, -$SF_5$, $N_3$, $C_{1-6}$ alkyl, -$COC_{1-4}$ alkyl, -$N(R_7')_2$, - $C(=O)N(R_7')_2$, -$NR_7'C(=O)$-$R_7'$, -$C(=O)$-$R_7'$, -$(CH_2)_r$-O-$(CH_2)_r$-$R_8'$, -S-$C_{1-6}$ alkyl, - S(O)-$C_{1-6}$ alkyl, -$S(O)_2$-$C_{1-6}$ alkyl, -S-$(CH_2)_r$-$R_8'$, -$NR_7'$-$(CH_2)_r$-$R_8'$ or -$(CH_2)_r$-$R_8'$, wherein the alkyl, alkenyl or alkynyl is optionally substituted with 1-5 groups selected from $R_{8''}$;

each $R_7'$ is independently H, D, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $C_{3-6}$ cycloalkyl, $C_{3-12}$ heterocycloalkyl, -$NHC_{1-6}$ alkyl, -$N(C_{1-6}$ alkyl$)_2$ or deuterated $C_{1-6}$ alkoxy, wherein the cycloalkyl or heterocycloalkyl is optionally substituted with 1 to 3 groups selected from deuterium, halogen, cyano, amino, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy or deuterated $C_{1-6}$ alkoxy;

each $R_8'$ is independently H, D, $C_{1-6}$ alkyl, $C_{1-4}$ alkoxy, $C_{3-12}$ carbocycle, or 4-to 12-membered heterocycle containing 1-3 heteroatoms selected from N, O or S, wherein the alkyl or alkoxy is optionally substituted with 1-3 groups selected from halogen, =O, D, OH, $NH_2$, CN, $C_{1-6}$ alkyl, halo $C_{1-6}$ alkyl, deuterated $C_{1-6}$ alkyl, - $C(O)C_{1-4}$ alkyl, -$C(O)NHC_{1-4}$ alkyl, -$C(O)NH_2$, -$NHC(O)C_{1-4}$ alkyl, $C_{1-6}$ alkoxy, halo $C_{1-6}$ alkoxy and deuterated $C_{1-6}$ alkoxy; the carbocycle or heterocycle is optionally substituted with 1-5 groups selected from $R_{8''}$;

$R_{8''}$ is selected from halogen, D, OH, $NH_2$, CN, $C_{1-6}$ alkyl and $C_{1-6}$ alkoxy, wherein the alkyl or alkoxy is optionally further substituted with 1 to 5 groups selected from halogen, deuterium, cyano, amino, hydroxyl, $C_{1-6}$ alkyl or $C_{1-6}$ alkoxy;

each r is independently 0, 1, 2 or 3;

p is 0, 1 or 2;

n and m are independently an integer of 0-5;

provided that:

1. the group

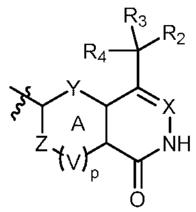

is not

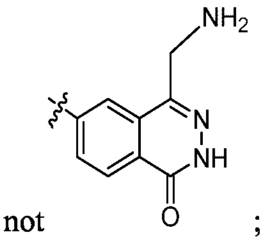

;

2. when

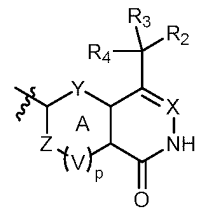

is

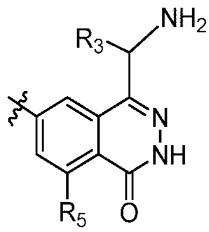

,

$R_3$ is H, $C_{1-4}$ alkyl or $C_{1-4}$ haloalkyl, $R_5$ is H, halogen, $C_{1-4}$ alkyl, $C_{1-4}$ alkoxy or $C_{1-4}$ haloalkyl, and $R_3$ and $R_5$ are not both H, the group

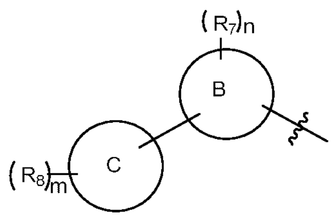

is

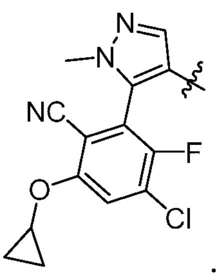

.

2. The compound represented by formula (I-a), or the stereoisomer, the deuterated compound, the solvate or the pharmaceutically acceptable salt thereof according to claim 1, **characterized in that** the compound has a structure of formula (I) below:

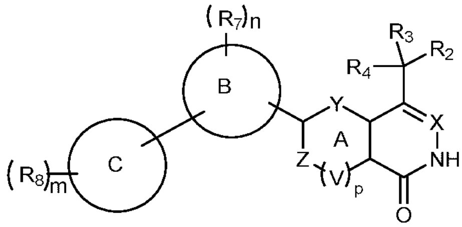

I

.

3. The compound, or the stereoisomer, the deuterated compound, the solvate or the pharmaceutically acceptable salt thereof according to claim 1, **characterized in that**
D is selected from

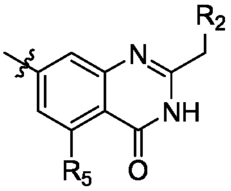 , 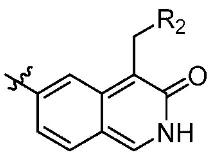 , 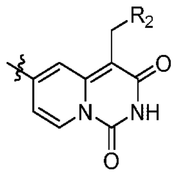 ,

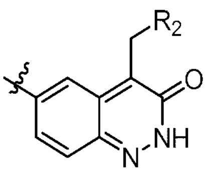 , 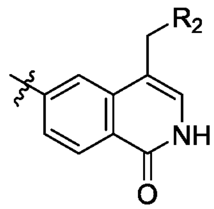 , 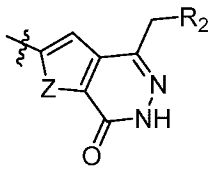 , 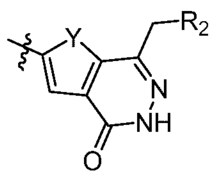 ,

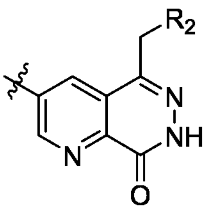 , 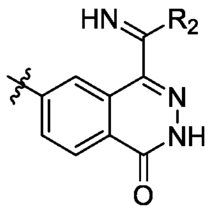 , 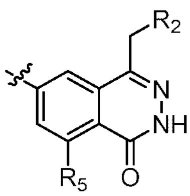 , 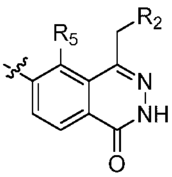 , 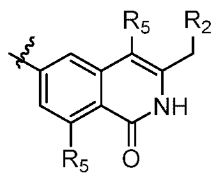 ,

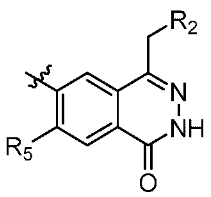 , 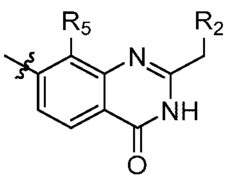 , 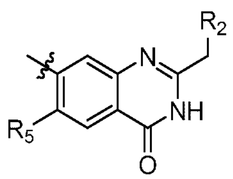 , 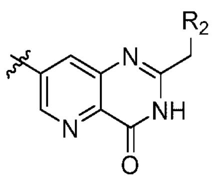 ,

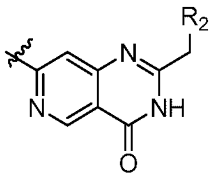 , 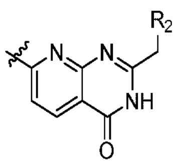 , 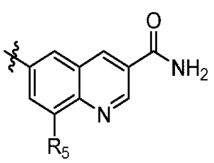 , 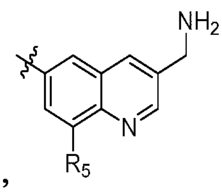 , 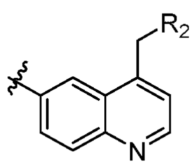 ,

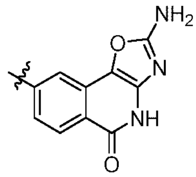, 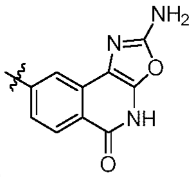, 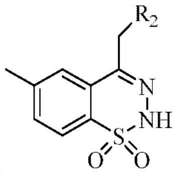.

4. The compound, or the stereoisomer, the deuterated compound, the solvate or the pharmaceutically acceptable salt thereof according to any one of claims 1-3, **characterized in that**

X is selected from $CR_1$ or N;
Y, Z and V are independently $CR_5$, N, O or S, and the total number of O and S is not greater than 1;
ring A is phenyl, or 5- to 6-membered heteroaryl containing 1-3 heteroatoms selected from N, O or S;
ring B is phenyl, $C_{3-6}$ cycloalkyl, 4- to 7-membered heterocycloalkyl containing 1-3 heteroatoms selected from N, O or S, or 5- to 6-membered heteroaryl containing 1-3 heteroatoms selected from N, O or S;
ring C is phenyl, $C_{3-6}$ cycloalkyl, 4- to 7-membered heterocycloalkyl containing 1-3 heteroatoms selected from N, O or S, or 5- to 6-membered heteroaryl containing 1-3 heteroatoms selected from N, O or S;
$R_1$ is H, D, $C_{1-4}$ alkyl, halogen or CN, wherein the alkyl is optionally substituted with 1-3 groups selected from halogen, D, CN and OH;
$R_2$ is OH, $NH_2$, $-CH_2NH_2$ or -NH-OH;
$R_3$ and $R_4$ are independently H, D, halogen, $C_{1-4}$ alkyl, CN, OH or $NH_2$, wherein the alkyl is optionally substituted with 1-3 groups selected from halogen, D, CN, OH and $NH_2$;
each $R_5$ is independently H, D, OH, $C_{1-4}$ alkyl, CN, halogen, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from halogen, D, CN, OH, $C_{1-4}$ alkyl, $C_{1-4}$ alkoxy and $NH_2$;
each $R_7$ is independently H, =O, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl, $-C(O)C_{1-4}$ alkyl, $-C(O)NHC_{1-4}$ alkyl, $-C(O)NH_2$, $-NHC(O)C_{1-4}$ alkyl and $C_{1-4}$ alkoxy;
each $R_8$ is independently H, D, =O, OH, CN, halogen, $C_{2-4}$ alkenyl, $C_{2-4}$ alkynyl, $N_3$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;
each $R_7'$ is independently H, $C_{1-4}$ alkyl or halo $C_{1-4}$ alkyl;
each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy;
each r is independently 0, 1 or 2;
p is 0 or 1;
n and m are independently 0, 1, 2, 3 or 4.

5. The compound, or the stereoisomer, the deuterated compound, the solvate or the pharmaceutically acceptable salt thereof according to claim 1, **characterized in that** ring B is selected from

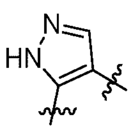 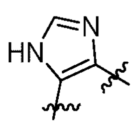 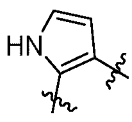 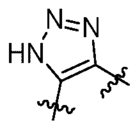 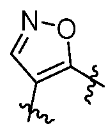.

6. The compound, or the stereoisomer, the deuterated compound, the solvate or the pharmaceutically acceptable salt thereof according to claim 1, **characterized in that** the compound has a structure of formula (II-a) or formula (II):

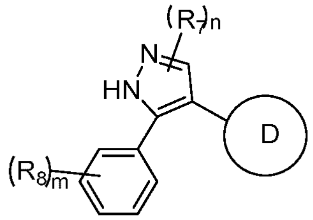

II-a

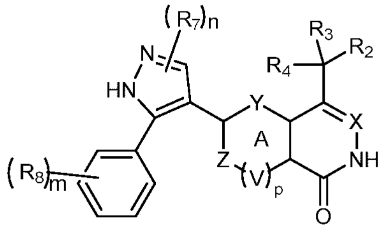

II

.

7. The compound, or the stereoisomer, the deuterated compound, the solvate or the pharmaceutically acceptable salt thereof according to claim 6, **characterized in that**

X is selected from $CR_1$ or N;
Y, Z and V are independently $CR_5$, N, O or S, and the total number of O and S is not greater than 1;
ring A is phenyl, thienyl, thiazolyl, pyrrolyl, imidazolyl, oxazolyl, pyridyl, pyrazinyl, pyrimidyl or pyridazinyl;
D is selected from

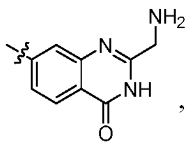 , 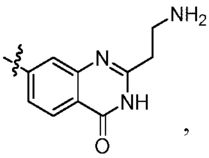 , 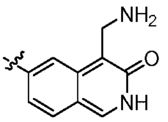 , 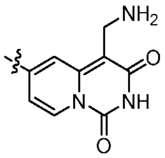 , 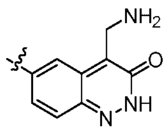 ,

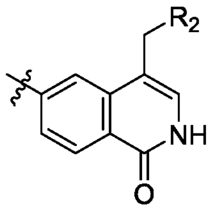 , 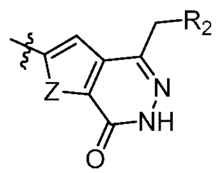 , 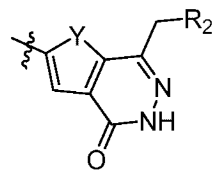 , 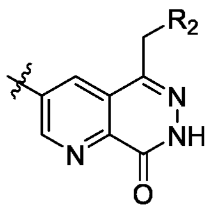 ,

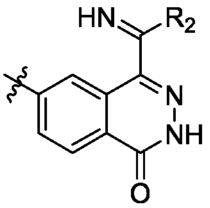 , 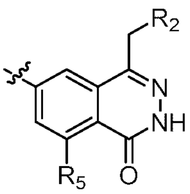 , 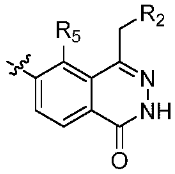 , 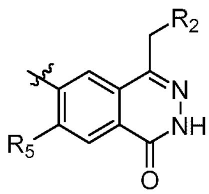 , 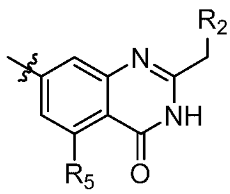 ,

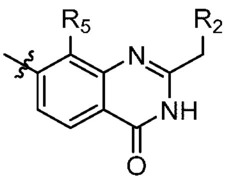 , 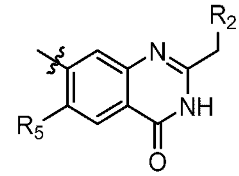 , 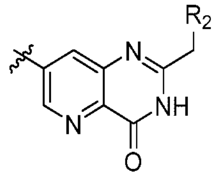 , 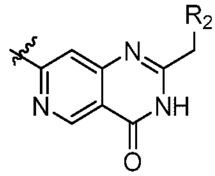 ,

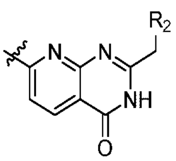 , 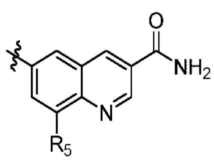 , 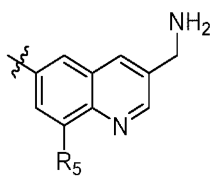 , 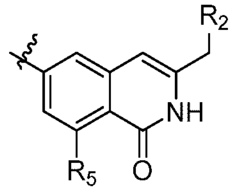 ,

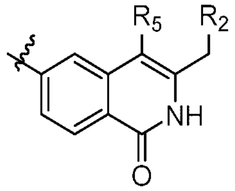 , 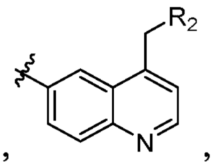 , 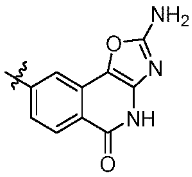 , 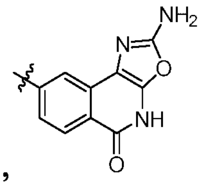 , 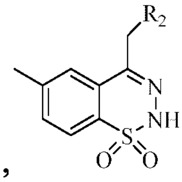 ;

$R_1$ is H, D, $C_{1-4}$ alkyl, F, Cl, Br, I or CN, wherein the alkyl is optionally substituted with 1-3 groups selected from F, Cl, Br, I, D, CN and OH;
$R_2$ is OH, $NH_2$, $-CH_2NH_2$ or -NH-OH;
$R_3$ and $R_4$ are independently H, D, F, Cl, Br, I, $C_{1-4}$ alkyl or CN, wherein the alkyl is optionally substituted with 1-3 groups selected from F, Cl, Br, I, D, CN, OH and $NH_2$;
each $R_5$ is independently H, D, OH, $C_{1-4}$ alkyl, CN, F, Cl, Br, I, $C_{1-4}$ alkoxy, $C_{2-4}$ alkenyl, $C_{2-4}$ alkynyl, 5-membered heteroaryl containing 1-3 heteroatoms selected from N, O or S, 4- to 5-membered heterocycloalkyl containing 1-3 heteroatoms selected from N, O or S, or $C_{3-4}$ cycloalkyl, wherein the alkyl, alkoxy, alkenyl, alkynyl, heteroaryl, heterocycloalkyl or cycloalkyl is optionally substituted with 1-3 groups selected from F, Cl, Br, I, D, CN, OH, methoxy and $NH_2$;
each $R_7$ is independently H, D, OH, CN, halogen, $C_{1-4}$ alkyl, $C_{1-4}$ alkoxy, $N(R_7')_2$, $C_{2-4}$ alkenyl or $C_{2-4}$ alkynyl, wherein the alkyl, alkoxy, alkenyl or alkynyl is optionally substituted with 1-3 groups selected from halogen, D, OH, $NH_2$, CN, $C_{1-4}$ alkyl and $C_{1-4}$ alkoxy;
each $R_8$ is independently H, D, OH, CN, F, Cl, Br, I, $C_{2-4}$ alkenyl, $C_{2-4}$ alkynyl, $N_3$, $C_{1-4}$ alkyl, $-COC_{1-4}$ alkyl, $-CONR_7'C_{1-4}$ alkyl, $-NR_7'COC_{1-4}$ alkyl, $N(R_7')_2$, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-4}$ alkyl and $C_{1-4}$ alkoxy;
each $R_7'$ is independently H, $C_{1-4}$ alkyl or halo $C_{1-4}$ alkyl;
each $R_8'$ is independently H, $C_{1-4}$ alkoxy, $C_{3-6}$ cycloalkyl, phenyl, 4- to 7-membered heterocycloalkyl containing 1-3 heteroatoms selected from N, O or S, or 5- to 6-membered heteroaryl containing 1-3 heteroatoms selected from N, O or S, wherein the cycloalkyl, phenyl, heterocycloalkyl or heteroaryl is optionally substituted with 1-3 groups selected from halogen, =O, D, OH, $NH_2$, CN, $C_{1-4}$ alkyl, halo $C_{1-4}$ alkyl and $C_{1-4}$ alkoxy;
each r is independently 0, 1 or 2;
p is 0 or 1;
n is 0, 1, 2 or 3;
m is 0, 1, 2, 3 or 4.

8. The compound, or the stereoisomer, the deuterated compound, the solvate or the pharmaceutically acceptable salt thereof according to claim 7, **characterized in that**
each $R_8$ is independently D, CN, F, Cl, Br, $C_{2-4}$ alkenyl, $C_{2-4}$ alkynyl, $N_3$, $C_{1-4}$ alkyl, $-O-(CH_2)r-R_8'$ or $-(CH_2)r-R_8'$, wherein the alkyl, alkenyl or alkynyl is optionally substituted with 1-3 groups selected from halogen, D, OH, $NH_2$, CN, $C_{1-2}$ alkyl and $C_{1-2}$ alkoxy.

9. The compound, or the stereoisomer, the deuterated compound, the solvate or the pharmaceutically acceptable salt thereof according to claim 6, **characterized in that**
$R_8'$ is independently $C_{1-4}$ alkoxy, cyclopropyl, cyclobutyl, cyclopentyl, phenyl, tetrahydropyrrolyl, azetidinyl, piperidyl, piperazinyl, morpholinyl, oxetanyl, tetrahydrofuryl, tetrahydropyranyl,

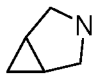 , 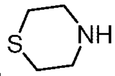 , 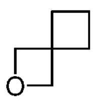 , 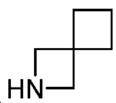 , 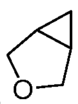 , 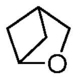

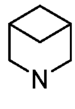 ,

thienyl, thiazolyl, pyrrolyl, imidazolyl, oxazolyl, pyridyl, pyrazinyl, pyrimidyl or pyridazinyl optionally substituted with 1-3 groups selected from F, Cl, Br, I, =O, D, OH, $NH_2$, CN, $C_{1-4}$ alkyl, halo $C_{1-2}$ alkyl and $C_{1-2}$ alkoxy.

10. The compound, or the stereoisomer, the deuterated compound, the solvate or the pharmaceutically acceptable salt

thereof according to claim 1, **characterized in that**

D1 is selected from:

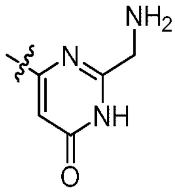

;

Ld is selected from $C_{2-4}$ alkenyl, preferably

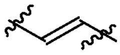

;

Rd1 is selected from 5-membered heteroaryl, wherein the heteroaryl is optionally substituted with 1-2 groups selected from $NH_2$, and Rd1 is preferably

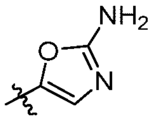

.

**11.** The compound, or the stereoisomer, the deuterated compound, the solvate or the pharmaceutically acceptable salt thereof according to claim 1, **characterized in that** the compound has a structure selected from those in Table 1.

**12.** A pharmaceutical composition, **characterized in** comprising the compound, or the stereoisomer, the deuterated compound, the solvate or the pharmaceutically acceptable salt thereof according to any one of claims 1-11, and a pharmaceutically acceptable carrier and/or excipient.

**13.** Use of the compound, or the stereoisomer, the deuterated compound, the solvate or the pharmaceutically acceptable salt thereof according to any one of claims 1-11, or the composition according to claim 12 in the preparation of a drug for treating/preventing PRMT5•MTA-mediated diseases.

**14.** The use according to claim 13, **characterized in that** the PRMT5•MTA-mediated diseases are selected from liver cancer, breast cancer, skin cancer, bladder cancer, pancreatic cancer, or head and neck cancer.

**15.** A pharmaceutical composition or pharmaceutical preparation, **characterized in that** the pharmaceutical composition or pharmaceutical preparation comprises 1-1500 mg of the compound according to any one of claims 1-11, or a stereoisomer, a deuterated compound, a solvate, a prodrug, a metabolite, a pharmaceutically acceptable salt or a eutectic crystal thereof, and a pharmaceutically acceptable excipient.

**16.** A method for treating a disease in a mammal, **characterized in that** the method comprises administering to a subject a therapeutically effective amount of the compound according to any one of claims 1-11, or a stereoisomer, a deuterated compound, a solvate, a prodrug, a metabolite, a pharmaceutically acceptable salt or a eutectic crystal thereof, wherein the therapeutically effective amount is preferably 1-1500 mg, and the disease is preferably liver cancer, breast cancer, skin cancer, bladder cancer, pancreatic cancer, or head and neck cancer.

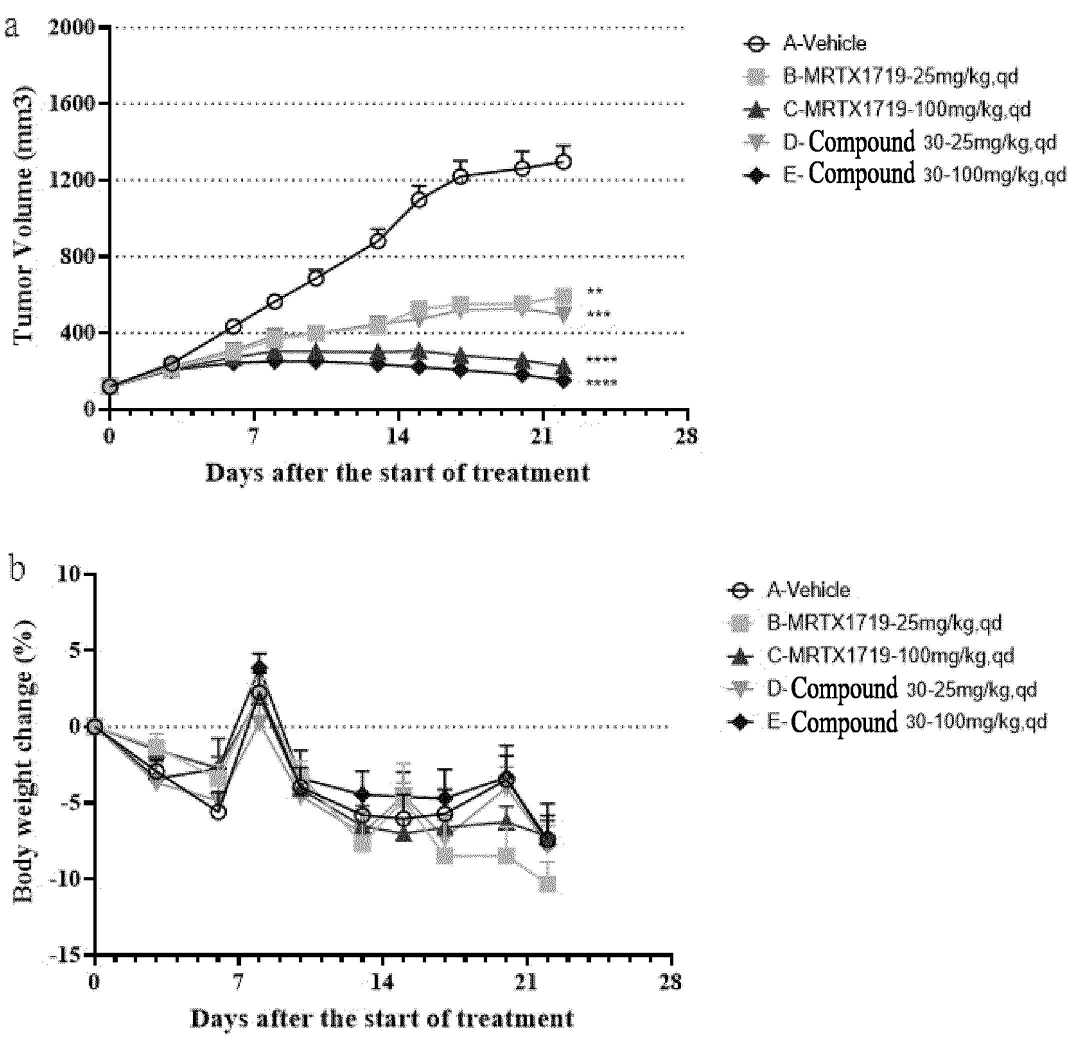
a
2000
1600
1200
800
400
0
Tumor Volume (mm3)
0
7
14
21
28
Days after the start of treatment
A-Vehicle
B-MRTX1719-25mg/kg,qd
C-MRTX1719-100mg/kg,qd
D-Compound 30-25mg/kg,qd
E-Compound 30-100mg/kg,qd
**
***
****
****
b
10
5
0
-5
-10
-15
Body weight change (%)
0
7
14
21
28
Days after the start of treatment
A-Vehicle
B-MRTX1719-25mg/kg,qd
C-MRTX1719-100mg/kg,qd
D-Compound 30-25mg/kg,qd
E-Compound 30-100mg/kg,qd

*FIG. 1*

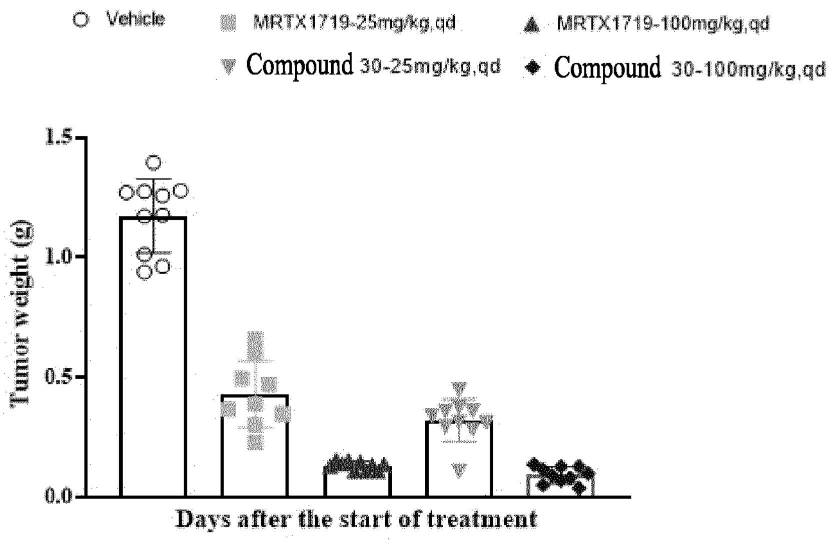
Vehicle
MRTX1719-25mg/kg,qd
MRTX1719-100mg/kg,qd
Compound 30-25mg/kg,qd
Compound 30-100mg/kg,qd
1.5
1.0
0.5
0.0
Tumor weight (g)
Days after the start of treatment

*FIG. 2*

## INTERNATIONAL SEARCH REPORT

International application No. **PCT/CN2023/106257**

### A. CLASSIFICATION OF SUBJECT MATTER

C07D 237/32(2006.01)i； C07D 403/02(2006.01)i； C07D 403/14(2006.01)i； C07D 471/04(2006.01)i； A61K 31/502(2006.01)i； A61K 31/517(2006.01)i； A61P 35/00(2006.01)i

According to International Patent Classification (IPC) or to both national classification and IPC

### B. FIELDS SEARCHED

Minimum documentation searched (classification system followed by classification symbols)

C07D237/- C07D403/- C07D471/- A61K31/- A61P35/-

Documentation searched other than minimum documentation to the extent that such documents are included in the fields searched

Electronic data base consulted during the international search (name of data base and, where practicable, search terms used)

DWPI, ENTXT, CNTXT, STN(registry): 吡唑, 酞嗪酮, 蛋白质精氨酸, 甲基转移酶, 抑制剂, 协同, pyrazole, phthalazinone, MTA, PRMT5, protein arginase, protein arginine, methyl transferase, inhibitor, cooperative, complex, 结构式检索, search for structural formula

### C. DOCUMENTS CONSIDERED TO BE RELEVANT

| Category* | Citation of document, with indication, where appropriate, of the relevant passages | Relevant to claim No. |
|---|---|---|
| X | WO 2021050915 A1 (MIRATI THERAPEUTICS, INC.) 18 March 2021 (2021-03-18)<br>description, paragraphs [0006]-[0606] | 1-16 |
| PX | WO 2022192745 A1 (MIRATI THERAPEUTICS, INC.) 15 September 2022 (2022-09-15)<br>description, paragraphs [0006]-[0342] | 1-16 |
| PX | CN 116178347 A (SUZHOU PUHE PHARMACEUTICAL TECHNOLOGY CO., LTD.) 30 May 2023 (2023-05-30)<br>description, paragraphs [0008]-[0342] | 1-16 |
| PX | WO 2023125540 A1 (MEDSHINE DISCOVERY INC.) 06 July 2023 (2023-07-06)<br>description, page 1 line 1 to page 32 line 3 | 1-16 |
| PX | WO 2023098439 A1 (SHANGHAI ABBISKO THERAPEUTICS CO., LTD.) 08 June 2023 (2023-06-08)<br>description, pages 2-18, embodiments 1 to 13 | 1-16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 September 2023** | **21 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/106257**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

Claim 16 relates to a method for treating diseases, including liver cancer, etc. in a mammal by using the compound of any one of claims 1-11. It can be seen that claim 16 relates to a treatment method implemented on a human/animal body, and thus said claim does not comply with PCT Rule 39.1. Nevertheless, a search is still conducted on the basis of the claimed effect of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/106257**

| Patent document cited in search report | Publication date (day/month/year) | Patent family member(s) | Publication date (day/month/year) |
|---|---|---|---|
| WO 2021050915 A1 | 18 March 2021 | US 2023054883 A1 | 23 February 2023 |
| | | IL 290341 A | 01 April 2022 |
| | | US 2021079003 A1 | 18 March 2021 |
| | | US 11492351 B2 | 08 November 2022 |
| | | AU 2020345899 A1 | 03 March 2022 |
| | | JP 2022548255 A | 17 November 2022 |
| | | NO 20220309 A1 | 11 March 2022 |
| | | TW 202122387 A | 16 June 2021 |
| | | CL 2022000603 A1 | 27 January 2023 |
| | | CA 3150515 A1 | 18 March 2021 |
| | | CO 2022004513 A2 | 21 June 2022 |
| | | US 2021078994 A1 | 18 March 2021 |
| | | US 11479551 B2 | 25 October 2022 |
| | | BR 112022004248 A2 | 31 May 2022 |
| | | KR 20220083691 A | 20 June 2022 |
| | | EP 4028388 A1 | 20 July 2022 |
| WO 2022192745 A1 | 15 September 2022 | None | |
| CN 116178347 A | 30 May 2023 | None | |
| WO 2023125540 A1 | 06 July 2023 | None | |
| WO 2023098439 A1 | 08 June 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2021050915 A1
- US 202178994 B

### Non-patent literature cited in the description

- **FIRESTONE** ; **SCHRAMM**. *J. Am. Chem Soc.*, 2017, vol. 139 (39), 13754-13760
- *CHEMICAL ABSTRACTS*, 131002-09-0
- *CHEMICAL ABSTRACTS*, 403850-89-5
- *Journal of Medicinal Chemistry*, 2022, vol. 65 (3), 1749-1766